# EUROPEAN PATENT APPLICATION

(11) **EP 4 810 062 A1**
(43) Date of publication of application: **23.09.2026**
(21) Application number: 24890722.2
(22) Date of filing: 13.11.2024
(51) Int. Cl.: A61K 47/68, A61K 47/64, C07K 5/062, A61P 35/00

(54) **LIGAND-DRUG CONJUGATE, LINKER FOR CONJUGATION, AND USE THEREOF**

(30) Priority: 14.11.2023 CN 202311515509; 06.02.2024 CN 202410173706
(71) Applicant: Simcere Zaiming Pharmaceutical Co., Ltd., Haikou, Hainan 570311 (CN)
(72) Inventor: LI, Zhen, Shanghai 201318 (CN); WANG, Xuefeng, Shanghai 201318 (CN); CHEN, Changyan, Shanghai 201318 (CN); FU, Yayuan, Shanghai 201318 (CN); WEI, Menghao, Shanghai 201318 (CN); LI, Zhengtao, Shanghai 201318 (CN); CEN, Chaochao, Shanghai 201318 (CN)
(74) Representative: Richly & Ritschel Patentanwälte PartG mbB
(86) International application number: PCT/CN2024/131800
(87) International publication number: WO 2025/103367

(57) **Abstract**

Provided are a ligand-drug conjugate represented by general formula Pc-(L-D)ₙ or a pharmaceutically acceptable salt thereof, and further provided is a linker for conjugation represented by formula (L). The linker can efficiently and highly selectively perform chemical conjugation with a thiol group on cysteine, and the antibody-drug conjugate formed has good stability.

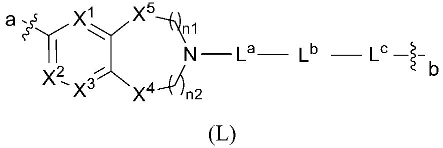

## Description

### CROSS-REFERENCE TO RELATED APPLICATION

The present application claims the benefit and priority to Chinese Patent Application No. 202311515509.5 filed with China National Intellectual Property Administration on Nov. 14, 2023 and Chinese Patent Application No. 202410173706.1 filed with China National Intellectual Property Administration on Feb. 06, 2024, which are incorporated herein by reference in their entirety.

### TECHNICAL FIELD

The present disclosure pertains to the field of biomedicine, and relates to a ligand-drug conjugate with a novel structure, a conjugation linker thereof, a pharmaceutical composition containing the conjugate, and use of the conjugate as an anti-tumor drug and in related fields.

### BACKGROUND

Antibody-drug conjugates (ADCs) have emerged as a novel drug treatment form in recent years. Structurally, an ADC consists of an antibody with a targeting effect, a payload exerting a pharmacological activity, and a linker chemically linking the antibody and the payload. Mechanistically, ADCs enhance their enrichment in the lesion tissue site with high expression of the antigen through the specific recognition of the antigen by the antibody, and achieve the targeted release of the active payload in the lesion tissue through endocytosis, lysosomal degradation, and the like, thereby producing pharmacological effects. Meanwhile, ADCs can maintain a relatively stable state in the systemic circulation, and only a small amount of payload can be released into the systemic circulation compared with the lesion tissue, thereby reducing the systemic toxicity of the highly active payload. Through such a selective release mechanism, ADCs further improve the therapeutic effect on diseases and reduce the risk of toxicity. The linker, as a bridge connecting the antibody and the payload, plays an important role in both controlling the release of the payload from target tissues and ensuring the stability of the payload in the systemic circulation.

In the linker of an ADC, the portion that connects the small molecule to the antibody via a chemical bond is defined as a conjugation linker. Conjugation with antibodies can be roughly divided into conjugation with natural amino acid residues on antibodies (e.g., lysine and cysteine) and site-specific conjugation by engineered antibodies via modifications (e.g., N297 glycosyl conjugation and unnatural amino acid conjugation). For different antibody conjugation methods, the corresponding conjugation linkers are also different. Among the ADCs currently on the market, conjugation via a sulfhydryl group on a cysteine is one of the most common conjugation methods. Generally, the antibody contains 4 pairs of interchain disulfide bonds, and 8 cysteines containing sulfhydryl groups can be obtained by reduction. By virtue of the nucleophilic activity of the sulfhydryl group, a chemical reaction can be performed with the conjugation linker to link the antibody to the linker-payload. The most commonly used conjugation linker is maleimide, which can efficiently form a carbon-sulfur bond with a sulfhydryl group on a cysteine through a Michael addition reaction, thereby linking the payload to the antibody. For example, Adcetris, Enhertu, and the like are ADC products obtained by cysteine conjugation using maleimide.

### SUMMARY

In a first aspect, the present disclosure provides a ligand-drug conjugate having a structural general formula of Pc-(L-D)ₙ or a pharmaceutically acceptable salt thereof, wherein
Pc is a ligand unit;
D is a drug unit;
L is a linker unit of the following structure, wherein the a-terminus is linked to the ligand unit, 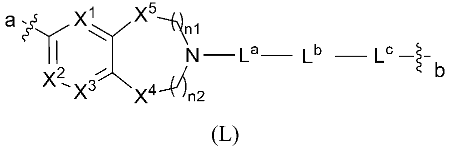
wherein
X¹, X², and X³ are each independently selected from N or CR^{a1}, and at least one of X¹, X², and X³ is selected from N, wherein R^{a1} is selected from H, halogen, CN, C₁-C₃ alkyl, C₂-C₃ alkenyl, C₂-C₃ alkynyl, C₃-C₆ cycloalkyl, or 4-to 7-membered heterocyclyl;
n1 and n2 are each independently selected from 0, 1, or 2;
X⁴ and X⁵ are each independently selected from C(=O), C(R^{b1})(R^{b2}), O, S, S(=O), S(=O)₂, NR^{b3}, or any combination thereof, wherein R^{b1}, R^{b2}, and R^{b3} are each independently selected from H or R^{c1}, or R^{b1} and R^{b2}, together with the carbon atom to which they are attached, form C₃-C₆ cycloalkyl or 4- to 7-membered heterocyclyl, wherein the C₃-C₆ cycloalkyl or 4- to 7-membered heterocyclyl is optionally substituted with one or more R^{c1};
each R^{c1} is independently selected from halogen, CN, C₁-C₆ alkyl, C₃-C₆ cycloalkyl, or a hydrophilic group;
L^{a} is selected from 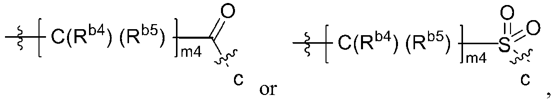 wherein R^{b4} and R^{b5} are each independently selected from H, halogen, CN, C₁-C₃ alkyl, OH, O(C₁-C₃ alkyl), NH₂, NH(C₁-C₃ alkyl), or N(C₁-C₃ alkyl)₂, or R^{b4} and R^{b5}, together with the carbon atom to which they are attached, form C₃-C₆ cycloalkyl or 4- to 7-membered heterocyclyl, m4 is selected from integers of 1-10, and the c-terminus of L^{a} is linked to L^{b};
L^{b} is selected from a chemical bond or 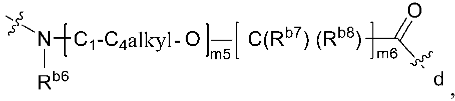 wherein m5 is selected from integers of 0-24, m6 is selected from integers of 1-10, each R^{b6}, each R^{b7}, and each R^{b8} are each independently selected from H or R^{c6}, wherein R^{c6} is selected from halogen, CN, C₁-C₆ alkyl, C₃-C₆ cycloalkyl, or a hydrophilic group, and the d-terminus of L^{b} is linked to L^{c};
L^{c} is selected from Y¹-Y²-Y³, wherein Y¹ is selected from a chemical bond or 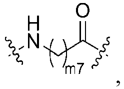 m7 is selected from integers of 1-6, Y² is selected from a peptide residue consisting of 2-10 amino acids, Y³ is selected from 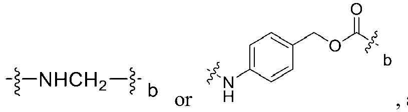 and the b-terminus of L^{c} is linked to drug unit D; and
n is a real number of 1-16.

In some embodiments, X¹ and X² are both selected from N, and X³ is selected from CH.

In some embodiments, n1 and n2 are each independently selected from 0 or 1.

In some embodiments, n1 is selected from 1, and n2 is selected from 0.

In some embodiments, X⁴ and X⁵ are each independently selected from C(=O) or C(R^{b1})(R^{b2}), wherein R^{b1} and R^{b2} are each independently selected from H or R^{c1}, or R^{b1} and R^{b2}, together with the carbon atom to which they are attached, form C₃-C₆ cycloalkyl or 4- to 7-membered heterocyclyl, wherein the C₃-C₆ cycloalkyl or 4- to 7-membered heterocyclyl is optionally substituted with one or more R^{c1}.

In some embodiments, X⁴ is selected from C(=O), and X⁵ is selected from C(R^{b1})(R^{b2}), wherein R^{b1} and R^{b2} are each independently selected from H or R^{c1}, or R^{b1} and R^{b2}, together with the carbon atom to which they are attached, form C₃-C₆ cycloalkyl or 4- to 7-membered heterocyclyl, wherein the C₃-C₆ cycloalkyl or 4- to 7-membered heterocyclyl is optionally substituted with one or more R^{c1}. In some embodiments, X⁴ is selected from C(=O), and X⁵ is selected from C(R^{b1})(R^{b2}), wherein R^{b1} and R^{b2} are each independently selected from H or R^{c1}, or R^{b1} and R^{b2}, together with the carbon atom to which they are attached, form C₄-C₆ cycloalkyl or 4- to 6-membered heterocyclyl containing one O atom or one N atom, wherein the 4- to 6-membered heterocyclyl containing one O atom or one N atom is optionally substituted with one R^{c1}, wherein each R^{c1} is independently selected from 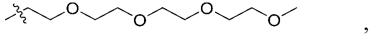 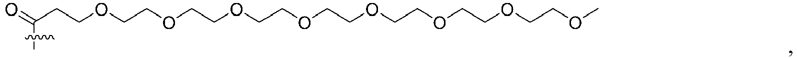 or

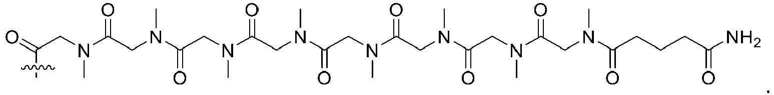

In some embodiments, L^{b} is selected from a chemical bond, 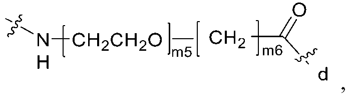 or 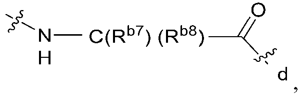 wherein m5 is selected from integers of 1-16, e.g., m5 is selected from 1, 2, 3, 4, 5, 6, 7, 8, 9, 10, 11, 12, 13, 14, 15, or 16; m6 is selected from integers of 1-6, e.g., m6 is selected from 1, 2, 3, 4, 5, or 6; R^{b7} and R^{b8} are each independently selected from H or R^{c6}; the d-terminus of L^{b} is linked to L^{c}.

In some embodiments, L^{b} is selected from a chemical bond or 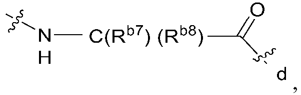 wherein R^{b7} is H, R^{b8} is H or R^{c6}, and the d-terminus of L^{b} is linked to L^{c}.

In some embodiments, each R^{c1} and each R^{c6} are each independently selected from halogen, CN, C₁-C₆ alkyl, C₃-C₆ cycloalkyl, or a hydrophilic group comprising one or more of the following atomic groups or structural fragments: C(=O), OH, O(C₁-C₃ alkyl), NH₂, NH(C₁-C₃ alkyl), N(C₁-C₃ alkyl)₂, C₁-C₄ alkyl-O, NHC(=O), N(CH₃)C(=O), C(=O)NH₂, S(=O)₂NH₂, C(=O)NH(C₁-C₃ alkyl), S(=O)₂NH(C₁-C₃ alkyl), C(=O)N(C₁-C₃ alkyl)₂, S(=O)₂N(C₁-C₃ alkyl)₂,

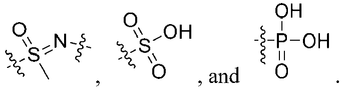

In some embodiments, each R^{c1} and each R^{c6} are independently selected from halogen, CN, C₁-C₆ alkyl, C₃-C₆ cycloalkyl, or W¹-W²-W³-W⁴-W⁵, wherein each W¹, each W², each W³, and each W⁴ are each independently selected from a chemical bond, A-(C₁-C₄ alkyl-O)ₘ₁, or [A-(CR^{d1}R^{d2})ₘ₂-NR^{d3}]ₘ₃-B, wherein each A and each B are independently selected from a chemical bond, C₁-C₁₀ alkylene, C₃-C₆ cycloalkylene, 4- to 7-membered heterocyclylene, O, S, NR^{d4}, C=O, C(=O)NR^{d5}, S(=O)₂, S(=O)₂NR^{d6}, S(=O)(=NR^{d7}), 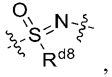 or any combination thereof, wherein R^{d1}, R^{d2}, R^{d3}, R^{d4}, R^{d5}, R^{d6}, R^{d7}, and R^{d8} are each independently selected from H, C₁-C₆ alkyl, C₃-C₆ cycloalkyl, 4- to 7-membered heterocyclyl, or (C₁-C₄ alkyl-O)ₖ₁-C₁-C₆ alkyl, wherein the C₁-C₆ alkyl, C₃-C₆ cycloalkyl, or 4- to 7-membered heterocyclyl is optionally substituted with halogen, CN, OH, O(C₁-C₃ alkyl), NH₂, NH(C₁-C₃ alkyl), N(C₁-C₃ alkyl)₂, C(=O)NH₂, NHC(=O)NH₂, or COOH; m1 is selected from integers of 0-30, m2 is selected from integers of 1-5, and m3 and k1 are each independently selected from integers of 1-30; W⁵ is selected from H, C₁-C₆ alkyl, C₃-C₆ cycloalkyl, 4- to 7-membered heterocyclyl, OH, O(C₁-C₃ alkyl), NH₂, NH(C₁-C₃ alkyl), N(C₁-C₃ alkyl)₂, C(=O)NH₂, S(=O)₂NH₂, C(=O)NH(C₁-C₃ alkyl), S(=O)₂NH(C₁-C₃ alkyl), C(=O)N(C₁-C₃ alkyl)₂, S(=O)₂N(C₁-C₃ alkyl)₂,

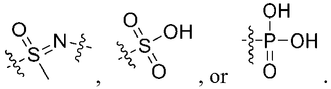

In some embodiments, each R^{c1} and each R^{c6} are independently selected from W¹-W²-W³-W⁴-W⁵, wherein W¹ and W² are both selected from a chemical bond, W³ is selected from a chemical bond or A-(C₁-C₄ alkyl-O)ₘ₁, and W⁴ is selected from A-(C₁-C₄ alkyl-O)ₘ₁ or [A-(CR^{d1}R^{d2})ₘ₂-NR^{d3}]ₘ₃-B, wherein each A and each B are independently selected from a chemical bond, C₁-C₁₀ alkylene, C₃-C₆ cycloalkylene, 4- to 7-membered heterocyclylene, O, S, NR^{d4}, C=O, C(=O)NR^{d5}, S(=O)₂, S(=O)₂NR^{d6}, S(=O)(-NR^{d7}), 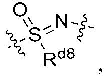 or any combination thereof, wherein R^{d1}, R^{d2}, R^{d3}, R^{d4}, R^{d5}, R^{d6}, R^{d7}, and R^{d8} are each independently selected from H, C₁-C₆ alkyl, C₃-C₆ cycloalkyl, 4-to 7-membered heterocyclyl, or (C₁-C₄ alkyl-O)ₖ₁-C₁-C₆ alkyl, wherein the C₁-C₆ alkyl, C₃-C₆ cycloalkyl, or 4- to 7-membered heterocyclyl is optionally substituted with halogen, CN, OH, O(C₁-C₃ alkyl), NH₂, NH(C₁-C₃ alkyl), N(C₁-C₃ alkyl)₂, C(=O)NH₂, NHC(=O)NH₂, or COOH; m1 is selected from integers of 0-30, m2 is selected from integers of 1-5, and m3 and k1 are each independently selected from integers of 1-30; W⁵ is selected from H, C₁-C₆ alkyl, C₃-C₆ cycloalkyl, 4- to 7-membered heterocyclyl, OH, O(C₁-C₃ alkyl), NH₂, NH(C₁-C₃ alkyl), N(C₁-C₃ alkyl)₂, C(=O)NH₂, S(=O)₂NH₂, C(=O)NH(C₁-C₃ alkyl), S(=O)₂NH(C₁-C₃ alkyl), C(=O)N(C₁-C₃ alkyl)₂, S(=O)₂N(C₁-C₃ alkyl)₂,

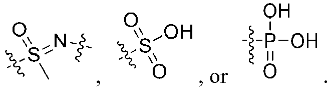

In some embodiments, each R^{c1} is independently selected from A-(C₁-C₄ alkyl-O)ₘ₁-W⁵ or [C(=O)-CH₂-NR^{d3}]ₘ₃-B-W⁵, wherein A is selected from a chemical bond or C=O, R^{d3} is selected from C₁-C₆ alkyl, B is selected from C₁-C₁₀ alkylene, C=O, or a combination thereof C(=O)-C₁-C₁₀ alkylene, W⁵ is selected from C₁-C₆ alkyl, C(=O)NH₂, or S(=O)₂NH₂, and m1 and m3 are independently selected from integers of 1-24.

In some embodiments, each R^{c1} is independently selected from 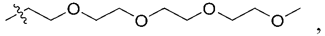 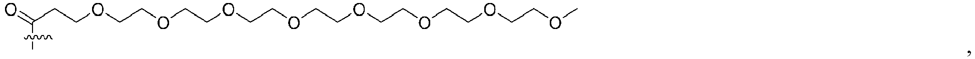 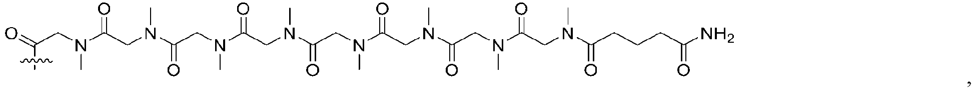 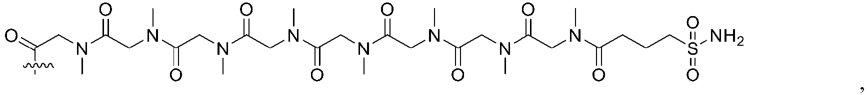 or

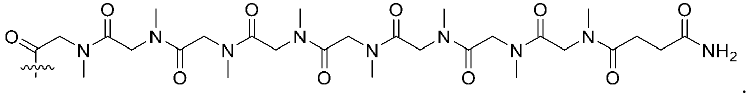

In some embodiments, each R^{c1} is independently selected from 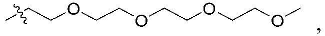 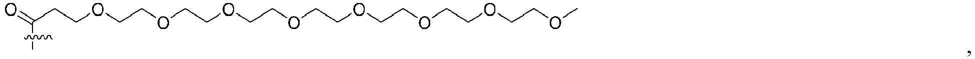 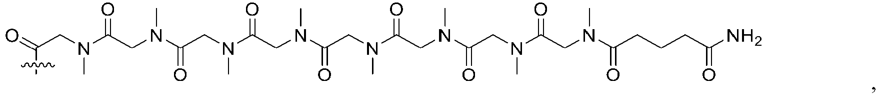 or

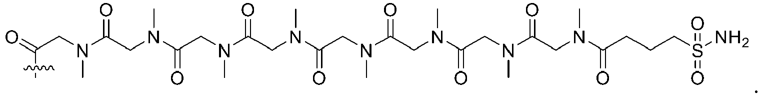

In some embodiments, each R^{c6} is independently selected from W¹-W²-W³-W⁴-W⁵, wherein W¹ and W² are both selected from a chemical bond, W³ is selected from a chemical bond or A-(C₁-C₄ alkyl-O)ₘ₁, and W⁴ is selected from A-(C₁-C₄ alkyl-O)ₘ₁ or [C(=O)-CH₂-NR^{d3}]ₘ₃-B, wherein each A and each B are independently selected from a chemical bond, C₁-C₆ alkylene, NR^{d4}, C(=O), C(=O)NH, S(=O)₂, S(=O)₂NR^{d6}, S(=O)(-NR^{d7}), 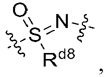 or any combination thereof, wherein R^{d1}, R^{d2}, R^{d3}, R^{d4}, R^{d6}, R^{d7}, and R^{d8} are each independently selected from H, C₁-C₆ alkyl, or (C₁-C₄ alkyl-O)ₖ₁-C₁-C₆ alkyl; m1 is selected from integers of 0-24, m2 is selected from integers of 1-5, and m3 and k1 are each independently selected from integers of 1-24; W⁵ is selected from C₁-C₆ alkyl, NH₂,

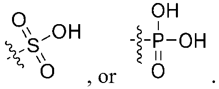

In some embodiments, R^{c6} is selected from 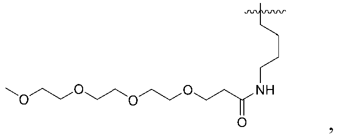 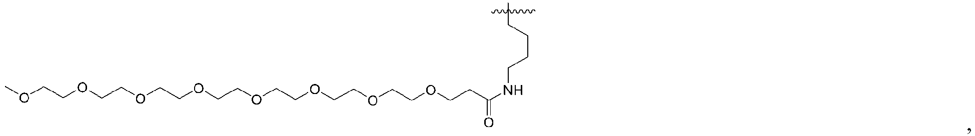 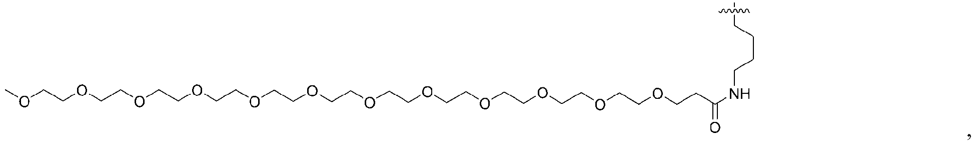 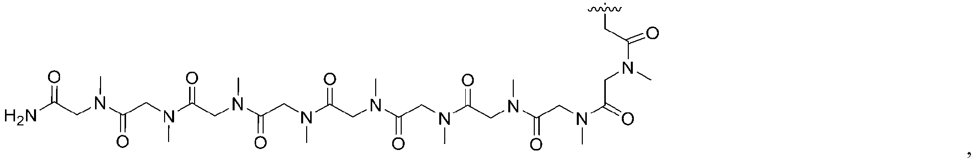 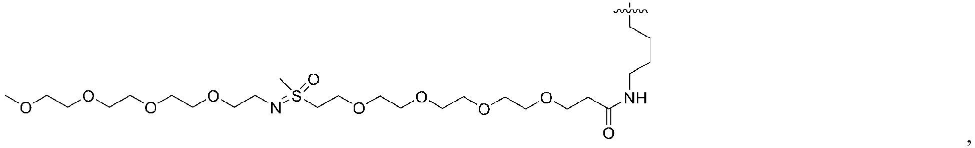 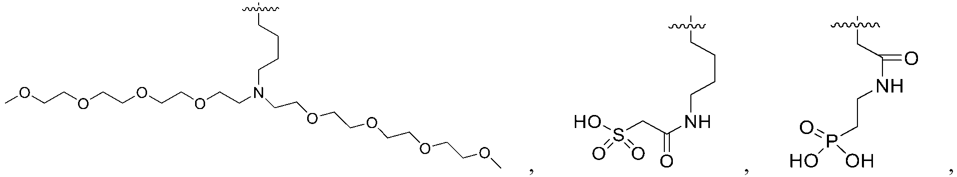 or

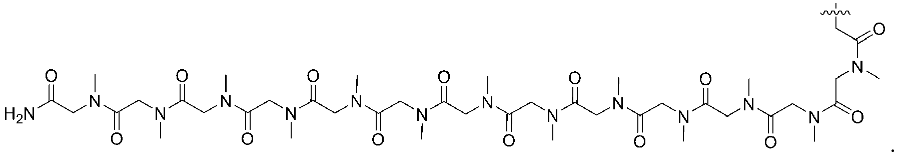

In some embodiments, R^{e6} is selected from

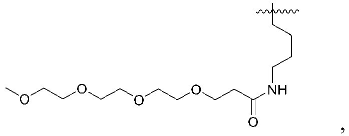

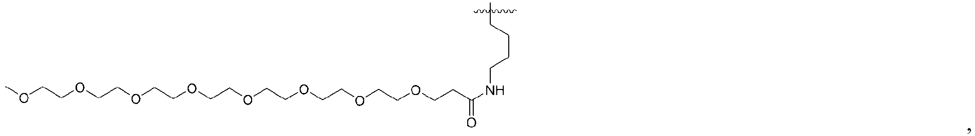

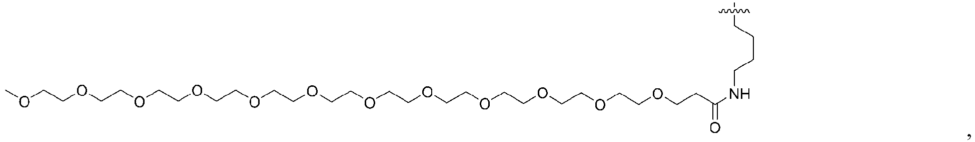

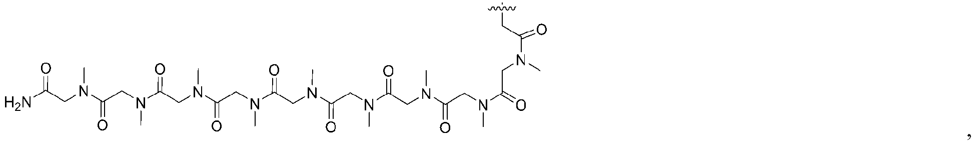

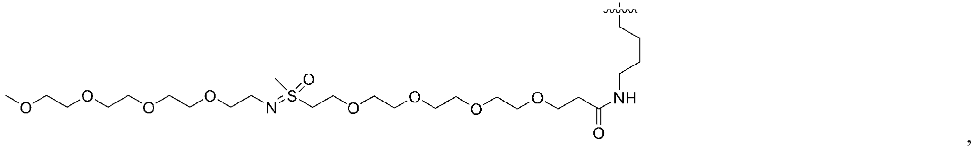

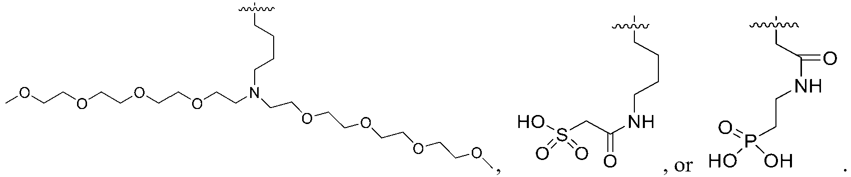

In some embodiments, L^{a} is selected from 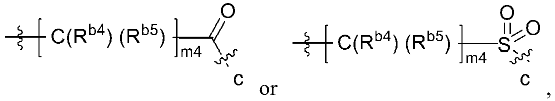 wherein R^{b4} and R^{b5} are each independently selected from H; m4 is selected from integers of 1-6, e.g., m4 is selected from 1, 2, 3, 4, 5, or 6; the c-terminus of L^{a} is linked to L^{b}. In some embodiments, L^{a} is selected from 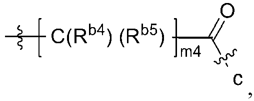 wherein R^{b4} and R^{b5} are each independently selected from H; m4 is selected from integers of 1-6, e.g., m4 is selected from 1, 2, 3, 4, 5, or 6; the c-terminus of L^{a} is linked to L^{b}. In some embodiments, L^{c} is selected from Y¹-Y²-Y³, wherein Y¹ is selected from a chemical bond or 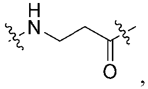 Y² is selected from dipeptide, tripeptide, or tetrapeptide residues of Val-Cit, Val-Ala, Gly-Gly-Phe-Gly, and Ala-Ala-Ala, Y³ is selected from 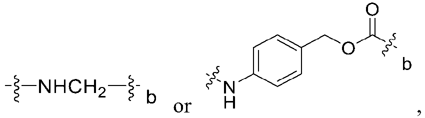 and the b-terminus of L^{c} is linked to drug unit D.

In some embodiments, L° is selected from Y¹-Y²-Y³, wherein Y¹ is selected from a chemical bond or 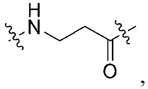 Y² is selected from dipeptide or tetrapeptide residues of Val-Cit, Val-Ala, and Gly-Gly-Phe-Gly, Y³ is selected from 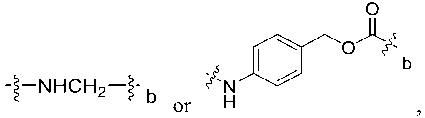 and the b-terminus of L^{c} is linked to drug unit D.

In some embodiments, L^{c} is selected from

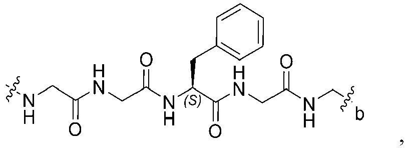

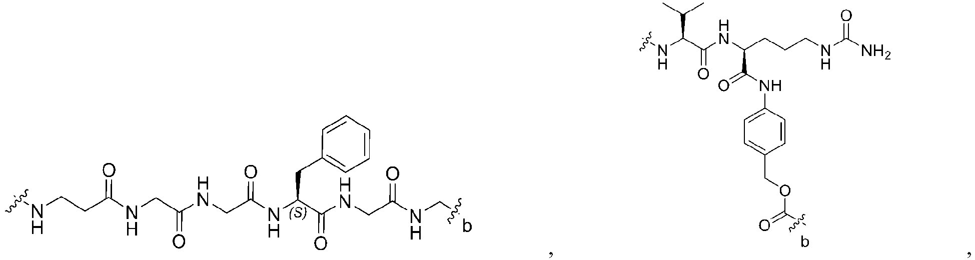

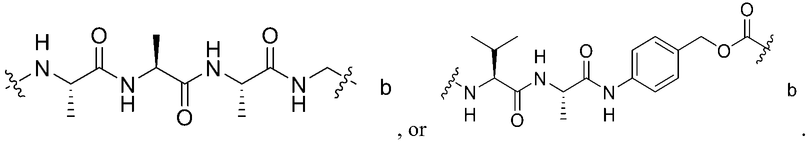

In some embodiments, L^{c} is selected from

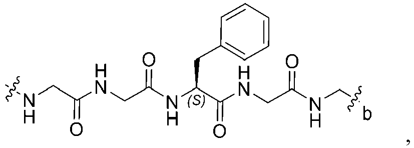

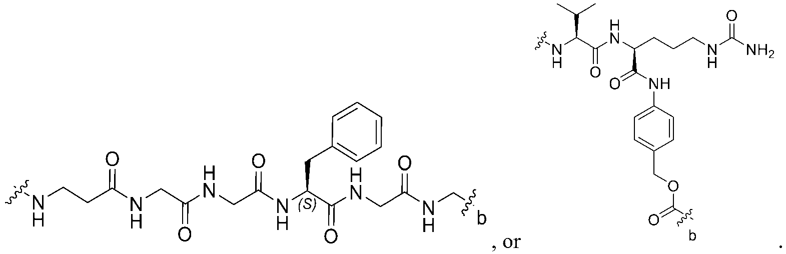

In some embodiments, linker unit L is selected from the following structure: 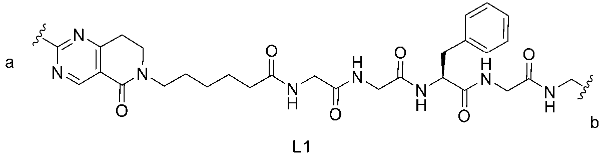 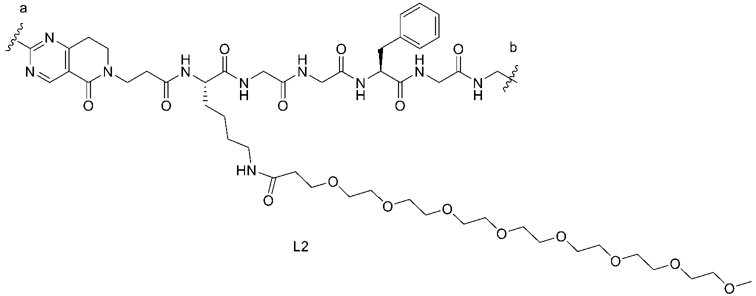 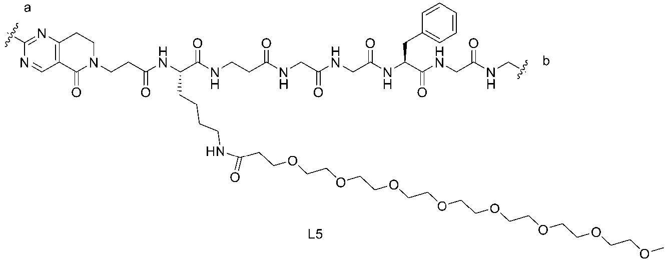 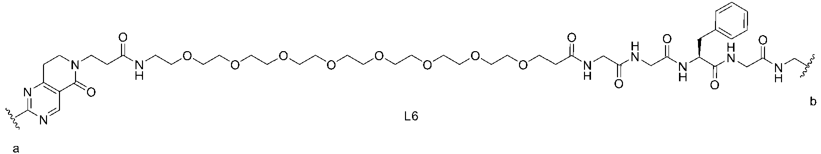 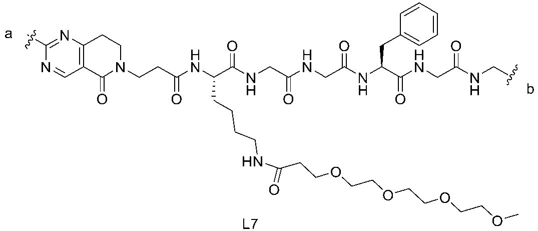 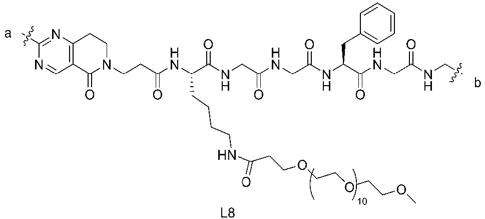 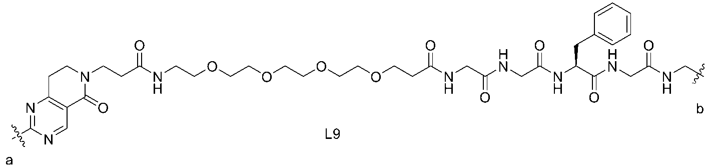 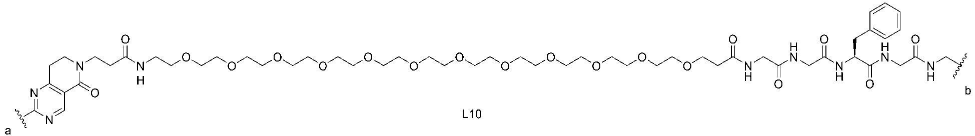 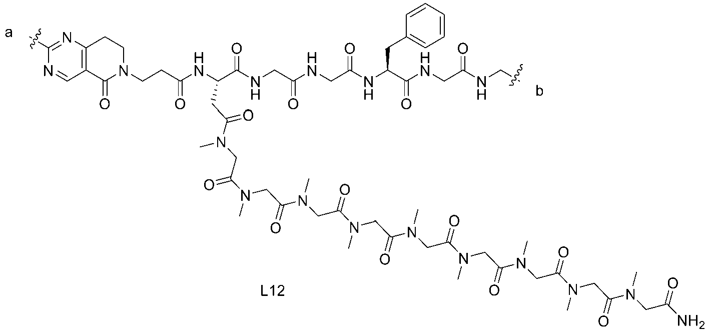 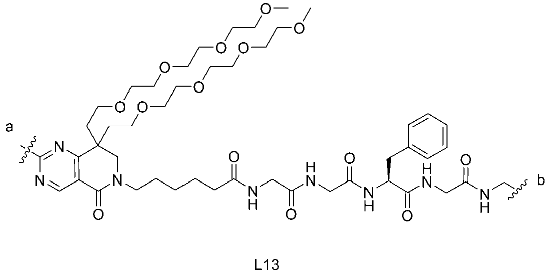 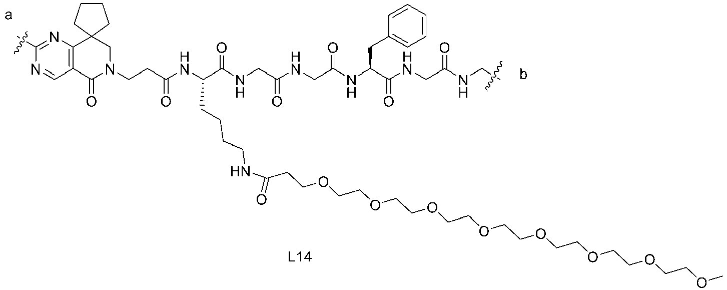 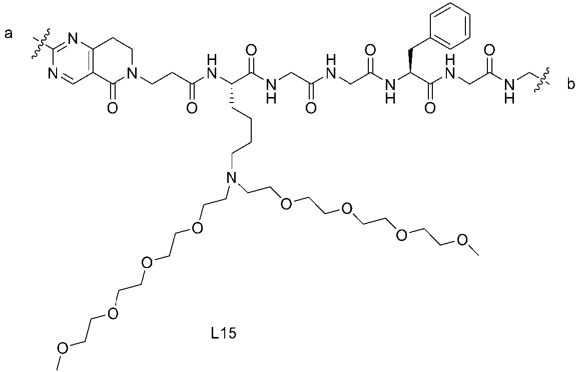 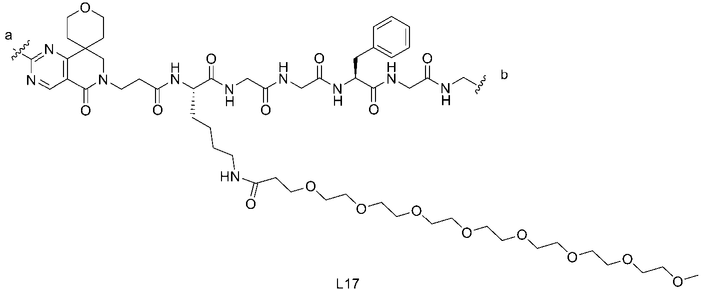 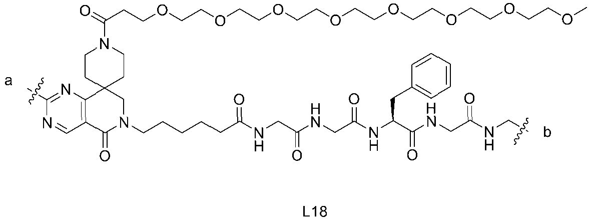 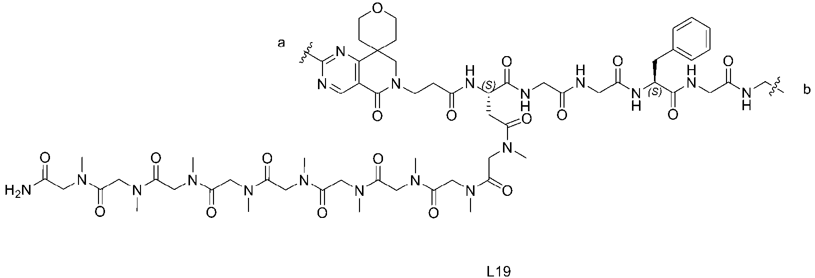 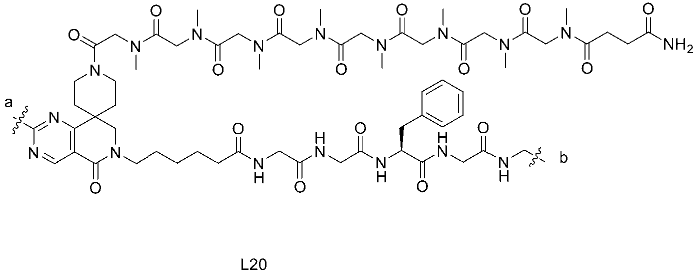 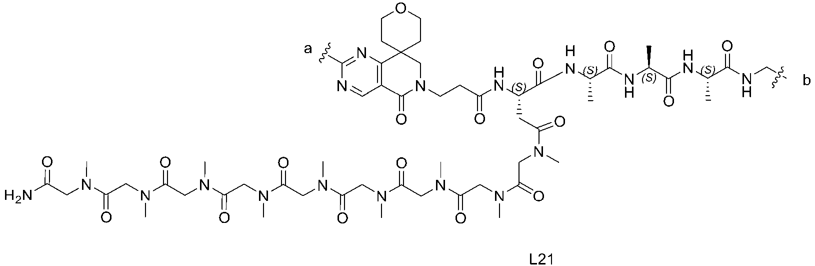 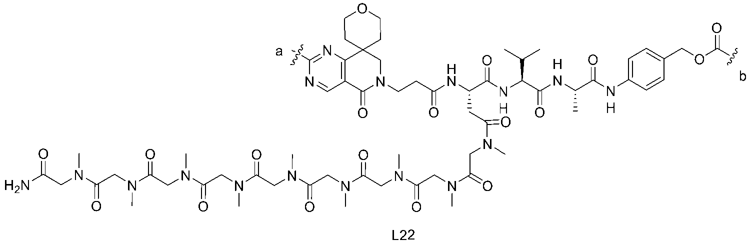 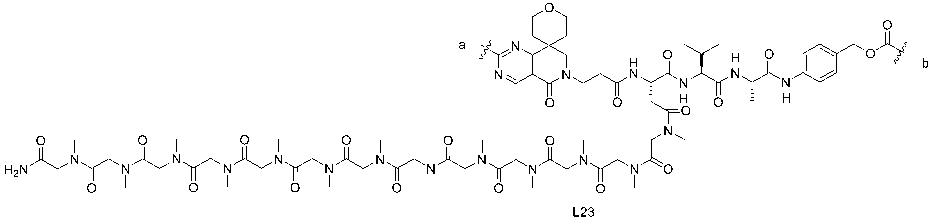 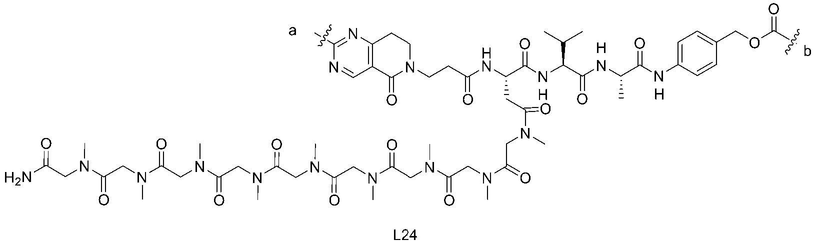 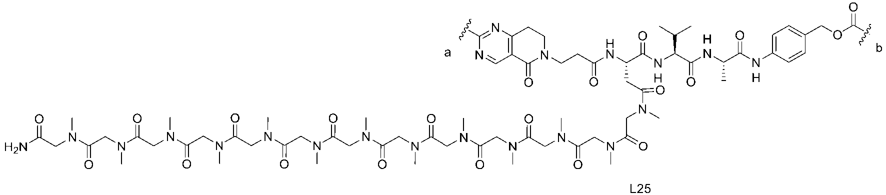 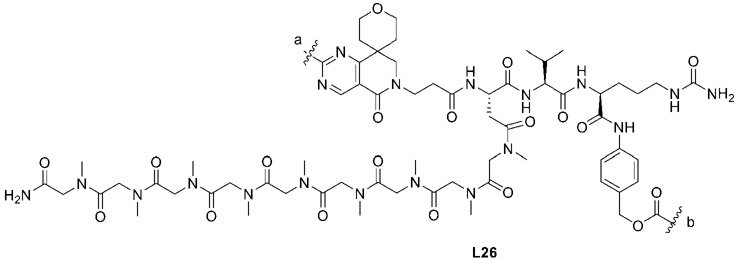 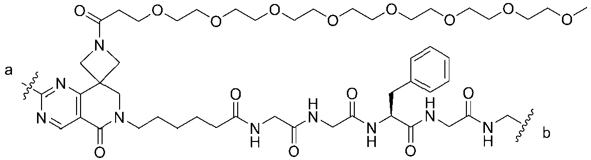 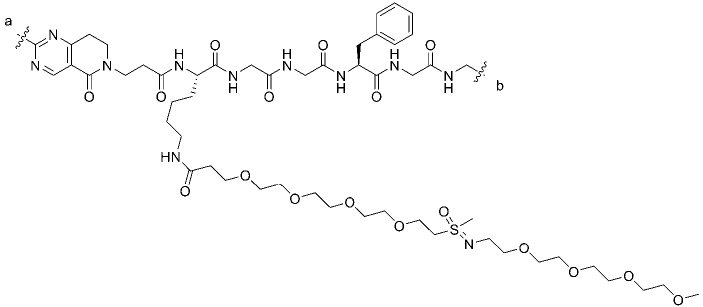 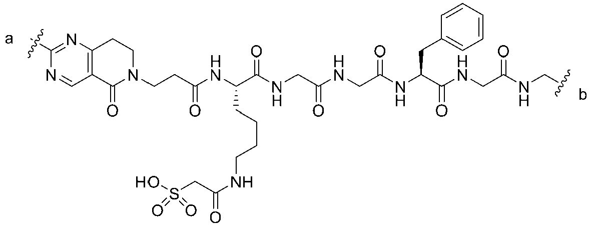 or

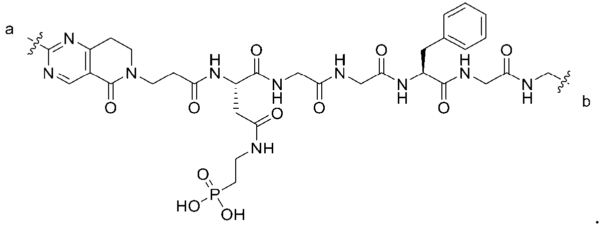

In some embodiments, drug unit D is selected from a cytotoxic drug or a protein degrader.

In some embodiments, the cytotoxic drug is selected from a tubulin inhibitor, a DNA damaging agent, or a topoisomerase inhibitor, wherein the tubulin inhibitor includes, but is not limited to, dolastatin, auristatin, maytansine, tubulysin, and cryptomycin drugs, the DNA damaging agent includes, but is not limited to, PBD, duocarmycin, and calicheamicin drugs, and the topoisomerase inhibitor includes, but is not limited to, a camptothecin drug.

In some embodiments, the cytotoxic drug includes, but is not limited to, camptothecin, hydroxycamptothecin, 9-aminocamptothecin, SN-38, irinotecan, topotecan, belotecan, rubitecan, actinomycin D, doxorubicin, duocarmycin, daunorubicin, mitoxantrone, podophyllotoxin, etoposide, vincristine, vinblastine, paclitaxel, docetaxel, cabazitaxel, maytansine, maytansinoid, DM1, DM3, DM4, calicheamicin, MMAE, MMAF, and tubulysin D.

In some embodiments, drug unit D is selected from a topoisomerase I inhibitor or an auristatin drug.

In some embodiments, the protein degrader is selected from a GSPT1 protein degrader. In some embodiments, D is selected from

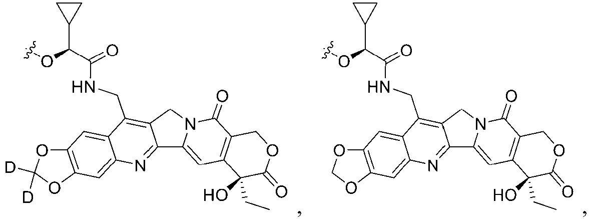

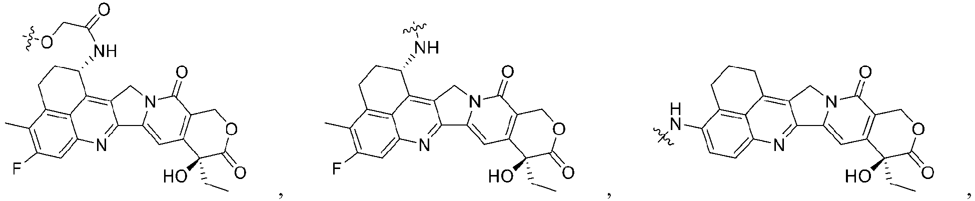

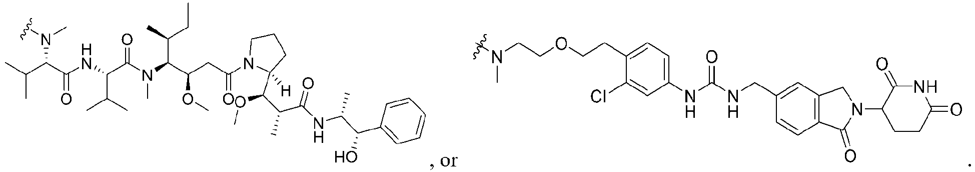

In some embodiments, the ligand-drug conjugate having the general formula of Pc-(L-D)ₙ or the pharmaceutically acceptable salt thereof of the present disclosure is selected from the following compound or a pharmaceutically acceptable salt thereof:

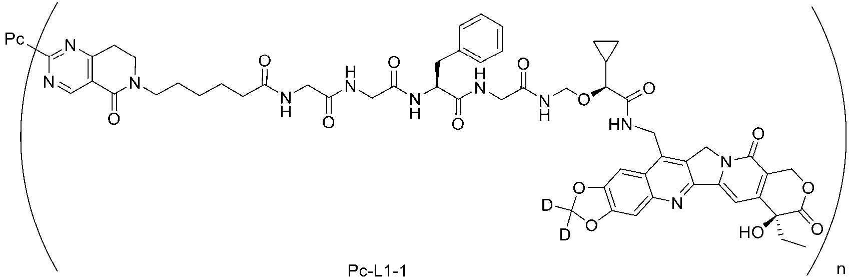

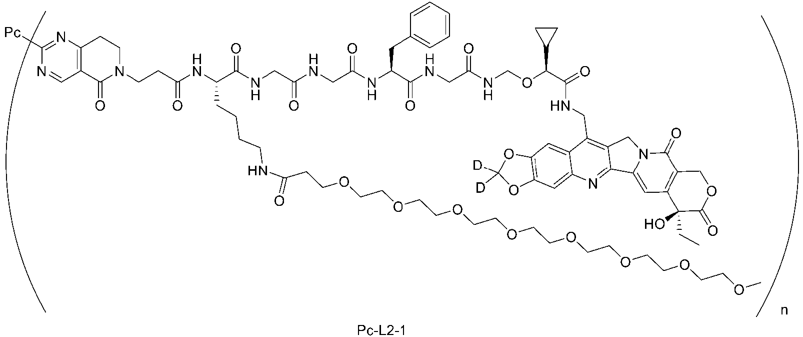

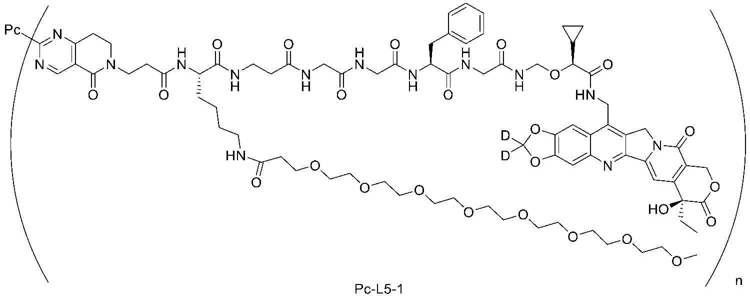

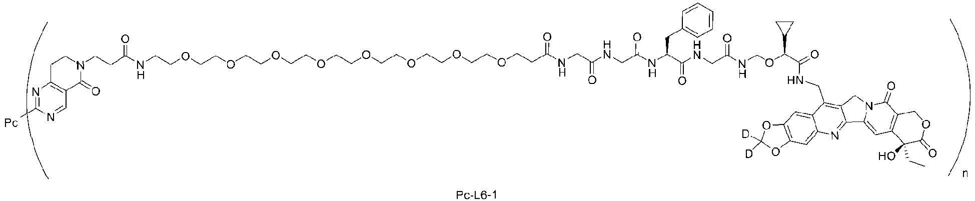

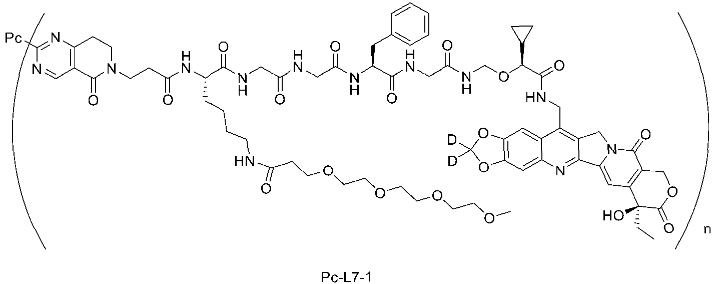

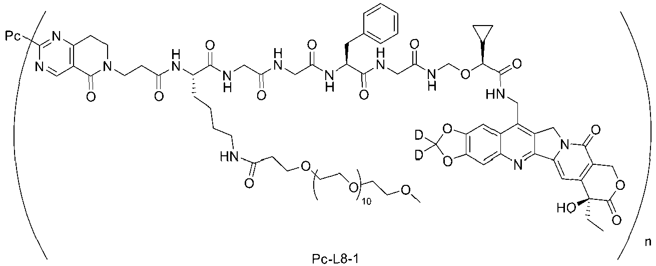

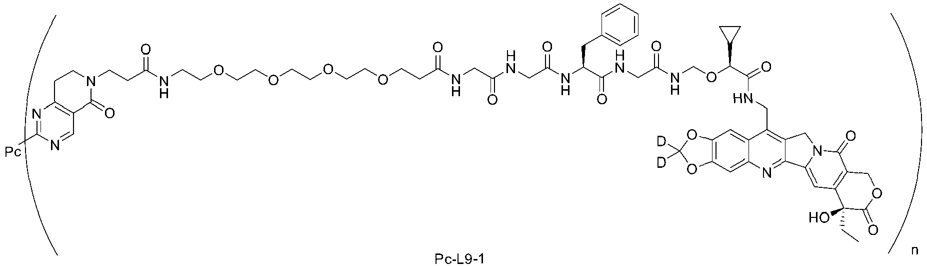

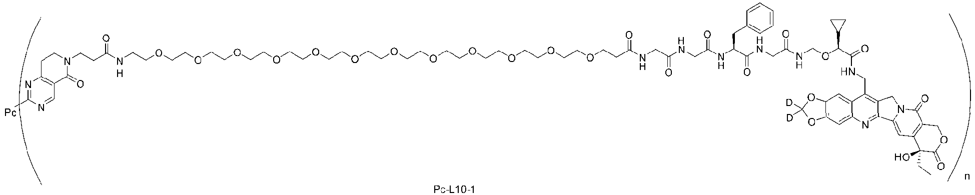

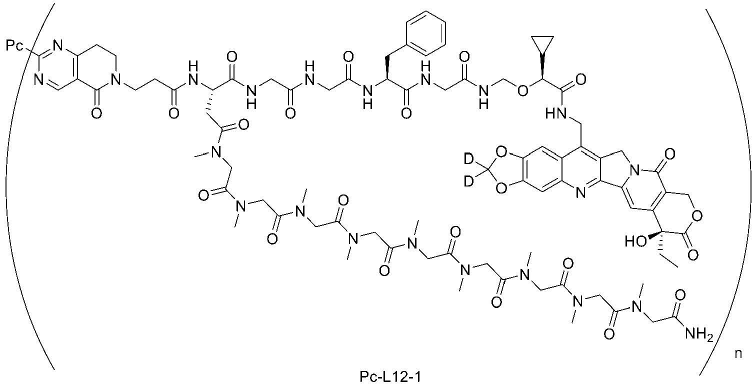

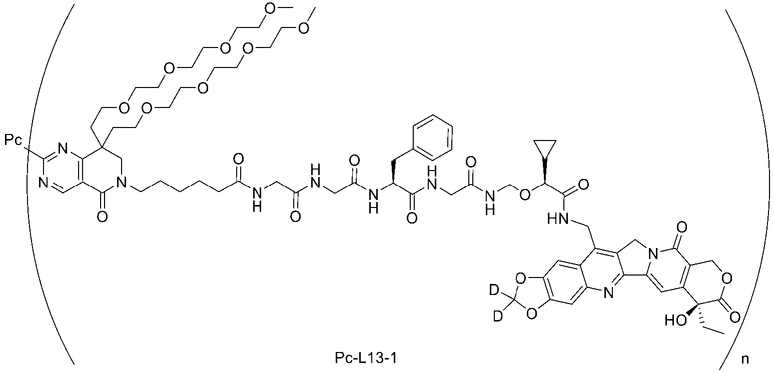

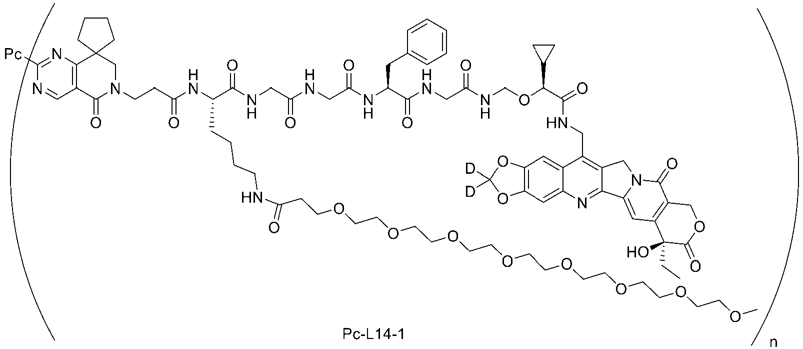

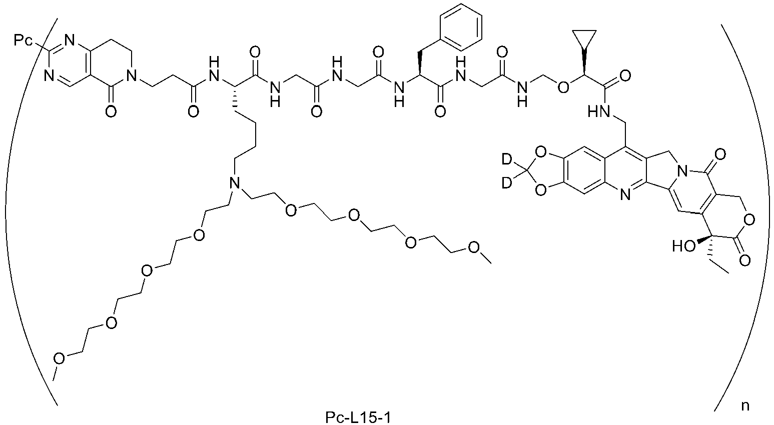

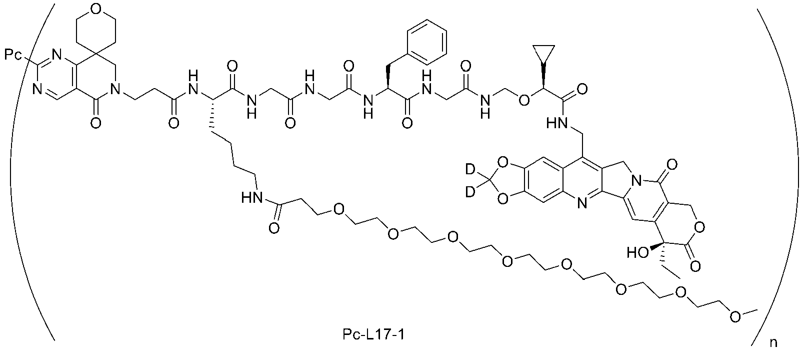

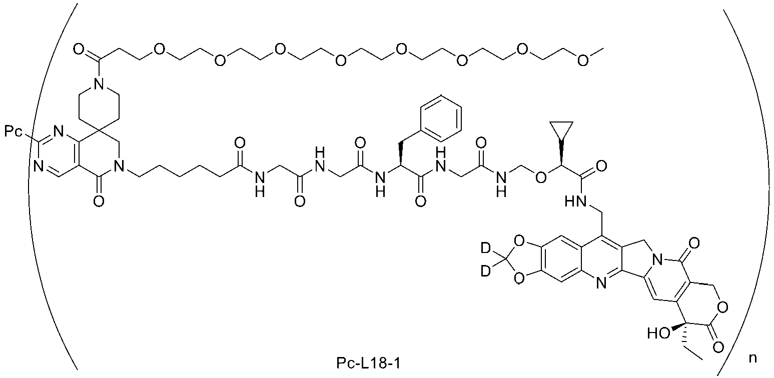

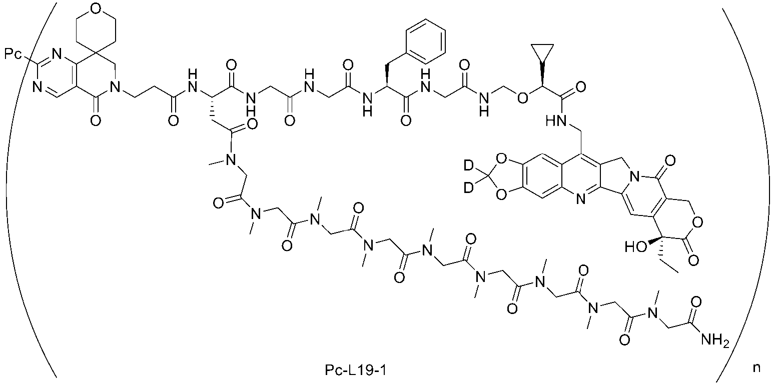

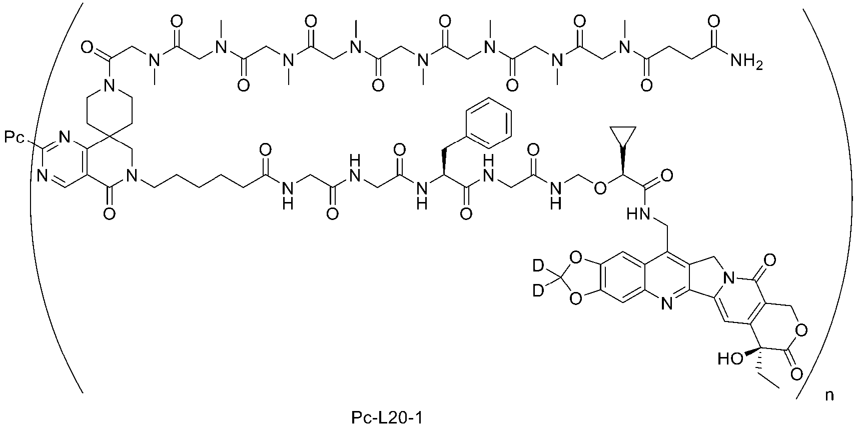

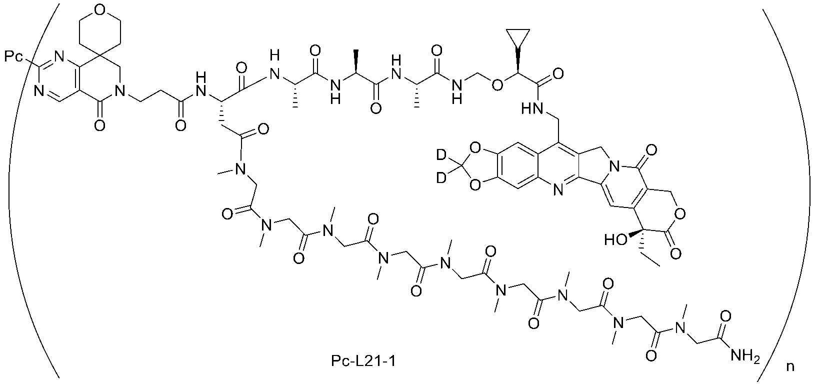

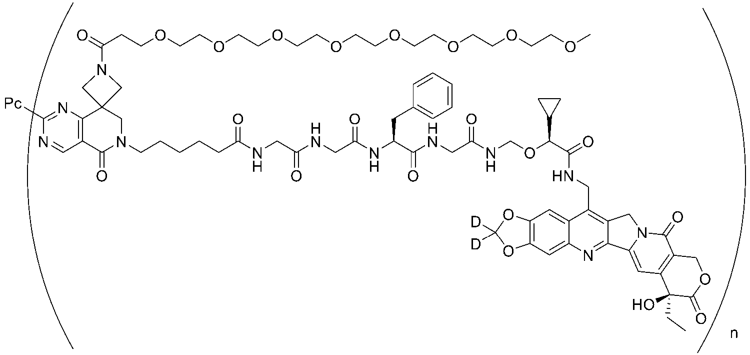

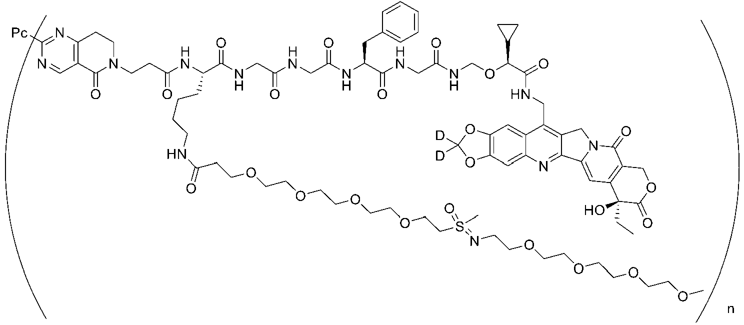

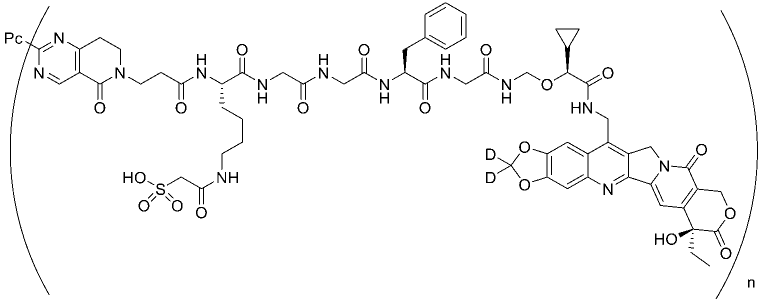

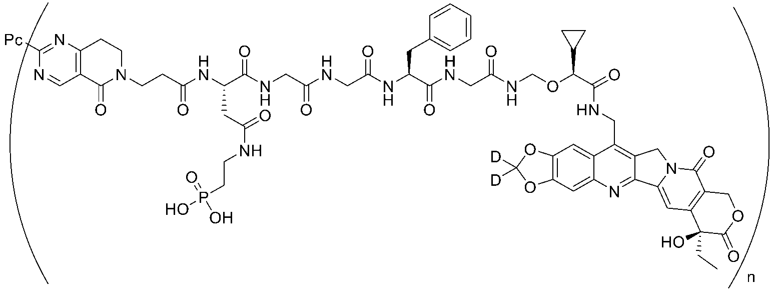

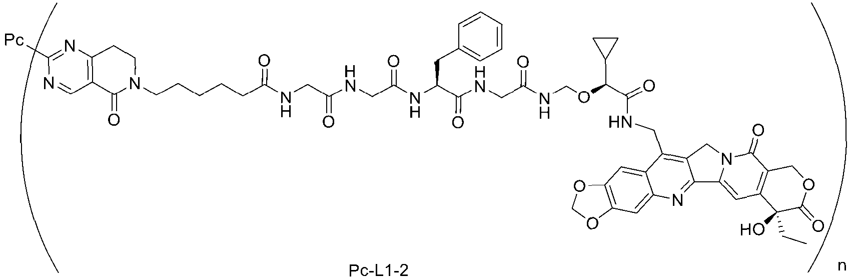

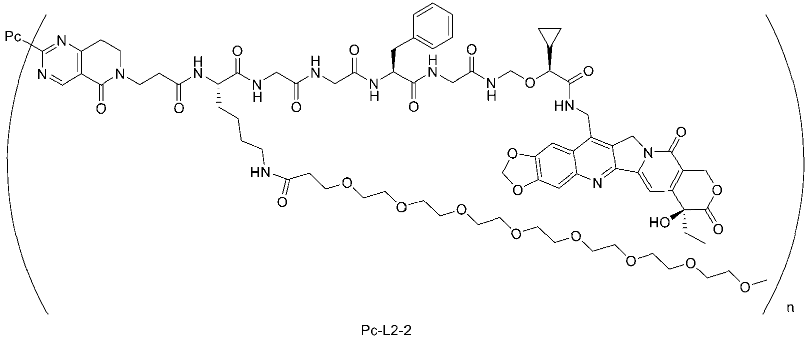

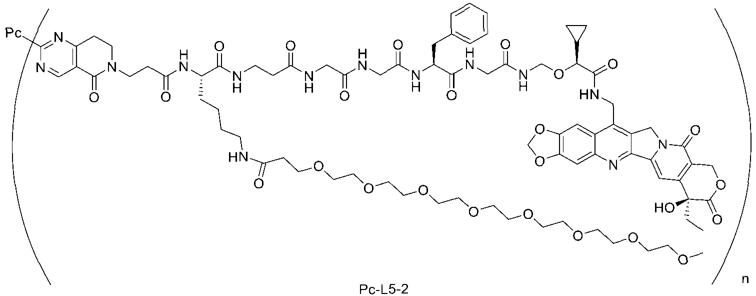

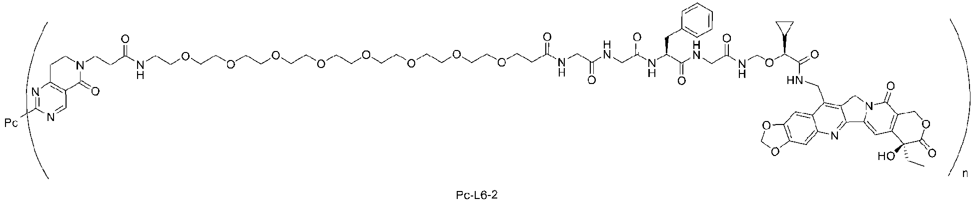

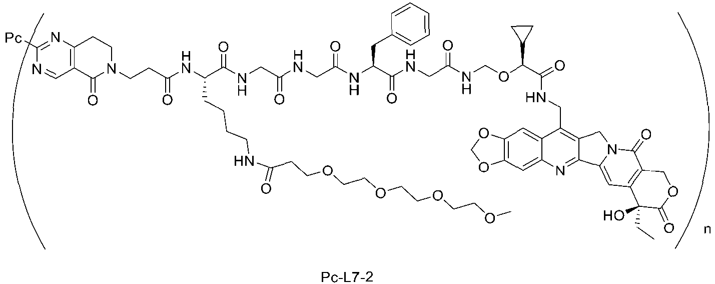

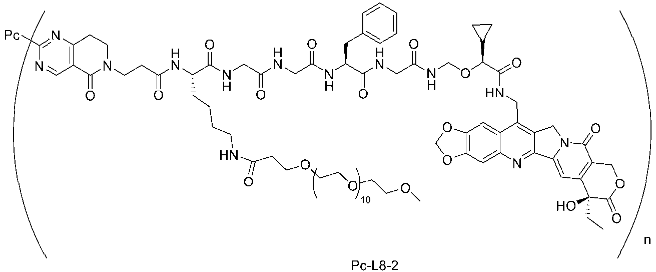

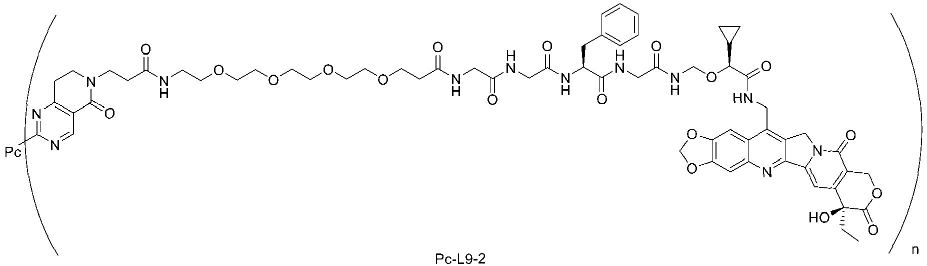

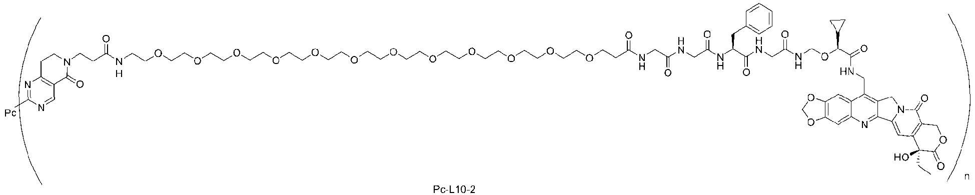

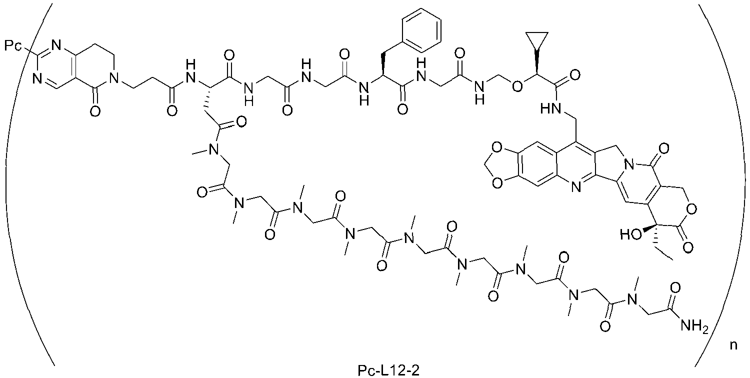

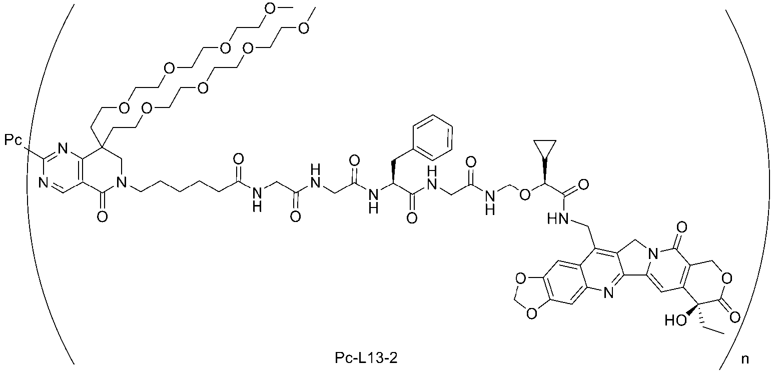

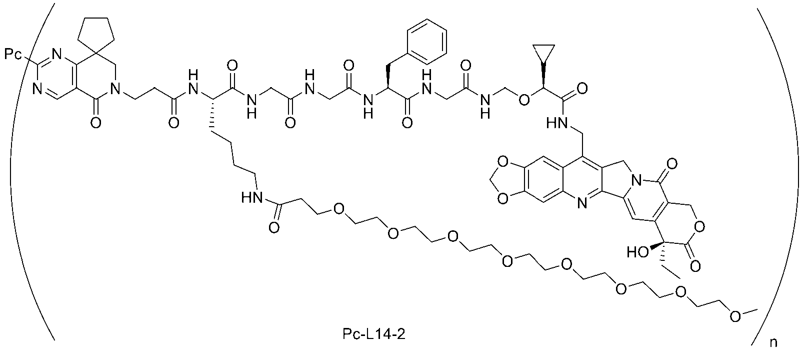

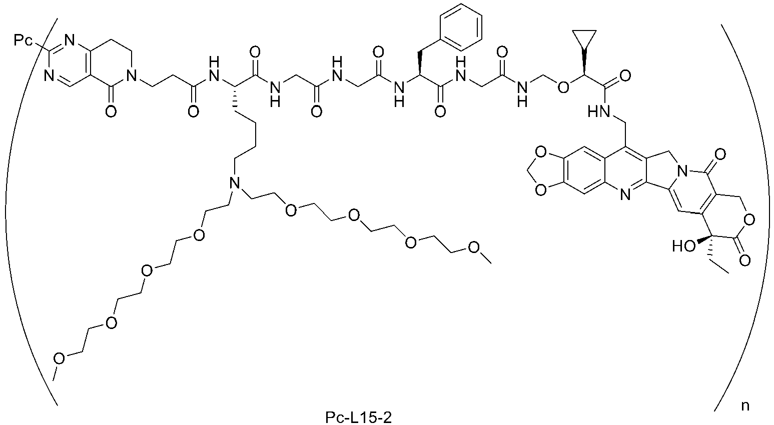

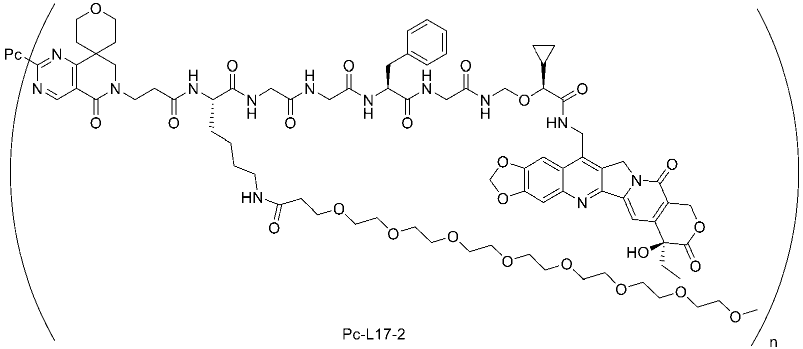

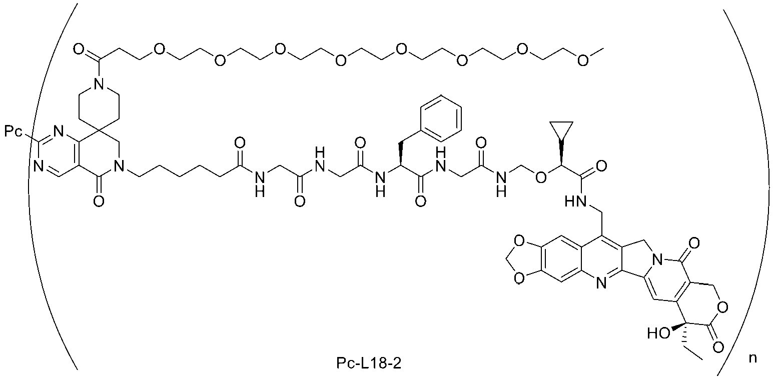

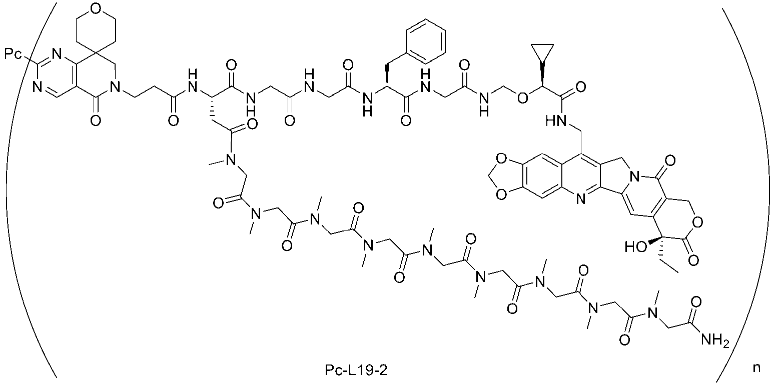

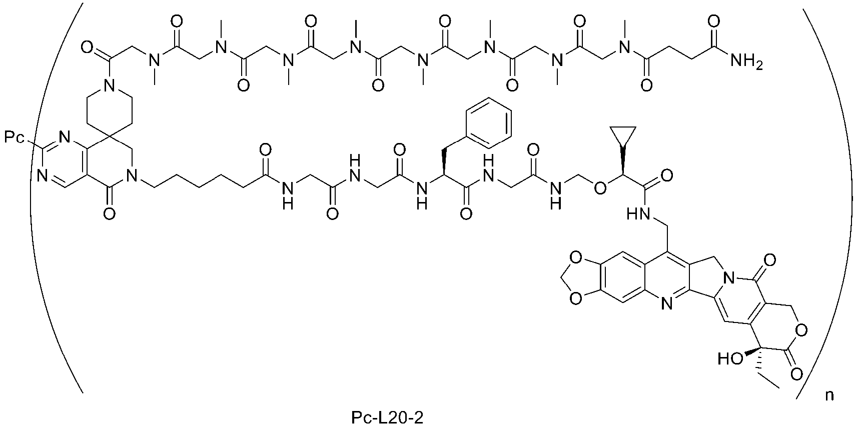

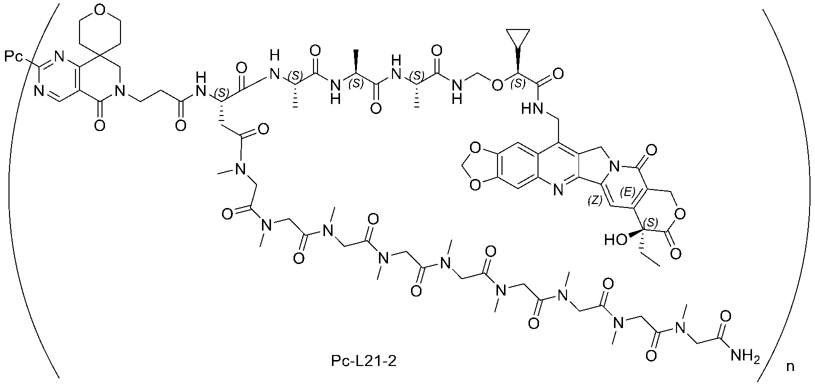

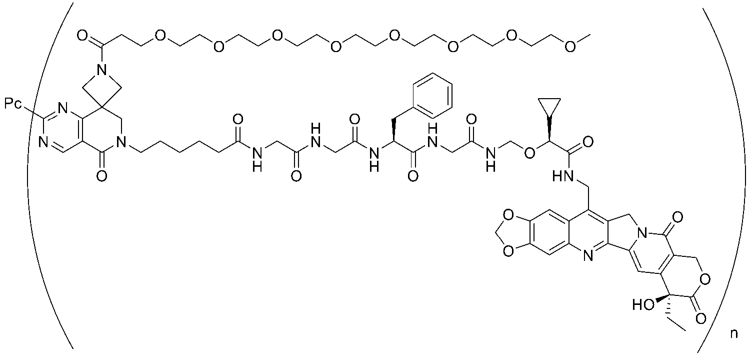

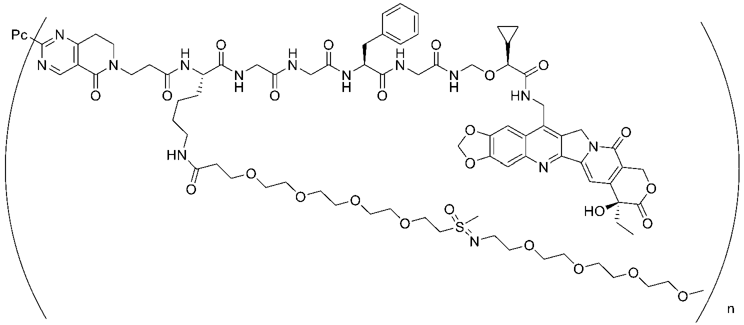

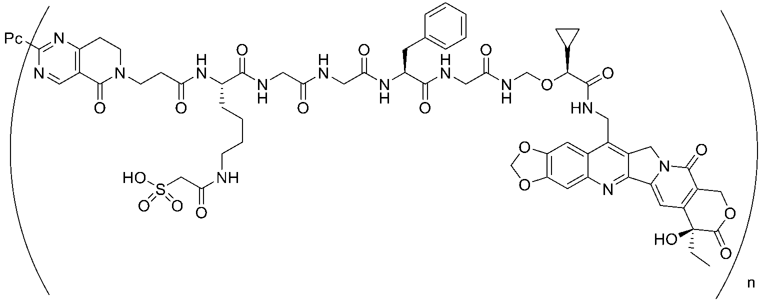

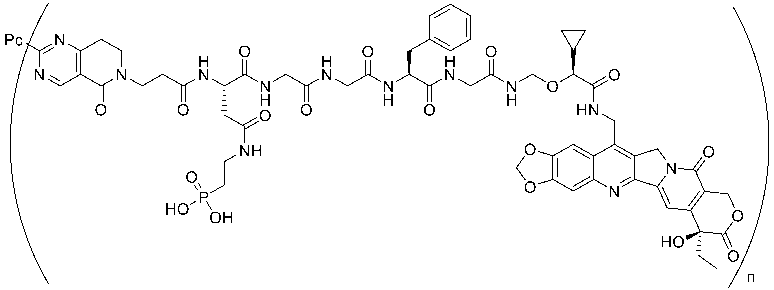

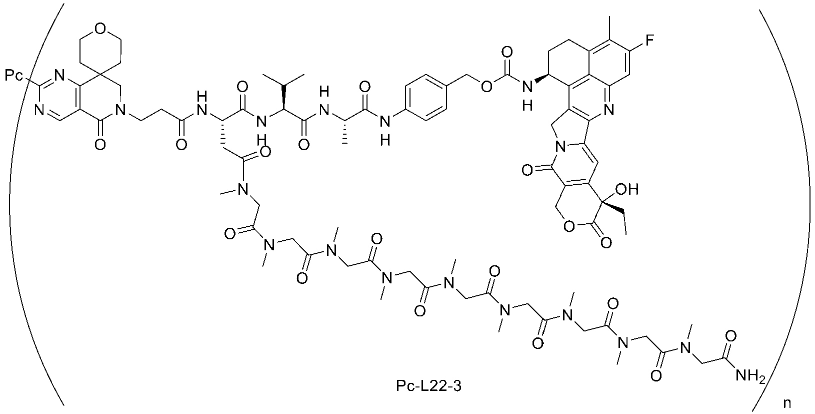

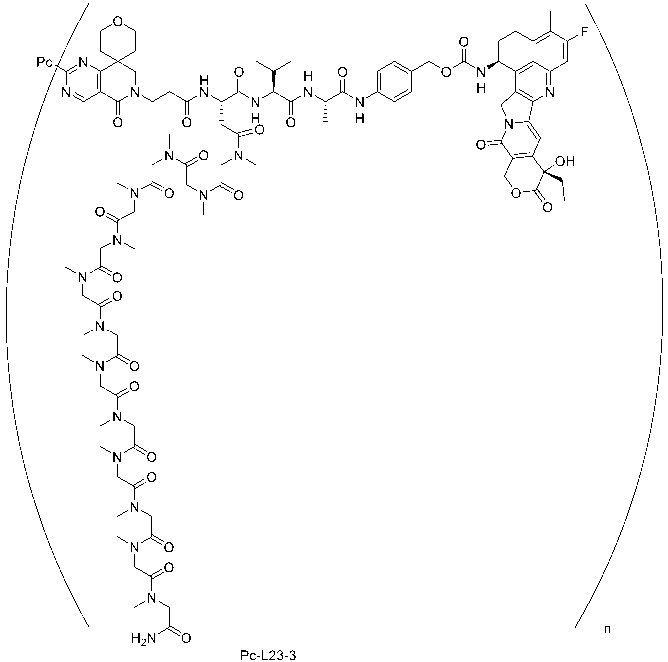

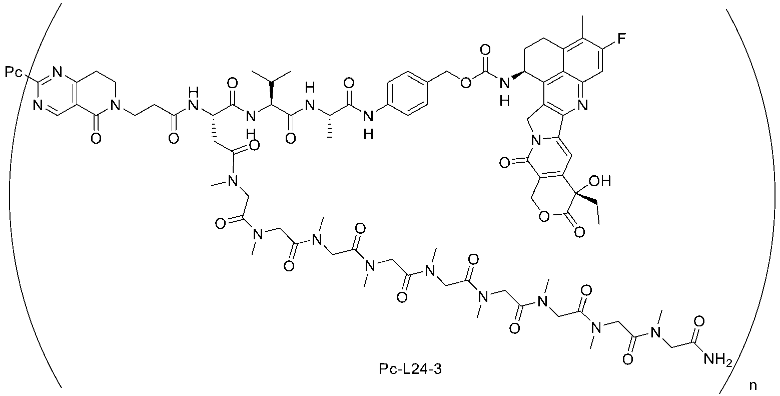

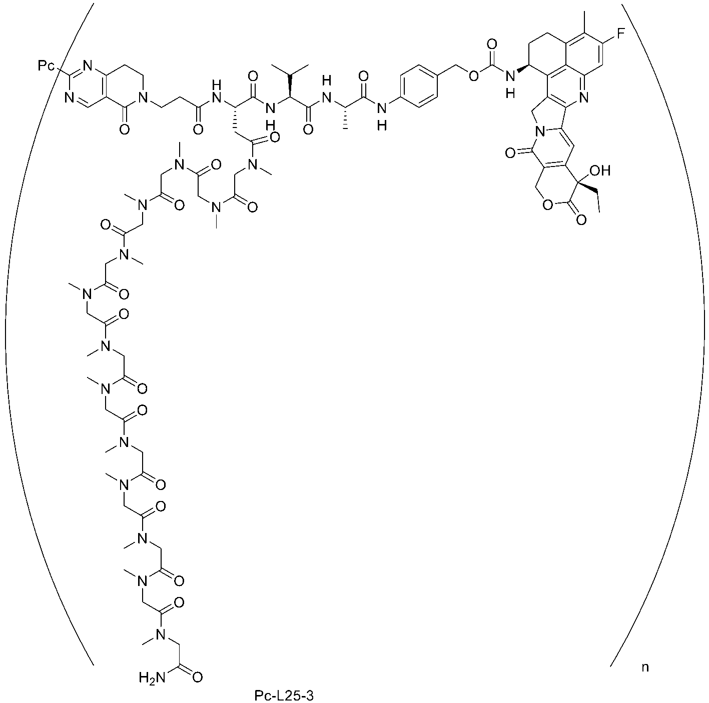

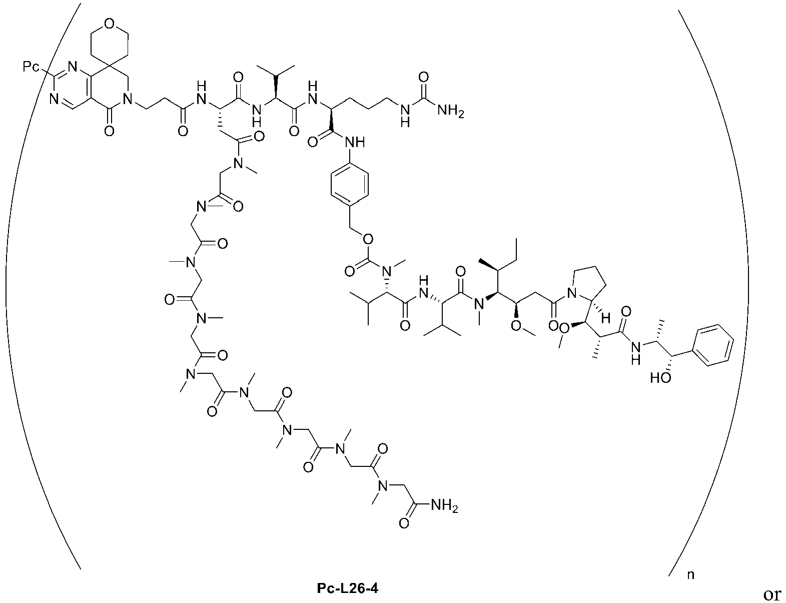

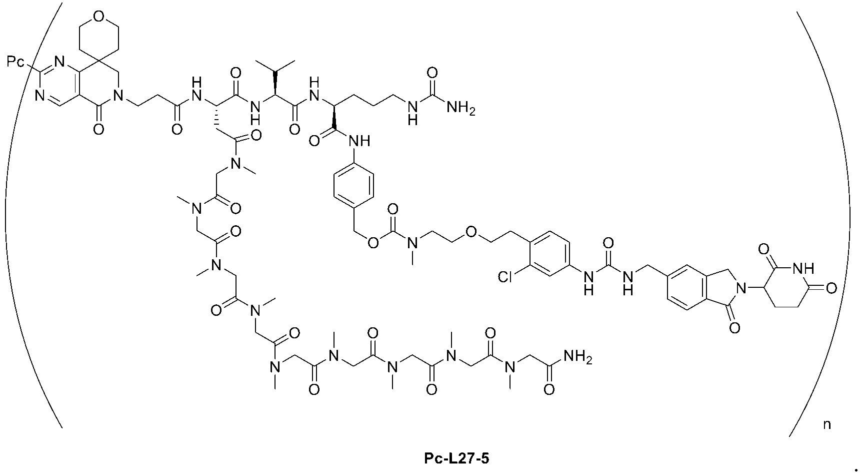

In some embodiments, ligand unit Pc may be selected from a polypeptide, an antibody, or an antigen-binding fragment thereof.

In some embodiments, ligand unit Pc may specifically bind to one or more antigens selected from the group consisting of: HER2 (ErbB2), p95HER2, HER3 (ErbB3), CD3, CD16, ROR1, DLL3, CDH6, CD70, CD5, CD20, BCMA, EGFR, VEGF, Trop-2, Claudin6, Claudin18.2, c-MET, CD30, MUC1, Nectin-4, CD22, CD74, CD19, CD79b, MSLN, TOPO2, EpCAM, CEACAM-5, Mesothelin, PD-L1, PSMA, ROR1, ROR2, CD25, CD33, CD123, FLT3, CD174, CD166, CD326, CD71, LIV-1, MUC16, ENPP3, TDGF1, ETBR, TIM1, TIM3, LRRC15, CanAg/AFP, FAP, SLITRK6, KIT/CD117, STEAP1, SLAMF7/CS1, NaPi2B/SLC34A2, GPNMB, AXL, B7-H3 (CD276), PTK7/CCK4, PRLR, EFNA4, 5T4, NOTCH3, CD142, CA6, GPR20, EphA2, LYPD3, FGFR2, FGFR3, FRα, CEACAMs, GCC, IntegrinAv, CAIX, P-cadherin, GD3, cadherin6, LAMP1, CD56, CD37, CD47, CD138, or CD352.

In some embodiments, ligand unit Pc may specifically bind to HER2.

In some embodiments, Pc comprises an antibody or an antigen-binding fragment thereof that specifically binds to HER2; the antibody or the antigen-binding fragment thereof comprises a heavy chain variable region (VH) or/and a light chain variable region (VL), wherein:
(1) the heavy chain variable region comprises an HCDR1 having the sequence set forth in SEQ ID NO. 5, an HCDR2 having the sequence set forth in SEQ ID NO. 6, and an HCDR3 having the sequence set forth in SEQ ID NO. 7, or/and the light chain variable region comprises an LCDR1 having the sequence set forth in SEQ ID NO. 8, an LCDR2 having the sequence set forth in SEQ ID NO. 9, and an LCDR3 having the sequence set forth in SEQ ID NO. 10; or,
(2) the heavy chain variable region comprises an amino acid sequence having at least 80%, 85%, 90%, 91%, 92%, 93%, 94%, 95%, 96%, 97%, 98%, 99%, or 100% identity to, or having at most 3, 2, or 1 insertion, deletion, or substitution mutations compared with each CDR of (1), and/or the light chain variable region comprises an amino acid sequence having at least 80%, 85%, 90%, 91%, 92%, 93%, 94%, 95%, 96%, 97%, 98%, 99%, or 100% identity to, or having at most 3, 2, or 1 insertion, deletion, or substitution mutations compared with each CDR of (1).

In some embodiments, the antibody or the antigen-binding fragment comprises a heavy chain variable region (VH) or/and a light chain variable region (VL), wherein:
(1) the heavy chain variable region comprises the sequence set forth in SEQ ID NO. 1, or/and the light chain variable region comprises the sequence set forth in SEQ ID NO. 2; or,
(2) the heavy chain variable region or/and the light chain variable region comprises a sequence having at least 80%, 85%, 90%, 91%, 92%, 93%, 94%, 95%, 96%, 97%, 98%, 99%, or 100% identity to, or having at most 3, 2, or 1 insertion, deletion, or substitution mutations compared with the heavy chain variable region or/and the light chain variable region in group (1) described above.

In some embodiments, ligand unit Pc may specifically bind to CDH6.

In some embodiments, Pc comprises an antibody or an antigen-binding fragment thereof that specifically binds to CDH6; the antibody or the antigen-binding fragment thereof comprises a heavy chain variable region (VH) or/and a light chain variable region (VL), wherein:
(1) the heavy chain variable region comprises an HCDR1 having the sequence set forth in SEQ ID NO. 17, an HCDR2 having the sequence set forth in SEQ ID NO. 18, and an HCDR3 having the sequence set forth in SEQ ID NO. 19, or/and the light chain variable region comprises an LCDR1 having the sequence set forth in SEQ ID NO. 20, an LCDR2 having the sequence set forth in SEQ ID NO. 21, and an LCDR3 having the sequence set forth in SEQ ID NO. 22; or,
(2) the heavy chain variable region comprises an amino acid sequence having at least 80%, 85%, 90%, 91%, 92%, 93%, 94%, 95%, 96%, 97%, 98%, 99%, or 100% identity to, or having at most 3, 2, or 1 insertion, deletion, or substitution mutations compared with each CDR of (1), and/or the light chain variable region comprises an amino acid sequence having at least 80%, 85%, 90%, 91%, 92%, 93%, 94%, 95%, 96%, 97%, 98%, 99%, or 100% identity to, or having at most 3, 2, or 1 insertion, deletion, or substitution mutations compared with each CDR of (1).

In some embodiments, the antibody or the antigen-binding fragment comprises a heavy chain variable region (VH) or/and a light chain variable region (VL), wherein:
(1) the heavy chain variable region comprises the sequence set forth in SEQ ID NO. 15, or/and the light chain variable region comprises the sequence set forth in SEQ ID NO. 16; or,
(2) the heavy chain variable region or/and the light chain variable region comprises a sequence having at least 80%, 85%, 90%, 91%, 92%, 93%, 94%, 95%, 96%, 97%, 98%, 99%, or 100% identity to, or having at most 3, 2, or 1 insertion, deletion, or substitution mutations compared with the heavy chain variable region or/and the light chain variable region in group (1) described above.

In some embodiments, the antibody or the antigen-binding fragment comprises a heavy chain constant region sequence and/or a light chain constant region sequence; optionally, the heavy chain constant region and/or the light chain constant region comprises an intact constant region sequence or a fragment thereof, and the constant region fragment comprises a CH1, a hinge region, a CH2, a CH3, or an Fc; optionally, the heavy chain constant region is selected from IgG1, IgG2, IgG3, or IgG4 constant regions of a human or murine origin, or/and, the light chain constant region is selected from kappa constant regions or lambda constant regions of a human and murine origin; optionally, the antibody or the antigen-binding fragment comprises an intact heavy chain and light chain, the heavy chain consists of the VH and the heavy chain constant region, the heavy chain constant region comprises the sequence set forth in SEQ ID NO. 3, the light chain consists of the VL and the light chain constant region, and the light chain constant region comprises the sequence set forth in SEQ ID NO. 4.

In some embodiments, the antibody or the antigen-binding fragment is:
(1) a chimeric antibody or a fragment thereof;
(2) a humanized antibody or a fragment thereof; and/or,
(3) a fully human antibody or a fragment thereof;
preferably, the antibody or the antigen-binding fragment is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', a F(ab')2, an Fd, an Fv, an scFv, a diabody, or a single domain antibody.

In some embodiments, the antigen-binding fragment is selected from one or more of a F(ab)2, a Fab', a Fab, an Fv, an scFv, a bispecific antibody, a nanobody, or an antibody minimal recognition unit.

In some embodiments, ligand unit Pc specifically binds to EGFR and c-Met.

In some embodiments, ligand unit Pc specifically binding to EGFR and c-Met comprises at least a bispecific antibody; preferably, the bispecific antibody is at least bivalent; preferably, the bispecific antibody comprises two different Fc regions, and the Fc comprises a heterodimerization-promoting mutation, such as a Knob-into-Hole mutation.

In some embodiments, ligand unit Pc comprises first, second, third, and fourth polypeptide chains:
(1) the first polypeptide chain comprises an antibody heavy chain variable region domain targeting EGFR, and the first polypeptide chain comprises the amino acid sequence set forth in SEQ ID NO. 11;
(2) the second polypeptide chain comprises an antibody heavy chain variable region domain targeting c-Met, and the second polypeptide chain comprises the amino acid sequence set forth in SEQ ID NO. 12;
(3) the third polypeptide chain comprises an antibody light chain variable region domain targeting EGFR, and the third polypeptide chain comprises the amino acid sequence set forth in SEQ ID NO. 13; and
(4) the fourth polypeptide chain comprises an antibody light chain variable region domain targeting c-Met, and the fourth polypeptide chain comprises the amino acid sequence set forth in SEQ ID NO. 14.

In some embodiments, n is selected from real numbers of 1-8, for example, n is 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0; preferably, n is selected from real numbers of 2-8, and more preferably, n is selected from real numbers of 6-8.

In a second aspect, the present disclosure provides a linker unit of formula (L), wherein the linker unit is used for linking a ligand unit and a drug unit to give a ligand-drug conjugate, 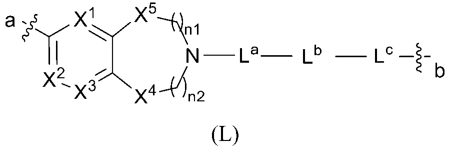 wherein X¹, X², X³, X⁴, X⁵, L^{a}, L^{b}, L^{c}, n1, and n2 are as defined in any one of the embodiments of the first aspect described above, the a-terminus of L is linked to the ligand unit, and the b-terminus is linked to the drug unit.

In a third aspect, the present disclosure provides a drug-linker compound of formula (II) or a pharmaceutically acceptable salt thereof, 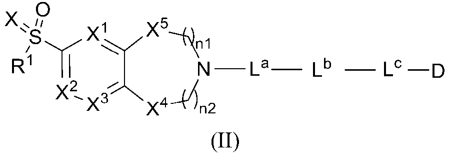 wherein X¹, X², X³, X⁴, X⁵, L^{a}, L^{b}, L^{c}, n1, n2, and drug unit D are as defined in any one of the items of the first aspect described above;
X is selected from being absent, O, or NR^{e1};
R¹ is selected from N(R^{e2})(R^{e3}), C₁-C₁₀ alkyl, C₃-C₁₀ cycloalkyl, 4- to 14-membered heterocyclyl, C₆-C₁₀ aryl, or 5-to 10-membered heteroaryl, wherein the C₁-C₁₀ alkyl, C₃-C₁₀ cycloalkyl, 4- to 14-membered heterocyclyl, C₆-C₁₀ aryl, or 5- to 10-membered heteroaryl is optionally substituted with one or more R^{f}, wherein each R^{f} is independently selected from halogen, CN, =O, C₁-C₃ alkyl, OH, O(C₁-C₃ alkyl), NH₂, NH(C₁-C₃ alkyl), or N(C₁-C₃ alkyl)₂;
R^{e1}, R^{e2}, and R^{e3} are each independently selected from H, C₁-C₆ alkyl, C₃-C₆ cycloalkyl, or 4- to 7-membered heterocyclyl.

In some embodiments, X is selected from O.

In some embodiments, R¹ is selected from C₁-C₆ alkyl or C₃-C₆ cycloalkyl, wherein the C₁-C₆ alkyl or C₃-C₆ cycloalkyl is optionally substituted with one or more R^{f}.

In some embodiments, R¹ is selected from methyl, *tert*-butyl, or cyclopentyl.

In some embodiments, the drug-linker compound of formula (II) or the pharmaceutically acceptable salt thereof is selected from the following compounds or pharmaceutically acceptable salts thereof:

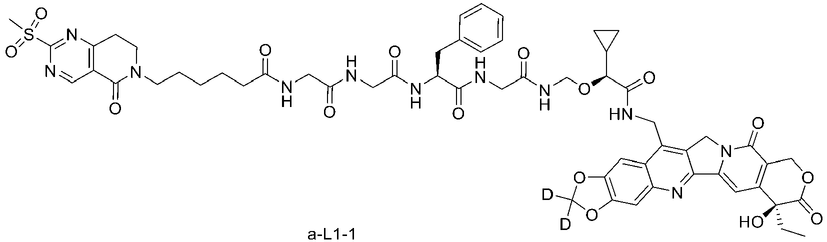

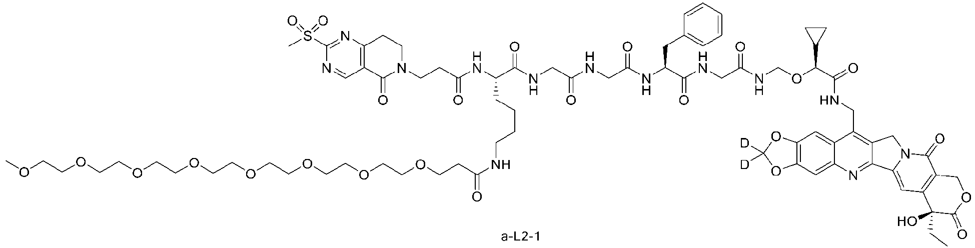

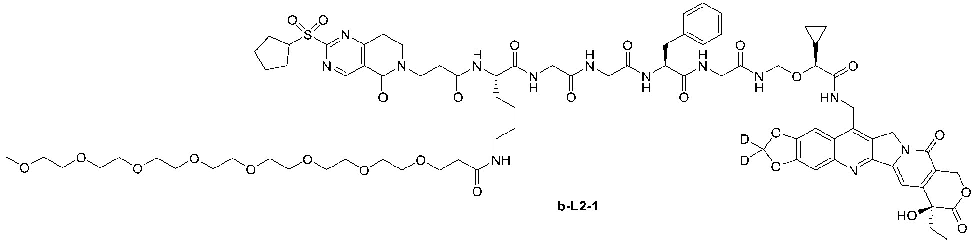

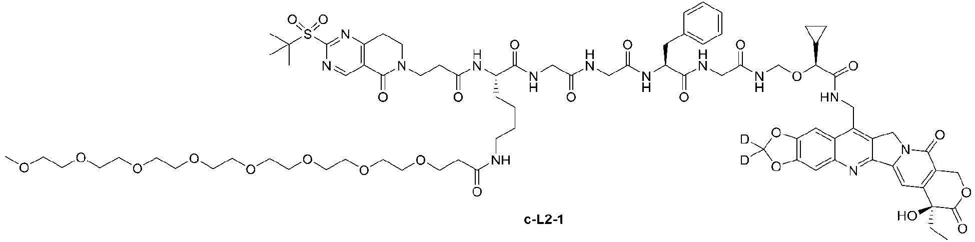

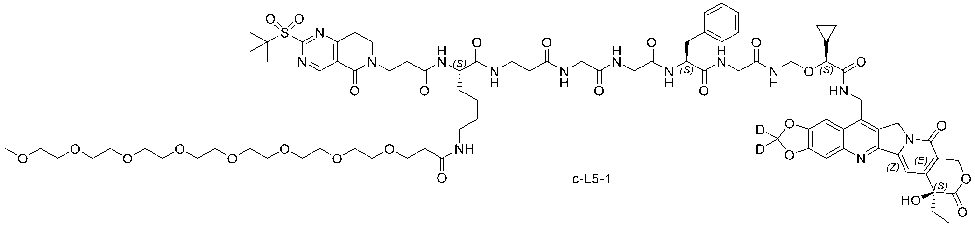

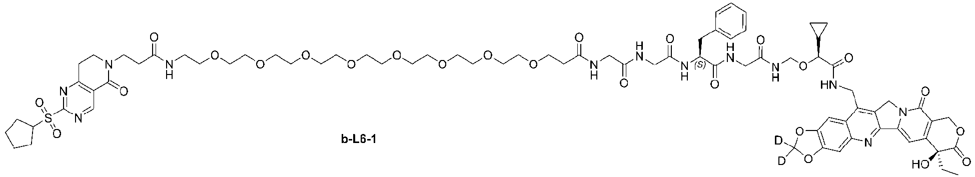

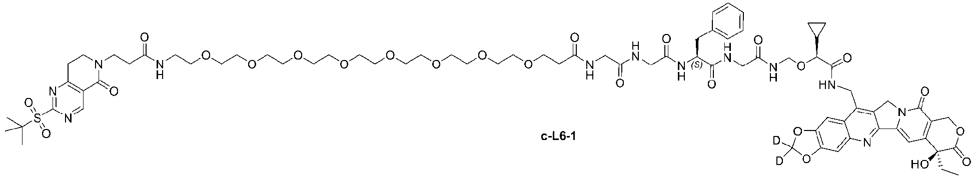

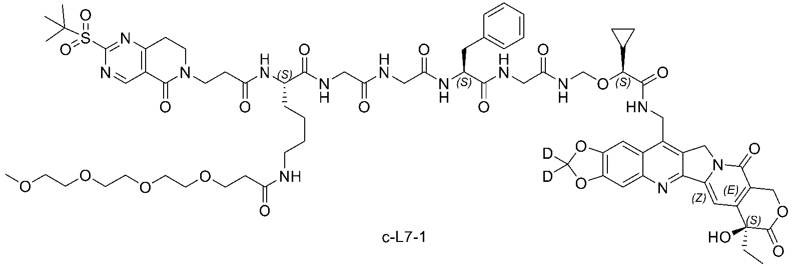

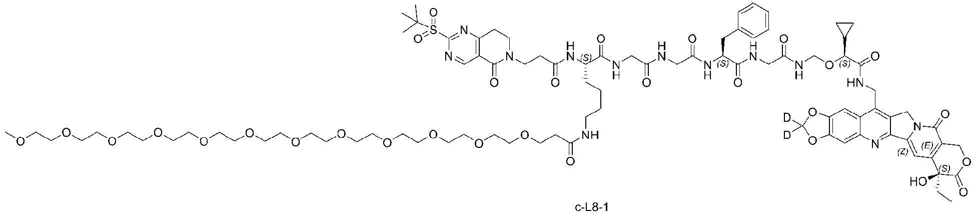

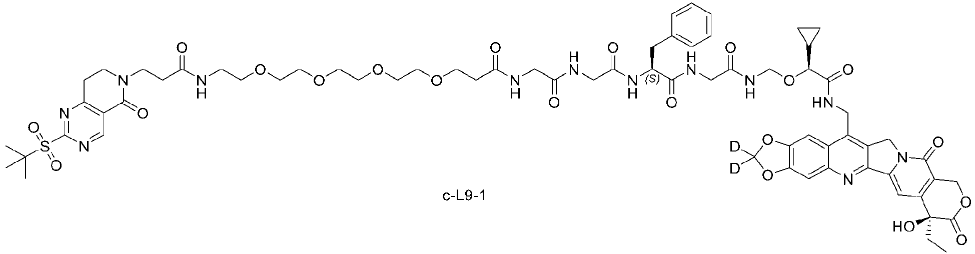

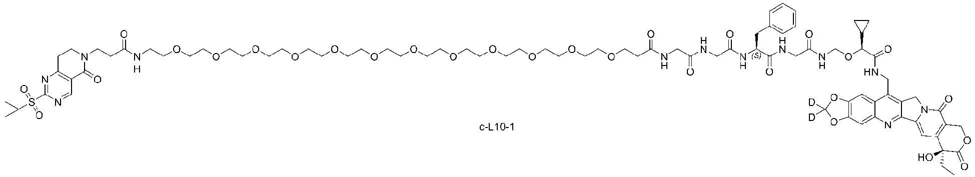

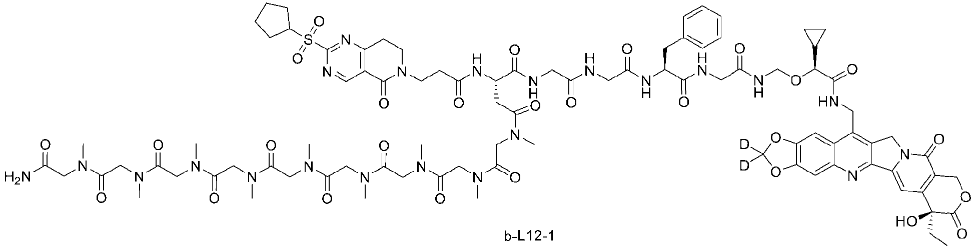

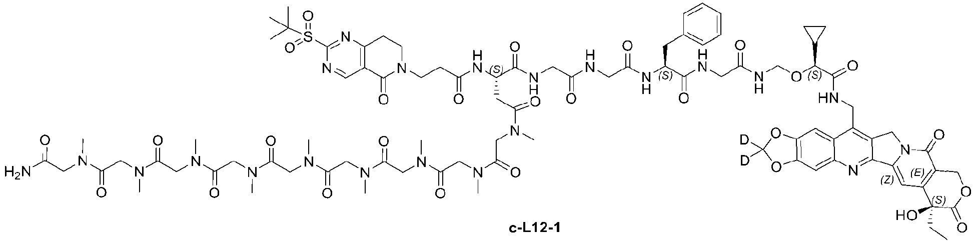

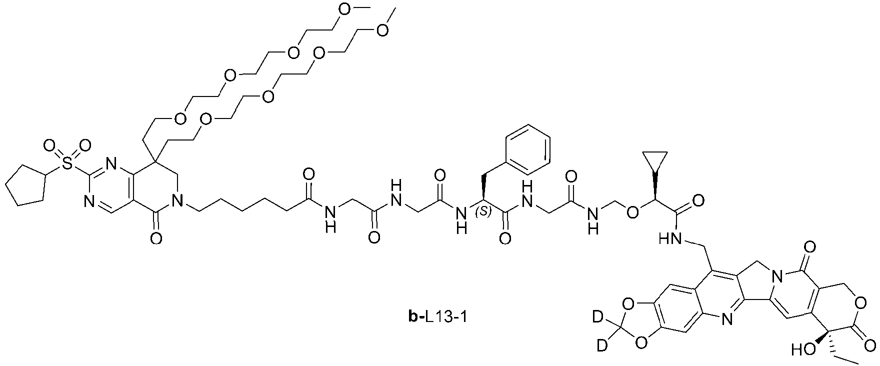

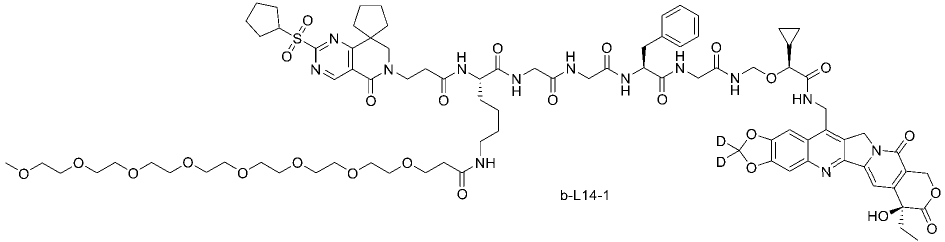

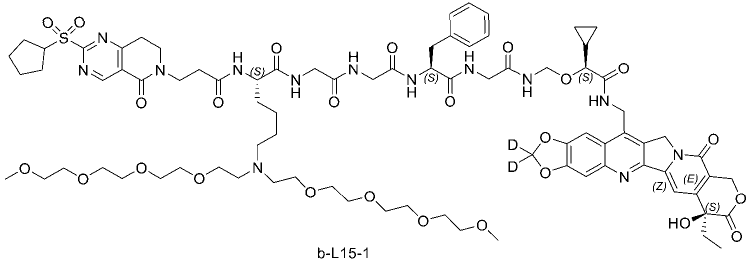

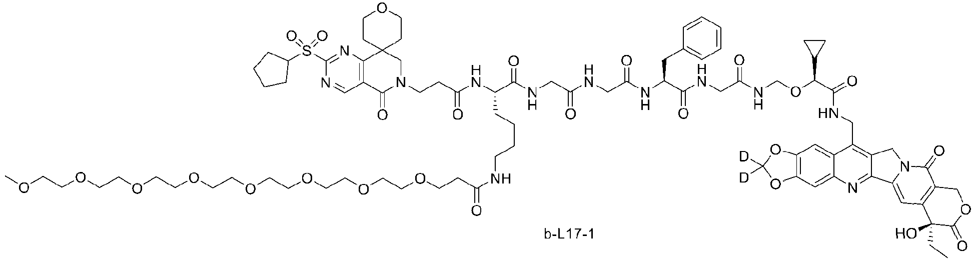

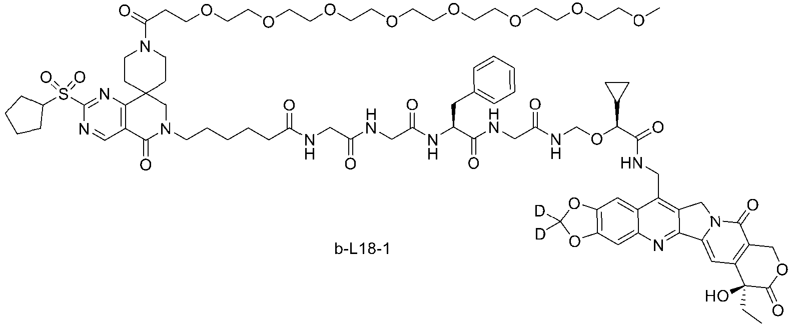

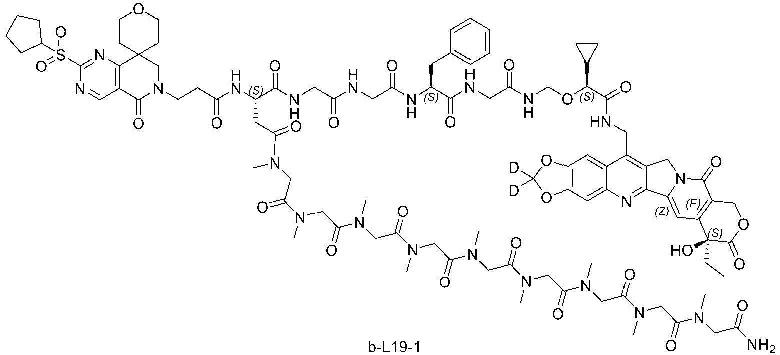

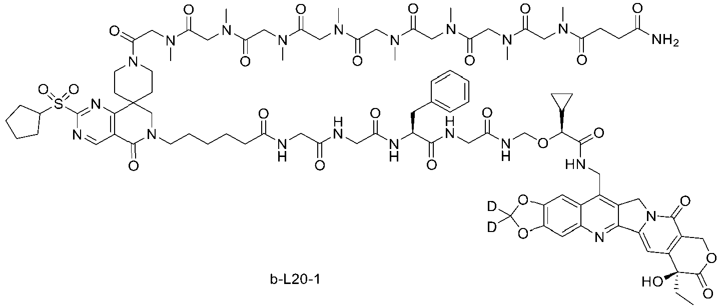

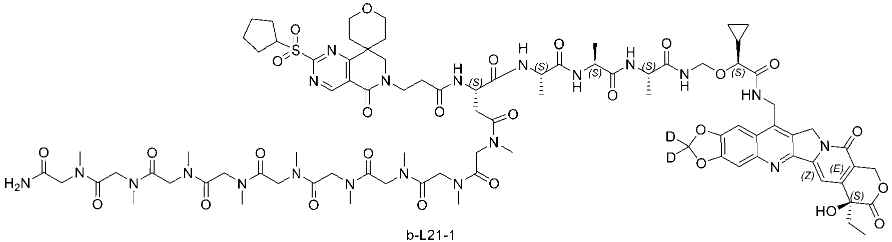

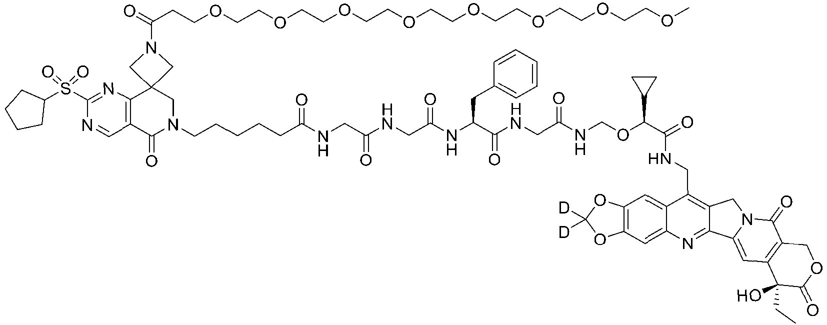

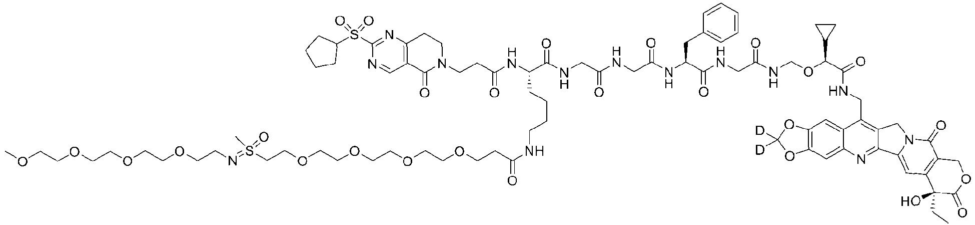

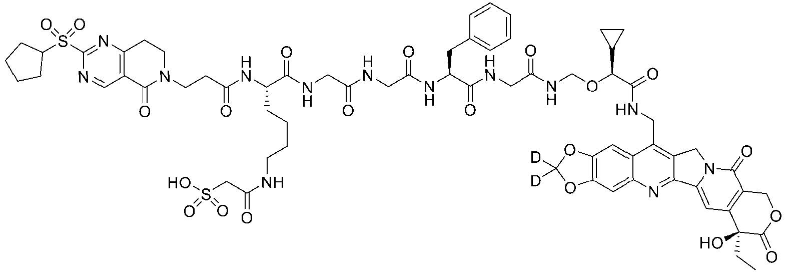

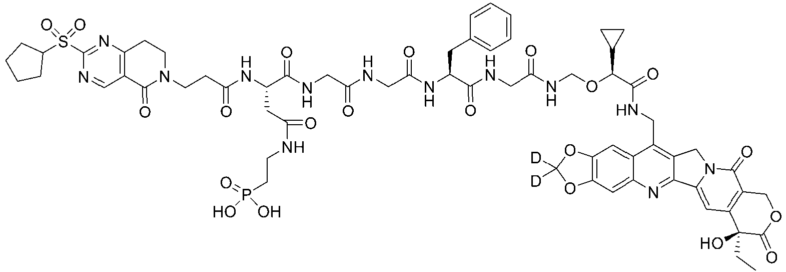

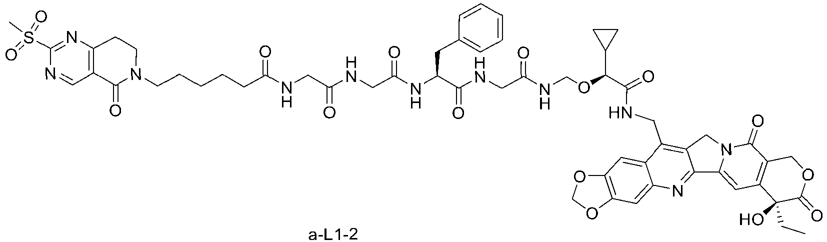

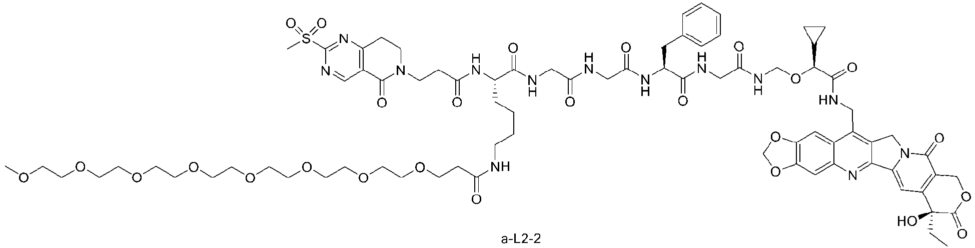

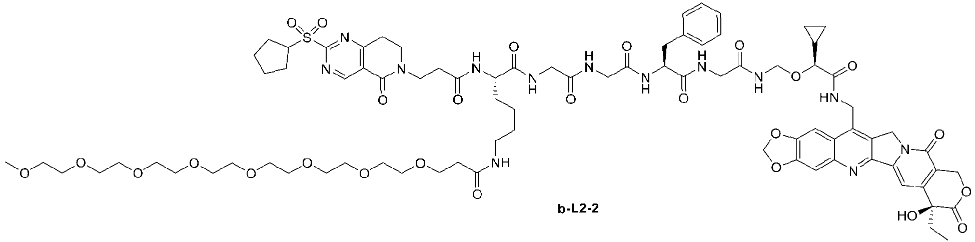

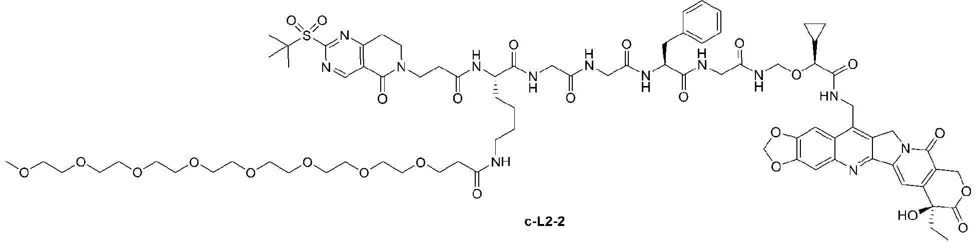

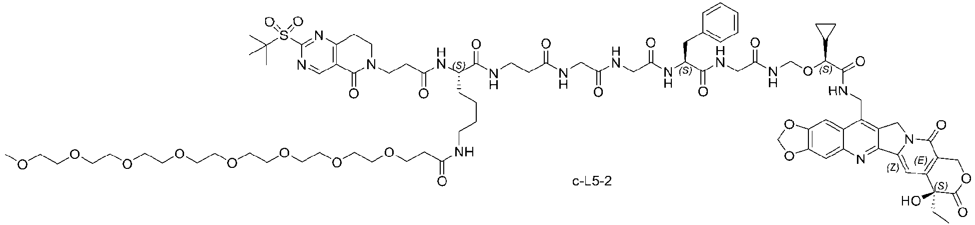

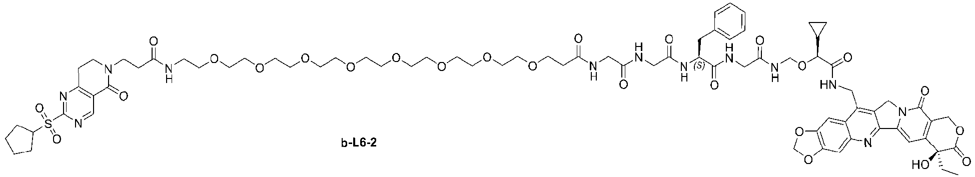

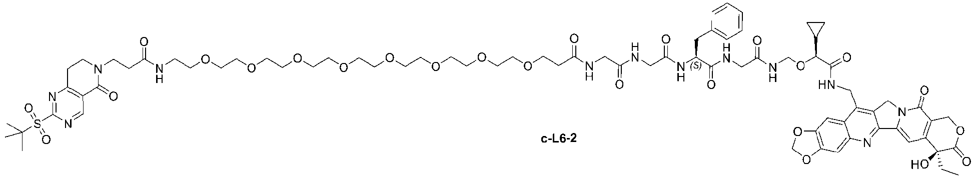

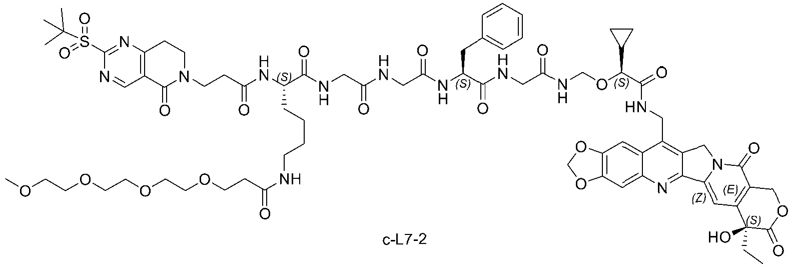

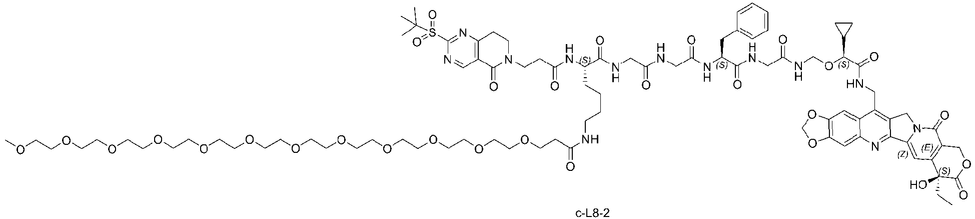

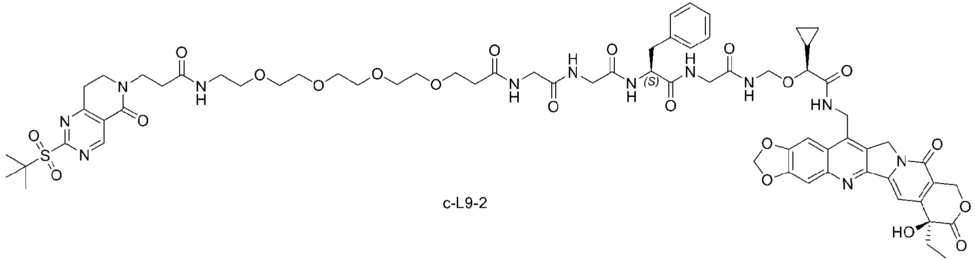

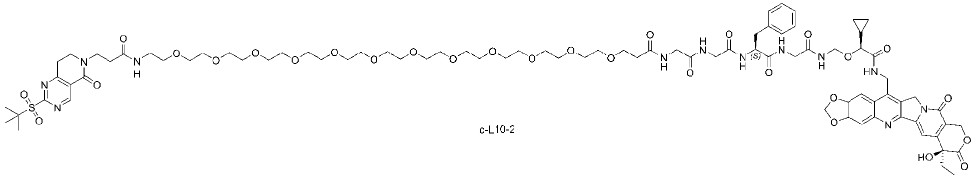

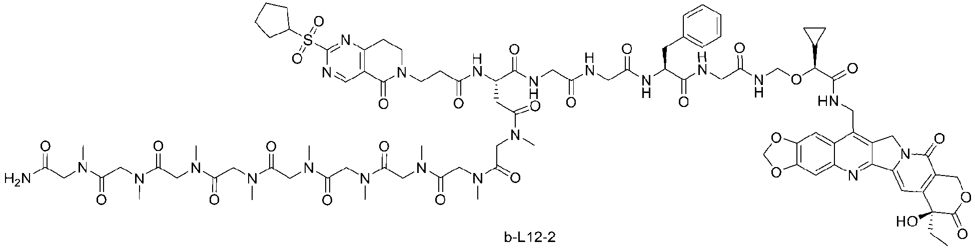

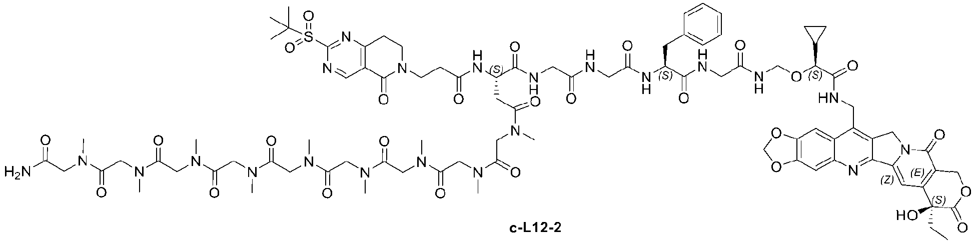

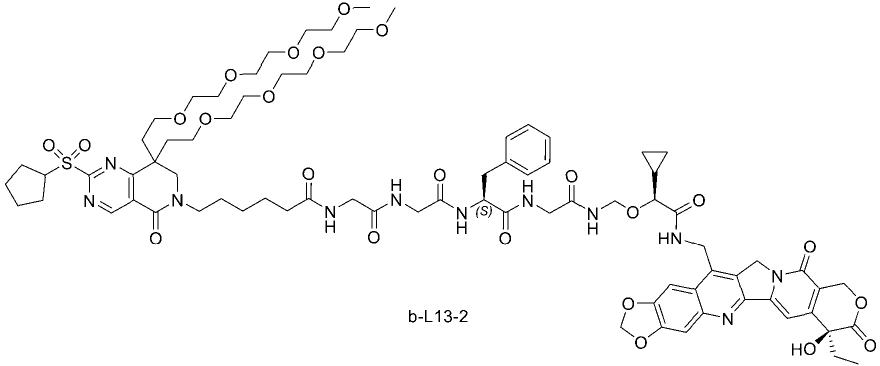

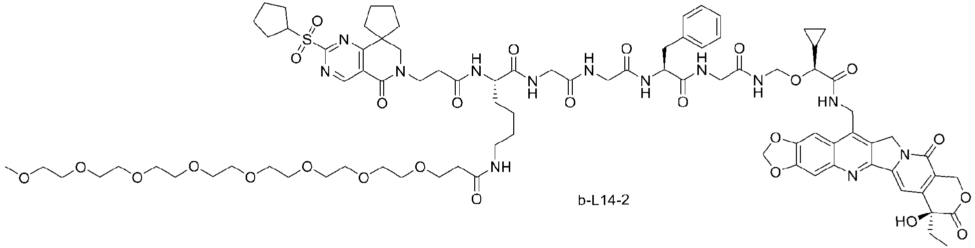

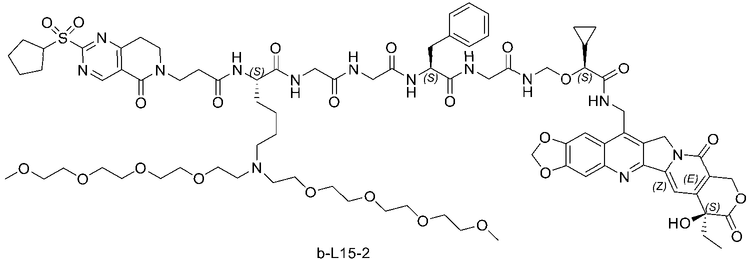

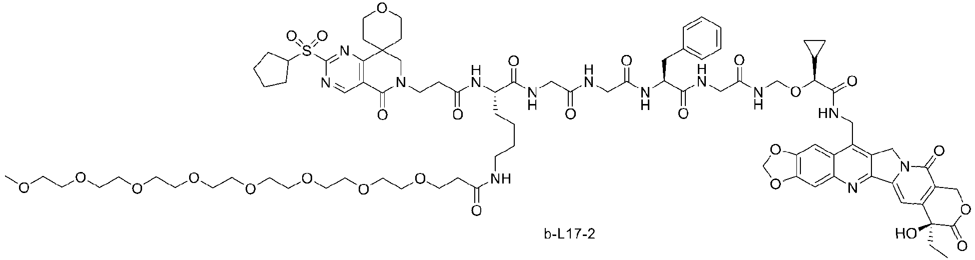

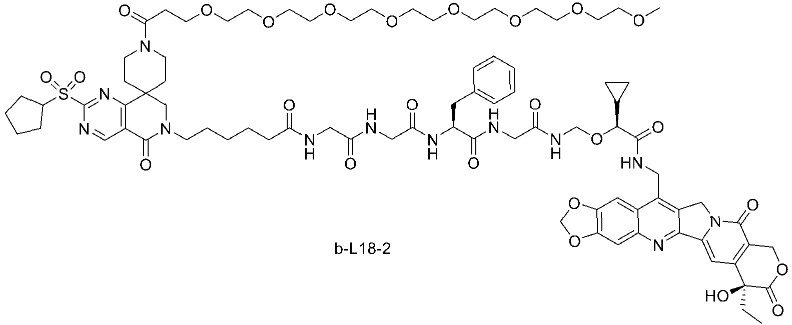

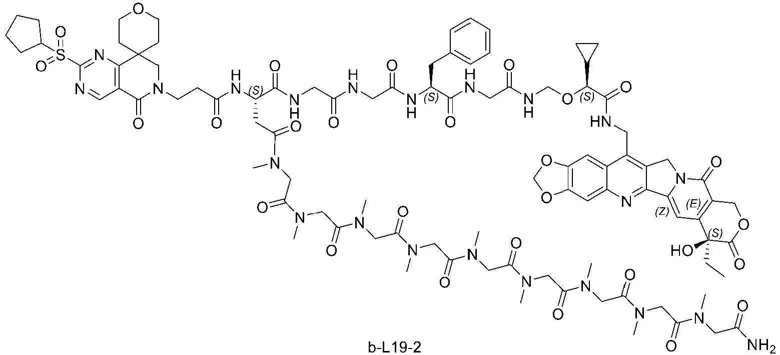

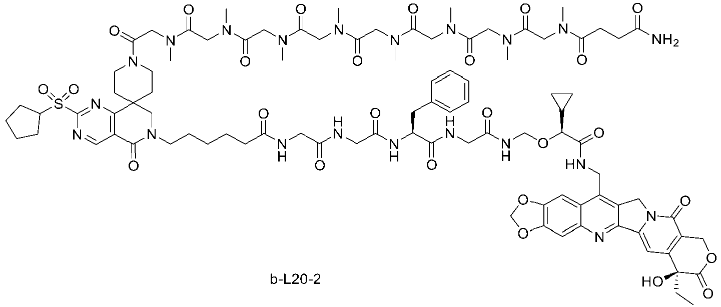

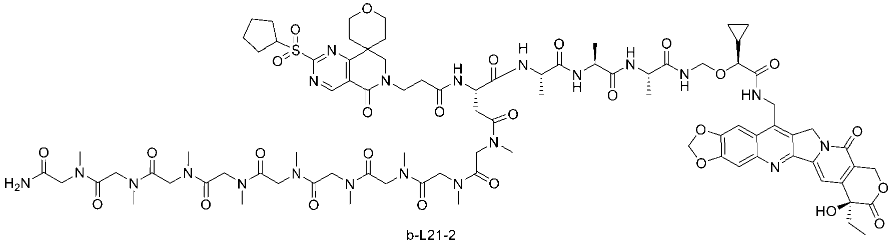

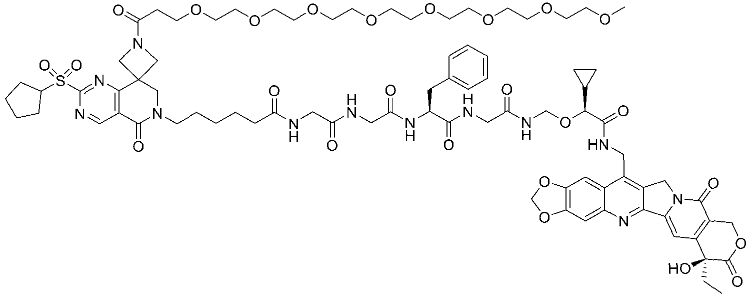

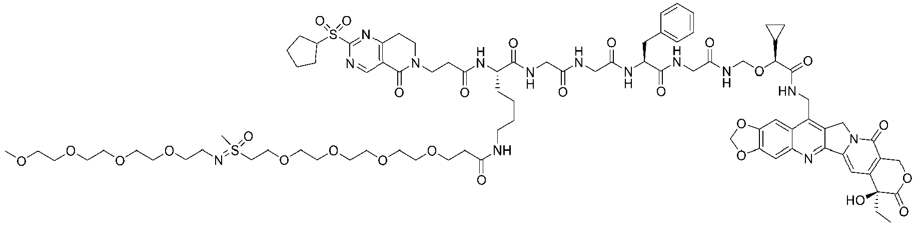

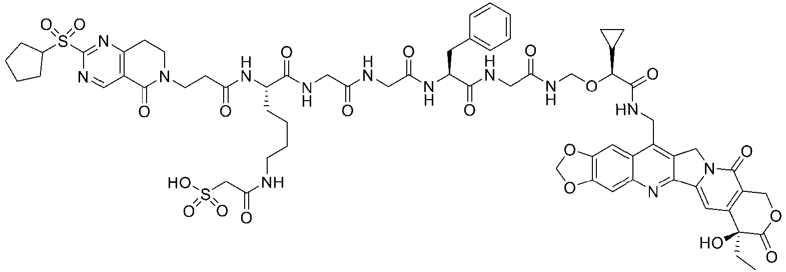

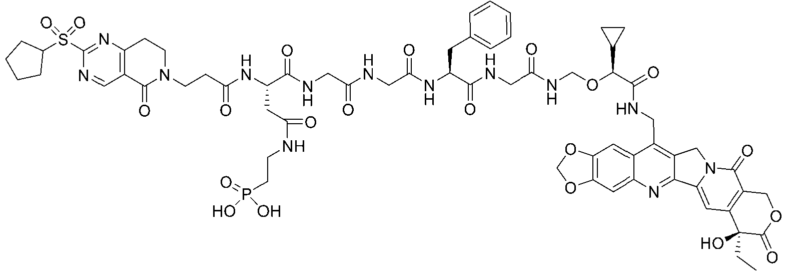

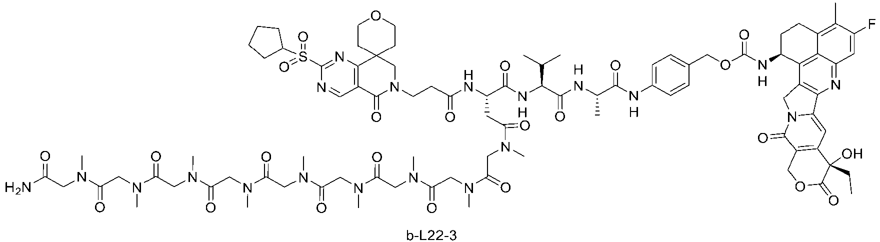

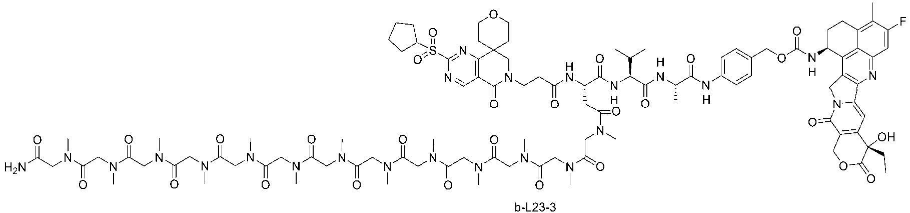

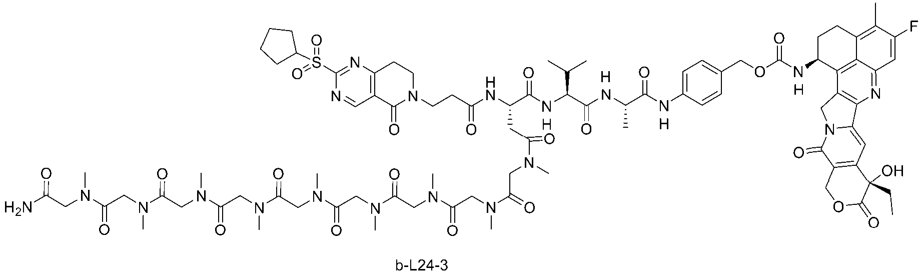

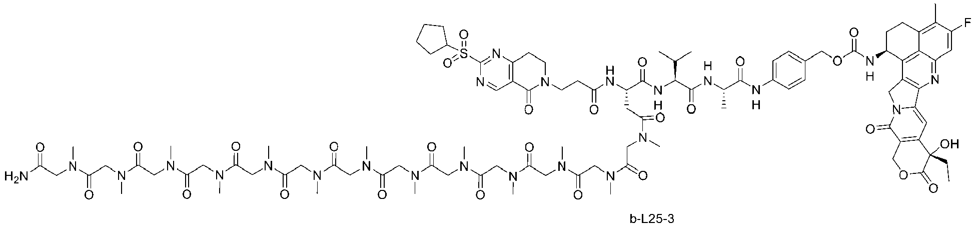

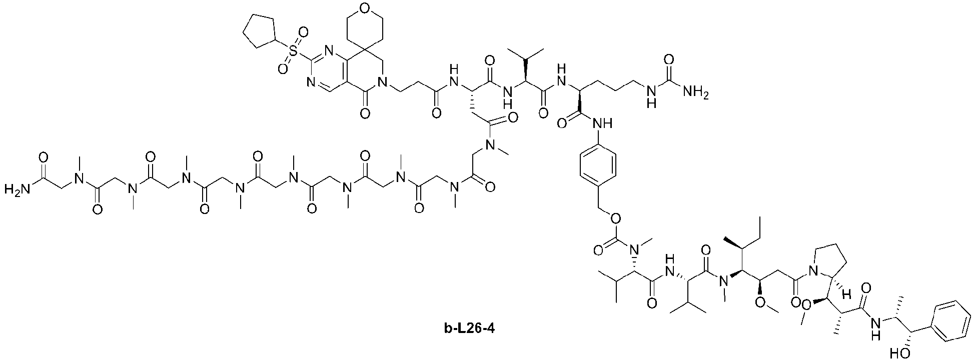

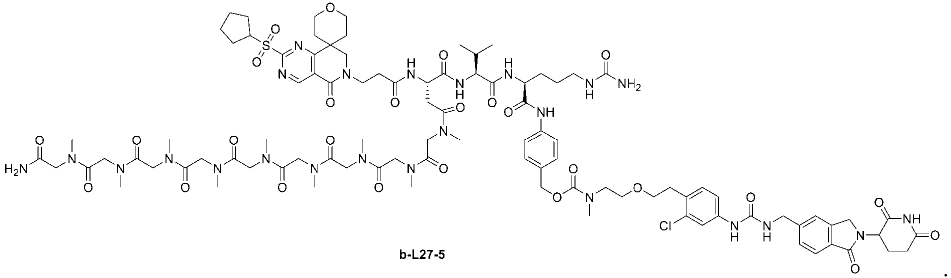

In another aspect, the present disclosure provides a pharmaceutical composition comprising the aforementioned ligand-drug conjugate having the general formula of Pc-(L-D)ₙ or the pharmaceutically acceptable salt thereof of the present disclosure, and a pharmaceutically acceptable excipient.

In another aspect, the present disclosure provides a method for treating a mammalian tumor, which comprises administering to a mammal, preferably a human, in need of the treatment a therapeutically effective amount of the aforementioned ligand-drug conjugate having the general formula of Pc-(L-D)ₙ or the pharmaceutically acceptable salt thereof, or a pharmaceutical composition thereof.

In another aspect, the present disclosure provides use of the aforementioned ligand-drug conjugate having the general formula of Pc-(L-D)ₙ or the pharmaceutically acceptable salt thereof, or a pharmaceutical composition thereof in the manufacture of a medicament for treating a tumor.

In another aspect, the present disclosure provides use of the aforementioned ligand-drug conjugate having the general formula of Pc-(L-D)ₙ or the pharmaceutically acceptable salt thereof, or a pharmaceutical composition thereof in treating a tumor.

In another aspect, the present disclosure provides the aforementioned ligand-drug conjugate having the general formula of Pc-(L-D)ₙ or the pharmaceutically acceptable salt thereof, or a pharmaceutical composition thereof for use in treating a tumor.

In another aspect, the present disclosure provides a method for preparing the ligand-drug conjugate having the general formula of Pc-(L-D)ₙ or the pharmaceutically acceptable salt thereof, which comprises a step of conjugating the aforementioned drug-linker compound having the general formula of (II) to the aforementioned ligand of the present disclosure, wherein optionally, the ligand is an antibody or an antigen-binding fragment thereof.

In another aspect, the present disclosure provides a method for preparing the ligand-drug conjugate having the general formula of Pc-(L-D)ₙ or the pharmaceutically acceptable salt thereof, which comprises a step of linking the aforementioned drug unit D to the aforementioned ligand unit Pc of the present disclosure, wherein optionally, the aforementioned drug unit D is linked to the aforementioned ligand unit Pc via the aforementioned linker unit L; optionally, ligand unit Pc is an antibody or an antigen-binding fragment thereof.

The ligand-drug conjugate provided by the present disclosure has significant anti-tumor activity and/or reduced toxic and side effects and/or improved product uniformity and/or higher stability.

### BRIEF DESCRIPTION OF THE DRAWINGS

FIG. 1 shows the inhibition of tumor growth in an HCC827 subcutaneous tumor model by an exemplary EGFR-cMet ADC of the present disclosure and control ADCs.
FIG. 2 shows the effect of an exemplary EGFR-cMetADC of the present disclosure and control ADCs on the mouse body weight in an HCC827 subcutaneous tumor model.
FIG. 3 shows the inhibition of tumor growth in an HCT116 subcutaneous tumor model by an exemplary EGFR-cMet ADC of the present disclosure and a control ADC.
FIG. 4 shows the effect of an exemplary EGFR-cMet ADC of the present disclosure and a control ADC on the mouse body weight in an HCT116 subcutaneous tumor model.
FIG. 5 shows the inhibition of tumor growth in a PC9-6 subcutaneous tumor model by an exemplary EGFR-cMet ADC of the present disclosure and a control ADC.
FIG. 6 shows the effect of an exemplary EGFR-cMet ADC of the present disclosure and a control ADC on the mouse body weight in a PC9-6 subcutaneous tumor model.
FIG. 7 shows the inhibition of tumor growth in a PA-1 subcutaneous tumor model by exemplary CDH-6 ADCs of the present disclosure and control ADCs.

### TERMINOLOGY AND DEFINITIONS

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be understood according to its common meaning in the field. When referring to a trade name, it is intended to refer to its corresponding commercial product or its active ingredient.

The term "ligand" refers to a macromolecular compound capable of recognizing and binding to an antigen or receptor associated with a target cell. The role of ligands is to present the drug to a target cell population to which the ligands bind, and these ligands include, but are not limited to, protein hormones, lectins, growth factors, antibodies, or other molecules capable of binding to the cells. In an embodiment of the present disclosure, the ligand or ligand unit is denoted as Pc, and the ligand may form a linking bond with the linker unit through a heteroatom on the ligand. In some embodiments of the present disclosure, ligand unit Pc may comprise a heteroatom group (e.g., an S atom or an N atom) on the ligand for linking to the linker unit, or may not comprise the heteroatom group described above for linking to the linker unit. In some embodiments of the present disclosure, the ligand is selected from an antibody or an antigen-binding fragment, wherein the antibody is selected from a chimeric antibody, a humanized antibody, a fully human antibody, or a murine antibody; in some embodiments of the present disclosure, the antibody is a monoclonal antibody.

The term "linker" or "linker unit" refers to a chemical structural fragment or chemical bond, which is linked to a ligand at one end and linked to a drug at the other end.

The term "drug" refers to a small-molecule compound that has biological activity in an organism. In some embodiments of the present disclosure, the drug includes a glucocorticoid receptor agonist with an antiinflammatory function, a cytotoxic drug that kills or inhibits cell growth, and a protein degrader that promotes the intracellular degradation of a target protein. In some embodiments of the present disclosure, the protein degrader is a small-molecule protein degrader.

The term "ligand-drug conjugate" means that a ligand is linked to a biologically active drug via a stable linker unit. In some embodiments of the present disclosure, a "ligand-drug conjugate" is an antibody-drug conjugate (ADC), which means that a monoclonal antibody or an antibody fragment is linked to a biologically active drug via a stable linker unit.

The term "DAR" or "drug/antibody ratio" refers to the average number of small-molecule glucocorticoid receptor agonist drugs linked to each antibody molecule. In the antibody-drug conjugates of the present disclosure, the DAR is defined by the variable "n", which may be either an integer or a decimal.

The term "antibody" is used in its broadest sense and refers to a polypeptide or a combination of polypeptides that comprises a sufficient sequence from an immunoglobulin heavy chain variable region and/or a sufficient sequence from an immunoglobulin light chain variable region to be capable of specifically binding to an antigen. "Antibody" herein encompasses various forms and various structures as long as they exhibit the desired antigen binding activity. "Antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer et al., 2003, Proteins: Structure, Function, and Bioinformatics, 53(1): 121-129 (2003); and Roque et al., Biotechnol. Prog. 20: 639-654 (2004). Such scaffolds may also include non-antibody-derived scaffolds, such as scaffold proteins known in the art to be useful for grafting CDRs, including but not limited to, tenascin, fibronectin, peptide aptamers, and the like.

"Antibody" herein includes a typical "tetrabody", which belongs to an immunoglobulin consisting of two heavy chains (HCs) and two light chains (LCs). The heavy chain refers to a polypeptide chain consisting of, from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is of IgE isoform, the heavy chain optionally further comprises a heavy chain constant region CH4 domain. The light chain is a polypeptide chain consisting of, from the N-terminus to the C-terminus, a light chain variable region (VL) and a light chain constant region (CL). The heavy chains are linked to each other and to the light chains through disulfide bonds to form a Y-shaped structure. The heavy chain constant regions of immunoglobulins differ in their amino acid composition and arrangement, and thus in their antigenicity. Accordingly, "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class can also be divided into different subclasses according to the differences in the amino acid composition of the hinge regions and the number and location of disulfide bonds in the heavy chains. For example, IgG may be divided into IgG1, IgG2, IgG3, and IgG4, and IgA can be divided into IgA1 and IgA2. The light chains can be divided into κ or λ chains according to the differences in the constant regions. Each of the five classes of Ig may have a κ chain or a λ chain.

"Antibody" herein also includes antibodies that do not comprise a light chain, e.g., heavy-chain antibodies (HCAbs) produced by *Camelus dromedarius, Camelus bactrianus, Lama glama, Lama guanicoe, Vicugna pacos,* and the like, as well as Ig new antigen receptors (IgNARs) found in *Chondrichthyes,* e.g., shark.

"Antibody" herein may be derived from any animal, including but not limited to, human and non-human animals, wherein the non-human animals may be selected from primates, mammals, rodents, or vertebrates, such as Camelidae species, *Lama glama, Lama guanicoe, Vicugna pacos,* sheep, rabbits, mice, rats, or *Chondrichthyes* (e.g., shark).

"Antibody" herein includes, but is not limited to, monoclonal antibodies, polyclonal antibodies, monospecific antibodies, multispecific antibodies (e.g., bispecific antibodies), monovalent antibodies, multivalent antibodies, intact antibodies, fragments of an intact antibody, naked antibodies, conjugated antibodies, chimeric antibodies, humanized antibodies, or fully human antibodies.

The term "monoclonal antibody" refers to an antibody obtained from a population of substantially homogeneous antibodies, that is, the individual antibodies constituting the population are identical and/or bind to the same epitope, except for possible variants (e.g., containing naturally occurring mutations or arising during the production of the formulation, such variants typically being present in minor amounts). In contrast to polyclonal antibody formulations, which generally comprise different antibodies directed against different determinants (epitopes), each monoclonal antibody in a monoclonal antibody formulation is directed against a single determinant on the antigen. The modifier "monoclonal" herein is not to be construed as requiring the production of the antibody or the antigen-binding molecule by any particular method. For example, monoclonal antibodies can be prepared by a variety of techniques, including (but not limited to) a hybridoma technique, a recombinant DNA method, a phage library display technique, methods that utilize transgenic animals containing all or part of human immunoglobulin loci, and other methods known in the art.

The term "natural antibody" refers to an antibody that is produced and paired by the immune system of a multicellular organism. The term "engineered antibody" herein refers to a non-natural antibody obtained by genetic engineering, antibody engineering, and the like. Illustratively, "engineered antibody" includes humanized antibodies, antibody fragments (e.g., scFv and sdAb), bispecific antibodies, and the like.

The term "HER2" herein, also known as "human epidermal growth factor receptor 2" or ErbB2, is a cell-derived oncogene. It is a transmembrane glycoprotein with tyrosine-protein kinase activity that can activate a signal transduction system regulated by intracellular tyrosine kinases. HER2 gene copy number amplification and protein overexpression will lead to over-activation of the signal. The biological function of the signal is closely related to the formation of various cancers, malignant proliferation, invasion and metastasis, and resistance to radio chemotherapy. The oncogene and its protein product (P185) are overexpressed and amplified in various tumors. Its positive expression can be found in various cancers, such as breast cancer, ovarian cancer, gastric cancer, esophageal cancer, salivary gland tumors, lung cancer, bile duct cancer, bladder cancer, prostate cancer, and colorectal cancer.

The term "CDH6" herein, also known as "cadherin 6", refers to a cell adhesion molecule, which is a member of the cadherin family of cell-cell adhesion molecules. CDH6 is a single transmembrane protein consisting of 790 amino acids. CDH6 is classified as the Type II Cadherin family and has an N-terminal extracellular domain and a C-terminal intracellular domain. The CDH6 protein used in the present disclosure may be directly purified from CDH6-expressing cells of a human or non-human mammal (e.g., rat, mouse, or monkey) and may be subsequently used, or a cell membrane fraction of the cells described above may be prepared and may be used as a CDH6 protein. Alternatively, CDH6 may also be obtained by allowing a host cell to produce CDH6 by *in vitro* synthesis or by genetic manipulation. According to this genetic manipulation, the CDH6 protein can be obtained. Specifically, CDH6 cDNA is incorporated into a vector capable of expressing CDH6 cDNA, and then CDH6 is synthesized in a solution containing enzymes, substrates, and energy materials required for transcription and translation, or CDH6 can be produced by transforming other prokaryotic or eukaryotic host cells, thereby allowing them to express CDH6. CDH6-expressing cells or CDH6-expressing cell lines based on the genetic manipulations described above can also be used to present the CDH6 protein. Alternatively, an expression vector into which CDH6 cDNA has been incorporated can be administered directly to the animal to be immunized, and CDH6 can be expressed in the animal so immunized.

The term "epidermal growth factor receptor" or "EGFR" herein refers to human EGFR (also known as HER1 or ErbB1) having the amino acid sequence set forth in GenBank Accession No. NP_005219 and a naturally occurring variant thereof.

The term "hepatocyte growth factor receptor" or "c-Met" herein refers to human c-Met having the amino acid sequence set forth in GenBank Accession No. NP_001120972 and a natural variant thereof.

The term "monospecific" means having one or more binding sites, each of which binds to the same epitope of the same antigen.

The term "multispecific antibody" means having at least two antigen-binding sites, each of which binds to a different epitope of the same antigen or a different epitope of a different antigen. Thus, the terms such as "bispecific", "trispecific", and "tetraspecific" refer to the number of different epitopes to which an antibody/antigen-binding molecule can bind.

The term "valent" refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

"Full-length antibody", "complete antibody", and "intact antibody" herein are used interchangeably and refer to an antibody having a structure substantially similar to that of a natural antibody.

"Antigen-binding fragment" and "antibody fragment" herein are used interchangeably and do not have the entire structure of an intact antibody, but comprise only a portion of the intact antibody or a variant of the portion that has the ability to bind to an antigen. "Antigen-binding fragment" or "antibody fragment" herein includes, but is not limited to, a Fab, a Fab', a Fab'-SH, a F(ab')2, an Fv, a VHH, and an scFv.

An intact antibody is digested by papain to produce two identical antigen-binding fragments, called "Fab" fragments, each of which contains a heavy chain variable domain and a light chain variable domain, as well as a light chain constant domain and a first heavy chain constant domain (CH1). Thus, the term "Fab fragment" herein refers to an antibody fragment comprising a light chain fragment comprising the VL domain and the constant domain (CL) of a light chain, and the VH domain and the first constant domain (CH1) of a heavy chain. A Fab' fragment differs from the Fab fragment by the addition of a few residues (including one or more cysteines from an antibody hinge region) at the carboxyl terminus of the heavy chain CH1 domain. Fab'-SH is a Fab' fragment in which the cysteine residue in the constant domain carries a free thiol group. Pepsin treatment produces a F(ab')2 fragment having two antigen-binding sites (two Fab fragments) and a portion of the Fc region.

An "Fv fragment" is the smallest fragment derived from IgG and IgM, comprising the intact antigen-binding site. An Fv fragment has the same binding properties and similar three-dimensional binding properties as Fab. The VH and VL chains of the Fv fragment are bound together by a non-covalent interaction.

The term "scFv" (single-chain variable fragment) refers to a single polypeptide chain comprising VL and VH domains, wherein the VL and VH are linked through a linker (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Roseburg and Moore Ed., Springer-Verlag, New York, pp 269-315 (1994)). Such scFv molecules may have a general structure: NH2-VL-linker-VH-COOH or NH2-VH-linker-VL-COOH. An appropriate linker in the prior art consists of GGGGS amino acid sequence repeats or a variant thereof. For example, a linker having the amino acid sequence (GGGGS)4 can be used, and variants thereof can also be used (Holliger et al. (1993), Proc. Natl. Acad. Sci. USA 90: 6444-6448). Other linkers that can be used in the present disclosure are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol*.* In some cases, there may also be a disulfide bond between the VH and VL of the scFv, forming a disulfide-linked Fv (dsFv).

The term "diabody" refers to an antibody having VH and VL domains that are expressed on a single polypeptide chain, but using a linker that is too short to allow the pairing of the two domains on the same chain, thereby forcing the domains to pair with the complementary domains of the other chain and generating two antigen-binding sites (see, e.g., Holliger P. et al., Proc. Natl. Acad. Sci. USA 90: 6444-6448 (1993), and Poljak R.J. et al., Structure 2: 1121-1123 (1994)).

The term "chimeric antibody" refers to an antibody in which a portion of the light chain or/and heavy chain is derived from one antibody (which may be derived from a particular species or belong to a particular antibody class or subclass) and another portion of the light chain or/and heavy chain is derived from another antibody (which may be derived from the same or a different species or belong to the same or a different antibody class or subclass), but which nevertheless retains binding activity to a target antigen (U.S.P 4,816,567 to Cabilly et al.; Morrison et al., Proc. Natl. Acad. Sci. USA, 81: 6851 6855(1984)). For example, the term "chimeric antibody" can include an antibody (e.g., a human-murine chimeric antibody) in which the heavy and light chain variable regions of the antibody are derived from a first antibody (e.g., a murine antibody) and the heavy and light chain constant regions of the antibody are derived from a second antibody (e.g., a human antibody).

The term "humanized antibody" refers to a genetically engineered non-human antibody that has an amino acid sequence modified to increase the homology to the sequence of a human antibody. Generally, all or part of the CDRs of a humanized antibody is derived from a non-human antibody (donor antibody), and all or part of the non-CDRs (e.g., FRs and/or constant regions in variable regions) is derived from a human immunoglobulin (receptor antibody). The humanized antibody generally retains or partially retains the desired properties of the donor antibody, including but not limited to, antigen specificity, affinity, reactivity, the ability to increase the activity of immune cells, the ability to enhance immune response, and the like.

The term "fully human antibody" refers to an antibody having variable regions in which both the FRs and CDRs are derived from human germline immunoglobulin sequences. Furthermore, if the antibody comprises constant regions, the constant regions are also derived from human germline immunoglobulin sequences. "Fully human antibody" herein may include amino acid residues that are not encoded by human germline immunoglobulin sequences (e.g., mutations introduced by random or site-specific mutagenesis *in vitro* or by somatic mutations *in vivo).* However, "fully human antibody" herein does not include antibodies in which CDR sequences derived from the germline of another mammalian species (e.g., mouse) have been grafted onto human framework sequences. The term "variable region" refers to a region contained in a heavy or light chain of an antibody enabling the binding of the antibody to an antigen. "Heavy chain variable region" is used interchangeably with "VH" and "HCVR", and "light chain variable region" is used interchangeably with "VL" and "LCVR". Heavy and light chain variable domains (VH and VL, respectively) of natural antibodies generally have similar structures, and each domain contains four conservative framework regions (FRs) and three hypervariable regions (HVRs). See, e.g., Kindt et al., Kuby Immunology, 6th ed., W. H. Freeman and Co., p.91 (2007). A single VH or VL domain may be sufficient to provide antigen binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region because it is precisely complementary to an antigen epitope in a spatial structure, wherein the heavy chain variable region CDR may be abbreviated as HCDR, and the light chain variable region CDR may be abbreviated as LCDR. The term "framework region" or "FR region" herein is used interchangeably and refers to those amino acid residues of an antibody heavy chain variable region or light chain variable region, other than CDRs. Generally, a typical antibody variable region consists of 4 FRs and 3 CDRs in the following order: FR1-CDR1-FR2-CDR2-FR3-CDR3-FR4.

"CDR" herein may be annotated and defined in a manner well known in the art, including but not limited to, the Kabat numbering scheme, Chothia numbering scheme, or IMGT numbering scheme; the tool sites used include, but are not limited to, AbRSA site (http://cao.labshare.cn/AbRSA/cdrs.php), abYsis site (www.abysis.org/abysis/sequence_input/key_annotation/key_annotation.cgi), and IMGT site (http://www.imgt.org/3Dstructure-DB/cgi/DomainGapAlign.cgi#results). The CDR herein includes overlaps and subsets of amino acid residues defined in different ways.

The term "heavy chain constant region" herein refers to the carboxyl-terminal portion of an antibody heavy chain that is not directly involved in the binding of the antibody to an antigen, but exhibits effector functions, such as interaction with an Fc receptor; the heavy chain constant region has a more conserved amino acid sequence relative to the variable domain of the antibody. "Heavy chain constant region" at least comprises: a CH1 domain, a hinge region, a CH2 domain, a CH3 domain, or a variant or fragment thereof. "Heavy chain constant region" includes a "full-length heavy chain constant region" having a structure substantially similar to that of a natural antibody constant region and a "heavy chain constant region fragment" including only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; when the antibody is a heavy chain antibody, it does not comprise a CH1 domain. Illustratively, a typical "heavy chain constant region fragment" may be selected from a CH1, Fc, or CH3 domain.

The term "light chain constant region" herein refers to the carboxyl-terminal portion of an antibody light chain that is not directly involved in the binding of the antibody to an antigen. The light chain constant region may be selected from a constant κ domain or a constant λ domain.

The term "Fc" herein refers to the carboxyl-terminal portion of an antibody that is formed by the hydrolysis of an intact antibody by papain, which typically comprises the CH3 and CH2 domains of the antibody. The Fc region includes, for example, an Fc region of native sequences, a recombinant Fc region, and a variant Fc region. Although the boundaries of the Fc region of an immunoglobulin heavy chain may vary slightly, the human IgG heavy chain Fc region is generally defined to stretch from an amino acid residue at position Cys226, or from Pro230, to the carboxyl terminus thereof. The C-terminal lysine of the Fc region (residue 447 according to the Kabat numbering scheme) may be removed, for example, during production or purification of the antibody, or by recombinant engineering of the nucleic acid encoding the heavy chain of the antibody, and thus, the Fc region may or may not include Lys447.

The term "identity" herein can be obtained by calculating as follows: to determine the percent "identity" of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

The percent identity between two sequences varies with the identical positions shared by the sequences, taking into account the number of gaps that need to be introduced and the length of each gap for optimal alignment of the two sequences.

A mathematical algorithm can be used to compare two sequences and calculate the percent identity between the sequences. For example, the percent identity between two amino acid sequences is determined with the Needleman and Wunsch algorithm ((1970) J. Mol. Biol., 48: 444-453; available at www.gcg.com) which has been integrated into the GAP program of the GCG software package, using the Blossum 62 matrix or PAM250 matrix and gap weight of 16, 14, 12, 10, 8, 6, or 4 and length weight of 1, 2, 3, 4, 5, or 6. For another example, the percent identity between two nucleotide acid sequences is determined with the GAP program of the GCG software package (available at www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossum62 scoring matrix with a gap penalty of 12, a gap extension penalty of 4, and a frameshift gap penalty of 5.

"n is a real number of 1-8" herein means that n is any real number greater than or equal to 1 and less than or equal to 8.

"Integers of x-y" herein refers to both the case of each integer (including x and y) in the interval and the case of range values with any 2 of the integers described above as endpoints. For example, "m4 is selected from integers of 1-10" means that m4 includes both the case where m4 is selected from 1, 2, 3, 4, 5, 6, 7, 8, 9, or 10 and the case where m4 is selected from range values with any 2 of the 10 integers described above as endpoints, for example, m4 is selected from 2-9, 3-9, 3-8, 4-6, or the like.

Herein, 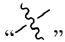 represents a connection site.

The illustration method for the racemic or enantiomerically pure compounds herein is from Maehr, J. Chem. Ed. 1985, 62:114-120. Unless otherwise stated, the absolute configuration of a stereogenic center is represented by a wedged bond and a wedged dashed bond ( 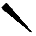 and 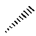 ), and the relative configuration of a stereogenic center (e.g., *cis-* or *trans*-configuration of an alicyclic compound) is represented by a black solid bond and a dashed bond ( 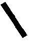 and 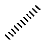 ).

The term "tautomer" refers to functional isomers resulting from the rapid movement of an atom in a molecule between two positions. The compounds of the present disclosure may exhibit the tautomerism. Tautomeric compounds may exist in two or more interconvertible forms. Tautomers generally exist in an equilibrium form. Trying to separate a single tautomer usually leads to a mixture, the physicochemical properties of which are consistent with the mixture of the compound. The position of the equilibrium depends on the chemical properties of the molecule. For example, in many aliphatic aldehydes and ketones, such as acetaldehyde, the keto form predominates; whereas in phenol, the enol form predominates. In the present disclosure, all tautomeric forms of the compounds are included.

The term "stereoisomer" refers to isomers resulting from different spatial arrangements of atoms in a molecule, including *cis-trans* isomers, enantiomers, and diastereoisomers.

The compounds of the present disclosure may have an asymmetric atom such as a carbon atom, a sulfur atom, a nitrogen atom, and a phosphorus atom, or an asymmetric double bond, and thus the compounds of the present disclosure may exist in the form of a particular geometric isomer or stereoisomer. The form of a particular geometric isomer or stereoisomer may be *cis* and *trans* isomers, E and Z geometric isomers, (-)- and (+)- enantiomers, (R)-and (S)- enantiomers, diastereoisomers, (D)-isomers, (L)-isomers, and racemic mixtures or other mixtures thereof, such as an enantiomer or diastereoisomer enriched mixture, and all of the above isomers, as well as mixtures thereof, are encompassed within the definition scope of the compounds of the present disclosure. An additional asymmetric carbon atom, asymmetric sulfur atom, asymmetric nitrogen atom, or asymmetric phosphorus atom may be present in substituents such as alkyl. All of these isomers and mixtures thereof referred to in the substituents are also encompassed within the definition scope of the compounds of the present disclosure. The compounds containing asymmetric atoms of the present disclosure can be separated in an optically active pure form or in a racemic form. The optically active pure form can be obtained by resolving a racemic mixture or by synthesis using chiral starting materials or chiral reagents.

The term "substituted" means that any one or more hydrogen atoms on a specific atom are substituted with substituents, as long as the valence of the specific atom is normal and the compound resulting from the substitution is stable. When the substituent is oxo (namely =O), it means that two hydrogen atoms are substituted, and oxo is not available in an aromatic group.

The term "optional" or "optionally" means that the subsequently described event or circumstance may or may not occur. The description includes instances where the event or circumstance occurs and instances where the event or circumstance does not occur. For example, ethyl being "optionally" substituted with halogen means that the ethyl may be unsubstituted (CH₂CH₃), monosubstituted (CH₂CH₂F, CH₂CH₂Cl, or the like), polysubstituted (CHFCH₂F, CH₂CHF₂, CHFCH₂Cl, CH₂CHCl₂, or the like), or fully substituted (CF₂CF₃, CF₂CCl₃, CCl₂CCl₃, or the like). It will be understood by those skilled in the art that for any group comprising one or more substituents, no substitution or substituting pattern that is spatially impossible and/or cannot be synthesized will be introduced.

When any variable (e.g., R^{a} or R^{b}) occurs once or more in the constitution or structure of a compound, the variable is independently defined in each case. For example, if a group is substituted with 2 R^{b}, the definition of each R^{b} is independent.

When the number of a linking group is 0, such as -(CH₂)₀-, it means that the linking group is a bond.

When one of the variables is selected from a chemical bond or is absent, it means that the two groups that it links are linked directly. For example, when L in A-L-Z represents a bond, it means that the structure is actually A-Z.

When the linking direction of the linking group referred to herein is not specified, the linking direction is arbitrary. For example, when W⁴ in structural unit W³-W⁴-W⁵ is selected from "[A-(CR^{d1}R^{d2})ₘ₂-NR^{d3}]ₘ₃-B", W⁴ may connect W³ and W⁵ either in a left-to-right orientation to form "W³-[A-(CR^{d1}R^{d2})ₘ₂-NR^{d3}]ₘ₃-B-W⁵" or in a right-to-left orientation to form "W³-B-[NR^{d3}-(CR^{d1}R^{d2})ₘ₂-A]ₘ₃-W⁵".

Cₘ-Cₙ herein means that the portion has an integer number of carbon atoms in the range of m-n.

The term "alkyl" refers to a monovalent hydrocarbon group with a general formula of CₙH₂ₙ₊₁. The alkyl may be linear or branched. The term "C₁-C₁₀ alkyl" refers to a linear or branched saturated hydrocarbon group having 1, 2, 3, 4, 5, 6, 7, 8, 9, or 10 carbon atoms. Specific examples of the alkyl include, but are not limited to, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl-, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or the like. The term "C₁-C₆ alkyl" refers to a linear or branched saturated hydrocarbon group having 1, 2, 3, 4, 5, or 6 carbon atoms. "C₁-C₁₀ alkyl" described herein may include the range of "C₁-C₆ alkyl", "C₁-C₄ alkyl", "C₁-C₃ alkyl", or the like, and the "C₁-C₆ alkyl" may further include "C₁-C₄ alkyl" or "C₁-C₃ alkyl".

The term "alkylene" refers to a linear or branched saturated divalent hydrocarbon group. "C₁-C₁₀ alkylene" refers to a linear or branched saturated divalent hydrocarbon group having 1, 2, 3, 4, 5, 6, 7, 8, 9, or 10 carbon atoms, including but not limited to methylene, CH₂CH₂, CH₂CH₂CH₂, or the like.

The term "cycloalkyl" refers to a fully saturated monovalent carbon ring that exists in the form of a monocyclic ring, fused ring, bridged ring, spiro ring, or the like. The term "C₃-C₆ cycloalkyl" should be understood as a saturated monocyclic ring, fused ring, spiro ring, or bridged ring having 3, 4, 5, or 6 carbon atoms. Specific examples include, but are not limited to, cyclopropyl, cyclobutyl, cyclopentyl, cyclohexyl, and the like.

The term "C₃-C₆ cycloalkylene" refers to a fully saturated divalent carbon ring that exists in the form of a monocyclic ring, fused ring, bridged ring, spiro ring, or the like, and has 3, 4, 5, or 6 ring carbon atoms.

The term "heterocyclyl" refers to a fully saturated or partially saturated monovalent group that exists in the form of a monocyclic ring, fused ring, spiro ring, bridged ring, or the like, and ring atoms of the group include 1-5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), -S(=O)₂-, -S(=O)-, -P(=O)₂-, -P(=O)-, -NH-, -S(=O)(=NH)-, -C(=O)NH-, - NHC(=O)NH-, or the like. The term "4- to 7-membered heterocyclyl" refers to a heterocyclyl group having a ring atom number of 4, 5, 6, or 7, and ring atoms of the heterocyclyl group include 1-3 heteroatoms or heteroatom groups independently selected from those described above. Examples of 4-membered heterocyclyl include, but are not limited to, azetidinyl or oxetanyl; examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1*H*-pyrrolyl; examples of 6-membered heterocyclyl include, but are not limited to, tetrahydropyranyl, piperidinyl, morpholinyl, dithianyl, thiomorpholinyl, piperazinyl, trithianyl, tetrahydropyridinyl, or 4*H-*[1,3,4]thiadiazinyl; examples of 7-membered heterocyclyl include, but are not limited to, diazepanyl. Preferably, "4- to 7-membered heterocyclyl" may include the ranges of "4- to 7-membered heterocycloalkyl", "5- to 6-membered heterocyclyl", "5- to 6-membered heterocycloalkyl", and the like.

The term "heterocyclylene" refers to a fully saturated or partially saturated divalent group that exists in the form of a monocyclic ring, fused ring, spiro ring, bridged ring, or the like, and ring atoms of the group include 1-5 heteroatoms or heteroatom groups. The "heteroatom or heteroatom group" includes, but is not limited to, a nitrogen atom (N), an oxygen atom (O), a sulfur atom (S), a phosphorus atom (P), a boron atom (B), -S(=O)₂-, -S(=O)-, - P(=O)₂-, -P(=O)-, -NH-, -S(=O)(=NH)-, -C(=O)NH-, -NHC(=O)NH-, or the like.

The term "C₂-C₃ alkenyl" refers to a linear or branched unsaturated hydrocarbon group containing one double bond and having 2 or 3 carbon atoms. Specific examples include, but are not limited to, vinyl, allyl, (*E*)-2-methylvinyl, and (*Z*)-2-methylvinyl.

The term "C₂-C₃ alkynyl" refers to a linear or branched unsaturated hydrocarbon group containing one triple bond and having 2 or 3 carbon atoms. Specific examples include, but are not limited to, ethynyl (-C≡CH), prop-1-ynyl (-C≡CCH₃), and prop-2-ynyl (-CH₂C≡CH).

The term "halo-" or "halogen" refers to fluorine, chlorine, bromine, and iodine.

The term "hydrophilic group" refers to a group that is capable of dissolving or ionizing into water and is easily compatible with water. The hydrophilic group generally contains one or more O, N, P, S, OH, NH, NH₂, C=O, S(=O)₂, P(=O)₂, and any combination thereof, for example, contains structural units such as polyether, polyhydroxy, polyamine, polysaccharide, peptide, carboxylic acid, sulfonic acid, and phosphoric acid.

The term "treatment" refers to surgical or therapeutic treatment for the purpose of preventing and slowing (reducing) the progression of an undesired physiological or pathological change, e.g., cancer, an autoimmune disease, and virus infection, in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

The term "effective amount" refers to an amount of a therapeutic agent that is effective to prevent or alleviate symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "subject" refers to an organism that receives treatment for a particular disease or disorder described in the present disclosure. Examples of subjects and patients include mammals, such as humans, primates (e.g., monkeys), or non-primate mammals, that receive treatment for a disease or disorder.

The amount of the compound of the present disclosure constituting the "therapeutically effective amount" varies depending on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvents, water, and the like.

The word "comprise" and variations thereof such as "comprises" or "comprising" may be understood in an open and non-exclusive sense, i.e., "including but not limited to".

The present disclosure also includes isotopically labeled compounds of the present disclosure which are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present disclosure (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron-emitting isotopes, such as ¹⁵O, ¹³N, ¹¹C, and ¹⁸F, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical compositions of the present disclosure may be suitable for parenteral administration, such as sterile solutions, suspensions or lyophilized products in suitable unit dosage forms. For example, the pharmaceutical composition of the present disclosure may be in the form of a sterile injectable aqueous solution for intramuscular or subcutaneous administration. Other vehicles or solvents such as water, Ringer's solution, or isotonic sodium chloride solution are acceptable when the pharmaceutical composition of the present disclosure is used.

In all of the administration methods of the compound described herein, the daily dose administered is from 0.001 mg/kg to 600 mg/kg body weight, preferably from 0.05 mg/kg to 200 mg/kg body weight, and more preferably from 0.1 mg/kg to 100 mg/kg body weight, given in individual or separated doses.

The compounds of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

An important consideration in synthetic route planning in the art is the selection of suitable protective groups for reactive functional groups (e.g., amino and carboxy in the present disclosure). For example, reference may be made to *Greene's Protective Groups in Organic Synthesis* (4th Ed.) Hoboken, New Jersey: John Wiley & Sons, Inc. All references cited in the present disclosure are incorporated herein in their entirety.

The following abbreviations are used in the present disclosure:

| Abbreviation | Chemical name | Abbreviation | Chemical name |
|---|---|---|---|
| BPin | 4,4,5,5-Tetramethyl-1,3,2-dioxaborane-2-yl | B₂Pin₂ | 4,4,5,5-Tetramethyl-2-(4,4,5,5-tetramethyl-1,3,2-dioxaborane-2-yl)-1,3,2-dioxaborane |
| HATU | *O*-(7-Azabenzotriazol-1-yl)-*N*,*N*,*N*'*,N*'-tetramethyluronium hexafluorophosphate | EDCI | 1-(3-Dimethylaminopropyl)-3-ethylcarbodiimide |
| DMAP | 4-Dimethylaminopyridine | 2-Me-THF | 2-Methyltetrahydrofuran |
| DCM | Dichloromethane | DMA | *N,N*-Dimethylacetamide |
| EtOH | Ethanol | AcOH | Acetic acid |
| KOAc | Potassium acetate | DMSO | Dimethyl sulfoxide |
| THF | Tetrahydrofuran | TFA | Trifluoroacetic acid |
| TEA | Triethylamine | MeCN | Acetonitrile |
| M | mol/L | T₃P | Propylphosphonic anhydride |
| *m*CPBA | *m*-Chloroperoxybenzoic acid | MeB(OH)₂ | Methylboronic acid |
| DMF | *N,N*-Dimethylformamide | Cu(OAc)₂ | Copper(II) acetate |
| TsOH | *p*-Toluenesulfonic acid | Ti(OiPr)₄ | Tetraisopropyl titanate |
| (R)-(S)-cy₂PF-P*t*Bu₂ | (*R*)*-*1*-*[(*S*)*-*2-(Dicyclohexylphosphino)ferrocenyl] ethyldi-*tert*-butylphosphine | tBuONO | *tert*-Butyl Nitrite |
| Pd(PPh₃)₄ | Tetrakis(triphenylphosphine)palladiu m(0) | BrettPhos Pd G₃ | Methanesulfonato(2-dicyclohexylphosphino-3,6-dimethoxy-2',4',6'-triisopropyl-1,1'-biphenyl)(2'-amino-1,1'-biphenyl-2-yl)palladium(II) |
| Boc | *tert*-Butyloxycarbonyl | Et₂O | Diethyl ether |
| DIEA | *N,N-*Diisopropylethylamine | NBS | *N*-Bromosuccinimide |
| PhI(OAc)₂ | Phenyliodosodiacetate | DME | Ethylene glycol dimethyl ether |
| MeOH | Methanol | LiHMDS | Lithium bis(trimethylsilyl)amide |
| MW | Microwave | THP | Tetrahydropyranyl |
| Xphos-Pd G₂ | Chloro(2-dicyclohexylphosphino-2',4',6'-triisopropyl- 1,1'-biphenyl)[2-(2'-amino-1,1'-biphenyl)]palladium(II) | Boc₂O | Di-*tert*-butyl dicarbonate |
| xantphos | 4,5-Bis(diphenylphosphino)-9,9-dimethylxanthene | t-BuBrettPhos-Pd G₃ | Methanesulfonato(2-(di-*tert-*butylphosphino)-3,6-dimethoxy-2',4',6'-triisopropyl-1,1'-biphenyl)(2'-amino-1,1'-biphenyl-2-yl)palladium(II) |
| Pd₂(dba)₃ | Tris(dibenzylideneacetone)dipalladiu m | DMEDA | *N,N*-Dimethylethylenediamine |
| Xphos | 2-Dicyclohexylphosphino-2',4',6'-triisopropylbiphenyl | NIS | *N*-Iodosuccinimide |
| Pd(OAc)₂ | Palladium(II) acetate | Pd(AmPhos)C l₂ | Dichlorobis[di-*tert*-butyl(4-dimethylaminophenyl)phosphine]p alladium(II) |
| EtOAc or EA | Ethyl acetate | n-BuOH | *n*-Butanol |
| PE | Petroleum ether | TFA | Trifluoroacetic acid |
| FA | Formic acid | Pd/C | Palladium on carbon |
| ACN or MeCN | Acetonitrile | DIPEA | *N,N-*Diisopropylethylamine |
| SEM | 2-(Trimethylsilyl)ethoxymethyl | Pd₂(dba)₃ | Tris(dibenzylideneacetone)dipallad ium |
| Boc | *tert*-Butoxycarbonyl | BINAP | 2,2'-Bis(diphenylphosphino)-1,1'-binaphthyl |
| Cbz | Benzyloxycarbonyl | Cs₂CO₃ | Cesium carbonate |
| DMSO | Dimethyl sulfoxide | Pd(dppf)Cl₂ | [1,1'-Bis(diphenylphosphino)ferrocene]d ichloropalladium(II) |
| Ti(OEt)₄ | Titanium ethoxide | NaH | Sodium hydride |
| NH₄Cl | Ammonium chloride | LiOH | Lithium hydroxide |
| SFC | Supercritical fluid chromatography | TLC | Thin layer chromatography |
| DHP | 3,4-Dihydro-2*H*-pyran | DEA | Diethylamine |
| Pd/C | Palladium on carbon | TsCl | *p*-Toluenesulfonyl chloride |
| Ph₃PMeBr | Methyltriphenylphosphonium bromide | iPrOH | Isopropanol |
| BrettPhos Pd G3 | Methanesulfonato(2-dicyclohexylphosphino-3,6-dimethoxy-2',4',6'-triisopropyl-1,1'-biphenyl)(2'-amino-1,1'-biphenyl-2-yl)palladium(II) | 9-BBN | 9-Borabicyclo[3.3.1]nonane |
| DMF-DMA | *N*,*N*-Dimethylformamide dimethyl acetal | T₃P | Propylphosphonic anhydride |
| HOBt | 1-Hydroxybenzotriazole | HBTU | *O*-(Benzotriazol-1-yl)-*N,N,N',N'-*tetramethyluronium hexafluorophosphate |
| HFIP | 1,1,1,3,3,3-Hexafluoroisopropanol | | |

### Examples

The present disclosure is described in detail below by way of examples. However, this is by no means disadvantageously limiting the scope of the present disclosure. Although the present disclosure has been described in detail herein and specific examples have also been disclosed, it will be apparent to those skilled in the art that various modifications can be made to the specific embodiments of the present disclosure without departing from the spirit and scope of the present disclosure. All reagents used in the present disclosure are commercially available and can be used without further purification.

Unless otherwise stated, the ratios expressed for mixed solvents are volume mixing ratios.

Unless otherwise stated, % refers to wt%.

Compounds are named either manually or by ChemDraw^{®} software, and supplier's catalog names are given for commercially available compounds.

The structures of the compounds are determined by nuclear magnetic resonance (NMR) and/or mass spectrometry (MS). NMR shifts are given in 10⁻⁶ (ppm). Solvents for NMR determination are deuterated dimethyl sulfoxide, deuterated chloroform, deuterated methanol, or the like, and internal standard is tetramethylsilane (TMS); "IC₅₀" refers to the half maximal inhibitory concentration, which is the concentration at which half of the maximal inhibitory effect is achieved; "EC₅₀" refers to the half maximal effect concentration, a concentration that causes 50% of the maximal effect.

### Example 1. tert-Butyl 3-(2-methylsulfonyl-5-oxo-7,8-dihydropyrido[4,3-d]pyrimidin-6(5H)-yl)propanoate (Compound 1)

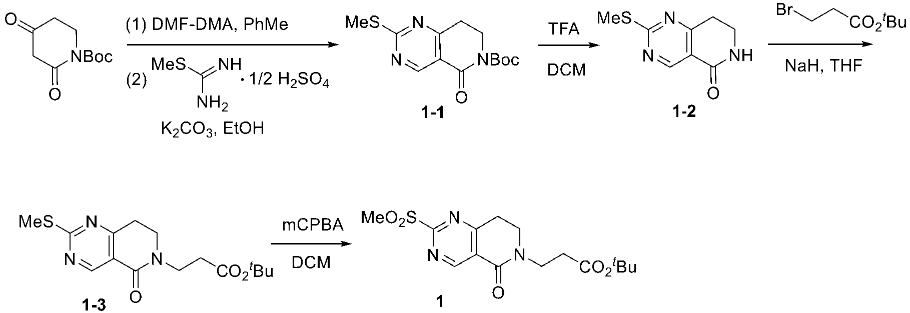

### Step 1: synthesis of tert-butyl 2-methylthio-5-oxo-7,8-dihydropyrido[4,3-d]pyrimidine-6(5H)-carboxylate (intermediate 1-1)

*N*,*N*-Dimethylformamide dimethyl acetal (DMF-DMA) (9.2 g) was added to a mixture of *tert*-butyl 2,4-dioxopiperidine-1-carboxylate (15 g) and toluene (150 mL) at room temperature under a nitrogen atmosphere. The resulting mixture was stirred at 25 °C for reaction for 1 h. The mixture was concentrated under reduced pressure to remove the solvent, and then methylisothiourea hemisulfate (10.8 g) was added. Potassium carbonate (14.7 g) and absolute ethanol (200 mL) were added, and the resulting mixture was stirred at 25 °C for reaction for 4 h. The mixture was then concentrated under reduced pressure to remove the solvent, and the residue was separated by column chromatography (EA/PE, 0-30%) to give a crude product of the title compound, which was directly used in the next step.

LC-MS: m/z (ESI): 296.1 [M+H]⁺.

### Step 2: synthesis of 2-methylthio-7,8-dihydropyrido[4,3-d]pyrimidin-5(6H)-one (intermediate 1-2)

**Intermediate 1-1** obtained in the previous step was dissolved in 100 mL of dichloromethane, and 25 mL of trifluoroacetic acid was added. The mixture was stirred at 25 °C for reaction for 2 h. The mixture was then concentrated under reduced pressure to remove the solvent, and the residue was slurried with 100 mL of ethyl acetate. The slurry was filtered and dried to give the title compound (10.0 g).

LC-MS: m/z (ESI): 196.1 [M+H]⁺.

### Step 3: synthesis of tert-butyl 3-(2-methylthio-5-oxo-7,8-dihydropyrido[4,3-d]pyrimidin-6(5H)-yl)propanoate (intermediate 1-3)

NaH (5 g, 60% mass fraction in mineral oil) was added to a mixture of **intermediate 1-2** (9.75 g) and THF (240 mL) at 0 °C, and the resulting mixture was reacted at 0 °C for 30 min. *tert*-Butyl 3-bromopropanoate (15.68 g) was then added to the reaction mixture, and the resulting mixture was warmed to 70 °C and reacted for 2 h. 2 mL of water was then added to the mixture, and the mixture was concentrated under reduced pressure to remove the solvent. The residue was separated by column chromatography (EA/PE, 0-50%) to give the title compound (9.75 g).

LC-MS: m/z (ESI): 324.4 [M+H]⁺.

### Step 4: synthesis of tert-butyl 3-(2-methylsulfonyl-5-oxo-7,8-dihydropyrido[4,3-d]pyrimidin-6(5H)-yl)propanoate (compound 1)

mCPBA (12.6 g, 85% purity) was added to a mixture of **intermediate 1-3** (5 g) and DCM (150 mL) at 0 °C, and the resulting mixture was reacted at room temperature for 30 min. The mixture was then concentrated under reduced pressure, and the residue was separated by column chromatography (EA/PE, 0-100%) to give the title compound (5.2 g).

LC-MS: m/z (ESI): 356.4 [M+H]⁺.

¹H NMR (400 MHz, DMSO-*d*₆) δ 9.24 (s, 1H), 3.76 (t, *J =* 6.7 Hz, 2H), 3.70 (t, *J =* 7.0 Hz, 2H), 3.43 (s, 3H), 3.24 (t, *J* = 6.7 Hz, 2H), 2.57 (t, *J =* 7.0 Hz, 2H), 1.39 (s, 9H).

### Example 2. tert-Butyl 3-(2-methylsulfonyl-5-oxo-5,7-dihydro-6H-pyrrolo[3,4-d]pyrimidin-6-yl)propanoate (Compound 2)

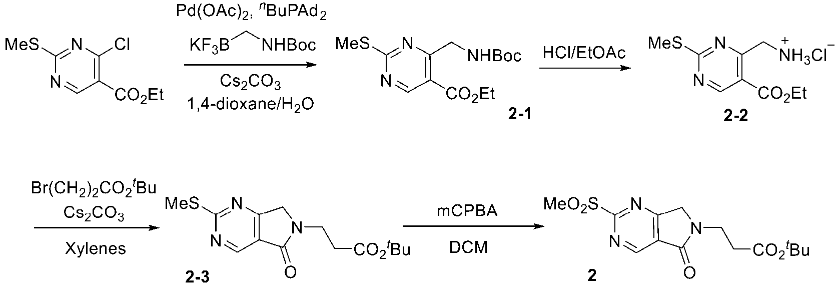

### Step 1: synthesis of ethyl 4-(((tert-butoxycarbonyl)amino)methyl)-2-methylthiopyrimidine-5-carboxylate (intermediate 2-1)

1,4-Dioxane (100 mL) and water (10 mL) were added to a mixture of ethyl 4-chloro-2-methylthiopyrimidine-5-carboxylate (5.82 g), potassium (((tert-butoxycarbonyl)amino)methyl)trifluoroborate (4.74 g), cesium carbonate (13.0 g), palladium(II) acetate (448 mg), and di(1-adamantyl)-n-butylphosphine (1.43 g) at room temperature under a nitrogen atmosphere. The resulting mixture was stirred at 90 °C for reaction for 6 h. The mixture was then concentrated under reduced pressure to remove the solvent. The residue was separated by column chromatography (EA/PE, 0-50%) to give the title compound (4147 mg).

LC-MS: m/z (ESI): 328.1 [M+H]⁺.

### Step 2: synthesis of (5-ethoxycarbonyl-2-methylthiopyrimidin-4-yl)methylamine hydrochloride (intermediate 2-2)

A solution of hydrogen chloride in ethyl acetate (4 M, 10 mL) was added to a solution of **intermediate 2-1** (1637 mg) in ethyl acetate (10 mL) at room temperature under a nitrogen atmosphere, and the resulting mixture was stirred at room temperature for reaction for 4 h. The mixture was then filtered, and the filter cake was washed with ethyl acetate to give the title compound (1231 mg).

LC-MS: m/z (ESI): 228.3 [M+H]⁺.

### Step 3: synthesis of tert-butyl 3-(2-methylthio-5-oxo-5,7-dihydro-6H-pyrrolo[3,4-d]pyrimidin-6-yl)propanoate (intermediate 2-3)

A mixture of **intermediate 2-2** (500 mg), cesium carbonate (2.14 g), *tert*-butyl 3-bromopropanoate (916 mg), and xylene (10 mL) was heated to 80 °C and reacted for 16 h under a nitrogen atmosphere. The mixture was then concentrated under reduced pressure to remove the solvent. The residue was separated by column chromatography (EA/PE, 0-30%) and thin layer chromatography (EA/PE = 1/1) to give the title compound (28 mg).

LC-MS: m/z (ESI): 310.0 [M+H]⁺.

### Step 4: synthesis of tert-butyl 3-(2-methylsulfonyl-5-oxo-5,7-dihydro-6H-pyrrolo[3,4-d]pyrimidin-6-yl)propanoate (compound 2)

*m*-Chloroperoxybenzoic acid (mCPBA) (78.74 mg, 85% purity) was added to a solution of **intermediate 2-3** (30 mg) dissolved in dichloromethane (1 mL) at 0 °C, and the resulting mixture was stirred at room temperature for 1 h. The mixture was then separated by column chromatography (EA/PE, 0-66%) to give the title compound (14.0 mg).

LC-MS: m/z (ESI): 342.2 [M+H]⁺.

¹H NMR (400 MHz, DMSO-*d*₆) δ = 9.37 (s, 1H), 4.76 (s, 2H), 3.78 (t, *J=* 7.1 Hz, 2H), 3.48 (s, 3H), 2.64 (t, *J=* 7.1 Hz, 2H), 1.39 (s, 9H)

### Example 3. tert-Butyl 3-(2-ethylsulfonyl-5-oxo-7,8-dihydropyrido[4,3-d]pyrimidin-6(5H)-yl)propanoate (Compound 3)

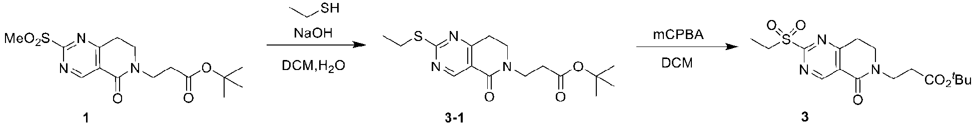

### Step 1: synthesis of tert-butyl 3-(2-ethylthio-5-oxo-7,8-dihydropyrido[4,3-d]pyrimidin-6(5H)-yl)propanoate (intermediate 3-1)

Dichloromethane (1 mL) and a 2 M sodium hydroxide solution (0.5 mL) were added to a mixture of **compound 1** (50 mg) and ethanethiol (180 mg) at room temperature. The resulting mixture was stirred at 25 °C for reaction for 3 h. The mixture was then concentrated under reduced pressure to remove the solvent. The residue was separated by column chromatography (THF/PE, 0-30%) to give the title compound (45 mg).

LC-MS: m/z (ESI): 338.1 [M+H]⁺.

### Step 2: synthesis of tert-butyl 3-(2-ethylsulfonyl-5-oxo-7,8-dihydropyrido[4,3-d]pyrimidin-6(5H)-yl)propanoate (compound 3)

m-Chloroperoxybenzoic acid (92.05 mg) was added to a solution of **intermediate 3-1** (45 mg) dissolved in dichloromethane (2 mL) at 0 °C, and the resulting mixture was stirred at room temperature for 1 h. The mixture was then separated by column chromatography (EA/PE, 0-100%) to give the title compound (15.6 mg).

LC-MS: m/z (ESI): 370.1 [M+H]⁺.

¹H NMR (400 MHz, DMSO-*d*₆) δ = 9.25 (s, 1H), 3.77 (t, *J=* 6.7 Hz, 2H), 3.71 (t, *J=* 7.0 Hz, 2H), 3.62 (q, *J =* 7.4 Hz, 2H), 3.25 (t, *J =* 6.7 Hz, 2H), 2.57 (t, *J =* 7.0 Hz, 2H), 1.40 (s, 9H), 1.26 (t, *J* = 7.3 Hz, 3H)

### Example 4. tert-Butyl 3-(2-butylsulfonyl-5-oxo-7,8-dihydropyrido[4,3-d]pyrimidin-6(5H)-yl)propanoate (Compound 4)

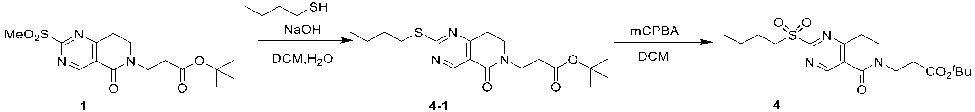

### Step 1: synthesis of tert-butyl 3-(2-butylthio-5-oxo-7,8-dihydropyrido[4,3-d]pyrimidin-6(5H)-yl)propanoate (intermediate 4-1)

Dichloromethane (1 mL) and a 2 M sodium hydroxide solution (0.5 mL) were added to a mixture of **compound 1** (50 mg) and 1-butanethiol (19 mg) at room temperature. The resulting mixture was stirred at 25 °C for reaction for 3 h. The mixture was then concentrated under reduced pressure to remove the solvent. The residue was separated by column chromatography (THF/PE, 0-18%) to give the title compound (47 mg).

LC-MS: m/z (ESI): 366.4 [M+H]⁺.

### Step 2: synthesis of tert-butyl 3-(2-butylsulfonyl-5-oxo-7,8-dihydropyrido[4,3-d]pyrimidin-6(5H)-yl)propanoate (compound 4)

*m*-Chloroperoxybenzoic acid (88.76 mg) was added to a solution of **intermediate 4-1** (47 mg) dissolved in dichloromethane (2 mL) at 0 °C, and the resulting mixture was stirred at room temperature for 1 h. The mixture was then separated by column chromatography (EA/PE, 0-100%) to give the title compound (10.7 mg).

LC-MS: m/z (ESI): 398.1 [M+H]⁺

¹H NMR (400 MHz, DMSO-*d₆*) δ = 9.25 (s, 1H), 3.77 (t, *J =* 6.7 Hz, 2H), 3.71 (t, *J =* 7.0 Hz, 2H), 3.63-3.58 (m, 2H), 3.25 (t, *J =* 6.7 Hz, 2H), 2.57 (t, *J=* 7.0 Hz, 2H), 1.72-1.63 (m, 2H), 1.45-1.41 (m, 2H), 1.40 (s, 9H), 0.89 (t, *J* = 7.3 Hz, 3H)

### Example 5. tert-Butyl 3-(2-isobutylsulfonyl-5-oxo-7,8-dihydropyrido[4,3-d]pyrimidin-6(5H)-yl)propanoate (Compound 5)

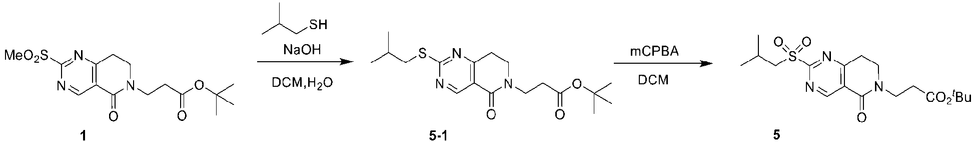

### Step 1: synthesis of tert-butyl 3-(2-isobutylthio-5-oxo-7,8-dihydropyrido[4,3-d]pyrimidin-6(5H)-yl)propanoate (intermediate 5-1)

Dichloromethane (1 mL) and a 2 M sodium hydroxide solution (0.5 mL) were added to a mixture of **compound 1** (50 mg) and 1-butanethiol (250 mg) at room temperature. The resulting mixture was stirred at 25 °C for reaction for 3 h. The mixture was then concentrated under reduced pressure to remove the solvent. The residue was separated by column chromatography (THF/PE, 0-18%) to give the title compound (47 mg).

LC-MS: m/z (ESI): 366.4 [M+H]⁺.

### Step 2: synthesis of tert-butyl 3-(2-isobutylsulfonyl-5-oxo-7,8-dihydropyrido[4,3-d]pyrimidin-6(5H)-yl)propanoate (compound 5)

m-Chloroperoxybenzoic acid (88.76 mg) was added to a solution of **intermediate 5-1** (47 mg) dissolved in dichloromethane (2 mL) at 0 °C, and the resulting mixture was stirred at room temperature for 1 h. The mixture was then separated by column chromatography (EA/PE, 0-100%) to give the title compound (7.6 mg).

### LC-MS: m/z (ESI): 398.1 [M+H]⁺

¹H NMR (400 MHz, DMSO-*d₆*) δ = 9.25 (s, 1H), 3.77 (t, *J=* 6.7 Hz, 2H), 3.71 (t, *J=* 7.0 Hz, 2H), 3.54 (d, *J* = 6.6 Hz, 2H), 3.25 (t, *J =* 6.7 Hz, 2H), 2.57 (t, *J =* 7.0 Hz, 2H), 2.21 (t, *J =* 6.7, 13.4 Hz, 1H), 1.40 (s, 9H), 1.04 (d, *J*= 6.7 Hz, 6H)

### Example 6. tert-Butyl 3-(2-isopropylsulfonyl-5-oxo-7,8-dihydropyrido[4,3-d]pyrimidin-6(5H)-yl)propanoate (Compound 6)

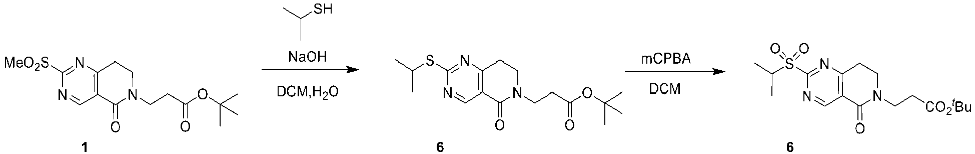

### Step 1: synthesis of tert-butyl 3-(2-isopropylthio-5-oxo-7,8-dihydropyrido[4,3-d]pyrimidin-6(5H)-yl)propanoate (intermediate 6-1)

Dichloromethane (1 mL) and a 2 M sodium hydroxide solution (0.5 mL) were added to a mixture of **compound 1** (50 mg) and isopropanethiol (500 mg) at room temperature. The resulting mixture was stirred at 25 °C for reaction for 3 h. The mixture was then concentrated under reduced pressure to remove the solvent. The residue was separated by column chromatography (THF/PE, 0-30%) to give the title compound (45 mg).

LC-MS: m/z (ESI): 352.2 [M+H]⁺.

### Step 2: synthesis of tert-butyl 3-(2-isopropylsulfonyl-5-oxo-7,8-dihydropyrido[4,3-d]pyrimidin-6(5H)-yl)propanoate (compound 6)

*m*-Chloroperoxybenzoic acid (88 mg) was added to a solution of **intermediate 6-1** (45 mg) dissolved in dichloromethane (2 mL) at 0 °C, and the resulting mixture was stirred at room temperature for 1 h. The mixture was then separated by column chromatography (EA/PE, 0-100%) to give the title compound (18.5 mg).

LC-MS: m/z (ESI): 384.1 [M+H]⁺.

¹H NMR (400 MHz, DMSO-*d₆*) δ = 9.25 (s, 1H), 3.99-3.94 (m, 1H), 3.77 (t, *J=* 6.7 Hz, 2H), 3.71 (t, *J =* 7.0 Hz, 2H), 3.25 (t, *J =* 6.7 Hz, 2H), 2.57 (t, *J=* 7.0 Hz, 2H), 1.40 (s, 9H), 1.28 (s, 3H), 1.27 (s, 3H)

### Example 7. tert-Butyl 3-(2-cyclopentylsulfonyl-5-oxo-7,8-dihydropyrido[4,3-d]pyrimidin-6(5H)-yl)propanoate (Compound 7)

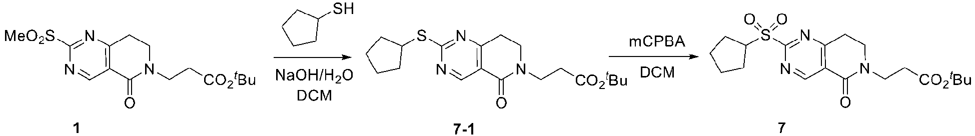

### Step 1: synthesis of tert-butyl 3-(2-cyclopentylthio-5-oxo-7,8-dihydropyrido[4,3-d]pyrimidin-6(5H)-yl)propanoate (intermediate 7-1)

Cyclopentanethiol (367 mg) and a 2 M sodium hydroxide solution (1.65 mL) were sequentially added to a solution of **compound 1** (1.066 g) dissolved in DCM (10 mL) at room temperature, and the resulting mixture was stirred at room temperature for reaction for 30 min. The mixture was then separated by column chromatography (EA/PE, 0-50%) to give a crude product of the title compound, which was directly used in the next step.

LC-MS: m/z (ESI): 378.4 [M+H]⁺.

### Step 2: synthesis of tert-butyl 3-(2-cyclopentylsulfonyl-5-oxo-7,8-dihydropyrido[4,3-d]pyrimidin-6(5H)-yl)propanoate (compound 7)

mCPBA (2.43 g, 85% purity) was added to a mixture of the crude product of the compound obtained in the previous step and DCM (50 mL) at 0 °C, and the resulting mixture was reacted at room temperature for 30 min. The mixture was then concentrated under reduced pressure, and the residue was separated by column chromatography (EA/PE, 0-100%) to give the title compound (890 mg).

LC-MS: m/z (ESI): 410.5 [M+H]⁺.

¹H NMR (400 MHz, DMSO-*d*₆) δ 9.23 (s, 1H), 4.30-4.17 (m, 1H), 3.76 (t*, J* = 6.7 Hz, 2H), 3.70 (t, *J=* 7.0 Hz, 2H), 3.24 (t, *J* = 6.7 Hz, 2H), 2.56 (t, *J =* 7.0 Hz, 2H), 2.04-1.85 (m, 4H), 1.74-1.57 (m, 4H), 1.39 (s, 9H).

### Example 8. tert-Butyl 3-(2-tert-butylsulfonyl-5-oxo-7,8-dihydropyrido[4,3-d]pyrimidin-6(5H)-yl)propanoate (Compound 8)

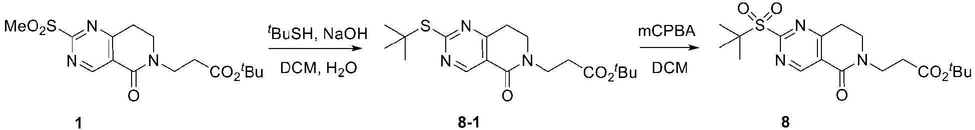

### Step 1: synthesis of tert-butyl 3-(2-tert-butylthio-5-oxo-7,8-dihydropyrido[4,3-d]pyrimidin-6(5H)-yl)propanoate (intermediate 8-1)

*tert*-Butanethiol (324 mg) and a 2 M sodium hydroxide solution (1.65 mL) were sequentially added to a solution of **compound 1** (1.066 g) dissolved in DCM (10 mL) at room temperature, and the resulting mixture was vigorously stirred at room temperature for reaction for 30 min. The mixture was then separated by column chromatography (EA/PE, 0-50%) to give a crude product of the title compound, which was directly used in the next step.

LC-MS: m/z (ESI): 366.4 [M+H]⁺.

### Step 2: synthesis of tert-butyl 3-(2-tert-butylsulfonyl-5-oxo-7,8-dihydropyrido[4,3-d]pyrimidin-6(5H)-yl)propanoate (compound 8)

mCPBA (2.43 g, 85% purity) was added to a mixture of the crude product of the compound obtained in the previous step and DCM (50 mL) at 0 °C, and the resulting mixture was reacted at room temperature for 30 min. The mixture was then concentrated under reduced pressure, and the residue was separated by column chromatography (EA/PE, 0-100%) to give the title compound (831 mg).

LC-MS: m/z (ESI): 398.4 [M+H]⁺.

¹H NMR (400 MHz, DMSO-*d*₆) δ 9.25 (s, 1H), 3.76 (t, *J =* 6.7 Hz, 2H), 3.70 (t, *J =* 7.0 Hz, 2H), 3.23 (t, *J=* 6.7 Hz, 2H), 2.57 (t, *J=* 7.0 Hz, 2H), 1.40 (s, 9H), 1.37 (s, 9H).

### Example 9. Synthesis of tert-Butyl 3-(2-(1-adamantylsulfonyl)-5-oxo-7,8-dihydropyrido[4,3-d]pyrimidin-6(5H)-yl)propanoate (Compound 9)

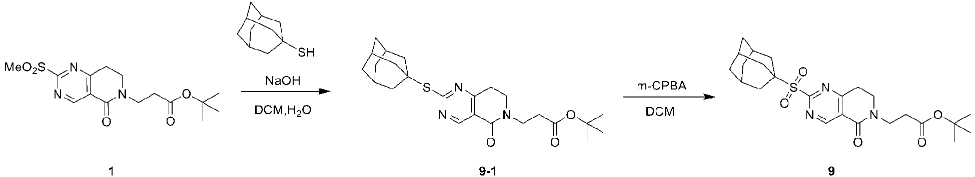

### Step 1: synthesis of tert-butyl 3-(2-(1-adamantylthio)-5-oxo-7,8-dihydropyrido[4,3-d]pyrimidin-6(5H)-yl)propanoate (intermediate 9-1)

A 2 M aqueous NaOH solution (0.692 mL) and 1-adamantanethiol (291 mg) were added to a solution of **compound** 1 (410 mg) in DCM (10 mL) at room temperature, and the resulting mixture was stirred at 25 °C for 2 h. The reaction mixture was then diluted with water (5 mL) and extracted with DCM (3 mL × 3). The organic phases were combined, washed with saturated brine, dried over anhydrous sodium sulfate, filtered, and concentrated under reduced pressure, and the residue was separated by column chromatography (THF/PE, 0-60%) to give the title compound (351 mg).

LC-MS: m/z (ESI): 444.0 [M+H]⁺

### Step 2: synthesis of tert-butyl 3-(2-(1-adamantylsulfonyl)-5-oxo-7,8-dihydropyrido[4,3-d]pyrimidin-6(5H)-yl)propanoate (compound 9)

mCPBA (546 mg, 85% purity) was added to a solution of **intermediate 9-1** (351 mg) in DCM (5 mL) at room temperature, and the mixture was stirred at 25 °C for 2 h. The reaction mixture was then concentrated under reduced pressure to remove the solvent, and the mixture was separated by column chromatography (THF/PE, 0-50%) to give the title compound (300 mg), which was then purified by high performance liquid chromatography (column: C18 150 × 30 mm × 5 µm; mobile phase: [A phase: water (0.225% formic acid), B phase: acetonitrile]; B%: 43%-63%, 11 min) to give the title compound (24.7 mg).

LC-MS: m/z (ESI): 476.1 [M+H]⁺

¹H NMR (400 MHz, DMSO-*d₆*) δ = 9.26 (s, 1H), 3.77-3.75 (m, 2H), 3.71-3.69 (m, 2H), 3.24 (t, *J =* 6.7 Hz, 2H), 2.60-2.55 (m, 2H), 2.08 (s, 3H), 2.00-1.92 (m, 6H), 1.68-1.58 (m, 6H), 1.41 (s, 9H).

### Example 10. tert-Butyl 3-(2-(4-methoxyphenylsulfonyl)-5-oxo-7,8-dihydropyrido[4,3-d]pyrimidin-6(5H)-yl)propanoate (Compound 10)

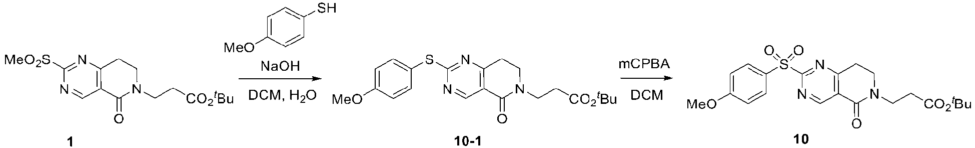

### Step 1: synthesis of tert-butyl 3-(2-(4-methoxyphenylthio)-5-oxo-7,8-dihydropyrido[4,3-d]pyrimidin-6(5H)-yl)propanoate (intermediate 10-1)

Dichloromethane (2 mL) and water (56 µL) were added to a mixture of **compound 1** (40.0 mg), 4-methoxybenzenethiol (23.7 mg), and sodium hydroxide (4.50 mg) at room temperature under a nitrogen atmosphere. The resulting mixture was stirred at room temperature for reaction for 1 h. The mixture was then concentrated under reduced pressure to remove the solvent. The residue was separated by column chromatography (EA/PE, 0-80%) to give the title compound (40.0 mg).

LC-MS: m/z (ESI): 416.3 [M+H]⁺.

### Step 2: synthesis of tert-butyl 3-(2-(4-methoxyphenylsulfonyl)-5-oxo-7,8-dihydropyrido[4,3-d]pyrimidin-6(5H)-yl)propanoate (compound 10)

mCPBA (117 mg, 85% purity) was added to a solution of **intermediate 10-1** (40 mg) dissolved in dichloromethane (1 mL) at 0 °C, and the resulting mixture was stirred at room temperature for 1 h. The mixture was then separated by column chromatography (THF/PE, 0-80%) and purified by high performance liquid chromatography (column: C18 150 × 30 mm × 5 µm; mobile phase: [A phase: water (0.225% formic acid), B phase: acetonitrile]; B%: 45%-75%, 9 min) to give the title compound (5.0 mg).

LC-MS: m/z (ESI): 448.2 [M+H]⁺.

¹H NMR (400 MHz, DMSO-*d*₆) δ 9.14 (s, 1H), 7.93 (d, *J=* 8.8 Hz, 2H), 7.20 (d, *J =* 8.8 Hz, 2H), 3.87 (s, 3H), 3.73-3.64 (m, 4H), 3.18 (t, *J =* 6.7 Hz, 2H), 2.55-2.51 (m, 2H), 1.38 (s, 9H).

### Example 11. tert-Butyl 4-((6-(3-tert-butoxy-3-oxopropyl)-5-oxo-5,6,7,8-tetrahydropyrido[4,3-d]pyrimidin-2-yl)sulfonyl)piperidine-1-carboxylate (Compound 11)

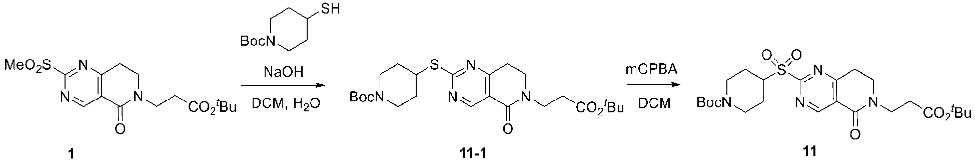

### Step 1: synthesis of tert-butyl 4-((6-(3-tert-butoxy-3-oxopropyl)-5-oxo-5,6,7,8-tetrahydropyrido[4,3-d]pyrimidin-2-yl)thio)piperidine-1-carboxylate (intermediate 11-1)

Dichloromethane (1 mL) and a 2 M sodium hydroxide solution (0.5 mL) were added to a mixture of **compound 1** (50 mg) and *tert*-butyl 4-mercaptopiperidine-1-carboxylate (30.57 mg) at room temperature, and the resulting mixture was stirred at 25 °C for reaction for 3 h. The reaction mixture was then concentrated under reduced pressure to remove the solvent. The mixture was separated by column chromatography (THF/PE, 0-30%) to give the title compound (50 mg).

LC-MS: m/z (ESI): 515.1 [M+Na]⁺.

### Step 2: synthesis of tert-butyl 4-((6-(3-tert-butoxy-3-oxopropyl)-5-oxo-5,6,7,8-tetrahydropyrido[4,3-d]pyrimidin-2-yl)sulfonyl)piperidine-1-carboxylate (compound 11)

mCPBA (87.57 mg) was added to a solution of **intermediate 11-1** (50 mg) in dichloromethane (1 mL) at 0 °C, and the resulting mixture was stirred at room temperature for reaction for 1 h. The mixture was then separated by column chromatography (EA/PE, 0-100%) to give the title compound (20.7 mg).

LC-MS: m/z (ESI): 547.2 [M+Na]⁺.

¹H NMR (400 MHz, DMSO-*d₆*) δ = 9.25 (s, 1H), 4.08-3.96 (m, 3H), 3.77 (t, *J =* 6.7 Hz, 2H), 3.73-3.66 (m, 2H), 3.25 (t, *J =* 6.8 Hz, 2H), 2.92-2.75 (m, 2H), 2.61-2.55 (m, 2H), 1.95 (d, *J =* 12.1 Hz, 2H), 1.58-1.46 (m, 2H), 1.41 (s, 9H), 1.40 (s, 9H).

### Example 12. tert-Butyl 3-(2-(4,4-difluorocyclohexylsulfonyl)-5-oxo-7,8-dihydropyrido[4,3-d]pyrimidin-6(5H)-yl)propanoate (Compound 12)

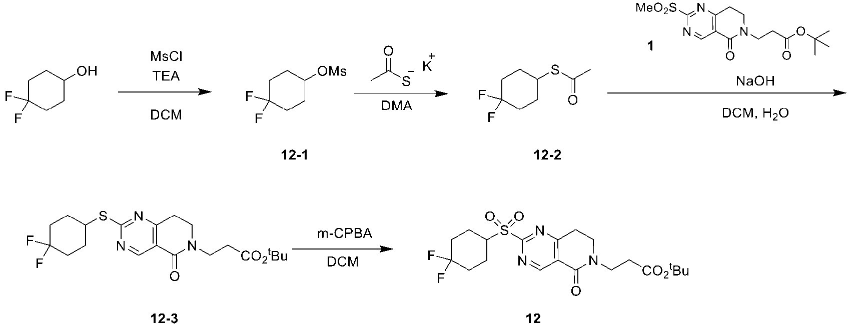

### Step 1: synthesis of 4,4-difluorocyclohexanol methanesulfonate (intermediate 12-1)

Methanesulfonyl chloride (1.01 g) was added dropwise to a solution of 4,4-difluorocyclohexanol (1 g) and triethylamine (1.23 mL) in DCM (36 mL) at 0 °C, and the resulting mixture was stirred at 25 °C for 16 h. After the reaction was completed, the mixture was poured into water (50 mL), and the resulting mixture was extracted with ethyl acetate (20 mL × 3). The organic phases were combined, dried over anhydrous sodium sulfate, and concentrated under reduced pressure to give a crude product of the title compound (1.57 g), which was directly used in the next step without further purification.

¹H NMR (400 MHz, DMSO-*d₆*) δ = 4.93-4.77 (m, 1H), 3.22 (s, 3H), 2.11-1.80 (m, 8H)

### Step 2: synthesis of S-(4,4-difluorocyclohexyl) ethanethioate (intermediate 12-2)

Potassium thioacetate (1.26 g) was added to a solution of intermediate 12-1 (785 mg) in DMA (18 mL), and the resulting mixture was heated at 80 °C for reaction for 18 h. The mixture was then cooled to room temperature. Ethyl acetate (50 mL) and water (50 mL) were added, and the mixture was extracted with ethyl acetate (3 × 20 mL). The organic phases were combined, dried over anhydrous sodium sulfate, and concentrated under reduced pressure, and the residue was purified by column chromatography (THF/PE, 0-6%) to give the title compound (368 mg).

¹H NMR (400 MHz, DMSO-*d*₆) δ = 3.58-3.46 (m, 1H), 2.32 (s, 3H), 2.08-1.85 (m, 8H).

### Step 3: synthesis of tert-butyl 3-(2-(4,4-difluorocyclohexylthio)-5-oxo-7,8-dihydropyrido[4,3-d]pyrimidin-6(5H)-yl)propanoate (intermediate 12-3)

NaOH (2 M, 1.54 mL) was added to a solution of **intermediate 12-2** (100 mg) in THF (1 mL), and the reaction mixture was stirred at 25 °C for 2 h. Compound **1** (90 mg) was then added. The mixture was stirred at 25 °C for 1 h and then poured into 5 mL of water. The resulting mixture was extracted with ethyl acetate (2 mL × 3). The organic phases were combined, dried over anhydrous sodium sulfate, and concentrated under reduced pressure, and the residue was separated by column chromatography (EA/PE, 0-31%) to give the title compound (80 mg).

LC-MS: m/z (ESI): 428.1 [M+H]⁺

### Step 4: synthesis of tert-butyl 3-(2-(4,4-difluorocyclohexylsulfonyl)-5-oxo-7,8-dihydropyrido[4,3-d]pyrimidin-6(5H)-yl)propanoate (compound 12)

mCPBA (151.96 mg, 85% purity) was added to a solution of **intermediate 12-3** (80 mg) in DCM (1 mL), and the mixture was stirred at 25 °C for 2 h. The mixture was then poured into 5 mL of water. The resulting mixture was extracted with ethyl acetate (2 mL × 3). The organic phases were combined, dried over anhydrous sodium sulfate, and concentrated under reduced pressure, and the residue was separated by column chromatography (EA/PE, 0-40%) and then purified by preparative thin layer chromatography (PE/EA = 1/2) to give the title compound (9.3 mg).

LC-MS: m/z (ESI): 460.2 [M+H]⁺

¹H NMR (400 MHz, DMSO-*d₆*) δ = 9.25 (s,1H), 4.05-3.95 (m, 1H), 3.82-3.73 (m, 2H), 3.70 (t, *J =* 6.8 Hz, 2H), 3.24 (t, *J=* 6.5 Hz, 2H), 2.64-2.54 (m, 2H), 2.19-1.93 (m, 6H), 1.71 (d, *J =* 12.4 Hz, 2H), 1.39 (s, 9H).

¹⁹F NMR (376 MHz, DMSO-*d₆*) δ = -91.16 (d, *J=* 235.6 Hz), -100.27 (d, *J =* 235.6 Hz).

### Example 13. tert-Butyl 3-(2-methylsulfonyl-5-oxo-8,8-dimethyl-7,8-dihydropyrido[4,3-d]pyrimidin-6(5H)-yl)propanoate (Compound 13)

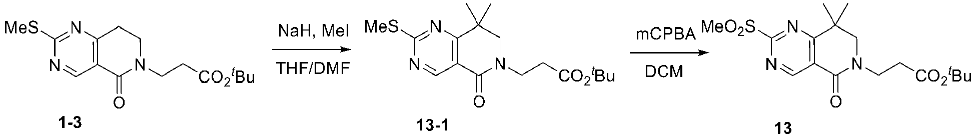

### Step 1: synthesis of tert-butyl 3-(2-methylthio-5-oxo-8,8-dimethyl-7,8-dihydropyrido[4,3-d]pyrimidin-6(5H)-yl)propanoate (intermediate 13-1)

NaH (1.76 g, 60% mass fraction in mineral oil) was added to a mixture of **intermediate 1-3** (4.75 g) and THF (100 mL) at 0 °C, and the resulting mixture was reacted at 0 °C for 30 min. Then, iodomethane (6.24 g) and DMF (20 mL) were added to the mixture obtained by the reaction, and the resulting mixture was reacted at room temperature for another 4 h. 2 mL of water was then added to the mixture, and the mixture was concentrated under reduced pressure to remove the solvent. The residue was separated by column chromatography (EA/PE, 0-50%) to give the title compound (2.77 g).

LC-MS: m/z (ESI): 352.4 [M+H]⁺.

### Step 2: synthesis of tert-butyl 3-(2-methylthio-5-oxo-8,8-dimethyl-7,8-dihydropyrido[4,3-d]pyrimidin-6(5H)-yl)propanoate (compound 13)

mCPBA (6.38 g, 85% purity) was added to a mixture of the crude product of the compound obtained in the previous step and DCM (100 mL) at 0 °C, and the resulting mixture was reacted at room temperature for 30 min. The mixture was then concentrated under reduced pressure, and the residue was separated by column chromatography (EA/PE, 0-100%) to give the title compound (2.87 g).

LC-MS: m/z (ESI): 384.4 [M+H]⁺.

¹H NMR (400 MHz, DMSO-*d*₆) δ 9.27 (s, 1H), 3.70 (t, *J=* 7.0 Hz, 2H), 3.60 (s, 2H), 3.45 (s, 3H), 2.58 (t, *J =* 7.0 Hz, 2H), 1.39 (s, 9H), 1.33 (s, 6H).

### Example 14. tert-Butyl 3-(2-tert-butylsulfonyl-5-oxo-8,8-dimethyl-7,8-dihydropyrido[4,3-d]pyrimidin-6(5H)-yl)propanoate (Compound 14)

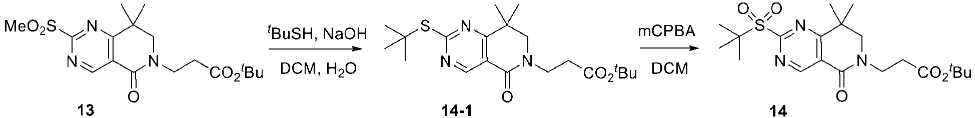

### Step 1: synthesis of tert-butyl 3-(2-tert-butylthio-5-oxo-8,8-dimethyl-7,8-dihydropyrido[4,3-d]pyrimidin-6(5H)-yl)propanoate (intermediate 14-1)

*tert*-Butanethiol (212 mg) and a 2 M sodium hydroxide solution (0.94 mL) were sequentially added to a solution of compound **13** (600 mg) dissolved in DCM (10 mL) at room temperature, and the resulting mixture was stirred at room temperature for reaction for 30 min. The mixture was then separated by column chromatography (EA/PE, 0-50%) to give a crude product of the title compound, which was directly used in the next step.

LC-MS: m/z (ESI): 394.4 [M+H]⁺.

### Step 2: synthesis of tert-butyl 3-(2-tert-butylsulfonyl-5-oxo-8,8-dimethyl-7,8-dihydropyrido[4,3-d]pyrimidin-6(5H)-yl)propanoate (compound 14)

mCPBA (1.27 g, 85% purity) was added to a mixture of the crude product of the compound obtained in the previous step and DCM (30 mL) at 0 °C, and the resulting mixture was reacted at room temperature for 30 min. The mixture was then concentrated under reduced pressure, and the residue was separated by column chromatography (EA/PE, 0-100%) to give the title compound.

LC-MS: m/z (ESI): 426.5 [M+H]⁺.

### Example 15. tert-Butyl 3-(2-tert-butylsulfonyl-5-oxopyrimido[5,4-c]quinolin-6(5H)-yl)propanoate (Compound 15)

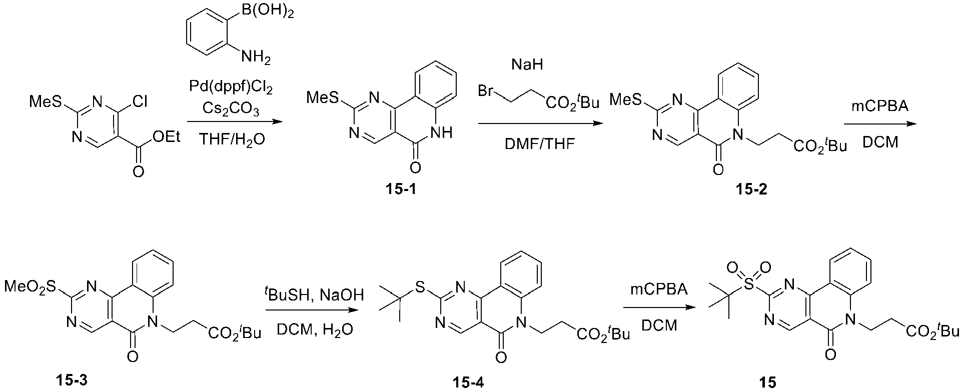

### Step 1: synthesis of 2-methylthiopyrimido[5,4-c]quinolin-5(6H)-one (intermediate 15-1)

THF (40 mL) and water (6 mL) were added to a mixture of ethyl 4-chloro-2-methylthio-5-pyrimidinecarboxylate (1.86 g), 2-aminophenylboronic acid (1.096 g), Pd(dppf)Cl₂ (585 mg), and Cs₂CO₃ (5.2 g) under a nitrogen atmosphere, and the resulting mixture was stirred at 70 °C for reaction for 4 h. The mixture was then cooled to room temperature overnight and filtered, and the filter cake was washed with water and petroleum ether to give the title compound (400 mg).

LC-MS: m/z (ESI): 244.3 [M+H]⁺.

### Step 2: synthesis of tert-butyl 3-(2-methylthio-5-oxopyrimido[5,4-c]quinolin-6(5H)-yl)propanoate (intermediate 15-2)

NaH (59 mg, 60% mass fraction in mineral oil) was added to a mixture of **intermediate 15-1** (90 mg) dissolved in DMF (3 mL) and THF (3 mL) under a nitrogen atmosphere, and the resulting mixture was stirred at room temperature for reaction for 30 min. *tert*-Butyl 3-bromopropanoate (309 mg) was then added, and the resulting mixture was reacted at 70 °C for 16 h. 0.5 mL of water was then added to the mixture, and the resulting mixture was concentrated under reduced pressure to remove the solvent. The residue was separated by column chromatography to give a crude product of the title compound, which was directly used in the next step.

LC-MS: m/z (ESI): 372.4 [M+H]⁺.

### Step 3: synthesis of tert-butyl 3-(2-methylsulfonyl-5-oxopyrimido[5,4-c]quinolin-6(5H)-yl)propanoate (intermediate 15-3)

mCPBA (200 mg, 85% purity) was added to a mixture of the crude product of the compound obtained in the previous step and DCM (10 mL) at 0 °C, and the resulting mixture was reacted at room temperature for 30 min. The mixture was then concentrated under reduced pressure, and the residue was separated by column chromatography (EA/PE, 0-100%) to give a crude product of the title compound, which was directly used in the next step.

LC-MS: m/z (ESI): 404.4 [M+H]⁺.

### Step 4: synthesis of tert-butyl 3-(2-tert-butylthio-5-oxopyrimido[5,4-c]quinolin-6(5H)-yl)propanoate (intermediate 15-4)

*tert*-Butanethiol (200 mg) and a 2 M sodium hydroxide solution (0.5 mL) were sequentially added to a solution of the crude product of the compound obtained in the previous step dissolved in DCM (10 mL) at room temperature, and the resulting mixture was vigorously stirred at room temperature for reaction for 30 min. The mixture was then separated by column chromatography (EA/PE, 0-50%) to give a crude product of the title compound.

LC-MS: m/z (ESI): 414.4 [M+H]⁺.

### Step 5: synthesis of tert-butyl 3-(2-tert-butylsulfonyl-5-oxopyrimido[5,4-c]quinolin-6(5H)-yl)propanoate (compound 15)

mCPBA (200 mg, 85% purity) was added to a mixture of the crude product of the compound obtained in the previous step and DCM (10 mL) at 0 °C, and the resulting mixture was reacted at room temperature for 30 min. The mixture was then concentrated under reduced pressure, and the residue was separated by column chromatography (EA/PE, 0-100%) to give the title compound (28.9 mg).

LC-MS: m/z (ESI): 446.5 [M+H]⁺.

¹H NMR (400 MHz, DMSO-*d*₆) δ 9.78 (s, 1H), 8.73-8.67 (m, 1H), 7.96-7.89 (m, 1H), 7.81 (d, *J=* 8.6 Hz, 1H), 7.55 (t, *J=* 7.5 Hz, 1H), 4.62-4.55 (m, 2H), 2.70-2.62 (m, 2H), 1.45 (s, 9H), 1.38 (s, 9H).

### Example 16. tert-Butyl 3-(2-tert-butylsulfonyl-5-oxo-5,7,8,9-tetrahydro-6H-pyrimido[5,4-c]azepin-6-yl)propanoate (Compound 16)

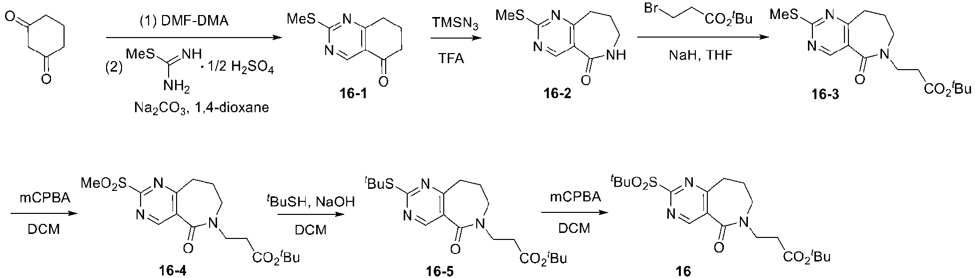

### Step 1: synthesis of 2-methylthio-7,8-dihydroquinazolin-5(6H)-one (intermediate 16-1)

A mixture of 1,3-cyclohexanedione (11.2 g) and DMF-DMA (35.7 g) was stirred at room temperature for reaction for 1 h under a nitrogen atmosphere, then heated to 50 °C, and reacted for 1 h. After the mixture was concentrated under reduced pressure to remove the solvent, methylisothiourea hemisulfate (13.9 g), sodium carbonate (12.7 g), and 1,4-dioxane (200 mL) were added, and the resulting mixture was stirred at 100 °C for reaction for 16 h. The mixture was then concentrated under reduced pressure to remove the solvent. The residue was separated by column chromatography (EA/PE, 0-30%) to give the title compound (5 g).

LC-MS: m/z (ESI): 195.4 [M+H]⁺.

### Step 2: synthesis of 2-methylthio-6,7,8,9-tetrahydro-5H-pyrimido[5,4-c]azepin-5-one (intermediate 16-2)

Trimethylazidosilane (2.3 g) was added to a solution of **intermediate 16-1** (1.94 g) dissolved in trifluoroacetic acid (20 mL) at 0 °C under a nitrogen atmosphere, and the resulting mixture was stirred at 25 °C for reaction for 10 days. The mixture was then poured into 200 mL of water, and the resulting mixture was extracted with dichloromethane (50 mL × 3). The organic phases were combined, dried over anhydrous sodium sulfate, and separated by column chromatography (EA/PE, 0-100%) to give the title compound (210 mg).

LC-MS: m/z (ESI): 210.5 [M+H]⁺.

### Step 3: synthesis of tert-butyl 3-(2-methylthio-5-oxo-5,7,8,9-tetrahydro-6H-pyrimido[5,4-c]azepin-6-yl)propanoate (intermediate 16-3)

NaH (80 mg, 60% mass fraction in mineral oil) was added to a mixture of **intermediate 16-2** (210 mg) and THF (5 mL) at 0 °C, and the resulting mixture was reacted at 0 °C for 30 min. *tert*-Butyl 3-bromopropanoate (418 mg) was then added to the mixture obtained by the reaction, and the resulting mixture was warmed to 70 °C and reacted for 16 h. The mixture was then concentrated under reduced pressure to remove the solvent, and the residue was separated by column chromatography (EA/PE, 0-70%) to give a crude product of the title compound, which was directly used in the next step.

LC-MS: m/z (ESI): 338.4 [M+H]⁺.

### Step 4: synthesis of tert-butyl 3-(2-methylsulfonyl-5-oxo-5,7,8,9-tetrahydro-6H-pyrimido[5,4-c]azepin-6-yl)propanoate (intermediate 16-4)

mCPBA (200 mg) was added to a mixture of the crude product of the compound obtained in the previous step and DCM (5 mL) at 0 °C, and the resulting mixture was reacted at room temperature for 30 min. The mixture was then separated by column chromatography (EA/PE, 0-100%) to give a crude product of the title compound, which was directly used in the next step.

LC-MS: m/z (ESI): 370.4 [M+H]⁺.

### Step 5: synthesis of tert-butyl 3-(2-tert-butylthio-5-oxo-5,7,8,9-tetrahydro-6H-pyrimido[5,4-c]azepin-6-yl)propanoate (intermediate 16-5)

*tert*-Butanethiol (0.5 mL) and a 2 M sodium hydroxide solution (1 mL) were added to a mixture of the crude product of the compound obtained in the previous step and DCM (5 mL) with vigorous stirring, and the resulting mixture was reacted at room temperature for 1 h. The mixture was then separated by column chromatography (EA/PE, 0-100%) to give a crude product of the title compound, which was directly used in the next step.

LC-MS: m/z (ESI): 380.4 [M+H]⁺.

### Step 6: synthesis of tert-butyl 3-(2-tert-butylsulfonyl-5-oxo-5,7,8,9-tetrahydro-6H-pyrimido[5,4-c]azepin-6-yl)propanoate (compound 16)

mCPBA (200 mg) was added to a mixture of the crude product of the compound obtained in the previous step and DCM (5 mL) at 0 °C, and the resulting mixture was reacted at room temperature for 30 min. The mixture was then separated by column chromatography (EA/PE, 0-100%) to give the title compound (14.2 mg).

LC-MS: m/z (ESI): 412.5 [M+H]⁺.

¹H NMR (400 MHz, DMSO-*d*₆) δ 9.04 (s, 1H), 3.75 (t, *J=* 7.0 Hz, 2H), 3.37-3.34 (m, 2H), 2.99 (t, *J=* 7.3 Hz, 2H), 2.61 (t, *J=* 7.0 Hz, 2H), 2.21-2.11 (m, 2H), 1.42 (s, 9H), 1.38 (s, 9H).

### Example 17. tert-Butyl 3-(2-(cyclopentylsulfonyl)-5-oxo-8,8-di(2,5,8,11-tetraoxatridecan-13-yl)-7,8-dihydropyrido[4,3-d]pyrimidin-6(5H)-yl)propanoate (Compound 17)

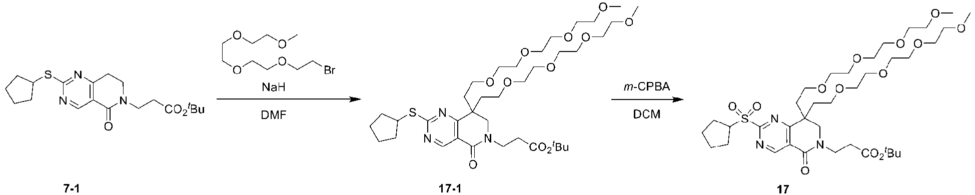

### Step 1: synthesis of tert-butyl 3-(2-cyclopentylthio-5-oxo-8,8-di(2,5,8,11-tetraoxatridecan-13-yl)-7,8-dihydropyrido[4,3-d]pyrimidin-6(5H)-yl)propanoate (intermediate 17-1)

**Intermediate 7-1** (50 mg) was dissolved in DMF (1 mL) at 0 °C under a nitrogen atmosphere, and NaH (26.5 mg, 60% effective content) was added thereto. The reaction mixture was stirred at 25 °C for 30 min, and 13-bromo-2,5,8,11-tetraoxatridecane (179.57 mg) was added thereto. The reaction mixture was stirred at 25 °C for 3 h under a nitrogen atmosphere. The mixture was then poured into 30 mL of water, and the resulting mixture was extracted with ethyl acetate (20 mL × 3). The organic phases were combined, dried over anhydrous sodium sulfate, and concentrated under reduced pressure, and the residue was separated by column chromatography (THF/PE, 0-100%) to give the title compound (25 mg).

LC-MS: m/z (ESI): 758.5 [M+H]⁺

### Step 2: synthesis of tert-butyl 3-(2-cyclopentylsulfonyl-5-oxo-8,8-di(2,5,8,11-tetraoxatridecan-13-yl)-7,8-dihydropyrido[4,3-d]pyrimidin-6(5H)-yl)propanoate (compound 17)

**Intermediate 17-1** (25 mg) was dissolved in anhydrous dichloromethane (1 mL) at 0 °C, and *m-*chloroperoxybenzoic acid (22.77 mg, 85% purity) was added thereto. The reaction mixture was stirred at 25 °C for 1 h. The mixture was then purified by reversed-phase high performance liquid chromatography (column: Boston Green ODS 150 × 30 mm × 5 µm; mobile phase: [A phase: water (0.225% formic acid), B phase: acetonitrile]; B%: 50%-80%, 10 min) to give the title compound (2.2 mg).

LC-MS: m/z (ESI): 790.4 [M+H]⁺.

¹H NMR (400 MHz, D₂O) δ = 9.28 (s, 1H), 4.36-4.24 (m, 1H), 3.84 (t, *J =* 6.3 Hz, 2H), 3.80 (s, 2H), 3.63-3.49 (m, 20H), 3.47-3.36 (m, 8H), 3.32 (s, 6H), 2.67 (t, *J =* 6.3 Hz, 2H), 2.33-2.22 (m, 2H), 2.20-2.10 (m, 2H), 2.09-1.97 (m, 4H), 1.70-1.62 (m, 4H), 1.39 (s, 9H)

### Example 18. tert-Butyl 6-(2-(cyclopentylsulfonyl)-5-oxo-8,8-di(2,5,8,11-tetraoxatridecan-13-yl)-7,8-dihydropyrido[4,3-d]pyrimidin-6(5H)-yl)hexanoate (Compound 18)

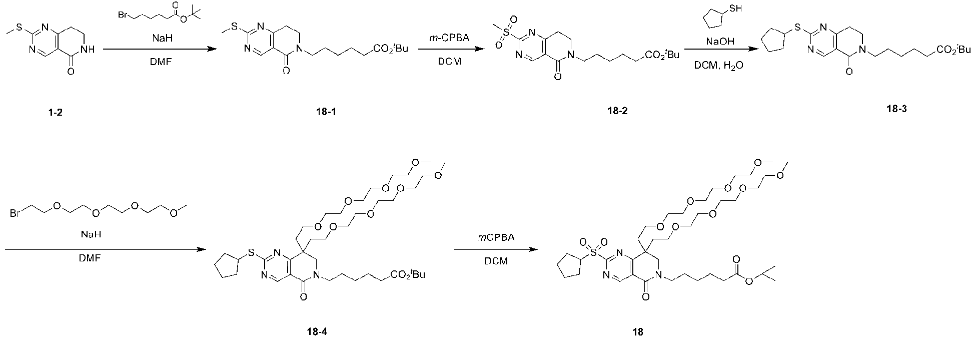

### Step 1: synthesis of tert-butyl 6-(2-methylthio-5-oxo-7,8-dihydropyrido[4,3-d]pyrimidin-6(5H)-yl)hexanoate (intermediate 18-1)

NaH (307.28 mg, 60% mass fraction in mineral oil) was added to a mixture of **intermediate 1-2** (1.0 g) and DMF (20 mL) at 0 °C under a nitrogen atmosphere. The reaction mixture was stirred at 25 °C for 30 min, and then *tert-*butyl 6-bromohexanoate (1.93 g) was added thereto. The reaction mixture was stirred at 25 °C for 1 h under a nitrogen atmosphere. The mixture was then poured into 50 mL of water, and the resulting mixture was extracted with ethyl acetate (30 mL × 3). The organic phases were combined, dried over anhydrous sodium sulfate, and concentrated under reduced pressure, and the residue was separated by column chromatography (THF/PE, 0-50%) to give the title compound (500 mg).

LC-MS: m/z (ESI): 366.2 [M+H]⁺.

### Step 2: synthesis of tert-butyl 6-(2-methylsulfonyl-5-oxo-7,8-dihydropyrido[4,3-d]pyrimidin-6(5H)-yl)hexanoate (intermediate 18-2)

**Intermediate 18-1** (500 mg) was dissolved in anhydrous dichloromethane (10 mL) at 0 °C, and *m-*chloroperoxybenzoic acid (944.30 mg, 85% purity) was added thereto. The reaction mixture was stirred at 25 °C for 1 h. The mixture was then separated by column chromatography (THF/PE, 0-50%) to give the title compound (500 mg).

LC-MS: m/z (ESI): 342.0 [M-56]⁺.

### Step 3: synthesis of tert-butyl 6-(2-cyclopentylthio-5-oxo-7,8-dihydropyrido[4,3-d]pyrimidin-6(5H)-yl)hexanoate (intermediate 18-3)

**Intermediate 18-2** (500 mg) and cyclopentanethiol (193 mg) were dissolved in dichloromethane (10 mL) and a 2 M sodium hydroxide solution (5 mL) at room temperature. The resulting mixture was stirred at 25 °C for reaction for 3 h. The mixture was then concentrated under reduced pressure to remove the solvent. The residue was separated by column chromatography (THF/PE, 0-30%) to give the title compound (460 mg).

LC-MS: m/z (ESI): 442.1 [M+Na]⁺.

### Step 4: synthesis of tert-butyl 6-(2-(cyclopentylthio)-5-oxo-8,8-di(2,5,8,11-tetraoxatridecan-13-yl)-7,8-dihydropyrido[4,3-d]pyrimidin-6(5H)-yl)hexanoate (intermediate 18-4)

**Intermediate 18-3** (350 mg) was dissolved in DMF (5 mL) at 0 °C under a nitrogen atmosphere, and NaH (167 mg, 60% effective content) was added thereto. The reaction mixture was stirred at 25 °C for 30 min, and 13-bromo-2,5,8,11-tetraoxatridecane (1.13 g) was added thereto. The reaction mixture was stirred at 25 °C for 3 h under a nitrogen atmosphere. The mixture was then poured into 30 mL of water, and the resulting mixture was extracted with ethyl acetate (20 mL × 3). The organic phases were combined, dried over anhydrous sodium sulfate, and concentrated under reduced pressure, and the residue was separated by column chromatography (THF/PE, 0-100%) to give the title compound (300 mg).

LC-MS: m/z (ESI): 800.6 [M+H]⁺.

### Step 5: synthesis of tert-butyl 6-(2-(cyclopentylsulfonyl)-5-oxo-8,8-di(2,5,8,11-tetraoxatridecan-13-yl)-7,8-dihydropyrido[4,3-d]pyrimidin-6(5H)-yl)hexanoate (compound 18)

**Intermediate 18-4** (450 mg) was dissolved in anhydrous dichloromethane (5 mL) at 0 °C, and m-chloroperoxybenzoic acid (388.25 mg, 85% purity) was added thereto. The reaction mixture was stirred at 25 °C for 1 h. The mixture was then separated by column chromatography (EA/PE, 0-100%) to give the title compound (300 mg).

LC-MS: m/z (ESI): 832.3 [M+H]⁺.

¹H NMR (400 MHz, DMSO-*d₆*) δ = 9.26 (s, 1H), 4.31-4.28 (m, 1H), 3.70 (s, 2H), 3.53-3.39 (m, 24H), 3.38-3.34 (m, 4H), 3.32-3.30 (m, 1H), 3.23 (s, 6H), 2.20 (t, *J =* 7.3 Hz, 2H), 2.13-1.93 (m, 8H), 1.73-1.63 (m, 4H), 1.59-1.55 (m, 4H), 1.39 (s, 9H), 1.33-1.29 (m, 2H)

### Example 19. tert-Butyl 3-(2'-(cyclopentylsulfonyl)-5'-oxo-5'H-spiro [cyclopentane-1,8'-pyrido[4,3-d]pyrimidin]-6'(7'H)-yl)propanoate (Compound 19)

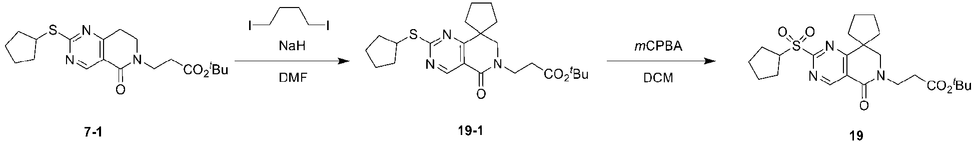

### Step 1: synthesis of tert-butyl 3-(2'-(cyclopentylthio)-5'-oxo-5'H-spiro[cyclopentane-1,8'-pyrido[4,3-d]pyrimidin]-6'(7'H)-yl)propanoate (intermediate 19-1)

**Intermediate 7-1** (1 g) was dissolved in *N,N-*dimethylformamide (10 mL) at 0 °C under a nitrogen atmosphere, and NaH (635.7 mg, 60% purity) was added thereto. The reaction mixture was stirred at 0 °C for 15 min, and 1,4-diiodobutane (2.46 g) was added thereto. The reaction mixture was stirred at 0 °C for 1 h under a nitrogen atmosphere. The mixture was then poured into 20 mL of ice water, and the resulting mixture was extracted with ethyl acetate (10 mL × 3). The organic phases were combined, dried over anhydrous sodium sulfate, and concentrated under reduced pressure, and the residue was separated by column chromatography (ethyl acetate/petroleum ether, 0-25%) to give the title compound (620 mg).

LC-MS: m/z (ESI): 432.1 [M+H]⁺

### Step 2: synthesis of tert-butyl 3-(2'-(cyclopentylsulfonyl)-5'-oxo-5'H-spiro[cyclopentane-1,8'-pyrido[4,3-d]pyrimidin]-6'(7'H)-yl)propanoate (compound 19)

**Intermediate 19-1** (100 mg) was dissolved in anhydrous dichloromethane (2 mL) at 0 °C, and m-chloroperoxybenzoic acid (188.16 mg, 85% purity) was added thereto. The reaction mixture was stirred at 0 °C for 1 h. The mixture was concentrated under reduced pressure, and the residue was separated by column chromatography (ethyl acetate/petroleum ether, 0-50%) to give the title compound (49.2 mg).

LC-MS: m/z (ESI): 464.3 [M+H]⁺.

¹H NMR (400 MHz, Chloroform-d) δ = 9.35 (s, 1H), 4.34-4.13 (m, 1H), 3.80 (t, *J =* 6.4 Hz, 2H), 3.61 (s, 2H), 2.66 (t, *J =* 6.3 Hz, 2H), 2.24-2.11 (m, 4H), 2.05 (m, 2H), 1.99-1.90 (m, 2H), 1.89-1.75 (m, 6H), 1.73-1.65 (m, 2H), 1.45 (s, 9H)

### Example 20. tert-Butyl 3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanoate (Compound 20)

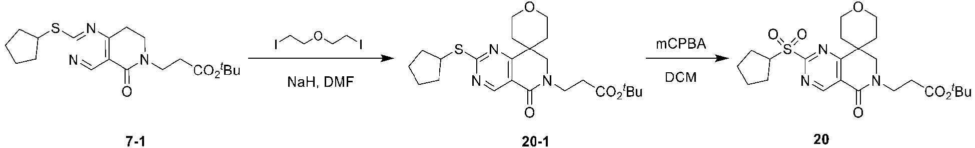

### Step 1: synthesis of tert-butyl 3-(2'-(cyclopentylthio)-5'-oxo-2,3,5,6-tetrahydro-5'H-spiro[pyran-4,8'-pyrido[4,3-d]pyrimidin]-6'(7'H)-yl)propanoate (intermediate 20-1)

**Intermediate 7-1** (200 mg) was dissolved in N,N-dimethylformamide (2 mL) at 0 °C under a nitrogen atmosphere, NaH (127.15 mg, 60% effective content) was added thereto, and 1-iodo-2-(2-iodoethoxy)ethane (518.01 mg) was added thereto. The reaction mixture was stirred at 0 °C for 1 h under a nitrogen atmosphere. Ice water (2 mL) was then added to the reaction mixture, and the resulting mixture was extracted with ethyl acetate (1 mL × 3). The organic phases were combined, dried over anhydrous sodium sulfate, and concentrated under reduced pressure, and the residue was separated by column chromatography (ethyl acetate/petroleum ether, 0-60%) to give the title compound (110 mg).

MS m/z (ESI): 448.2 [M+H]⁺.

### Step 2: synthesis of tert-butyl 3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanoate (compound 20)

**Intermediate 20-1** (60 mg) was dissolved in anhydrous dichloromethane (1 mL) at 0 °C, and m-chloroperoxybenzoic acid (108.86 mg, 85% purity) was added thereto. The reaction mixture was stirred at 25 °C for 1 h. After the reaction was completed, the mixture was concentrated under reduced pressure, and the residue was separated by column chromatography (ethyl acetate/petroleum ether, 0-55%) to give the title compound (9.8 mg). MS m/z (ESI): 480.3 [M+H]⁺.

**¹H NMR (400 MHz, Chloroform-d)** δ = 9.40 (s, 1H), 4.35-4.18 (m, 1H), 4.00 (m, 2H), 3.86-3.72 (m, 6H), 2.68 (t, *J* = 6.3 Hz, 2H), 2.31-2.23 (m, 2H), 2.18 (m, 2H), 2.06 (m, 2H), 1.93-1.81 (m, 2H), 1.77-1.65 (m, 4H), 1.45 (s, 9H)

### Example 21. tert-Butyl 3-(2-methylsulfonyl-7-oxo-5,7-dihydro-6H-pyrrolo[3,4-d]pyrimidin-6-yl)propanoate (Compound 21)

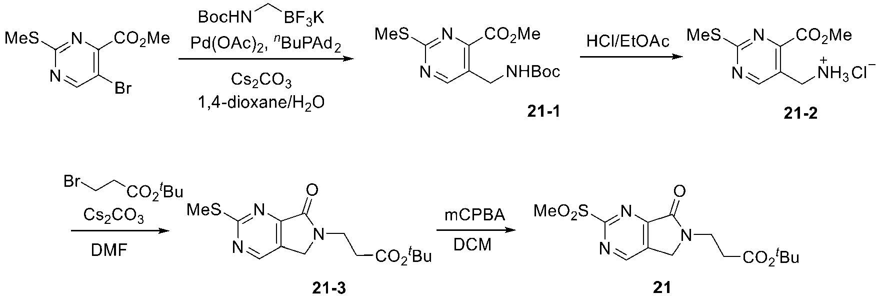

### Step 1: synthesis of methyl 5-(((tert-butoxycarbonyl)amino)methyl)-2-methylthiopyrimidine-4-carboxylate (intermediate 21-1)

1,4-Dioxane (50 mL) and water (5 mL) were added to a mixture of methyl 5-bromo-2-methylsulfanylpyrimidine-4-carboxylate (4 g), potassium (((tert-butoxycarbonyl)amino)methyl)trifluoroborate (5.41 g), cesium carbonate (9.91 g), palladium(II) acetate (341.34 mg), and di(1-adamantyl)-n-butylphosphine (817.62 mg) at room temperature under a nitrogen atmosphere. The resulting mixture was stirred at 80 °C for reaction for 5 h. The mixture was then concentrated under reduced pressure to remove the solvent. The residue was separated by column chromatography (tetrahydrofuran/petroleum ether, 0-30%) to give the title compound (1300 mg).

LC-MS: m/z (ESI): 314.0 [M+H]⁺.

### Step 2: synthesis of (4-methoxycarbonyl-2-methylthiopyrimidin-5-yl)methylamine hydrochloride (intermediate 21-2)

A solution of hydrogen chloride in ethyl acetate (4 M, 20 mL) was added to a solution of **intermediate 21-1** (1300 mg) in ethyl acetate (20 mL) at room temperature under a nitrogen atmosphere, and the resulting mixture was stirred at room temperature for reaction for 4 h. After the reaction was completed as detected by LCMS, the mixture was filtered, and the filter cake was washed with ethyl acetate to give the title compound (750 mg).

LC-MS: m/z (ESI): 214.0 [M+H]⁺.

### Step 3: synthesis of tert-butyl 3-(2-methylthio-7-oxo-5,7-dihydro-6H-pyrrolo[3,4-d]pyrimidin-6-yl)propanoate (intermediate 21-3)

A mixture of **intermediate 21-2** (100 mg), cesium carbonate (195.71 mg), *tert*-butyl 3-bromopropanoate (83.73 mg), and *N*,*N*-dimethylformamide (4 mL) was heated to 80 °C and reacted for 16 h under a nitrogen atmosphere. The mixture was then concentrated under reduced pressure to remove the solvent. The residue was separated by column chromatography (tetrahydrofuran/petroleum ether, 0-50%) to give the title compound (20 mg).

LC-MS: m/z (ESI): 310.0 [M+H]⁺

### Step 4: synthesis of tert-butyl 3-(2-methylsulfonyl-7-oxo-5,7-dihydro-6H-pyrrolo[3,4-d]pyrimidin-6-yl)propanoate (compound 21)

*m*-Chloroperoxybenzoic acid (33.47 mg) was added to a solution of **intermediate 21-3** (20 mg) dissolved in dichloromethane (4 mL) at 0 °C, and the resulting mixture was stirred at room temperature for 2 h. The reaction mixture was directly purified by high performance liquid chromatography (column: Phenomenex Gemini NX 150 × 30 mm, 5 µm; mobile phase: [A: water (ammonium bicarbonate), B: acetonitrile]; B%: 13%-43%, 11 min) to give the title compound (2.9 mg).
(Preparation of mobile phase A: 30 L of pure water + 24 g of ammonium bicarbonate)

¹H NMR (400 MHz, DMSO-*d*₆) δ = 9.44 (s, 1H), 4.77-4.66 (m, 2H), 3.90-3.78 (m, 2H), 3.50-3.45 (m, 3H), 2.69-2.63 (m, 2H), 1.41-1.38 (m, 9H).

### Preparation Examples of Drug-Linker (Examples 22-40)

### Example 22. N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-6-(2-methylsulfonyl-5-oxo-7,8-dihydropyrido[4,3-d]pyrimidin-6(5H)-yl)hexanamide (a-L1-1)

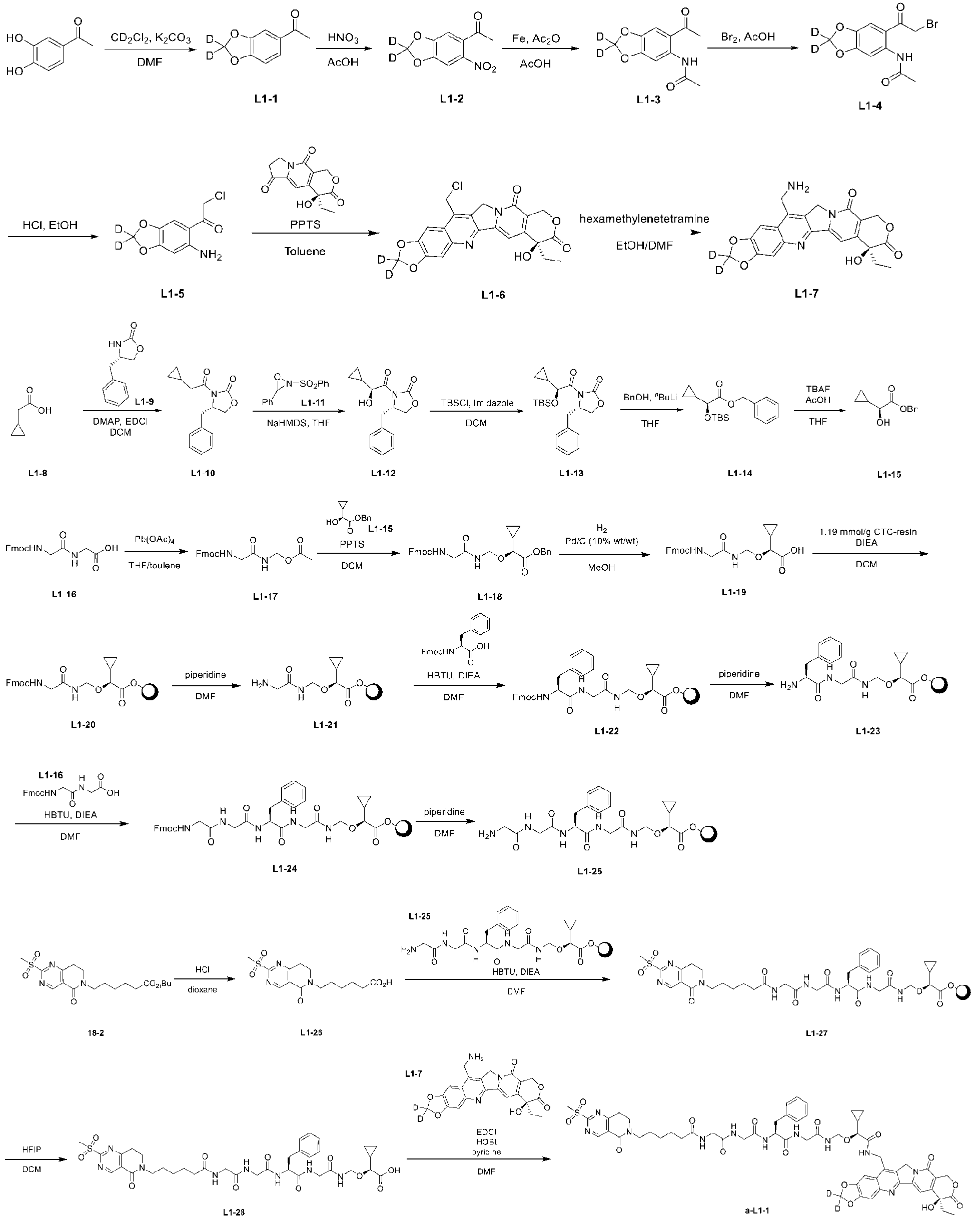

### Step 1: synthesis of 1-(benzo[d][1,3]dioxol-5-yl-2,2-d₂)ethan-1-one (intermediate L1-1)

3',4'-Dihydroxyacetophenone (3 g) was dissolved in anhydrous DMF (25 mL), and deuterated dichloromethane (8.57 g) and potassium carbonate (8.18 g) were added. After the addition, the mixture was warmed to 90 °C and stirred for 16 h. The reaction mixture was then added to water (100 mL), and the mixture was extracted with ethyl acetate (200 mL × 2). The organic phases were combined, washed with saturated brine (100 mL), and dried over anhydrous sodium sulfate. After filtration, the filtrate was concentrated to dryness under reduced pressure, and the residue was purified by column chromatography (ethyl acetate/petroleum ether = 5:1) to give the title compound (2.4 g).

MS m/z (ESI): 167.1 [M+H]⁺.

### Step 2: synthesis of 1-(6-nitrobenzo[d][1,3]dioxol-5-yl-2,2-d₂)ethan-1-one (intermediate L1-2)

**Intermediate L1-1** (2.4 g) was dissolved in anhydrous acetic acid (10 mL), concentrated nitric acid (32.50 g, 70% content) was added dropwise at 0 °C, and then the mixture was stirred at 0 °C for 10 min. Then the mixture was warmed to room temperature and stirred for 1 h. After the reaction was completed, the reaction mixture was added dropwise to ice water (200 mL), and after filtration, the filter cake was dried to give the title compound (1.9 g).

MS m/z (ESI): 212.0 [M+H]⁺.

**¹H NMR (400 MHz, DMSO-*d*₆)** δ 7.69 (s, 1H), 7.30 (s, 1H), 2.49 (s, 3H).

### Step 3: synthesis of N-(6-acetylbenzo[d][1,3]dioxol-5-yl-2,2-d₂)acetamide (intermediate L1-3)

**Intermediate L1-2** (1.8 g) was dissolved in acetic acid (25 mL), acetic anhydride (1.84 g) and reduced iron powder (4.76 g) were added, and the mixture was stirred at room temperature for 1 h. After the reaction was completed, the mixture was filtered, the filtrate was concentrated to dryness under reduced pressure, and the residue was purified by column chromatography (ethyl acetate/petroleum ether = 5:1) to give the title compound (1.5 g).

MS m/z (ESI): 224.1 [M+H]⁺.

### Step 4: synthesis of N-(6-(2-bromoacetyl)benzo[d][1,3]dioxol-5-yl-2,2-d₂)acetamide (intermediate L1-4)

A solution of HBr in acetic acid (2.39 g, 33% content) was added dropwise to a solution of **intermediate L1-3** (1.45 g) in anhydrous acetic acid (25 mL), and Br₂ (1.07 g) was then added dropwise. After the dropwise addition was completed, the mixture was stirred at room temperature for 1 h. After the reaction was completed, the reaction mixture was concentrated to dryness under reduced pressure. The residue was added to water (50 mL), and the mixture was extracted with ethyl acetate (50 mL × 2). The organic phase was washed with saturated brine (50 mL), dried over anhydrous sodium sulfate, and filtered. The residue was purified by column chromatography (ethyl acetate/petroleum ether = 5:1) to give the title compound (1.3 g).

MS m/z (ESI): 302.1 [M+H]⁺.

### Step 5: synthesis of 1-(6-aminobenzo[d][1,3]dioxol-5-yl-2,2-d₂)-2-chloroethan-1-one (intermediate L1-5)

**Intermediate L1-4** (1.2 g) and concentrated hydrochloric acid (144.82 mg) were dissolved in ethanol (15 mL), and the reaction mixture was stirred at 60 °C for 16 h. After the reaction was completed, the reaction mixture was concentrated to dryness under reduced pressure. The residue was purified by high performance liquid chromatography (YMC-Actus Triart C18 column, 5 µm silica, 30 mm diameter, 150 mm length; a mixture of water (containing 0.05% NH₄HCO₃) and acetonitrile with decreasing polarity was used as the eluent (acetonitrile gradient: 40%-50%)) to give the title compound (577 mg).

MS m/z (ESI): 216.0 [M+H]⁺.

### Step 6: synthesis of (S)-14-(chloromethyl)-7-ethyl-7-hydroxy-10,13-dihydro-11H-[1,3]dioxolo[4,5-g]pyrano[3',4':6,7]indolizino[1,2-b]quinoline-8,11(7H)-dione-2,2-d₂ (intermediate L1-6)

**Intermediate L1-5** (100.0 mg) and (*S*)-4-ethyl-4-hydroxy-7,8-dihydro-1*H*-pyrano[3,4-*f*]indolizine-3,6,10(4*H*)-trione (109.87 mg) were dissolved in toluene (1 mL) and acetic acid (1 mL), and pyridinium *p*-toluenesulfonate (5.24 mg) was added thereto. The reaction mixture was stirred at 100 °C for 16 h. After the reaction was completed, the reaction mixture was cooled to room temperature and directly concentrated to dryness under reduced pressure. Ethanol (5 mL) was added, and the reaction mixture was stirred at 25 °C for 0.5 h. The reaction mixture was filtered, and the filter cake was washed with ethanol (5 mL × 2) to give the title compound (100.0 mg).

MS m/z (ESI): 443.0 [M+H]⁺.

### Step 7: synthesis of (S)-14-(aminomethyl)-7-ethyl-7-hydroxy-10,13-dihydro-11H-[1,3]dioxolo[4,5-g]pyrano[3',4':6,7]indolizino[1,2-b]quinoline-8,11(7H)-dione-2,2-d₂ (intermediate L1-7)

**Intermediate L1-6** (100.00 mg) was dissolved in absolute ethanol (1.5 mL) and anhydrous DMF (1.5 mL), and hexamethylenetetramine (94.97 mg) was added thereto. The reaction mixture was stirred at 50 °C for 6 h. After the reaction was completed, the reaction mixture was concentrated to dryness under reduced pressure, and the residue was purified by high performance liquid chromatography (column: Boston Green ODS 150 × 30 mm × 5 µm; mobile phase: [A: water (formic acid), B: acetonitrile]; B%: 0%-30%, 12 min) to give the title compound (25.0 mg).

MS m/z (ESI): 424.0 [M+H]⁺.

### Step 8: synthesis of (S)-4-benzyl-3-(2-cyclopropylacetyl)oxazolidin-2-one (intermediate L1-10)

Compound **L1-8** (150.0 g), DMAP (160.15 g), and compound **L1-9** (221.2 g) were weighed out and dissolved in 1500 mL of dichloromethane, and the mixture was stirred at room temperature for 15 min. EDCI (359.0 g) was weighed out and added to the reaction mixture in batches. After the addition, the mixture was stirred at room temperature for about 5 h. After the reaction was completed, dichloromethane (1500 mL) was added to the reaction mixture for dilution. The mixture was then sequentially washed twice with water (500 mL), washed once with 2 N HCl (500 mL), washed once with a saturated sodium bicarbonate solution (500 mL), and washed once with a saturated brine solution (500 mL). The organic phase was dried over anhydrous sodium sulfate and filtered. The filtrate was concentrated to dryness under reduced pressure to give the title compound (302 g).

**¹H NMR (400 MHz, CDCl₃)** δ 7.32-7.35 (m, 2H), 7.26-7.29 (m, 1H), 7.21-7.23 (m, 2H), 4.68-4.71 (m, 1H), 4.17-4.23 (m, 2H), 3.30-3.33 (dd, 1H), 2.91-2.95 (dd, 1H), 2.78-2.82 (m, 2H), 1.14-1.18 (m, 1H), 0.59-0.63 (m, 2H), 0.21-0.26 (m, 2H).

### Step 9: synthesis of (S)-4-benzyl-3-((S)-2-cyclopropyl-2-hydroxyacetyl)oxazolidin-2-one (intermediate L1-12)

**Intermediate L1-10** (200 g) was weighed out and dissolved in 2000 mL of anhydrous THF, and the mixture was stirred at -78 °C for 15 min under a nitrogen atmosphere. Subsequently, sodium bis(trimethylsilyl)amide (443.5 mL, 2 M in THF) was added dropwise to the reaction mixture. After the dropwise addition was completed, the reaction mixture was stirred at -78 °C for 30 min. **Intermediate L1-11** (201.5 g) was completely dissolved in 700 mL of THF, and the solution was added dropwise and slowly to the reaction mixture. After the dropwise addition was completed, the mixture was stirred at -78 °C for 2 h. Subsequently, 220 mL of glacial acetic acid was added to the reaction mixture to quench the reaction. After the dropwise addition was completed, the mixture was gradually warmed to room temperature, 600 mL of 2 N HCl was added to the reaction mixture, and the mixture was stirred at room temperature (20-25 °C) for 10 h. Subsequently, the reaction mixture was concentrated under reduced pressure, ethyl acetate (1000 mL) and water (200 mL) were added to the residue, and the mixture was stirred for 20 min. The separated aqueous phase was extracted twice with ethyl acetate (500 mL × 2). The organic phases were combined, and sequentially washed twice with 400 mL of a saturated NaHCO₃ solution, twice with 400 mL of a saturated Na₂S₂O₃ solution, and twice with saturated brine. The resulting organic phase was dried over anhydrous sodium sulfate and filtered. The filtrate was concentrated to dryness under reduced pressure, and the residue was purified by silica gel column chromatography (petroleum ether:ethyl acetate = 1/30) to give the title compound (128.7 g).

**¹H NMR (400 MHz, CDCl₃)** δ 7.37 (dd, J = 8.1, 6.8 Hz, 2H), 7.33-7.29 (m, 1H), 7.27-7.23 (m, 2H), 4.81 (dd, J = 7.9, 5.9 Hz, 1H), 4.72 (ddt, J = 10.0, 7.5, 2.9 Hz, 1H), 4.33 (t, J = 8.3Hz, 1H), 4.28 (dd, J = 9.1, 2.5 Hz, 1H), 3.48 (dd, J = 8.2, 3.9 Hz, 1H), 3.35 (dd, J = 13.5, 3.4 Hz,1H), 2.88 (dd, J = 13.5, 9.4 Hz, 1H), 1.36-1.29 (m, 1H), 0.62-0.44 (m, 4H).

### Step 10: synthesis of (S)-4-benzyl-3-((S)-2-((tert-butyldimethylsilyl)oxy)-2-cyclopropylacetyl)oxazolidin-2-one (intermediate L1-13)

**Intermediate L1-12** (128.7 g) was weighed out and dissolved in 1300 mL of DCM. Imidazole (56.17 g) was added, and the mixture was stirred for 15 min under an ice bath. TBSCl (107.3 g) was then added to the reaction mixture in batches, and the mixture was stirred at room temperature for 3 h. 200 mL of 2 N HCl was added to the reaction mixture. The mixture was stirred for 20 min and subjected to liquid separation. The organic phase was sequentially washed twice with 200 mL of a saturated NaHCO₃ solution and twice with saturated brine. The organic phase was dried over anhydrous sodium sulfate and filtered, the filtrate was concentrated to dryness under reduced pressure, and the residue was purified by silica gel column chromatography (petroleum ether:ethyl acetate = 80:1) to give the title compound (160 g).

**¹H NMR (400 MHz, CDCl₃)** δ 7.35-7.37 (m, 2H), 7.30-7.32 (m, 1H), 7.26-7.29 (m, 2H), 5.26-5.31 (m, 1H), 4.67-4.71 (m, 1H), 4.20-4.26 (m, 2H), 3.41-3.44 (dd, 1H), 2.72-2.76 (dd, 1H), 1.25-1.31 (m, 1H), 0.94 (s, 9H), 0.54-0.56 (m, 2H), 0.46-0.48 (m, 2H), 0.12 (s, 6H).

### Step 11: synthesis of benzyl (S)-2-((tert-butyldimethylsilyl)oxy)-2-cyclopropylacetate (intermediate L1-14)

Benzyl alcohol (62.18 g) was weighed out and dissolved in 500 mL of THF, and the mixture was stirred at -25 °C. *n*-Butyllithium (213.6 mL, 2.5 M in THF) was measured out and added dropwise and slowly to the reaction mixture. After the dropwise addition was completed, the mixture was stirred at -25 °C for 1 h. **Intermediate L1-13** (160 g) was weighed out and dissolved in 320 mL of THF, and the mixture was added dropwise and slowly to the reaction mixture at -25 °C. After the dropwise addition was completed, the mixture was stirred at -15 °C for 3 h. A saturated NH₄Cl (200 mL) solution was added to the reaction mixture to quench the reaction. The reaction mixture was then concentrated under reduced pressure, and 400 mL of methyl *tert*-butyl ether and water (150 mL) were added to the reaction mixture. The mixture was stirred for 30 min and subjected to liquid separation. The aqueous phase was extracted twice with methyl *tert*-butyl ether (200 mL × 2). The organic phases were combined, washed once with saturated brine (250 mL), dried over anhydrous sodium sulfate, and filtered. The filtrate was concentrated under reduced pressure, and the residue was purified by silica gel column chromatography (petroleum ether:ethyl acetate = 100:1) to give the title compound (125 g).

**¹H NMR (400 MHz, CDCl₃)** δ 7.28-7.40 (m, 5H), 5.17-5.26 (m, 2H), 3.86-3.89 (m, 1H), 1.21-1.46 (m, 1H), 0.90 (s, 9H), 0.45-0.51 (m, 4H), 0.05 (s, 6H).

### Step 12: synthesis of benzyl (S)-2-cyclopropyl-2-hydroxyacetate (intermediate L1-15)

**Intermediate L1-14** (125 g) was weighed out and dissolved in 1200 mL of THF. Glacial acetic acid (35.1 g) was added, and the mixture was stirred at room temperature for 5 min. Subsequently, TBAF (585 mL, 1 M in THF) was added to the reaction mixture, and the reaction mixture was reacted at 45 °C for 4 h. The reaction mixture was concentrated under reduced pressure to remove the solvent. 300 mL of water and 400 mL of methyl *tert-*butyl ether were added to the residue. The mixture was stirred for 20 min and subjected to liquid separation. The aqueous phase was extracted twice with methyl *tert*-butyl ether (200 mL). The organic phases were combined, and sequentially washed twice with 200 mL of a saturated NaHCO₃ solution and twice with saturated brine. The organic phase was dried over anhydrous sodium sulfate and filtered, the filtrate was concentrated to dryness under reduced pressure, and the residue was purified by silica gel column chromatography (petroleum ether:ethyl acetate = 60:1) to give the title compound (68.9 g).

**¹H NMR (400 MHz, CDCl₃)** δ 7.27-7.39 (m, 5H), 5.22-5.28 (m, 2H), 3.82 (d, 1H), 2.7 (brs, 1H), 1.11-1.15 (m, 1H), 0.41-0.56 (m, 4H).

**Intermediate L1-15** was further analyzed by the following chiral supercritical fluid chromatographic conditions.

| | | |
|---|---|---|
| Column | Chiralpak AD-3 150*4.6mm I.D., 3um | |
| Mobile phase | A: carbon dioxide | |
| | B: methanol (0.05% diethylamine) | |
| | Time (min) | B phase% |
| | 0 | 5 |
| | 4 | 40 |
| | 0.2 | 5 |
| | 1.8 | 5 |
| Flow rate | 2.5 mL/min | |
| Wavelength | PDA 220nm | |
| Column temperature | 35°C | |
| Column pressure | 1500 Psi | |
| Instrument model | Waters UPCC with PDA Detector | |

Under the chiral supercritical fluid chromatographic conditions described above, the retention time of **intermediate L1-15** was 3.013 min.

### Step 13: synthesis of (2-((((9H-fluoren-9-yl)methoxy)carbonyl)amino)acetamido)methyl acetate (intermediate L1-17)

**Compound L1-16** (25 g), lead acetate (43.79 g), and pyridine (6.98 g) were dissolved in a mixed solvent of tetrahydrofuran (600 mL) and toluene (200 mL). The reaction mixture was purged 3 times with nitrogen, heated to 85 °C under a nitrogen atmosphere, and stirred for reaction for 18 h. The reaction mixture was then cooled to room temperature and filtered, the filtrate was concentrated to dryness under reduced pressure, and the crude product was purified by a flash silica gel column (ISCO^{®}; 330 g SepaFlash^{®} Silica Flash Column, mobile phase gradient: 0-75% ethyl acetate/petroleum ether, flow rate: 100 mL/min) to give the title compound (18 g).

MS m/z (ESI): 391.1 [M+Na]⁺.

### Step 14: synthesis of benzyl (S)-10-cyclopropyl-1-(9H-fluoren-9-yl)-3,6-dioxo-2,9-dioxa-4,7-diazaundecane-11-carboxylate (intermediate L1-18)

**Intermediate L1-17** (5 g), **intermediate L1-15** (8.40 g), and pyridinium p-toluenesulfonate (341.09 mg) were dissolved in dichloromethane (150 mL). The reaction mixture was purged 3 times with nitrogen, heated to 65 °C under a nitrogen atmosphere, and stirred for reaction for 48 h. The reaction mixture was then cooled to room temperature and filtered, and the filtrate was concentrated to dryness under reduced pressure. The crude product was purified by a flash silica gel column (ISCO^{®}; 120 g SepaFlash^{®} Silica Flash Column, mobile phase gradient: 0-45% ethyl acetate/petroleum ether, flow rate: 80 mL/min) and further purified by high performance liquid chromatography (column: Boston Prime C18 150 × 30 mm × 5 µm; mobile phase: [A: water (formic acid), B: acetonitrile]; B%: 42%-82%, 13 min) to give the title compound (1.4 g).

MS m/z (ESI): 537.2 [M+Na]⁺.

### Step 15: synthesis of (S)-10-cyclopropyl-1-(9H-fluoren-9-yl)-3,6-dioxo-2,9-dioxa-4,7-diazaundecane-11-carboxylic acid (intermediate L1-19)

**Intermediate L1-18** (1.4 g) was dissolved in a mixed solvent of methanol (15 mL) and water (15 mL), and wet palladium on carbon (10% mass content, 0.15 g) was added under a nitrogen atmosphere. The reaction mixture was purged 3 times with hydrogen, and then stirred at 25 °C for reaction for 16 h under a hydrogen atmosphere (15 Psi). After the reaction was completed, the reaction mixture was filtered, and the filtrate was concentrated to dryness under reduced pressure. The crude product was purified by high performance liquid chromatography (column: Boston Prime C18 150 × 30 mm × 5 µm; mobile phase: [A: water (formic acid), B: acetonitrile]; B%: 24%-64%, 13 min) to give the title compound.

LCMS m/z (ESI): 447.5 [M+Na]⁺.

### Step 16: synthesis of resin-supported (S)-10-cyclopropyl-1-(9H-fluoren-9-yl)-3,6-dioxo-2,9-dioxa-4,7-diazaundecane-11-carboxylic acid (intermediate L1-20)

CTC resin (specification: about 1.19 mmol/g) (257 mg) was added to dichloromethane (3 mL), and **intermediate L1-19** (130 mg) and diisopropylethylamine (59.37 mg) were then added. The reaction mixture was purged 3 times with nitrogen and then shaken on a shaker at 25 °C for reaction for 16 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (330 mg).

### Step 17: synthesis of resin-supported (S)-2-((2-aminoacetamido)methoxy)-2-cyclopropylacetic acid (intermediate L1-21)

**Intermediate L1-20** (330 mg) was dissolved in *N*,*N*-dimethylformamide (5 mL), and piperidine (1.08 g) was added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (220 mg).

### Step 18: synthesis of resin-supported (5S)-5-benzyl-13-cyclopropyl-1-(9H-fluoren-9-yl)-3,6,9-trioxy-2,12-dioxa-4,7,10-triazatetradecan-14-oic acid (intermediate L1-22)

**Intermediate L1-21** (220 mg) and (((9*H*-fluoren-9-yl)methoxy)carbonyl)-*L*-phenylalanine (230.17 mg) were dissolved in *N*,*N*-dimethylformamide (5 mL), and *O*-benzotriazole-tetramethyluronium hexafluorophosphate (225.31 mg) and diisopropylethylamine (99.96 mg) were added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (377 mg).

### Step 19: synthesis of resin-supported (S)-2-((2-((S)-2-amino-3-phenylpropanamido))acetamido)methoxy)-2-cyclopropylacetic acid (intermediate L1-23)

**Intermediate L1-22 (**377 mg) was dissolved in *N*,*N*-dimethylformamide (5 mL), and piperidine (37.22 mg, 437.15 µmol, 43.17 µL) was added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (270 mg).

### Step 20: synthesis of resin-supported (11S,19S)-11-benzyl-19-cyclopropyl-1-(9H-fluoren-9-yl)-3,6,9,12,15-pentaoxo-2,18-dioxa-4,7,10,13,16-pentaazaeicosane-20-carboxylic acid (intermediate L1-24)

**Intermediate L1-23** (270 mg) was dissolved in *N,N-*dimethylformamide (5 mL), and **intermediate L1-16** (209.58 mg), O-benzotriazole-tetramethyluronium hexafluorophosphate (224.29 mg), and diisopropylethylamine (99.51 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (403 mg).

### Step 21: synthesis of resin-supported (10S)-16-amino-10-benzyl-2-cyclopropyl-6,9,12,15-tetraoxy-3-oxy-5,8,11,14-tetraazahexadecane-1-carboxylic acid (intermediate L1-25)

**Intermediate L1-24** (403 mg) was dissolved in *N,N-*dimethylformamide (5 mL), and piperidine (1.08 g) was added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (300 mg).

### Step 22: synthesis of 6-(2-methylsulfonyl-5-oxo-5H,6H,7H,8H-pyrido[4,3-d]pyrimidin-6-yl)hexanoic acid (intermediate L1-26)

**Intermediate 18-2** (2.4 g) was dissolved in dichloromethane (50 mL), and trifluoroacetic acid (10 mL) was added. The reaction mixture was stirred at 25 °C for reaction for 1 h. The reaction mixture was then concentrated under reduced pressure to remove the solvent, and water (20 mL) was added. The mixture was stirred at room temperature for 5 min and then filtered to give the title compound (1.5 g).

(MS m/z (ESI): 342.1 [M+H]⁺.

### Step 23: synthesis of resin-supported (2S,10S)-10-benzyl-2-cyclopropyl-23-(2-methylsulfonyl-5-oxo-7,8-dihydropyrido[4,3-d]pyrimidin-6(5H)-yl)-6,9,12,15,18-pentaoxo-3-oxa-5,8,11,14,17-pentaazatricosanoic acid (intermediate L1-27)

Intermediate **L1-25** (200 mg) was added to DMF (3 mL), and **intermediate L1-26** (127 mg), O-benzotriazole-tetramethyluronium hexafluorophosphate (141 mg), and diisopropylethylamine (69 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (220 mg).

### Step 24: synthesis of (2S,10S)-10-benzyl-2-cyclopropyl-23-(2-methylsulfonyl-5-oxo-7,8-dihydropyrido[4,3-d]pyrimidin-6(5H)-yl)-6,9,12,15,18-pentaoxo-3-oxa-5,8,11,14,17-pentaazatricosanoic acid (intermediate L1-28)

**Intermediate L1-27** (220 mg) was added to a mixture of dichloromethane (4 mL) and 1,1,1,3,3,3-hexafluoroisopropanol (1 mL), and the resulting mixture was shaken at 25 °C for 1 h. After the reaction was completed, the reaction mixture was filtered to remove the resin, and the filtrate was concentrated to dryness under reduced pressure to give the title compound (17 mg).

### Step 25: synthesis of N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-6-(2-methylsulfonyl-5-oxo-7,8-dihydropyrido[4,3-d]pyrimidin-6(5H)-yl)hexanamide (a-L1-1)

**Intermediate L1-28** (17 mg), **intermediate L1-7** (10.06 mg), 1-(3-dimethylaminopropyl)-3-ethylcarbodiimide hydrochloride (8.28 mg), pyridine (5.13 mg), and 1-hydroxybenzotriazole (5.84 mg) were dissolved in DMF (0.7 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: Welch Xtimate C18 150 × 25 mm × 5 µm; mobile phase: [A: water (0.225% formic acid), B: acetonitrile]; B%: 25%-55%, 11 min) to give the title compound (2.1 mg).

**¹H NMR (400 MHz, DMSO-*d₆*)** δ = 9.22 (s, 1H), 8.67 (t, *J* = 6.0 Hz, 1H), 8.60 (t, *J =* 6.5 Hz, 1H), 8.30 (t, *J =* 5.7 Hz, 1H), 8.17-8.05 (m, 2H), 8.05-7.97 (m, 1H), 7.80 (s, 1H), 7.52 (s, 1H), 7.29-7.20 (m, 5H), 7.18-7.16 (m, 1H), 6.54-6.46 (m, 1H), 5.44 (d, *J =* 8.7 Hz, 4H), 4.85-4.71 (m, 2H), 4.68-4.66 (m, 1H), 4.55-4.45 (m, 1H), 4.42-4.39 (m, 1H), 3.77-3.63 (m, 7H), 3.62-3.53 (m, 1H), 3.51-3.45 (m, 3H), 3.43 (s, 3H), 3.25 (t, *J =* 6.7 Hz, 2H), 3.05-3.03 (m, 1H), 2.80-2.78 (m, 1H), 2.13 (t, *J =* 7.5 Hz, 2H), 1.94-1.79 (m, 2H), 1.61-1.46 (m, 4H), 1.33-1.21 (m, 2H), 1.01-0.92 (m, 1H), 0.87 (t, *J =* 7.3 Hz, 3H), 0.43-0.26 (m, 4H)

MS m/z (ESI): 1192.6 [M+H]⁺.

### Example 23. N-((4S,12S,21S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-21-(3-(2-(methylsulfonyl)-5-oxo-7,8-dihydropyrido[4,3-d]pyrimidin-6(5H)-yl)propanamido)-3,8,11,14,17,20-hexaoxo-5-oxa-2,7,10,13,16,19-hexaazapentacosan-25-yl)-2,5,8,11,14,17,20,23-octaoxahexacosan-26-amide (a-L2-1)

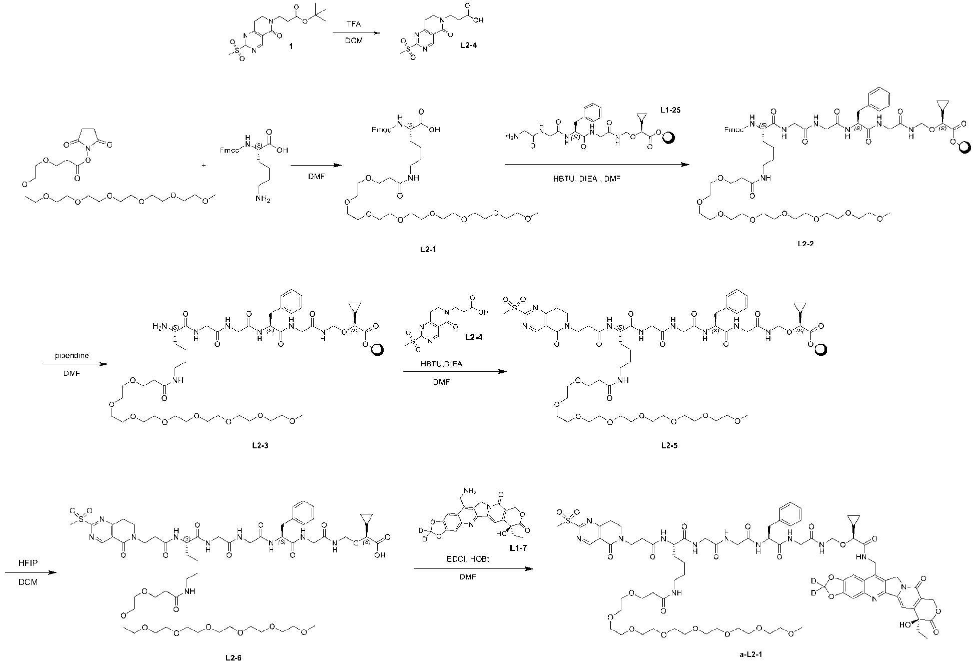

### Step 1: synthesis of (S)-32-((((9H-fluoren-9-yl)methoxy)carbonyl)amino)-26-oxo-2,5,8,11,14,17,20,23-octaoxa-27-azatritriacontan-33-oic acid (intermediate L2-1)

2,5-Dioxopyrrolidin-1-yl 2,5,8,11,14,17,20,23-octaoxahexacosan-26-oate (4.5 g) was dissolved in DMF (70 mL), and (((9*H*-fluoren-9-yl)methoxy)carbonyl)-*L*-lysine (3.25 g) was added. The reaction mixture was stirred at 25 °C for 16 h. After the reaction was completed, water (300 mL) was added to the reaction mixture. The mixture was extracted with ethyl acetate (50 mL × 3 times). The organic phases were combined and dried over anhydrous sodium sulfate. After filtration, the organic phase was concentrated under reduced pressure to remove the solvent, and the residue was purified by a flash silica gel column (ISCO^{®}; 40 g of SepaFlash^{®} Silica Flash chromatographic column; gradient 0-4% methanol/dichloromethane @ 60 mL/min) to give the title compound (4.1 g).

MS m/z (ESI): 763.3 [M+H]⁺.

### Step 2: synthesis of resin-supported (32S,41S,49S)-32-((((9H-fluoren-9-yl)methoxy)carbonyl)amino)-41-benzyl-49-cyclopropyl-26,33,36,39,42,45-hexaoxo-2,5,8,11,14,17,20,23,48-nonaoxa-27,34,37,40,43,46-hexaazapentacontan-50-oic acid (intermediate L2-2)

**Intermediate L1-25** (1.5 g) was dissolved in DMF (15 mL), and **intermediate L2-1** (2.02 g), *O*-benzotriazole-tetramethyluronium hexafluorophosphate (1.01 g), and diisopropylethylamine (771.05 mg) were sequentially added. The reaction mixture was shaken on a shaker at 25 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (1.67 g).

MS m/z (ESI): 1230.5 [M+Na]⁺

### Step 3: synthesis of resin-supported (32S,41S,49S)-32-amino-41-benzyl-49-cyclopropyl-26,33,36,39,42,45-hexaoxo-2,5,8,11,14,17,20,23,48-nonaoxa-27,34,37,40,43,46-hexaazapentacontan-50-oic acid (intermediate L2-3)

**Intermediate L2-2** (1.67 g) was dissolved in DMF (10 mL), and piperidine (2.5 mL) was added. The reaction mixture was shaken on a shaker at 25 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (1.5 g).

MS m/z (ESI): 986.5 [M+H]⁺.

### Step 4: synthesis of 3-(2-(methylsulfonyl)-5-oxo-7,8-dihydropyrido[4,3-d]pyrimidin-6(5H)-yl)propionic acid (intermediate L2-4)

**Compound 1** (400 mg) was dissolved in 20 mL of anhydrous dichloromethane under a nitrogen atmosphere, and the mixture was cooled to -78 °C. 5 mL of trifluoroacetic acid was added at this temperature, and the mixture was slowly and naturally warmed to room temperature. The solvent was then removed under a nitrogen stream. 5 mL of anhydrous acetonitrile was added to the residue, and the mixture was filtered. The filter cake was washed with a mixture of petroleum ether and ethyl acetate (1:1) to give the title compound (220 mg).

### Step 5: synthesis of resin-supported (32S,41S,49S)-41-benzyl-49-cyclopropyl-32-(3-(2-(methylsulfonyl)-5-oxo-7,8-dihydropyrido[4,3-d]pyrimidin-6(5H)-yl)propanamido)-26,33,36,39,42,45-hexaoxo-2,5,8,11,14,17,20,23,48-nonaoxa-27,34,37,40,43,46-hexaazapentacontan-50-oic acid (intermediate L2-5)

**Intermediate L2-3** (300 mg) was dissolved in DMF (5 mL), and **intermediate L2-4** (93.52 mg), *O*-benzotriazole-tetramethyluronium hexafluorophosphate (119.29 mg), and diisopropylethylamine (91.09 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (325 mg).

### Step 6: synthesis of (32S,41S,49S)-41-benzyl-49-cyclopropyl-32-(3-(2-(methylsulfonyl)-5-oxo-7,8-dihydropyrido[4,3-d]pyrimidin-6(5H)-yl)propanamido)-26,33,36,39,42,45-hexaoxo-2,5,8,11,14,17,20,23,48-nonaoxa-27,34,37,40,43,46-hexaazapentacontan-50-oic acid (intermediate L2-6)

**Intermediate L2-5** (325 mg) was added to a mixed solvent of dichloromethane (10 mL) and 1,1,1,3,3,3-hexafluoroisopropanol (2.5 mL), and the resulting mixture was shaken at 25 °C for 0.5 h. After the reaction was completed, the reaction mixture was filtered to remove the resin, and the filtrate was concentrated to dryness under reduced pressure to give the title compound (75 mg).

MS m/z (ESI): 562.0 [(M+H⁺)/2]⁺.

### Step 7: synthesis of N-((4S,12S,21S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-21-(3-(2-(methylsulfonyl)-5-oxo-7,8-dihydropyrido[4,3-d]pyrimidin-6(5H)-yl)propanamido)-3,8,11,14,17,20-hexaoxo-5-oxa-2,7,10,13,16,19-hexaazapentacosan-25-yl)-2,5,8,11,14,17,20,23-octaoxahexacosan-26-amide (a-L2-1)

**Intermediate L2-6** (70 mg), **intermediate L1-7** (28.06 mg), 1-(3-dimethylaminopropyl)-3-ethylcarbodiimide hydrochloride (21.18 mg), 1-hydroxybenzotriazole (14.93 mg), and pyridine (13.11 mg) were dissolved in DMF (0.5 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: C18 100 × 10 mm; mobile phase: [A: water (trifluoroacetic acid), B: acetonitrile]; B%: 16%-46%, 8 min) to give the title compound (4.4 mg).

**¹H NMR** (400 MHz, DMSO-*d₆*) δ = 9.22 (s, 1H), 8.67 (t, *J =* 5.9 Hz, 1H), 8.60 (t, *J =* 6.3 Hz, 1H), 8.32 (s, 1H), 8.26-8.16 (m, 2H), 8.11 (d, *J* = 7.9 Hz, 1H), 7.97 (t, *J =* 5.4 Hz, 1H), 7.83-7.74 (m, 2H), 7.54-7.49 (m, 1H), 7.28-7.20 (m, 5H), 7.19-7.14 (m, 1H), 7.12 (s, 1H), 6.50 (s, 1H), 5.43 (d, *J =* 5.3 Hz, 4H), 4.84-4.62 (m, 3H), 4.55-4.47 (m, 1H), 4.42-4.39 (m, 1H), 4.26-4.13 (m, 1H), 3.78-3.61 (m, 10H), 3.57 (t, *J =* 6.6 Hz, 3H), 3.53-3.45 (m, 23H), 3.45-3.40 (m, 6H), 3.24 (s, 4H), 3.14-3.01 (m, 3H), 2.96 (d, *J =* 5.8 Hz, 2H), 2.84-2.74 (m, 1H), 2.28 (t, *J =* 6.4 Hz, 2H), 1.95-1.80 (m, 2H), 1.62 (s, 1H), 1.48 (d, *J* = 9.4 Hz, 1H), 1.33 (s, 2H), 1.24 (s, 1H), 1.18 (t, *J =* 7.3 Hz, 3H), 1.02-0.93 (m, 1H), 0.88 (t, *J* = 7.3 Hz, 3H), 0.41-0.29 (m, 4H)

MS m/z (ESI): 837.4 [(M+2H)/2]⁺.

### Example 24. N-((4S,12S,21S)-12-Benzyl-21-(3-(2-(cyclopentylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (b-L2-1)

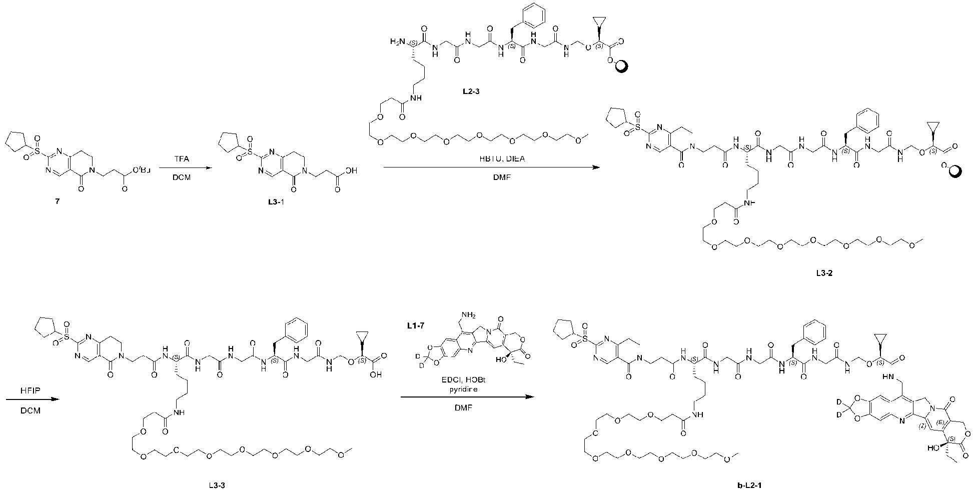

### Step 1: synthesis of 3-(2-cyclopentylsulfonyl-5-oxo-7,8-dihydropyrido[4,3-d]pyrimidin-6(5H)-yl)propionic acid (intermediate L3-1)

Trifluoroacetic acid (2 mL) was added to a mixture of **compound 7** (135 mg) and DCM (10 mL) at 0 °C, and the resulting mixture was warmed to room temperature and reacted for 2 h. The reaction mixture was then concentrated under reduced pressure to remove the solvent. The mixture was separated by column chromatography (EA/PE, 0-100%) to give the title compound (80 mg).

LC-MS: m/z (ESI): 354.4 [M+H]⁺.

### Step 2: synthesis of resin-supported (32S,41S,49S)-41-benzyl-32-(3-(2-(cyclopentylsulfonyl)-5-oxo-7,8-dihydropyrido[4,3-d]pyrimidin-6(5H)-yl)propanamido)-49-cyclopropyl-26,33,36,39,42,45-hexaoxo-2,5,8,11,14,17,20,23,48-nonaoxa-27,34,37,40,43,46-hexaazapentacontan-50-oic acid (intermediate L3-2)

**Intermediate L2-3** (200 mg) was dissolved in DMF (5 mL), and **intermediate L3-1** (73.62 mg), *O*-benzotriazole-tetramethyluronium hexafluorophosphate (79.53 mg), and diisopropylethylamine (60.73 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (285 mg).

MS m/z (ESI): 1344.7 [M+Na]⁺.

### Step 3: synthesis of (32S,41S,49S)-41-benzyl-32-(3-(2-(cyclopentylsulfonyl)-5-oxo-7,8-dihydropyrido[4,3-d]pyrimidin-6(5H)-yl)propanamido)-49-cyclopropyl-26,33,36,39,42,45-hexaoxo-2,5,8,11,14,17,20,23,48-nonaoxa-27,34,37,40,43,46-hexaazapentacontan-50-oic acid (intermediate L3-3)

**Intermediate L3-2** (200 mg) was added to a mixed solvent of dichloromethane (10 mL) and 1,1,1,3,3,3-hexafluoroisopropanol (2.5 mL), and the resulting mixture was shaken at 25 °C for 0.5 h. After the reaction was completed, the reaction mixture was filtered to remove the resin, and the filtrate was concentrated to dryness under reduced pressure to give the title compound (55 mg).

MS m/z (ESI): 1344.7 [M+Na]⁺.

### Step 4: synthesis of N-((4S,12S,21S)-12-benzyl-21-(3-(2-(cyclopentylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (b-L2-1)

**Intermediate L3-3** (55 mg), **intermediate L1-7** (21.15 mg), 1-(3-dimethylaminopropyl)-3-ethylcarbodiimide hydrochloride (15.96 mg), 1-hydroxybenzotriazole (11.25 mg), and pyridine (9.88 mg) were dissolved in DMF (0.5 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: C18 150 × 30 mm × 5 µm; mobile phase: [A: water (trifluoroacetic acid), B: acetonitrile]; B%: 27%-67%, 11 min) to give the title compound (6.45 mg).

**¹H NMR (400 MHz, DMSO-*d₆*)** δ = 9.23 (s, 1H), 8.70-8.64 (m, 1H), 8.63-8.55 (m, 1H), 8.36-8.28 (m, 1H), 8.26-8.15 (m, 2H), 8.14-8.08 (m, 1H), 8.00-7.94 (m, 1H), 7.82-7.74 (m, 2H), 7.54-7.48 (m, 1H), 7.27-7.21 (m, 6H), 7.19-7.13 (m, 1H), 6.51-6.47 (m, 1H), 5.48-5.39 (m, 4H), 4.84-4.72 (m, 2H), 4.71-4.64 (m, 1H), 4.55-4.49 (m, 1H), 4.41-4.39 (m, 1H), 4.27-4.16 (m, 2H), 3.76-3.63 (m, 11H), 3.57 (t, *J* = 6.5 Hz, 2H), 3.51-3.47 (m, 26H), 3.43 (d, *J* = 5.3 Hz, 3H), 3.24 (s, 3H), 3.22-3.17 (m, 1H), 3.07-2.96 (m, 3H), 2.84-2.76 (m, 1H), 2.31-2.26 (m, 2H), 2.01-1.84 (m, 6H), 1.71-1.59 (m, 5H), 1.53-1.44 (m, 1H), 1.38-1.21 (m, 4H), 1.01-0.94 (m, 1H), 0.88 (t, *J =* 7.3 Hz, 3H), 0.39-0.32 (m, 4H)

MS m/z (ESI): 864.0 [(M+2H)/2]⁺.

### Example 25. N-((4S,12S,21S)-12-Benzyl-21-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (c-L2-1)

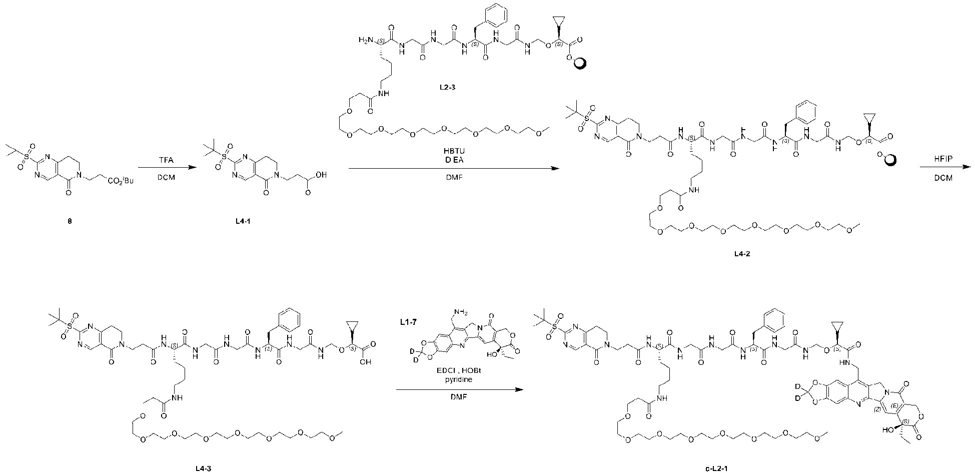

### Step 1: synthesis of 3-(2-(tert-butylsulfonyl)-5-oxo-7,8-dihydropyrido[4,3-d]pyrimidin-6(5H)-yl)propionic acid (intermediate L4-1)

**Compound 8** (200 mg) was dissolved in anhydrous dichloromethane (10 mL) under a nitrogen atmosphere, and the mixture was cooled to -78 °C. Trifluoroacetic acid (2.5 mL) was added at this temperature, and the mixture was slowly warmed to room temperature and stirred for 2 h. After the reaction was completed, the solvent was removed under a nitrogen stream. The residue was washed with a mixture of petroleum ether and ethyl acetate (5:1) and filtered to give the title compound (150 mg).

MS m/z (ESI): 342.1 [M+H]⁺.

### Step 2: synthesis of resin-supported (32S,41S,49S)-41-benzyl-32-(3-(2-(tert-butylsulfonyl)-5-oxo-7,8-dihydropyrido[4,3-d]pyrimidin-6(5H)-yl)propanamido)-49-cyclopropyl-26,33,36,39,42,45-hexaoxo-2,5,8,11,14,17,20,23,48-nonaoxa-27,34,37,40,43,46-hexaazapentacontan-50-oic acid (intermediate L4-2)

**Intermediate L2-3** (300 mg) was dissolved in DMF (5 mL), and **intermediate L4-1** (106.67 mg), *O*-benzotriazole-tetramethyluronium hexafluorophosphate (178.20 mg), and diisopropylethylamine (91.09 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (300 mg).

MS m/z (ESI): 583.0 [(M+H)/2]⁺.

### Step 3: synthesis of (32S,41S,49S)-41-benzyl-32-(3-(2-(tert-butylsulfonyl)-5-oxo-7,8-dihydropyrido[4,3-d]pyrimidin-6(5H)-yl)propanamido)-49-cyclopropyl-26,33,36,39,42,45-hexaoxo-2,5,8,11,14,17,20,23,48-nonaoxa-27,34,37,40,43,46-hexaazapentacontan-50-oic acid (intermediate L4-3)

**Intermediate L4-2** (300 mg) was added to a mixed solvent of dichloromethane (10 mL) and 1,1,1,3,3,3-hexafluoroisopropanol (2.5 mL), and the resulting mixture was shaken at 25 °C for 0.5 h. After the reaction was completed, the reaction mixture was filtered to remove the resin. The filtrate was concentrated to dryness under reduced pressure, and the crude product was lyophilized with a lyophilizer to give the title compound (80 mg).

MS m/z (ESI): 583.0 [(M+H)/2]⁺.

### Step 4: synthesis of N-((4S,12S,21S)-12-benzyl-21-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (c-L2-1)

**Intermediate L4-3** (70 mg), **intermediate L1-7** (24.57 mg), 1-(3-dimethylaminopropyl)-3-ethylcarbodiimide hydrochloride (20.5 mg), 1-hydroxybenzotriazole (14.44 mg), and pyridine (12.68 mg) were dissolved in DMF (0.5 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: C18 100 × 10 mm; mobile phase: [A: water (trifluoroacetic acid), B: acetonitrile]; B%: 18%-48%, 8 min) to give the title compound (6.3 mg).

**¹H NMR** (400 MHz, DMSO-*d*₆) δ = 9.25 (s, 1H), 8.69-8.64 (m, 1H), 8.60 (t, *J =* 6.4 Hz, 1H), 8.32 (t, *J =* 5.6 Hz, 1H), 8.25-8.16 (m, 1H), 8.11 (d, *J =* 7.8 Hz, 1H), 7.96 (t, *J =* 5.3 Hz, 1H), 7.82-7.75 (m, 1H), 7.52 (s, 1H), 7.29-7.19 (m, 5H), 7.19-7.12 (m, 1H), 6.50 (s, 1H), 5.44 (d, *J =* 8.8 Hz, 4H), 4.86-4.71 (m, 2H), 4.71-4.63 (m, 1H), 4.55-4.47 (m, 1H), 4.41-4.39 (m, 1H), 4.22-4.15 (m, 1H), 3.81-3.60 (m, 9H), 3.57 (t, *J =* 6.6 Hz, 3H), 3.53-3.44 (m, 24H), 3.43-3.40 (m, 12H), 3.24-3.20 (m, 4H), 3.08-3.08 (m, 1H), 3.08-2.94 (m, 2H), 2.79-2.77 (m, 1H), 2.28 (t, *J =* 6.5 Hz, 2H), 1.93-1.79 (m, 2H), 1.62 (s, 1H), 1.48 (d, *J =* 9.3 Hz, 1H), 1.37 (s, 9H), 1.24 (s, 2H), 1.02-0.93 (m, 1H), 0.88 (t, *J* = 7.1 Hz, 3H), 0.40-0.28 (m, 4H)

MS m/z (ESI): 858.4 [(M+2H)/2]⁺.

### Example 26. N-((4S,12S,25S)-12-Benzyl-25-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20,24-heptaoxo-5-oxa-2,7,10,13,16,19,23-heptaazanonacosan-29-yl)-2,5,8,11,14,17,20,23-octaoxahexacosan-26-amide (c-L5-1)

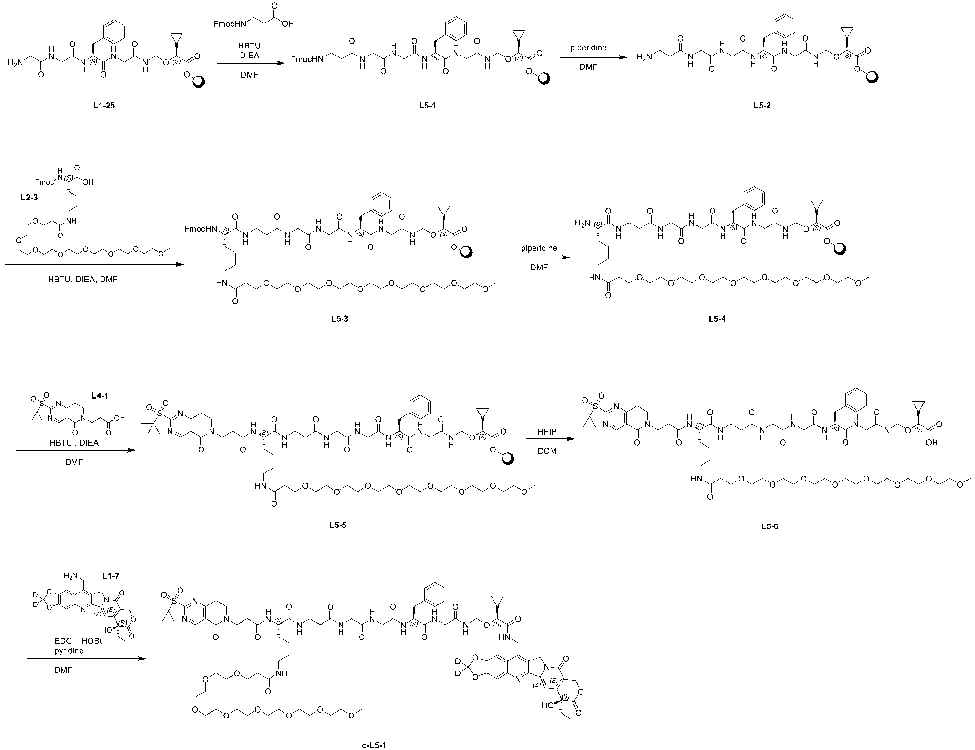

### Step 1: synthesis of resin-supported (15S,23S)-15-benzyl-23-cyclopropyl-1-(9H-fluoren-9-yl)-3,7,10,13,16,19-hexaoxo-2,22-dioxa-4,8,11,14,17,20-hexaazatetracosan-24-oic acid (intermediate L5-1)

3-(9*H*-Fluoren-9-yl)methoxy)carbonyl)amino)propionic acid (274.48 mg) was dissolved in DMF (5 mL), and **L1-25** (the total weight of the substrate and the resin: 500 mg, the content: about 0.22 mmol), O-benzotriazole-tetramethyluronium hexafluorophosphate (336.58 mg), and diisopropylethylamine (257.02 mg) were sequentially added. The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (528.4 mg).

MS m/z (ESI): 779.5 [M+Na]⁺.

### Step 2: synthesis of resin-supported (2S,10S)-20-amino-10-benzyl-2-cyclopropyl-6,9,12,15,18-pentaoxo-3-oxa-5,8,11,14,17-pentaazaeicosane-1-carboxylic acid (intermediate L5-2)

**Intermediate L5-1** (528.4 mg) was dissolved in DMF (5 mL) and piperidine (42.97 mg). The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (480.6 mg). MS m/z (ESI): 535.1 [M+H]⁺.

### Step 3: synthesis of resin-supported (32S,45S,53S)-32-((((9H-fluoren-9-yl)methoxy)carbonyl)amino)-45-benzyl-53-cyclopropyl-26,33,37,40,43,46,49-heptaoxo-2,5,8,11,14,17,20,23,52-nonaoxa-27,34,38,41,44,47,50-heptaazatetrapentacontane-54-carboxylic acid (intermediate L5-3)

**Intermediate L2-3** (590.82 mg) was dissolved in DMF (5 mL), and **compound L5-2** (480.6 mg), *O*-benzotriazole-tetramethyluronium hexafluorophosphate (295.66 mg), and diisopropylethylamine (225.77 mg) were sequentially added. The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (502 mg).

### Step 4: synthesis of resin-supported (32S,45S,53S)-32-amino-45-benzyl-53-cyclopropyl-26,33,37,40,43,46,49-heptaoxo-2,5,8,11,14,17,20,23,52-nonaoxa-27,34,38,41,44,47,50-heptaazatetrapentacontane-54-carboxylic acid (intermediate L5-4)

**Intermediate L5-3** (502 mg) was dissolved in DMF (5 mL) and piperidine (26.98 mg). The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (468.5 mg).

### Step 5: synthesis of resin-supported methyl (32S,45S,53S)-45-benzyl-32-(3-(2-(tert-butylsulfonyl)-5-oxo-7,8-dihydropyrido[4,3-d]pyrimidin-6(5H)-yl)propanamido)-53-cyclopropyl-26,33,37,40,43,46,49-heptaoxo-2,5,8,11,14,17,20,23,52-nonaoxa-27,34,38,41,44,47,50-heptaazatetrapentacontane-54-carboxylic acid (intermediate L5-5)

**Intermediate L5-4** (220.0 mg) was dissolved in DMF (5 mL), and **intermediate L4-1** (74.10 mg), *O*-benzotriazole-tetramethyluronium hexafluorophosphate (82.87 mg), and diisopropylethylamine (63.28 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (220 mg).

MS m/z (ESI): 713.0 [M+Na]⁺.

### Step 6: synthesis of (32S,45S,53S)-45-benzyl-32-(3-(2-(tert-butylsulfonyl)-5-oxo-7,8-dihydropyrido[4,3-d]pyrimidin-6(5H)-yl)propanamido)-53-cyclopropyl-26,33,37,40,43,46,49-heptaoxo-2,5,8,11,14,17,20,23,52-nonaoxa-27,34,38,41,44,47,50-heptaazatetrapentacontane-54-carboxylic acid (intermediate L5-6)

**Intermediate L5-5** (220.00 mg) was added to a mixed solvent of dichloromethane (16 mL) and 1,1,1,3,3,3-hexafluoroisopropanol (4 mL), and the resulting mixture was shaken at 25 °C for 15 min. After the reaction was completed, the reaction mixture was filtered to remove the resin, and the filtrate was concentrated to dryness under reduced pressure to give the title compound (30 mg).

MS m/z (ESI): 691.0 [(M+2H)/2]⁺.

### Step 7: synthesis of N-((4S,12S,25S)-12-benzyl-25-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20,24-heptaoxo-5-oxa-2,7,10,13,16,19,23-heptaazanonacosan-29-yl)-2,5,8,11,14,17,20,23-octaoxahexacosan-26-amide (c-L5-1)

**Intermediate L5-5** (30 mg), **intermediate L1-7** (17.49 mg), 1-(3-dimethylaminopropyl)-3-ethylcarbodiimide hydrochloride (8.33 mg), 1-hydroxybenzotriazole (5.87 mg), and pyridine (5.16 mg) were dissolved in DMF (0.5 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: C18 150 × 30 mm × 5 µm; mobile phase: [A: water (0.225% formic acid), B: acetonitrile]; B%: 25%-55%, 11 min) to give the title compound (7.1 mg).

MS m/z (ESI): 893.7 [(M+2H)/2]⁺.

**¹H NMR (400 MHz,** DMSO-*d₆*) δ = 9.25 (s, 1H), 8.67 (s, 1H), 8.63-8.57 (m, 1H), 8.30 (s, 1H), 8.22-8.09 (m, 2H), 8.03 (s, 1H), 7.96 (s, 1H), 7.83-7.75 (m, 2H), 7.52 (s, 1H), 7.28-7.19 (m, 5H), 7.17 (d, *J =* 6.6 Hz, 1H), 6.50 (s, 1H), 5.44 (d, *J* = 9.0 Hz, 4H), 4.85-4.71 (m, 2H), 4.68-4.66 (m, 1H), 4.51 (s, 1H), 4.41-4.39 (m, 1H), 4.18-4.09 (m, 1H), 4.06 (s, 1H), 3.77-3.65 (m, 12H), 3.61-3.55 (m, 9H), 3.51-3.49 (m, 22H), 3.24 (s, 5H), 3.04 (d, *J =* 9.8 Hz, 1H), 2.97 (d, *J* = 6.1 Hz, 2H), 2.79-2.77 (m, 1H), 2.32-2.24 (m, 3H), 1.93-1.80 (m, 2H), 1.56 (s, 2H), 1.45 (d, *J* = 8.5 Hz, 2H), 1.37 (s, 9H), 1.34-1.28 (m, 2H), 1.22 (d, *J =* 15.6 Hz, 3H), 0.96 (d, *J =* 6.8 Hz, 1H), 0.87 (t, *J =* 7.3 Hz, 3H), 0.40-0.30 (m, 4H)

### Example 27. N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(tert-butylsulfonyl)-5-oxo-7,8-dihydropyrido[4,3-d]pyrimidin-6(5H)-yl)propanamido)-3,6,9,12,15,18,21,24-octaoxaheptacosan-27-amide (c-L6-1)

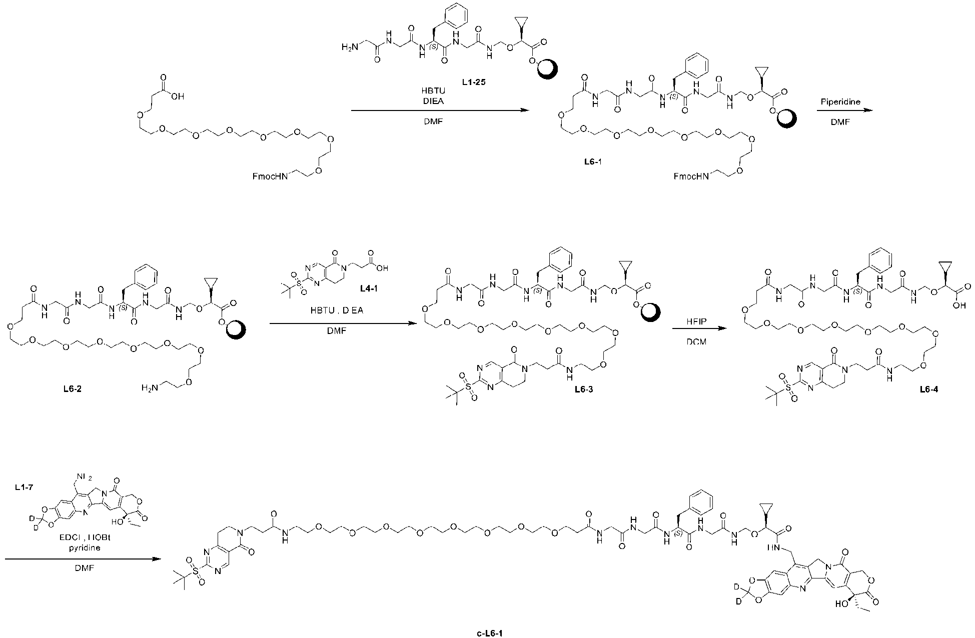

### Step 1: synthesis of resin-supported (39S,47S)-39-benzyl-47-cyclopropyl-1-(9H-fluoren-9-yl)-3,31,34,37,40,43-hexaoxo-2,7,10,13,16,19,22,25,28,46-decaoxa-4,32,35,38,41,44-hexaazaoctatetracontan-48-oic acid (intermediate L6-1)

1-(9*H*-Fluoren-9-yl)-3-oxo-2,7,10,13,16,19,22,25,28-nonaoxa-4-azahentriacontane-31-carboxylic acid (468.16 mg) was dissolved in DMF (5 mL), and **intermediate L1-25** (400.0 mg), O-benzotriazole-tetramethyluronium hexafluorophosphate (269.26 mg), and diisopropylethylamine (205.61 mg) were sequentially added. The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (450 mg).

MS m/z (ESI): 1109.4 [M+H]⁺.

### Step 2: synthesis of resin-supported (2S,10S)-44-amino-10-benzyl-2-cyclopropyl-6,9,12,15,18-pentaoxo-3,21,24,27,30,33,36,39,42-nonaoxa-5,8,11,14,17-pentaazatetratetracontanoic acid (compound L6-2)

**Intermediate L6-1** (440 mg) was dissolved in DMF (10 mL) and piperidine (2 mL). The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (365 mg). MS m/z (ESI): 887.5 [M+H]⁺.

### Step 3: synthesis of resin-supported (2S,10S)-10-benzyl-48-(2-(tert-butylsulfonyl)-5-oxo-7,8-dihydropyrido [4,3-d]pyrimidin-6(5H)-yl)-2-cyclopropyl-6,9,12,15,18,46-hexaoxo-3,21,24,27,30,33,36,39,42-nonaoxa-5,8,11,14,17,45-hexaazaoctatetracontanoic acid (intermediate L6-3)

**Intermediate L6-2** (365.0 mg) was dissolved in DMF (5 mL), and **intermediate L4-1** (140.71 mg), O-benzotriazole-tetramethyluronium hexafluorophosphate (157.35 mg), and diisopropylethylamine (120.16 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (285 mg).

MS m/z (ESI): 1210.4 [M+H]⁺.

### Step 4: synthesis of (2S,10S)-10-benzyl-48-(2-(tert-butylsulfonyl)-5-oxo-7,8-dihydropyrido[4,3-d]pyrimidin-6(5H)-yl)-2-cyclopropyl-6,9,12,15,18,46-hexaoxo-3,21,24,27,30,33,36,39,42-nonaoxa-5,8,11,14,17,45-hexaazaoctatetracontanoic acid (intermediate L6-4)

**Intermediate L6-3** (380.00 mg) was added to a mixed solvent of dichloromethane (8 mL) and 1,1,1,3,3,3-hexafluoroisopropanol (2 mL), and the resulting mixture was shaken at 25 °C for 15 min. After the reaction was completed, the reaction mixture was filtered to remove the resin, and the filtrate was concentrated to dryness under reduced pressure to give the title compound (110 mg).

MS m/z (ESI): 606.0 [(M+2H)/2]⁺.

### Step 5: synthesis of N-((45,125)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(tert-butylsulfonyl)-5-oxo-7,8-dihydropyrido[4,3-d]pyrimidin-6(5H)-yl)propanamido)-3,6,9,12,15,18,21,24-octaoxaheptacosan-27-amide (c-L6-1)

**Intermediate L6-4** (50 mg), intermediate **L1-7** (17.49 mg), 1-(3-dimethylaminopropyl)-3-ethylcarbodiimide hydrochloride (15.84 mg), 1-hydroxybenzotriazole (11.16 mg), and pyridine (9.80 mg) were dissolved in DMF (0.5 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: C18 150 × 30 mm × 5 µm; mobile phase: [A phase: water (0.225% formic acid), B phase: acetonitrile]; B%: 25%-55%, 9 min) to give the title compound (14.1 mg).

MS m/z (ESI): 808.6 [(M+2H)/2]⁺.

**¹H NMR (400 MHz,** DMSO-*d₆*) δ = 9.26 (s, 1H), 8.69-8.56 (m, 2H), 8.32-8.25 (m, 1H), 8.19-8.14 (m, 1H), 8.13-8.04 (m, 2H), 8.02-7.97 (m, 1H), 7.80 (s, 1H), 7.52 (s, 1H), 7.28-7.21 (m, 5H), 7.19-7.17 (m, 1H), 6.50 (s, 1H), 5.44 (d, *J* = 8.3 Hz, 4H), 4.79 (d, *J =* 7.8 Hz, 2H), 4.68-4.66 (m, 1H), 4.54-4.46 (m, 1H), 4.41-4.39 (m, 1H), 3.77-3.66 (m, 10H), 3.62-3.57 (m, 3H), 3.51-3.46 (m, 30H), 3.22 (d, *J =* 6.8 Hz, 4H), 3.09-3.01 (m, 1H), 2.80-2.78 (m, 1H), 2.46-2.37 (m, 4H), 1.93-1.80 (m, 2H), 1.37 (s, 9H), 1.01-0.94 (m, 1H), 0.88 (t, *J =* 7.3 Hz, 3H), 0.39-0.31 (m, 4H).

### Example 28. N-((4S,12S,21S)-12-Benzyl-21-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11-tetraoxatetradecan-14-amide (c-L7-1)

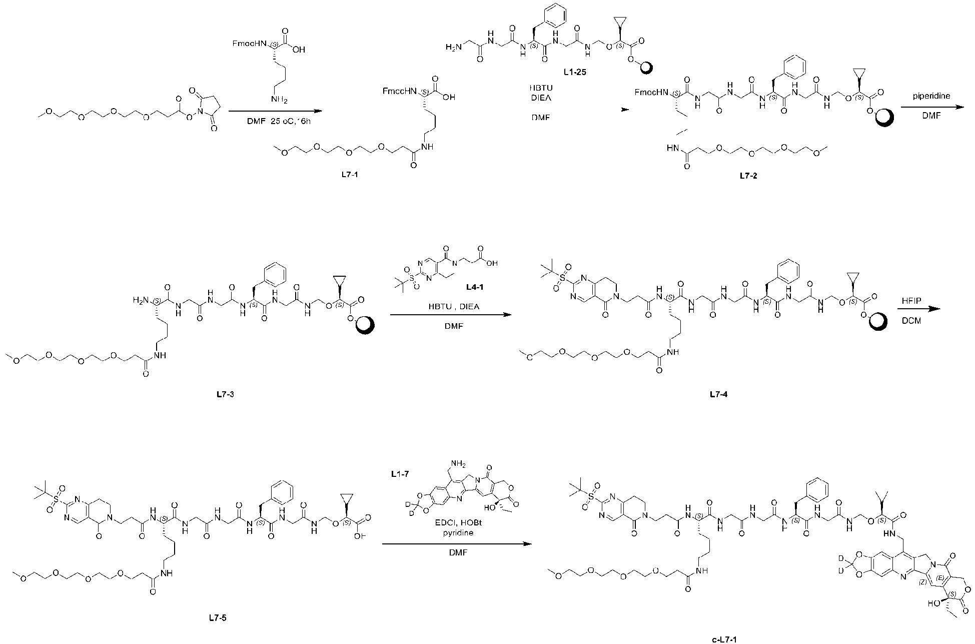

### Step 1: synthesis of (S)-20-((((9H-fluoren-9-yl)methoxy)carbonyl)amino)-14-oxo-2,5,8,11-tetraoxa-15-azaheneicosan-21-oic acid (intermediate L7-1)

(((9*H*-Fluoren-9-yl)methoxy)carbonyl)-*L*-lysine (2.1 g) and *O*-(2,5-dioxopyrrolidin-1-yl)-2,5,8,11-tetraoxatetradecan-14-oate (1.9 g) were dissolved in DMF (40 mL), and the reaction mixture was purged 3 times with nitrogen and stirred at 25 °C for 16 h. After the reaction was completed, the reaction mixture was separated by column chromatography (ethyl acetate/petroleum ether, 0-80%) to give the title compound (2.3 g).

MS m/z (ESI): 587.1 [M+H]⁺.

### Step 2: synthesis of resin-supported (20S,29S,37S)-20-((((9H-fluoren-9-yl)methoxy)carbonyl)amino)-29-benzyl-37-cyclopropyl-14,21,24,27,30,33-hexaoxo-2,5,8,11,36-pentaoxa-15,22,25,28,31,34-hexaazaoctatriacontan-38-oic acid (intermediate L7-2)

**Intermediate L7-1** (517.24 mg) was dissolved in DMF (5 mL), and **intermediate L1-25 (**the total weight of the supported substrate and the resin: 500 mg, the content: about 0.22 mmol), *O*-benzotriazole-tetramethyluronium hexafluorophosphate (336.58 mg), and diisopropylethylamine (257.02 mg) were sequentially added. The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (555 mg).

MS m/z (ESI): 1032.3 [M+H]⁺.

### Step 3: synthesis of resin-supported (20S,29S,37S)-20-amino-29-benzyl-37-cyclopropyl-14,21,24,27,30,33-hexaoxo-2,5,8,11,36-pentaoxa-15,22,25,28,31,34-hexaazaoctatriacontan-38-oic acid (intermediate L7-3)

**Intermediate L7-2** (555 mg) was dissolved in DMF (5 mL) and piperidine (35.72 mg). The reaction mixture was shaken on a shaker at 25 °C for 1 h. After the reaction was completed, the resin was washed sequentially with methanol (15 mL) and dichloromethane (15 mL). This process was repeated 3 times. The resin was then filtered under vacuum until it was dry, and the filter cake was dried under vacuum to give the title compound (428 mg).

MS m/z (ESI): 810.2 [M+H]⁺.

### Step 4: synthesis of resin-supported (20S,29S,37S)-29-benzyl-20-(3-(2-(tert-butylsulfonyl)-5-oxo-7,8-dihydropyrido[4,3-d]pyrimidin-6(5H)-yl)propanamido)-37-cyclopropyl-14,21,24,27,30,33-hexaoxo-2,5,8,11,36-pentaoxa-15,22,25,28,31,34-hexaazaoctatriacontan-38-oic acid (intermediate L7-4)

**Intermediate L7-3 (**428 mg) was dissolved in DMF (5 mL), and **intermediate L4-1** (176.55 mg), *O*-benzotriazole-tetramethyluronium hexafluorophosphate (197.43 mg), and diisopropylethylamine (150.76 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (438 mg).

MS m/z (ESI): 1155.3 [M+Na]⁺.

### Step 5: synthesis of (20S,29S,37S)-29-benzyl-20-(3-(2-(tert-butylsulfonyl)-5-oxo-7,8-dihydropyrido[4,3-d]pyrimidin-6(5H)-yl)propanamido)-37-cyclopropyl-14,21,24,27,30,33-hexaoxo-2,5,8,11,36-pentaoxa-15,22,25,28,31,34-hexaazaoctatriacontan-38-oic acid (intermediate L7-5)

**Intermediate L7-4** (438 mg) was added to a mixed solvent of dichloromethane (8 mL) and 1,1,1,3,3,3-hexafluoroisopropanol (2 mL), and the resulting mixture was shaken at 25 °C for 15 min. After the reaction was completed, the reaction mixture was filtered to remove the resin, and the filtrate was concentrated to dryness under reduced pressure to give the title compound (50 mg).

MS m/z (ESI): 1155.3 [M+Na]⁺.

### Step 6: synthesis of N-((4S,12S,21S)-12-benzyl-21-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11-tetraoxatetradecan-14-amide (c-L7-1)

**Intermediate L7-5 (**50 mg), intermediate **L1-7** (18.68 mg), 1-(3-dimethylaminopropyl)-3-ethylcarbodiimide hydrochloride (16.92 mg), 1-hydroxybenzotriazole (11.92 mg), and pyridine (10.47 mg) were dissolved in DMF (1 mL), and the reaction mixture was purged 3 times with nitrogen and stirred at 25 °C for reaction for 1 h under a nitrogen atmosphere. After the reaction was completed, the reaction mixture was directly purified by high performance liquid chromatography (column: Boston Prime C18 100 × 10 mm; mobile phase: [A phase: water (0.225% formic acid), B phase: acetonitrile]; B%: 20%-42%, 15 min) to give the title compound (5.7 mg).

MS m/z (ESI): 770.0 [(M+2H)/2]⁺.

**¹H NMR (400 MHz, DMSO-*d₆*) δ** = 9.23 (s, 1H), 8.76-8.64 (m, 1H), 8.59 (t, *J =* 6.4 Hz, 1H), 8.31 (t, *J =* 5.6 Hz, 1H), 8.28-8.14 (m, 2H), 8.14-8.04 (m, 1H), 7.96 (t, *J =* 5.2 Hz, 1H), 7.86-7.69 (m, 2H), 7.51 (s, 1H), 7.28-7.19 (m, 4H), 7.16 (*J* = 2.6, 5.7 Hz, 1H), 6.63-6.51 (m, 1H), 6.50 (s, 1H), 5.43 (d, *J =* 7.5 Hz, 3H), 4.90-4.57 (m, 3H), 4.50 (*J* = 5.1, 8.5 Hz, 1H), 4.39 (*J* = 6.5, 9.9 Hz, 1H), 4.23-4.12 (m, 1H), 3.81-3.44 (m, 27H), 3.22 (s, 3H), 3.08-2.91 (m, 4H), 2.86-2.72 (m, 2H), 2.27 (t, *J =* 6.4 Hz, 2H), 1.96-1.78 (m, 2H), 1.67-1.56 (m, 1H), 1.53-1.44 (m, 1H), 1.43-1.27 (m, 9H), 1.23 (s, 3H), 0.99-0.91 (m, 1H), 0.91-0.81 (m, 3H), 0.43-0.26 (m, 4H)

### Example 29. N-((4S,12S,21S)-12-Benzyl-21-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23,26,29,32,35-dodecaoxaoctatriacontan-38-amide (c-L8-1)

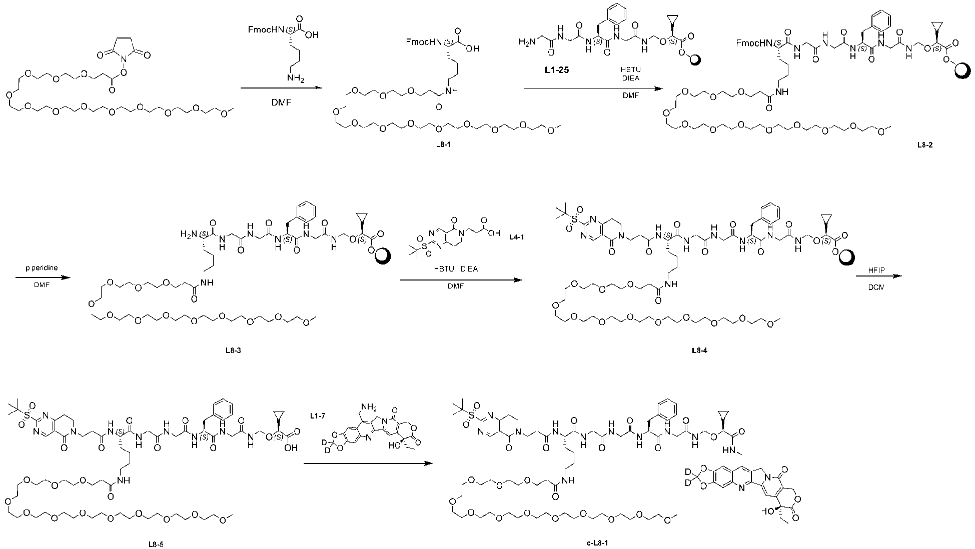

### Step 1: synthesis of (S)-44-((((9H-fluoren-9-yl)methoxy)carbonyl)amino)-38-oxo-2,5,8,11,14,17,20,23,26,29,32,35-dodecaoxa-39-azapentatetracontan-45-oic acid (intermediate L8-1)

(((9*H*-Fluoren-9-yl)methoxy)carbonyl)-*L*-lysine (800 mg) and *O*-(2,5-dioxopyrrolidin-1-yl)-2,5,8,11,14,17,20,23,26,29,32,35-dodecaoxaoctatriacontanoate (429 mg) were dissolved in DMF (4 mL), and the reaction mixture was purged 3 times with nitrogen and stirred at 25 °C for 16 h. After the reaction was completed, the crude product was purified by preparative reversed-phase high performance liquid chromatography (ISCO^{®}; 80 g of C18 column, mobile phase gradient: 0-50% acetonitrile/water, flow rate: 40 mL/min) to give the title compound (650 mg).

MS m/z (ESI): 940.0 [M+H]⁺.

### Step 2: synthesis of resin-supported (44S,53S,61S)-44-((((9H-fluoren-9-yl)methoxy)carbonyl)amino)-53-benzyl-61-cyclopropyl-38,45,48,51,54,57-hexaoxo-2,5,8,11,14,17,20,23,26,29,32,35,60-tridecaoxa-39,46,49,52,55,58-hexaazadohexacontan-62-oic acid (intermediate L8-2)

**Intermediate L8-1** (650 mg) was added to DMF (10 mL), and **intermediate L1-25** (400 mg), *O*-benzotriazole-tetramethyluronium hexafluorophosphate (336.58 mg), and diisopropylethylamine (205.62 mg) were then added. The reaction mixture was purged 3 times with nitrogen and then shaken on a shaker at 25 °C for reaction for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (430 mg).

### Step 3: synthesis of resin-supported (44S,53S,61S)-44-amino-53-benzyl-61-cyclopropyl-38,45,48,51,54,57-hexaoxo-2,5,8,11,14,17,20,23,26,29,32,35,60-tridecaoxa-39,46,49,52,55,58-hexaazadohexacontan-62-oic acid (intermediate L8-3)

**Intermediate L8-2** (430 mg) was dissolved in DMF (5 mL), and piperidine (1.08 g, 1.25 mL) was added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (410 mg).

### Step 4: synthesis of resin-supported (44S,53S,61S)-53-benzyl-44-(3-(2-(tert-butylsulfonyl)-5-oxo-7,8-dihydropyrido[4,3-d]pyrimidin-6(5H)-yl)propanamido)-61-cyclopropyl-38,45,48,51,54,57-hexaoxo-2,5,8,11,14,17,20,23,26,29,32,35,60-tridecaoxa-39,46,49,52,55,58-hexaazadohexacontan-62-oic acid (intermediate L8-4)

**Intermediate L8-3** (410 mg) and **intermediate L4-1** (144.48 mg) were dissolved in DMF (10 mL), ando *O-*benzotriazole-tetramethyluronium hexafluorophosphate (214.89 mg) and diisopropylethylamine (182.33 mg) were added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (420 mg).

### Step 5: synthesis of (44S,53S,61S)-53-benzyl-44-(3-(2-(tert-butylsulfonyl)-5-oxo-7,8-dihydropyrido[4,3-d]pyrimidin-6(5H)-yl)propanamido)-61-cyclopropyl-38,45,48,51,54,57-hexaoxo-2,5,8,11,14,17,20,23,26,29,32,35,60-tridecaoxa-39,46,49,52,55,58-hexaazadohexacontan-62-oic acid (intermediate L8-5)

**Intermediate L8-4** (420 mg) was added to a mixed solvent of dichloromethane (8 mL) and 1,1,1,3,3,3-hexafluoroisopropanol (2 mL), and the resulting mixture was shaken at 25 °C for 0.5 h. After the reaction was completed, the reaction mixture was filtered to remove the resin, and the filtrate was concentrated to dryness under reduced pressure to give the title compound (130 mg).

### Step 6: synthesis of N-((4S,12S,21S)-12-benzyl-21-(3-(2-(tert-butylsulfonyl)-5-oxo-7,8-dihydropyrido[4,3-d]pyrimidin-6(5H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23,26,29,32,35-dodecaoxaoctatriacontan-38-amide (c-L8-1)

**Intermediate L8-5** (70 mg), **intermediate L1-7** (21.94 mg), 1-(3-dimethylaminopropyl)-3-ethylcarbodiimide hydrochloride (18.20 mg), pyridine (11.26 mg), and 1-hydroxybenzotriazole (12.83 mg) were dissolved in DMF (2 mL), and the reaction mixture was purged 3 times with nitrogen and stirred at 25 °C for reaction for 2 h under a nitrogen atmosphere. After the reaction was completed, the reaction mixture was directly purified by high performance liquid chromatography (column: C 18 100 × 10 mm; mobile phase: [A phase: water (0.225% formic acid), B phase: acetonitrile]; B%: 28%-35%, 20 min) to give the title compound (5.1 mg).

**¹H NMR (**400 MHz, DMSO-*d₆*) δ = 9.27-9.23 (m, 1H), 8.70 (d, *J =* 5.3 Hz, 1H), 8.68-8.61 (m, 1H), 8.40 (s, 2H), 8.28-8.23 (m, 1H), 8.22-8.13 (m, 1H), 8.04-7.97 (m, 1H), 7.84-7.76 (m, 2H), 7.54-7.50 (m, 1H), 7.26 (s, 1H), 7.23-7.21 (m, 4H), 7.19-7.12 (m, 1H), 6.61-6.44 (m, 1H), 5.46-5.40 (m, 4H), 4.86-4.72 (m, 2H), 4.71-4.63 (m, 2H), 4.57-4.46 (m, 2H), 4.46-4.35 (m, 2H), 4.25-4.14 (m, 2H), 3.82-3.55 (m, 28H), 3.49-3.37 (m, 22H), 3.28-3.18 (m, 9H), 3.10-2.95 (m, 5H), 2.85-2.74 (m, 2H), 2.28 (t, *J =* 6.5 Hz, 2H), 1.92-1.80 (m, 2H), 1.66-1.59 (m, 1H), 1.51-1.42 (m, 1H), 1.42-1.30 (m, 9H), 1.28-1.16 (m, 2H), 1.00-0.95 (m, 1H), 0.89-0.85 (m, 3H), 0.38-0.32 (m, 4H)

MS m/z (ESI): 946.5 [M/2+H]⁺.

### Example 30. N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(tert-butylsulfonyl)-5-oxo-7,8-dihydropyrido[4,3-d]pyrimidin-6(5H)-yl)propanamido)-3,6,9,12-tetraoxapentadecan-15-amide (c-L9-1)

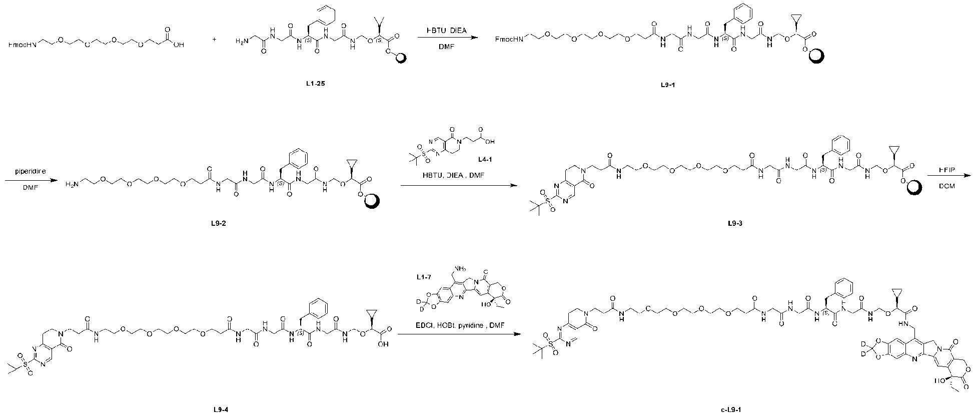

### Step 1: synthesis of resin-supported (27S,35S)-27-benzyl-35-cyclopropyl-1-(9H-fluoren-9-yl)-3,19,22,25,28,31-hexaoxo-2,7,10,13,16,34-hexaoxa-4,20,23,26,29,32-hexaazahexatriacontan-36-oic acid (intermediate L9-1)

DMF (5 mL) was added to a mixture of 1-(9*H*-fluoren-9-yl)-3-oxo-2,7,10,13,16-pentaoxa-4-azanonadecane-19-carboxylic acid (420 mg), **intermediate L1-25** (the total weight of the supported substrate and the resin: 500 mg, the content: about 0.22 mmol), *N*,*N*-diisopropylethylamine (257 mg), and *O*-(benzotriazol-1-yl)-*N*,*N*,*N'*,*N'-*tetramethyluronium hexafluorophosphate (497 mg) at room temperature under a nitrogen atmosphere. The resulting mixture was shaken on a shaker at 20 °C for 1 h. The resin was then washed sequentially with methanol (10 mL) and dichloromethane (10 mL). This process was repeated 3 times. The resin was then filtered under vacuum until it was dry, and the filter cake was dried under vacuum to give the title compound (550 mg).

### Step 2: synthesis of resin-supported (2S,10S)-32-amino-10-benzyl-2-cyclopropyl-6,9,12,15,18-pentaoxo-3,21,24,27,30-pentaoxa-5,8,11,14,17-pentaazadotriacontanoic acid (intermediate L9-2)

Piperidine (500 µL) was added to a solution of **intermediate L9-1** (550 mg) in DMF (5 mL) at room temperature under a nitrogen atmosphere, and the resulting mixture was shaken on a shaker at 20 °C for 1 h. The resin was then washed sequentially with methanol (10 mL) and dichloromethane (10 mL). This process was repeated 3 times. The resin was then filtered under vacuum until it was dry, and the filter cake was dried under vacuum to give the title compound (500 mg).

LC-MS: m/z (ESI): 733.2 [M+Na]⁺.

### Step 3: synthesis of resin-supported (2S,10S)-10-benzyl-36-(2-(tert-butylsulfonyl)-5-oxo-7,8-dihydropyrido[4,3-d]pyrimidin-6(5H)-yl)-2-cyclopropyl-6,9,12,15,18,34-hexaoxo-3,21,24,27,30-pentaoxa-5,8,11,14,17,33-hexaazahexatriacontanoic acid (intermediate L9-3)

DMF (5 mL) was added to a mixture of **intermediate L9-2** (500 mg), **intermediate L4-1** (187 mg), *N,N-*diisopropylethylamine (193 mg), and *O*-(benzotriazol-1-yl)-*N*,*N*,*N'*,*N'*-tetramethyluronium hexafluorophosphate (253 mg) under a nitrogen atmosphere. The resulting mixture was shaken on a shaker at 20 °C for 1 h. The resin was then washed sequentially with methanol (10 mL) and dichloromethane (10 mL). This process was repeated 3 times. The resin was then filtered under vacuum until it was dry, and the filter cake was dried under vacuum to give the title compound (500 mg).

### Step 4: synthesis of (2S,10S)-10-benzyl-36-(2-(tert-butylsulfonyl)-5-oxo-7,8-dihydropyrido[4,3-d]pyrimidin-6(5H)-yl)-2-cyclopropyl-6,9,12,15,18,34-hexaoxo-3,21,24,27,30-pentaoxa-5,8,11,14,17,33-hexaazahexatriacontanoic acid (intermediate L9-4)

**Intermediate L9-3 (**500 mg) was dissolved in dichloromethane (16 mL) and 1,1,1,3,3,3-hexafluoroisopropanol (4 mL) under a nitrogen atmosphere. The resulting mixture was shaken on a shaker at 20 °C for 1 h. The reaction mixture was then filtered to remove the resin, and the filtrate was concentrated to dryness under reduced pressure. Acetonitrile (4 mL) and pure water (8 mL) were added, and the mixture was lyophilized with a lyophilizer to give the title compound (150 mg).

### Step 5: synthesis of N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(tert-butylsulfonyl)-5-oxo-7,8-dihydropyrido[4,3-d]pyrimidin-6(5H)-yl)propanamido)-3,6,9,12-tetraoxapentadecan-15-amide (c-L9-1)

DMF (2 mL) was added to a mixture of **intermediate L9-4** (100 mg), **intermediate L1-7** (45.0 mg), 1-(3-dimethylaminopropyl)-3-ethylcarbodiimide hydrochloride (37.1 mg), 1-hydroxybenzotriazole (26.1 mg), and pyridine (23.0 mg) under a nitrogen atmosphere. The resulting mixture was stirred at room temperature for 1 h. Then the reaction mixture was directly purified by high performance liquid chromatography (column: C18 100 × 10 mm; mobile phase: [A: water (0.225% formic acid), B: acetonitrile]; B%: 15%-40%, 18 min) to give the title compound (10.6 mg).

LC-MS: m/z (ESI): 1439.4 [M+H]⁺

**¹H NMR (400 MHz, DMSO-*d₆*)** δ 9.29-9.24 (m, 1H), 8.60 (t, *J =* 6.4 Hz, 2H), 8.33-8.26 (m, 1H), 8.21-8.13 (m, 1H), 8.13-8.04 (m, 2H), 8.00 (t, *J =* 5.5 Hz, 1H), 7.80 (s, 1H), 7.52 (s, 1H), 7.26-7.22 (m, 5H), 7.19-7.14 (m, 1H), 6.50 (s, 1H), 5.44 (d, *J* = 8.4 Hz, 4H), 4.83-4.72 (m, 2H), 4.70-4.63 (m, 1H), 4.55-4.47 (m, 1H), 4.45-4.37 (m, 1H), 3.77-3.67 (m, 10H), 3.61-3.55 (m, 3H), 3.50-3.45 (m, 14H), 3.26-3.18 (m, 4H), 3.05-3.03 (m, 1H), 2.84-2.74 (m, 1H), 2.46-2.35 (m, 4H), 1.92-1.81 (m, 2H), 1.41-1.33 (m, 9H), 1.00-0.94 (m, 1H), 0.90-0.86 (m, 3H), 0.39-0.36 (m, 2H), 0.34-0.30 (m, 2H).

### Example 31. N-((4S,12S)-12-Benzyl-4-cyclopropyl-1-((S)-7-ethyl-7-hydroxy-8,11-dioxo-7,8,11,13-tetrahydro-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(tert-butylsulfonyl)-5-oxo-7,8-dihydropyrido[4,3-d]pyrimidin-6(5H)-yl)propanamido)-3,6,9,12,15,18,21,24,27,30,33,36-dodecaoxanonatriacontan-39-amide (c-L10-1)

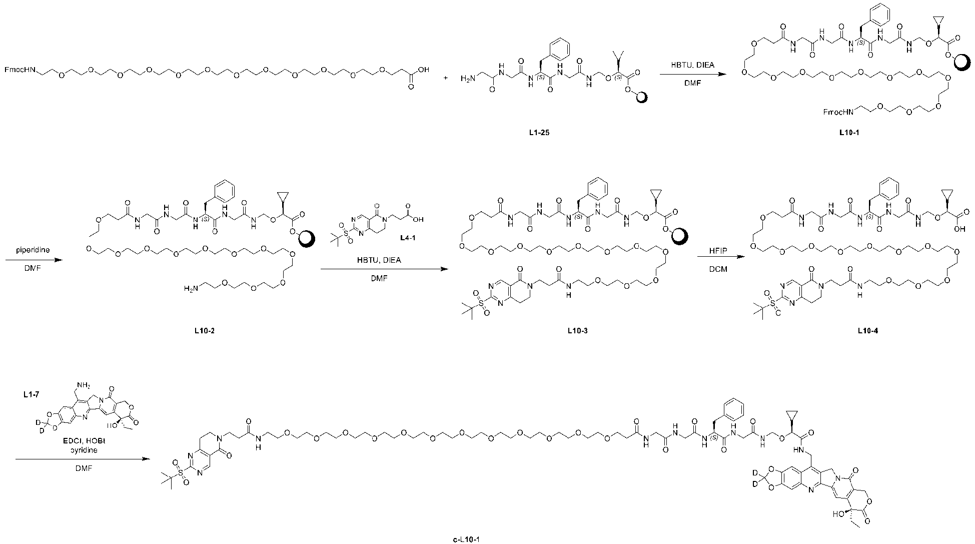

### Step 1: synthesis of (51S,59S)-51-benzyl-59-cyclopropyl-1-(9H-fluoren-9-yl)-3,43,46,49,52,55-hexaoxo-2,7,10,13,16,19,22,25,28,31,34,37,40,58-tetradecaoxa-4,44,47,50,53,56-hexaazahexacontanoic acid (intermediate L10-1)

**Intermediate L1-25** (the total weight of the supported substrate and the resin: 500 mg, the content: about 0.22 mmol) was dissolved in DMF (5 mL), and 1-(9*H*-fluoren-9-yl)-3-oxo-2,7,10,13,16,19,22,25,28,31,34,37,40-tridecaoxa-4-azatritetracontanoic acid (723.85 mg), O-benzotriazole-tetramethyluronium hexafluorophosphate (336.58 mg), and diisopropylethylamine (257.02 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (500 mg).

MS m/z (ESI): 1307.4 [M+Na]⁺.

### Step 2: synthesis of resin-supported (2S,10S)-56-amino-10-benzyl-2-cyclopropyl-6,9,12,15,18-pentaoxo-3,21,24,27,30,33,36,39,42,45,48,51,54-tridecaoxa-5,8,11,14,17-pentaazahexapentacontanoic acid (intermediate L10-2)

**Intermediate L10-1** (500 mg) was dissolved in DMF (2 mL), and piperidine (0.5 mL) was added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (470 mg).

### Step 3: synthesis of resin-supported (2S,10S)-10-benzyl-60-(2-(tert-butylsulfonyl)-5-oxo-7,8-dihydropyrido[4,3-d]pyrimidin-6(5H)-yl)-2-cyclopropyl-6,9,12,15,18,58-hexaoxo-3,21,24,27,30,33,36,39,42,45,48,51,54-tridecaoxa-5,8,11,14,17,57-hexaazahexacontanoic acid (intermediate L10-3)

**Intermediate L10-2** (470 mg) was dissolved in DMF (5 mL), and **intermediate L4-1** (192.54 mg), *O-*benzotriazole-tetramethyluronium hexafluorophosphate (176.25 mg), and diisopropylethylamine (134.59 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (460 mg).

### Step 4: synthesis of (2S,10S)-10-benzyl-60-(2-(tert-butylsulfonyl)-5-oxo-7,8-dihydropyrido[4,3-d]pyrimidin-6(5H)-yl)-2-cyclopropyl-6,9,12,15,18,58-hexaoxo-3,21,24,27,30,33,36,39,42,45,48,51,54-tridecaoxa-5,8,11,14,17,57-hexaazahexacontanoic acid (intermediate L10-4)

**Intermediate L10-3** (460 mg) was added to a mixed solvent of dichloromethane (4 mL) and 1,1,1,3,3,3-hexafluoroisopropanol (1 mL), and the resulting mixture was shaken at 25 °C for 0.5 h. After the reaction was completed, the reaction mixture was filtered to remove the resin, the filtrate was concentrated under reduced pressure, and the residue was lyophilized to give the title compound (130 mg).

### Step 5: synthesis of N-((4S,12S)-12-benzyl-4-cyclopropyl-1-((S)-7-ethyl-7-hydroxy-8,11-dioxo-7,8,11,13-tetrahydro-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(tert-butylsulfonyl)-5-oxo-7,8-dihydropyrido[4,3-d]pyrimidin-6(5H)-yl)propanamido)-3,6,9,12,15,18,21,24,27,30,33,36-dodecaoxanonatriacontan-39-amide (c-L10-1)

**Intermediate L10-4** (50 mg), **intermediate L1-7** (16.80 mg), 1-(3-dimethylaminopropyl)-3-ethylcarbodiimide hydrochloride (13.83 mg), pyridine (8.56 mg), and 1-hydroxybenzotriazole (9.75 mg) were dissolved in DMF (1 mL), and the reaction mixture was purged 3 times with nitrogen and stirred at 25 °C for reaction for 2 h under a nitrogen atmosphere. After the reaction was completed, the reaction mixture was directly purified by high performance liquid chromatography (column: Boston Prime 100 × 10 mm; mobile phase: [A: water (0.225% formic acid), B: acetonitrile]; B%: 20%-42%, 15 min) to give the title compound (3.3 mg).

**¹H NMR (400 MHz, DMSO-*d₆*)** δ 9.25 (s, 1H), 8.70-8.58 (m, 2H), 8.34-8.26 (m, 1H), 8.19-8.15 (m, 1H), 8.15-8.04 (m, 2H), 8.03-7.97 (m, 1H), 7.79 (s, 1H), 7.51 (s, 1H), 7.27-7.21 (m, 5H), 7.19-7.15 (m, 1H), 6.82-6.73 (m, 1H), 6.50 (s, 1H), 5.50-5.37 (m, 4H), 4.84-4.70 (m, 2H), 4.67-4.65 (m, 1H), 4.51-4.45 (m, 1H), 4.42-4.36 (m, 1H), 3.76-3.66 (m, 10H), 3.59 (s, 3H), 3.50-3.46 (m, 45H), 3.24-3.17 (m, 4H), 3.06-3.01 (m, 1H), 2.82-2.75 (m, 1H), 2.45-2.35 (m, 4H), 1.92-1.80 (m, 2H), 1.36 (s, 9H), 0.93 (s, 1H), 0.87 (t, *J=* 7.3 Hz, 3H), 0.38-0.30 (m, 4H). MS m/z (ESI): 896.6 [M/2+H]⁺.

### Example 32. N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(cyclopentylsulfonyl)-5-oxo-7,8-dihydropyrido[4,3-d]pyrimidin-6(5H)-yl)propanamido)-3,6,9,12,15,18,21,24-octaoxaheptacosan-27-amide (b-L6-1)

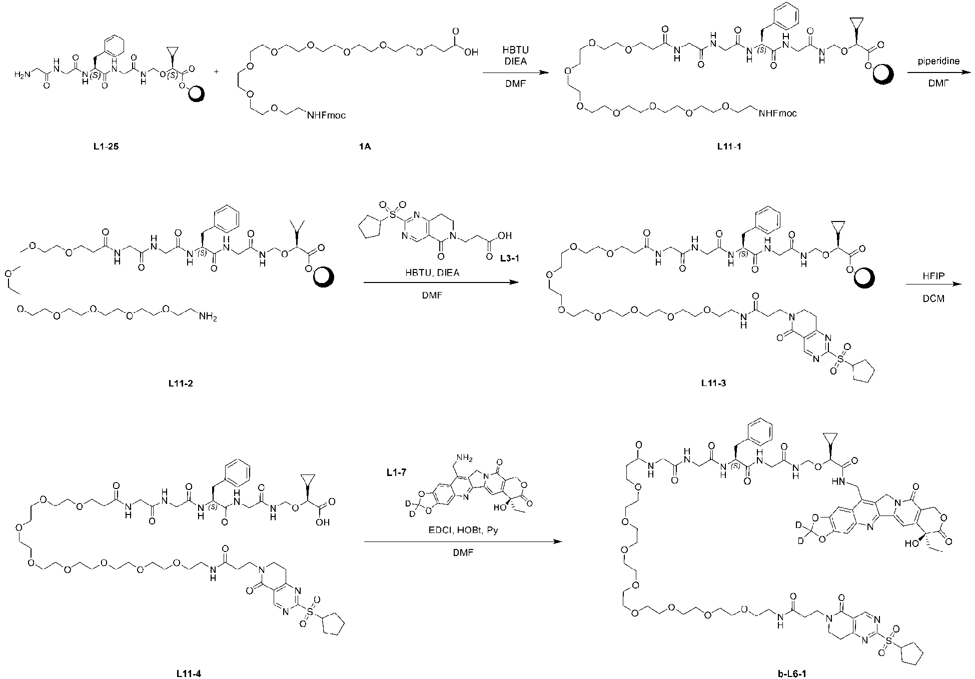

### Step 1: synthesis of resin-supported (39S,47S)-39-benzyl-47-cyclopropyl-1-(9H-fluoren-9-yl)-3,31,34,37,40,43-hexaoxo-2,7,10,13,16,19,22,25,28,46-decaoxa-4,32,35,38,41,44-hexaazaoctatetracontan-48-oic acid (L11-1)

*N*,*N*-Dimethylformamide (10 mL) was added to a mixture of **intermediate 1A** (1.17 g), **intermediate L1-25** (the total weight of the supported substrate and the resin: 1 g, the content: about 0.43 mmol), N,N-diisopropylethylamine (514.05 mg), and *O*-(benzotriazol-1-yl)-*N*,*N*,*N'*,*N'*-tetramethyluronium hexafluorophosphate (1.01 g) at room temperature under a nitrogen atmosphere. The resulting mixture was shaken on a shaker at 20 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (1.03 g).

### Step 2: synthesis of resin-supported (2S,108)-44-amino-10-benzyl-2-cyclopropyl-6,9,12,15,18-pentaoxo-3,21,24,27,30,33,36,39,42-nonaoxa-5,8,11,14,17-pentaazatetratetracontanoic acid (L11-2)

Piperidine (2.5 mL) was added to a solution of **intermediate L11-1** (1.03 g) in *N*,*N*-dimethylformamide (10 mL) at room temperature under a nitrogen atmosphere, and the resulting mixture was shaken on a shaker at 20 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (993 mg).

LC-MS: m/z (ESI): 887.6 [M+H]⁺.

### Step 3: synthesis of resin-supported (2S,10S)-10-benzyl-48-(2-(cyclopentylsulfonyl)-5-oxo-7,8-dihydropyrido[4,3-d]pyrimidin-6(5H)-yl)-2-cyclopropyl-6,9,12,15,18,46-hexaoxo-3,21,24,27,30,33,36,39,42-nonaoxa-5,8,11,14,17,45-hexaazaoctatetracontanoic acid (intermediate L11-3)

*N*,*N*-Dimethylformamide (15 mL) was added to a mixture of **intermediate L11-2** (993 mg), **intermediate L3-1** (396.28 mg), *N*,*N*-diisopropylethylamine (326.90 mg), and *O*-(benzotriazol-1-yl)-*N*,*N*,*N'*,*N'*-tetramethyluronium hexafluorophosphate (639.49 mg) under a nitrogen atmosphere. The resulting mixture was shaken on a shaker at 20 °C for 1 h. After the starting materials were completely reacted as detected by LCMS and the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (1.02 g).

LC-MS: m/z (ESI): 1245.2 [M+Na]⁺

### Step 4: synthesis of (2S,10S)-10-benzyl-48-(2-(cyclopentylsulfonyl)-5-oxo-7,8-dihydropyrido[4,3-d]pyrimidin-6(5H)-yl)-2-cyclopropyl-6,9,12,15,18,46-hexaoxo-3,21,24,27,30,33,36,39,42-nonaoxa-5,8,11,14,17,45-hexaazaoctatetracontanoic acid (intermediate L11-4)

**Intermediate L11-3** (1.02 g) was dissolved in dichloromethane (16 mL) and 1,1,1,3,3,3-hexafluoroisopropanol (4 mL) under a nitrogen atmosphere. The resulting mixture was shaken on a shaker at 20 °C for 1 h. After the reaction was completed, the reaction mixture was filtered to remove the resin, and the filtrate was concentrated to dryness under reduced pressure. Acetonitrile (4 mL) and pure water (8 mL) were added, and the mixture was lyophilized with a lyophilizer to give the title compound (230 mg).

LC-MS: m/z (ESI): 1244.7 [M+Na]⁺

### Step 5: synthesis of N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-1-(3-(2-(cyclopentylsulfonyl)-5-oxo-7,8-dihydropyrido[4,3-d]pyrimidin-6(5H)-yl)propanamido)-3,6,9,12,15,18,21,24-octaoxaheptacosan-27-amide (b-L6-1)

*N*,*N*-Dimethylformamide (2 mL) was added to a mixture of **intermediate L11-4** (230 mg), **intermediate L1-7** (87.64 mg), 1-(3-dimethylaminopropyl)-3-ethylcarbodiimide hydrochloride (72.14 mg), 1-hydroxybenzotriazole (50.85 mg), and pyridine (44.65 mg) under a nitrogen atmosphere. The resulting mixture was stirred at room temperature for 1 h. After the starting materials were completely reacted as detected by LCMS, the reaction mixture was directly purified by high performance liquid chromatography (column: C18 Welch Xtimate C18 150 × 30 mm × 5 µm; mobile phase: [A: water (0.225% formic acid), B: acetonitrile]; B%: 27%-47%, 22 min) to give the title compound (41.4 mg).

LC-MS: m/z (ESI): 814.8 [(M+2H)/2]⁺

**¹H NMR (400 MHz, DMSO-*d₆*)** δ = 9.24 (s, 1H), 8.70-8.64 (m, 1H), 8.60 (t, *J =* 6.6 Hz, 1H), 8.29 (t, *J =* 5.9 Hz, 1H), 8.17 (t, *J =* 5.6 Hz, 1H), 8.11 (d, *J =* 8.0 Hz, 1H), 8.07 (t, *J =* 5.5 Hz, 1H), 8.00 (t, *J =* 5.6 Hz, 1H), 7.79 (s, 1H), 7.52 (s, 1H), 7.27-7.21 (m, 6H), 7.16 (s, 1H), 6.51 (s, 1H), 5.43 (s, 4H), 4.84-4.71 (m, 2H), 4.68-4.66 (m, 1H), 4.54-4.46 (m, 1H), 4.43-4.37 (m, 1H), 4.28-4.19 (m, 1H), 3.76-3.66 (m, 11H), 3.59 (t, *J =* 6.4 Hz, 3H), 3.50-3.46 (m, 25H), 3.26-3.18 (m, 5H), 3.05-3.03 (m, 1H), 2.80-2.78 (m, 1H), 2.42-2.40 (m, 5H), 2.00-1.85 (m, 6H), 1.74-1.58 (m, 5H), 0.99-0.96 (m, 1H), 0.88 (t, *J* = 7.3 Hz, 3H), 0.38-0.31 (m, 4H)

### Example 33. (S)-N⁴-(26-Amino-3,6,9,12,15,18,21,24-octamethyl-2,5,8,11,14,17,20,23,26-nonaoxo-3,6,9,12,15,18,21,24-octaazahexacosanyl)-N¹-((4S,12S)-12-benzyl-4-cyclopropyl-1-((S)-7-ethyl-7-hydroxy-8,11-dioxo-8,10,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-2-(3-(2-(tert-butylsulfonyl)-5-oxo-7,8-dihydropyrido[4,3-d]pyrimidin-6(5H)-yl)propanamido)-N⁴-methylbutanediamide (c-L12-1)

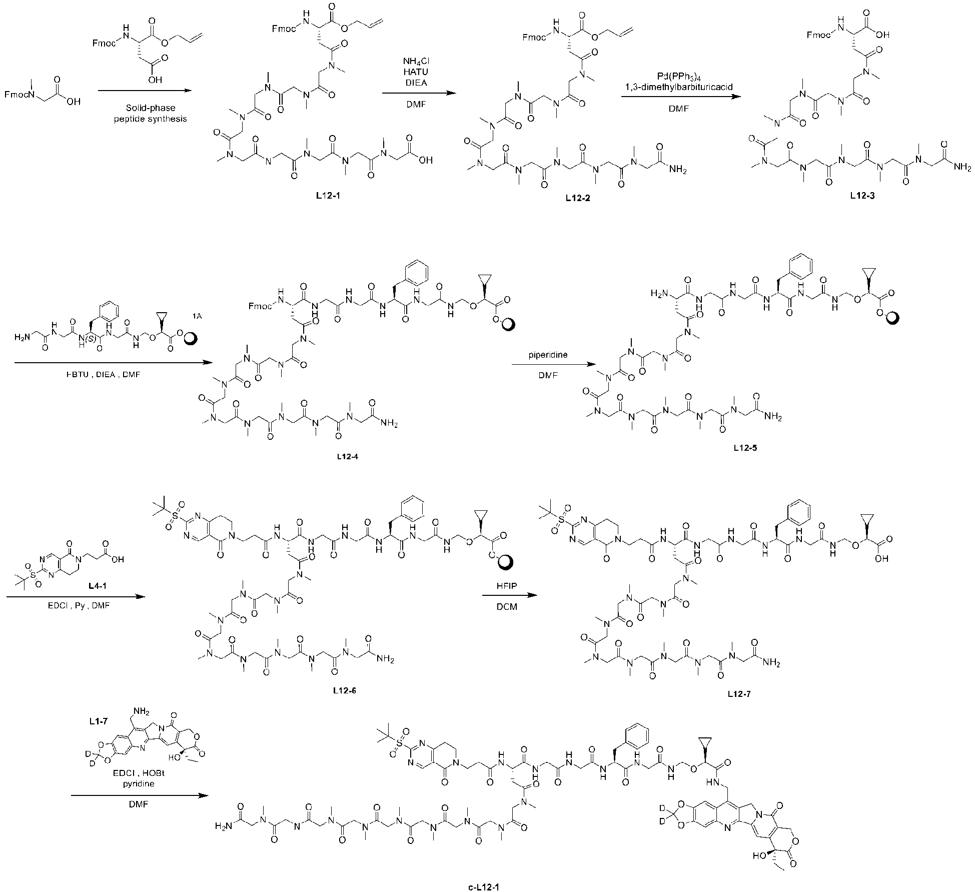

### Step 1: synthesis of (S)-5-allyloxycarbonyl-1-(9H-fluoren-9-yl)-8,11,14,17,20,23,26,29,32-nonamethyl-3,7,10,13,16,19,22,25,28,31-decaoxo-2-oxa-4,8,11,14,17,20,23,26,29,32-decaazatetratriacontan-34-carboxylic acid (intermediate L12-1)

The compound was synthesized by a polypeptide solid-phase synthesis method, and the steps are as follows:
1) Dichloromethane was added to a mixture of CTC resin (0.64 mmol/g, 12.5 g) and N-(((9H-fluoren-9-yl)methoxy)carbonyl)-N-methylglycine (2.50 g) under a nitrogen atmosphere;
2) diisopropylethylamine (DIEA, 6.2 g) was added dropwise, and the mixture was mixed and stirred for 2 h;
3) methanol (13 mL) was added, and the mixture was mixed and stirred for 0.5 h;
4) the resulting mixture was filtered and washed three times with DMF;
5) a 20% piperidine/DMF solution was added, and the mixture was reacted for 30 min;
6) the resulting mixture was filtered and washed five times with DMF;
7) the substances in the "Starting material" column in the table below were added and mixed for 30 s, and then the substances in the "Reagent" column in the table below were added. The reaction was carried out under nitrogen bubbling for 1 h;
8) steps 4-7 were repeated to complete the desired polypeptide synthesis.

### Note:

| # | **Starting material** | **Reagent** |
|---|---|---|
| 1 | Fmoc-Sar-OH(1.0 eq.) | DIEA(6.0 eq.) |
| 2 | Fmoc-Sar-OH(3.0 eq.) | HBTU(2.85 eq.)+ DIEA(6.0 eq.) |
| 3 | Fmoc-Sar-OH(3.0 eq.) | HBTU(2.85 eq.)+ DIEA(6.0 eq.) |
| 4 | Fmoc-Sar-OH(3.0 eq.) | HBTU(2.85 eq.)+ DIEA(6.0 eq.) |
| 5 | Fmoc-Sar-OH(3.0 eq.) | HBTU(2.85 eq.)+ DIEA(6.0 eq.) |
| 6 | Fmoc-Sar-OH(3.0 eq.) | HBTU(2.85 eq.)+ DIEA(6.0 eq.) |
| 7 | Fmoc-Sar-OH(3.0 eq.) | HATU(2.85 eq.)+ DIEA(6.0 eq.) |
| 8 | Fmoc-Sar-OH(3.0 eq.) | HATU(2.85 eq.)+ DIEA(6.0 eq.) |
| 9 | Fmoc-Sar-OH(3.0 eq.) | HATU(2.85 eq.)+ DIEA(6.0 eq.) |
| 10 | Fmoc-Asp-OAll-OH(2.0 eq.) | HATU(1.90 eq.)+ DIEA(4.0 eq.) |

After the solid phase was prepared, a 20% HFIP/DCM solution was added to the resulting mixture, and the mixture was stirred for reaction for 1.5 h. The reaction mixture was then filtered, and the filtrate was collected and concentrated. The resulting mixture was purified by preparative liquid chromatography (A: 0.075% TFA in H₂O, B: acetonitrile) to give the title compound (2.15 g).

MS m/z (ESI): 1035.6 [M+H]⁺.

### Step 2: synthesis of allyl (S)-30-((((9H-fluoren-9-yl)methoxy)carbonyl)amino)-1-amino-3,6,9,12,15,18,21,24,27-nonamethyl-1,4,7,10,13,16,19,22,25,28-decaoxo-3,6,9,12,15,18,21,24,27-nonaazahentriacontane-31-carboxylate (intermediate L12-2)

**Intermediate L12-1 (**1 g), ammonium chloride (155.09 mg), diisopropylethylamine (499.44 mg), and HATU (728.95 mg) were added to DMF (5 mL), and the reaction mixture was purged 3 times with nitrogen and stirred at 25 °C for 3 h. After the reaction was completed, the crude product was purified by preparative reversed-phase high performance liquid chromatography (ISCO^{®}; 80 g of C 18 Column, mobile phase gradient: 0-40% acetonitrile/water, flow rate: 40 mL/min) to give the title compound (800 mg).

MS m/z (ESI): 1034.6 [M+H]⁺.

### Step 3: synthesis of (S)-30-((((9H-fluoren-9-yl)methoxy)carbonyl)amino)-1-amino-3,6,9,12,15,18,21,24,27-nonamethyl-1,4,7,10,13,16,19,22,25,28-decaoxo-3,6,9,12,15,18,21,24,27-nonaazahentriacontane-31-carboxylic acid (intermediate L12-3)

**Intermediate L12-2** (800 mg), tetrakis(triphenylphosphine)palladium(0) (201.84 mg), and 1,3-dimethylbarbituric acid (108.71 mg) were added to DMF (5 mL), and the reaction mixture was purged 3 times with nitrogen and stirred at 25 °C for 4 h. After the reaction was completed, the crude product was purified by preparative reversed-phase high performance liquid chromatography (ISCO^{®}; 80 g of C 18 Column, mobile phase gradient: 0-40% acetonitrile/water, flow rate: 40 mL/min) to give the title compound (700 mg).

MS m/z (ESI): 994.9 [M+H]⁺.

### Step 4: synthesis of resin-supported (2S,10S,19S)-19-((((9H-fluoren-9-yl)methoxy)carbonyl)amino)-48-amino-10-benzyl-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontanoic acid (intermediate L12-4)

**Intermediate L12-3** (700 mg) was added to DMF (10 mL), and **intermediate L1-25** (the total weight of the resin and the substrate: 400 mg, the content: about 0.18 mmol), O-benzotriazole-tetramethyluronium hexafluorophosphate (336.58 mg), and diisopropylethylamine (205.62 mg) were then added. The reaction mixture was purged 3 times with nitrogen and then shaken on a shaker at 25 °C for reaction for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (420 mg).

MS m/z (ESI): 1461.4 [M+Na]⁺.

### Step 5: synthesis of resin-supported (2S,10S,19S)-19,48-diamino-10-benzyl-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontanoic acid (intermediate L12-5)

**Intermediate L12-4** (420 mg) was dissolved in DMF (5 mL), and piperidine (1.25 mL) was added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (400 mg).

MS m/z (ESI): 1217.4 [M+H]⁺.

### Step 6: synthesis of resin-supported (2S,10S,19S)-48-amino-10-benzyl-19-(3-(2-(tert-butylsulfonyl)-5-oxo-7,8-dihydropyrido[4,3-d]pyrimidin-6(5H)-yl)propanamido)-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontane-1-carboxylic acid (intermediate L12-6)

**Intermediate L12-5** (360 mg) and **intermediate L4-1** (108.39 mg) were dissolved in DMF (10 mL), and 1-(3-dimethylaminopropyl)-3-ethylcarbodiimide hydrochloride (114 mg) and pyridine (108 mg) were added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (390 mg).

### Step 7: synthesis of (2S,10S,19S)-48-amino-10-benzyl-19-(3-(2-(tert-butylsulfonyl)-5-oxo-7,8-dihydropyrido[4,3-d]pyrimidin-6(5H)-yl)propanamido)-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontane-1-carbolrylic acid (intermediate L12-7)

**Intermediate L12-6** (390 mg) was added to a mixed solvent of dichloromethane (8 mL) and 1,1,1,3,3,3-hexafluoroisopropanol (2 mL), and the resulting mixture was shaken at 25 °C for 0.5 h. After the reaction was completed, the reaction mixture was filtered to remove the resin, the filtrate was concentrated to dryness under reduced pressure, and the crude product was directly purified by high performance liquid chromatography (column: Boston Green ODS 150 × 30 mm × 5 µm; mobile phase: [A: water (0.225% formic acid), B: acetonitrile]; B%: 10%-50%, 10 min) to give the title compound (25 mg).

### Step 8: synthesis of (S)-N⁴-(26-amino-3,6,9,12,15,18,21,24-octamethyl-2,5,8,11,14,17,20,23,26-nonaoxo-3,6,9,12,15,18,21,24-octaazahexacosanyl)-N¹-((4S,12S)-12-benzyl-4-cyclopropyl-1-((S)-7-ethyl-7-hydroxy-8,11-dioxo-8,10,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-2-(3-(2-(tert-butylsulfonyl)-5-oxo-7,8-dihydropyrido[4,3-d]pyrimidin-6(5H)-yl)propanamido)-N⁴-methylbutanediamide (c-L12-1)

**Intermediate L12-7** (16 mg), **intermediate L1-7** (4.4 mg), 1-(3-dimethylaminopropyl)-3-ethylcarbodiimide hydrochloride (3.98 mg), pyridine (2.46 mg), and 1-hydroxybenzotriazole (2.81 mg) were dissolved in DMF (2 mL), and the reaction mixture was purged 3 times with nitrogen and stirred at 25 °C for reaction for 2 h under a nitrogen atmosphere. After the reaction was completed, the reaction mixture was directly purified by high performance liquid chromatography (column: C18 100 × 10 mm; mobile phase: [A: water (ammonium bicarbonate), B: acetonitrile]; B%: 10%-38%, 16 min) to give the title compound (4.5 mg).
(Preparation of mobile phase A: 30 L of pure water + 24 g of ammonium bicarbonate)

**¹H NMR (400 MHz, DMSO-*d₆*)** δ = 9.24 (s, 1H), 8.66 (d, *J =* 6.4 Hz, 1H), 8.59 (s, 1H), 8.27 (s, 2H), 8.01 (d, *J =* 16.5 Hz, 2H), 7.81 (s, 1H), 7.52 (s, 1H), 7.26 (s, 1H), 7.22 (s, 5H), 7.16 (s, 1H), 6.50 (s, 1H), 5.44 (d, *J* = 9.9 Hz, 4H), 4.85-4.71 (m, 3H), 4.70-4.63 (m, 1H), 4.57 (s, 1H), 4.49 (s, 1H), 4.43-4.27 (m, 6H), 4.23 (d, *J =* 16.1 Hz, 3H), 4.16 (s, 1H), 4.13-3.99 (m, 5H), 3.92 (s, 4H), 3.86 (d, *J =* 13.0 Hz, 2H), 3.75-3.64 (m, 10H), 3.59 (s, 2H), 3.49 (d, *J* = 7.0 Hz, 1H), 3.22 (t, *J* = 6.4 Hz, 2H), 2.99-2.84 (m, 14H), 2.84-2.71 (m, 14H), 1.92-1.79 (m, 2H), 1.37 (s, 9H), 1.24 (s, 2H), 0.96 (d, *J =* 7.7 Hz, 1H), 0.91-0.84 (m, 3H), 0.39-0.30 (m, 4H)

MS m/z (ESI): 973.9 [M/2+H]⁺.

### Example 34. N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-6-(2-(cyclopentylsulfonyl)-5-oxo-8,8-di(2,5,8,11-tetraoxatridecan-13-yl)-7,8-dihydropyrido[4,3-d]pyrimidin-6(5H)-yl)hexanamide (b-L13-1)

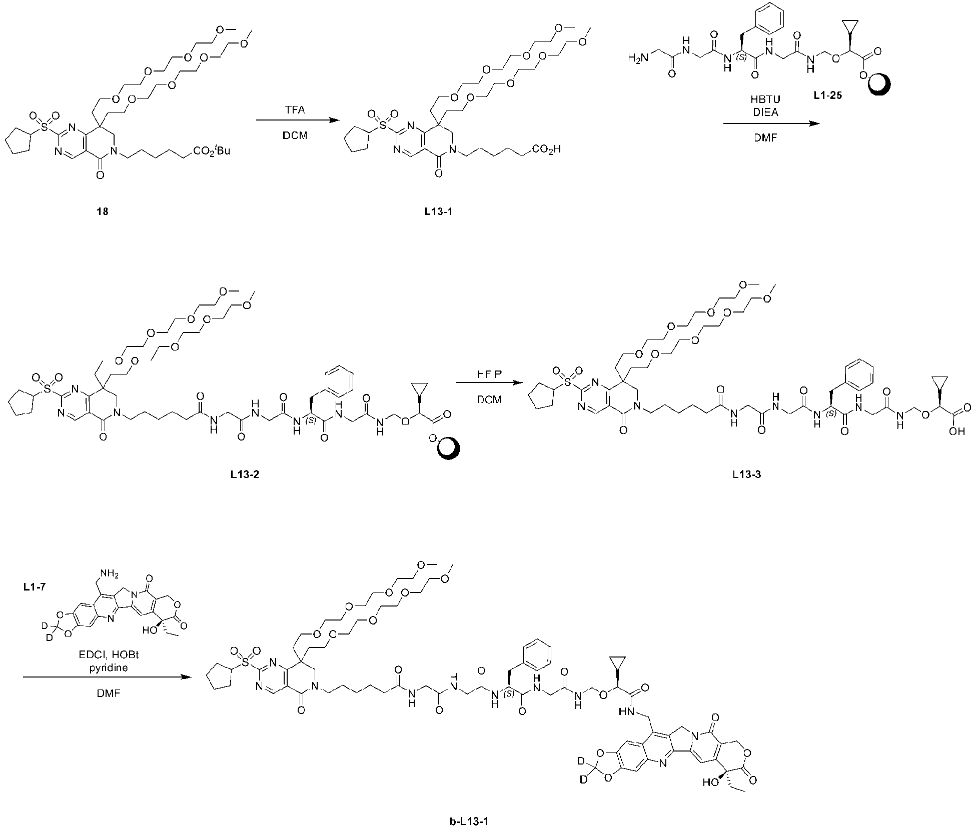

### Step 1: synthesis of 6-(2-cyclopentylsulfonyl-5-oxo-8,8-di(2,5,8,11-tetraoxatridecan-13-yl)-7,8-dihydropyrido[4,3-d]pyrimidin-6(5H)-yl)hexanoic acid (intermediate L13-1)

**Compound 18** (800 mg) was dissolved in dichloromethane (8 mL), and trifluoroacetic acid (1.5 mL) was added. The reaction mixture was stirred at 25 °C for reaction for 2 h under a nitrogen atmosphere. After the reaction was completed, the mixture was concentrated under reduced pressure to remove the solvent. The residue was purified by flash silica gel column chromatography (ISCO^{®}; 12 g of SepaFlash^{®} flash silica gel column, gradient 0-30% tetrahydrofuran/petroleum ether, flow rate: 50 mL/min) to give the title compound (280 mg).

MS m/z (ESI): 776.6 (M+H)⁺.

### Step 2: synthesis of resin-supported (2S,10S)-10-benzyl-23-(2-cyclopentylsulfonyl-5-oxo-8,8-di(2,5,8,11-tetraoxatridecan-13-yl)-7,8-dihydropyrido[4,3-d]pyrimidin-6(5H)-yl)-2-cyclopropyl-6,9,12,15,18-pentaoxo-3-oxa-5,8,11,14,17-pentaazatricosanoic acid (intermediate L13-2)

**Intermediate L13-1** (280 mg) and intermediate L1-25 (440 mg) were dissolved in *N*,*N*-dimethylformamide (5 mL), and *O*-benzotriazole-tetramethyluronium hexafluorophosphate (183.22 mg) and *N*,*N*-diisopropylethylamine (139.91 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (470 mg).

MS m/z (ESI): 1221.1 (M+H)⁺.

### Step 3: synthesis of (2S,10S)-10-benzyl-23-(2-cyclopentylsulfonyl-5-oxo-8,8-di(2,5,8,11-tetraoxatridecan-13-yl)-7,8-dihydropyrido[4,3-d]pyrimidin-6(5H)-yl)-2-cyclopropyl-6,9,12,15,18-pentaoxo-3-oxa-5,8,11,14,17-pentaazatricosanoic acid (intermediate L13-3)

**Intermediate L13-2** (450 mg) was dissolved in dichloromethane (8 mL) and 1,1,1,3,3,3-hexafluoroisopropanol (2 mL), and the reaction mixture was reacted in a shaker at 25 °C for 0.5 h. After the reaction was completed, the mixture was filtered, and the filtrate was concentrated under reduced pressure to remove the solvent. The residue was purified by preparative high performance liquid chromatography (C18 column, 5 µm particle size, 30 mm diameter, 150 mm length; a mixture of water (containing 0.225% formic acid) and acetonitrile with decreasing polarity used as the eluent; acetonitrile gradient: 25%-45%, elution time: 22 min) to give the title compound (34 mg).

MS m/z (ESI): 1221.4 (M+H)⁺.

### Step 4: synthesis of N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-6-(2-(cyclopentylsulfonyl)-5-oxo-8,8-di(2,5,8,11-tetraoxatridecan-13-yl)-7,8-dihydropyrido[4,3-d]pyrimidin-6(5H)-yl)hexanamide (b-L13-1)

**Intermediate L13-3** (34 mg) and **intermediate L1-7** (12.38 mg) were dissolved in *N*,*N*-dimethylformamide (0.5 mL), and 1-(3-dimethylaminopropyl)-3-ethylcarbodiimide hydrochloride (10.67 mg), 1-hydroxybenzotriazole (7.52 mg), and pyridine (6.61 mg) were sequentially added. The mixture was purged 3 times with nitrogen. The reaction mixture was stirred at 25 °C for reaction for 1 h under a nitrogen atmosphere. After the reaction was completed, the reaction mixture was directly purified by high performance liquid chromatography (C18 column, 5 µm particle size, 30 mm diameter, 150 mm length; a mixture of water (containing 0.225% formic acid) and acetonitrile with decreasing polarity used as the eluent; acetonitrile gradient: 32%-52%, elution time: 22 min) to give the title compound (26.8 mg).

MS m/z (ESI): 814.1 [(M+2H)/2]⁺.

**¹H NMR (400 MHz, DMSO-d₆)** δ = 9.24 (s, 1H), 8.71-8.64 (m, 1H), 8.62-8.57 (m, 1H), 8.33-8.25 (m, 1H), 8.12 (d, J = 7.9 Hz, 1H), 8.09-8.04 (m, 1H), 8.04-7.98 (m, 1H), 7.80 (s, 1H), 7.52 (s, 1H), 7.26-7.23 (m, 5H), 7.19-7.12 (m, 1H), 6.49 (s, 1H), 5.44 (d, J = 8.3 Hz, 4H), 4.84-4.71 (m, 2H), 4.70-4.63 (m, 1H), 4.54-4.45 (m, 1H), 4.44-4.37 (m, 1H), 4.31-4.21 (m, 1H), 3.80-3.63 (m, 8H), 3.63-3.57 (m, 1H), 3.53-3.36 (m, 30H), 3.22 (s, 6H), 3.09-3.00 (m, 1H), 2.84-2.75 (m, 1H), 2.13 (d, J = 7.5 Hz, 2H), 2.11-1.93 (m, 8H), 1.90 (s, 2H), 1.68 (m, 4H), 1.55 (d, J = 7.7 Hz, 4H), 1.33-1.25 (m, 2H), 1.01-0.93 (m, 1H), 0.90-0.85 (m, 3H), 0.39-0.28 (m, 4H)

### Example 35. N-((4S,12S,21S)-12-Benzyl-21-(3-(2'-(cyclopentylsulfonyl)-5'-oxo-5'H-spiro[cyclopenta-1,8'-pyrido[4,3-d]pyrimidin]-6'(7'H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (b-L14-1)

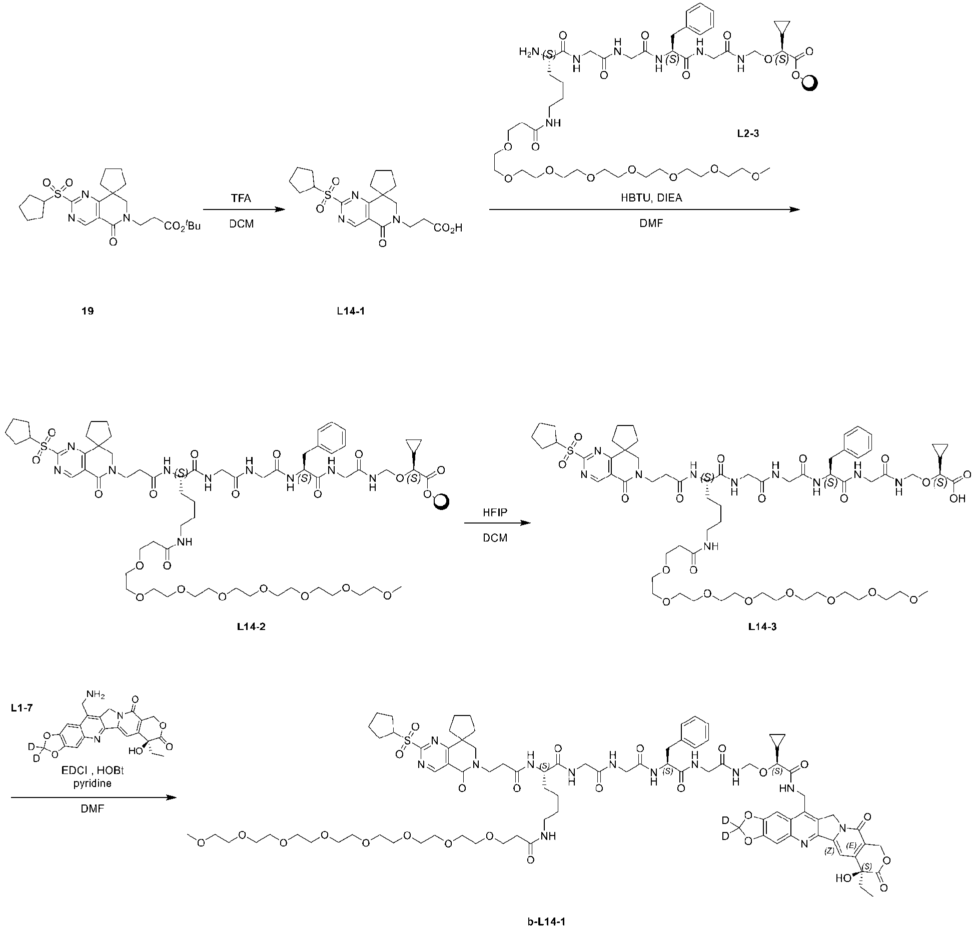

### Step 1: synthesis of 3-(2'-(cyclopentylsulfonyl)-5'-oxo-5'H-spiro[cyclopentane-1,8'-pyrido[4,3-d]pyrimidin]-6'(7'H)-yl)propionic acid (intermediate L14-1)

**Compound 19** (630 mg) was dissolved in dichloromethane (3 mL), and trifluoroacetic acid (0.6 mL) was added. The reaction mixture was stirred at 25 °C for 1 h. After the reaction was completed, the mixture was concentrated under reduced pressure, and the residue was slurried with petroleum ether (1 mL) and ethyl acetate (1 mL). The slurry was dried under vacuum to give the title compound (380 mg).

MS m/z (ESI): 408.2 [M+H]⁺.

### Step 2: synthesis of resin-supported (32S,41S,49S)-41-benzyl-32-(3-(2'-cyclopentylsulfonyl-5'-oxo-5'H-spiro[cyclopentane-1,8'-pyrido[4,3-d]pyrimidin]-6'(7'H)-yl)propanamido)-49-cyclopropyl-26,33,36,39,42,45-hexaoxo-2,5,8,11,14,17,20,23,48-nonaoxa-27,34,37,40,43,46-hexaazapentacontane-50-carboxylic acid (intermediate L14-2)

**Intermediate L14-1** was dissolved in *N*,*N*-dimethylformamide (3 mL), and **intermediate L2-3** (195.59 mg), O-benzotriazole-tetramethyluronium hexafluorophosphate (243.72 mg), and N,N-diisopropylethylamine (250.82 µL) were sequentially added. The reaction mixture was shaken on a shaker at 25 °C for 1 h. After the reaction was completed, the resin was washed sequentially with methanol (5 mL) and dichloromethane (5 mL). This process was repeated 3 times. The resin was then filtered under vacuum until it was dry, and the filter cake was dried under vacuum to give the title compound (430 mg).

### Step 3: synthesis of (32S,41S,49S)-41-benzyl-32-(3-(2'-cyclopentylsulfonyl-5'-oxo-5'H-spiro[cyclopentane-1,8'-pyrido[4,3-d]pyrimidin]-6'(7'H)-yl)propanamido)-49-cyclopropyl-26,33,36,39,42,45-hexaoxo-2,5,8,11,14,17,20,23,48-nonaoxa-27,34,37,40,43,46-hexaazapentacontane-50-carboxylic acid (intermediate L14-3)

**Intermediate L14-2** (430 mg) was added to a mixed solvent of dichloromethane (4 mL) and 1,1,1,3,3,3-hexafluoroisopropanol (1 mL), and the reaction mixture was shaken on a shaker at 25 °C for 1 h. After the reaction was completed, the reaction mixture was filtered to remove the resin. The filtrate was concentrated under reduced pressure, and the residue was purified by high performance liquid chromatography (column: Welch Xtimate C18 150 × 30 mm × 5 µm; mobile phase: [A: water (0.225% formic acid), B: acetonitrile]; B%: 25%-45%, 15 min) to give the title compound (17 mg).

### Step 4: synthesis of N-((4S,12S,21S)-12-benzyl-21-(3-(2'-cyclopentylsulfonyl-5'-oxo-5'H-spiro[cyclopentane-1,8'-pyrido[4,3-d]pyrimidin]-6'(7'H)-yl)propionylamino)-4-cyclopropyl-1-((S)-7-ethyl-7-hydroxy-8,11-dioxo-7,8,11,13-tetrahydro-10H-[1,3]dioxadideuteriopentacyclo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (b-L14-1)

**Intermediate L14-3** (17 mg), **intermediate L1-7** (5.76 mg), 1-(3-dimethylaminopropyl)-3-ethylcarbodiimide hydrochloride (4.74 mg), pyridine (8.56 mg), and 1-hydroxybenzotriazole (3.34 mg) were dissolved in *N,N-*dimethylformamide (0.5 mL), and the reaction mixture was purged 3 times with nitrogen and stirred at 25 °C for reaction for 2 h under a nitrogen atmosphere. After the reaction was completed, the reaction mixture was directly purified by high performance liquid chromatography (column: Welch Xtimate C18 150 × 30 mm × 5 µm; mobile phase: [A: water (0.225% formic acid), B: acetonitrile]; B%: 32%-52%, 22 min) to give the title compound (9.8 mg).

MS m/z (ESI): 891.2 [M/2+H]⁺.

**¹H NMR (400 MHz, DMSO-d₆)** δ = 9.23 (s, 1H), 8.73-8.55 (m, 2H), 8.36-8.29 (m, 1H), 8.25-8.16 (m, 2H), 8.13 (d, J = 7.9 Hz, 1H), 7.99 (t, J = 5.1 Hz, 1H), 7.81-7.74 (m, 2H), 7.50 (s, 1H), 7.25 (s, 1H), 7.23-7.19 (m, 4H), 7.18-7.12 (m, 1H), 6.49 (s, 1H), 5.42 (s, 4H), 4.84-4.71 (m, 2H), 4.66 (m, J = 6.7, 9.8 Hz, 1H), 4.55-4.45 (m, 1H), 4.39 (m, J = 6.5, 10.1 Hz, 1H), 4.31-4.16 (m, 2H), 3.70-3.66 (m, 7H), 3.60-3.53 (m, 5H), 3.51-3.47 (m, 25H), 3.40 (s, 3H), 3.23 (s, 3H), 3.08-2.90 (m, 4H), 2.78 (m, 1H), 2.27 (t, J = 6.5 Hz, 2H), 2.07-1.91 (m, 7H), 1.90-1.82 (m, 2H), 1.76 (s, 6H), 1.71-1.57 (m, 6H), 1.54-1.43 (m, 1H), 1.36-1.30 (m, 2H), 1.28-1.23 (m, 2H), 1.00-0.92 (m, 1H), 0.87 (t, J = 7.3 Hz, 3H), 0.38-0.34 (m, 2H), 0.31 (d, J = 4.5 Hz, 2H).

### Example 36. (S)-N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-19-(3-(2-cyclopentylsulfonyl-5-oxo-7,8-dihydropyrido[4,3-d]pyrimidin]-6(5H)-yl)propanamido)-14-(2,5,8,11-tetraoxatridecan-13-yl)-2,5,8,11-tetraoxa-14-azaeicosan-20-amide (b-L15-1)

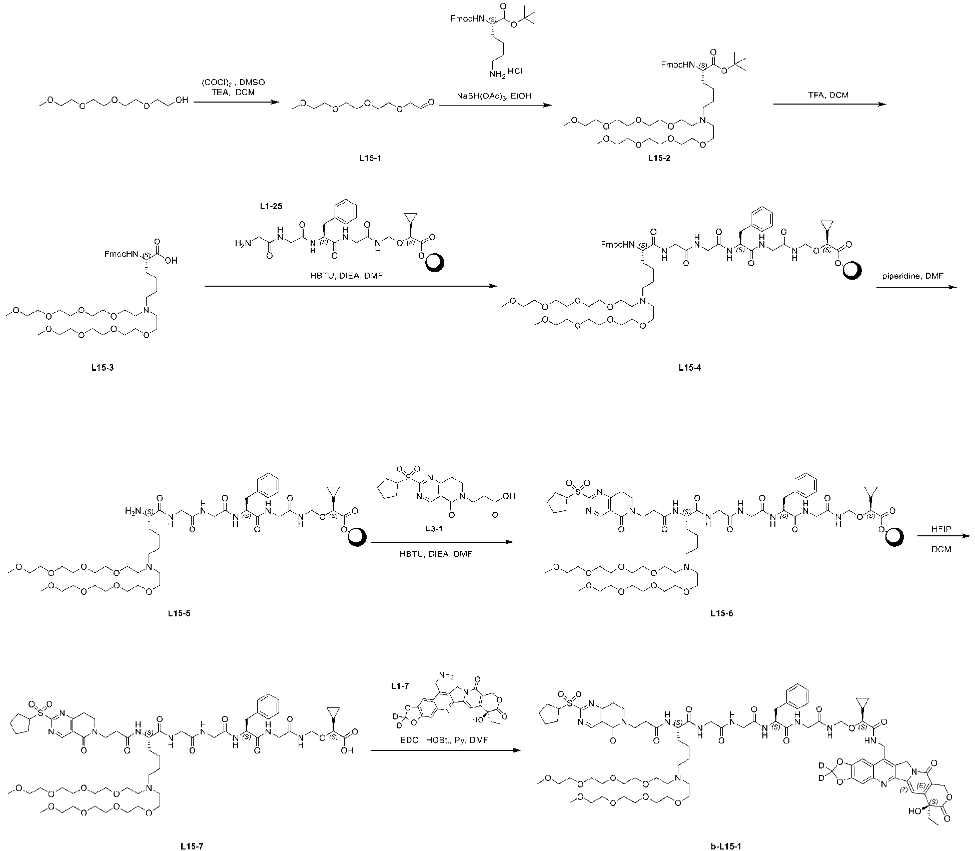

### Step 1: synthesis of 2,5,8,11-tetraoxatridecan-13-al (intermediate L15-1)

Dimethyl sulfoxide (1.02 mL) was added dropwise and slowly to a solution of oxalyl chloride (914 mg) in dichloromethane (25 mL) at -70 °C under a nitrogen atmosphere, and the reaction mixture was stirred at -70 °C for 10 min. A solution of 2,5,8,11-tetraoxatridecan-13-ol (1.00 g) in dichloromethane (5 mL) was added dropwise at - 70 °C, and the reaction mixture was stirred at -70 °C for 10 min. Triethylamine (4.02 mL) was added dropwise at - 70 °C, and the reaction mixture was warmed to room temperature and stirred at 20 °C for reaction for 0.5 h. After the reaction was completed, a solution of the title compound in dichloromethane (30 mL) was obtained, and the solution was filtered and then directly used in the next step.

### Step 2: synthesis of tert-butyl (S)-19-((((9H-fluoren-9-yl)methoxy)carbonyl)amino)-14-(2,5,8,11-tetraoxatridecan-13-yl)-2,5,8,11-tetraoxa-14-azaeicosane-20-carboxylate (intermediate L15-2)

Sodium triacetoxyborohydride (4.07 g) was added to a solution of Fmoc-lysine *tert*-butyl ester hydrochloride (407.58 mg) in ethanol (20 mL) at room temperature under a nitrogen atmosphere, and the solution of **intermediate L15-1** in dichloromethane (30 mL) obtained in the previous step was added dropwise. The resulting mixture was stirred at 20 °C for reaction for 3 h. After the reaction was completed, the mixture was concentrated under reduced pressure to remove the solvent. The residue was purified by flash silica gel column chromatography (ISCO^{®}; 20 g of SepaFlash^{®} flash silica gel column, gradient 0-15% methanol/dichloromethane, flow rate: 60 mL/min) to give the title compound (1.00 g).

LC-MS: m/z (ESI): 805.6 [M+H]⁺.

### Step 3: synthesis of (S)-19-((((9H-fluoren-9-yl)methoxy)carbonyl)amino)-14-(2,5,8,11-tetraoxatridecan-13-yl)-2,5,8,11-tetraoxa-14-azaeicosane-20-carboxylic acid (intermediate L15-3)

**Intermediate L15-2** (1.00 g) was dissolved in dichloromethane (5 mL) under a nitrogen atmosphere, and trifluoroacetic acid (5 mL) was added at room temperature. The reaction mixture was reacted at 20 °C for 2 h. After the reaction was completed, the mixture was concentrated under reduced pressure to remove the solvent. The residue was purified by flash silica gel column chromatography (ISCO^{®}; 12 g of SepaFlash^{®} flash silica gel column, gradient 0-15% methanol/dichloromethane, flow rate: 60 mL/min) to give the title compound (330 mg).

LC-MS: m/z (ESI): 749.4 [M+H]⁺.

### Step 4: synthesis of resin-supported (19S,28S,36S)-19-((((9H-fluoren-9-yl)methoxy)carbonyl)amino)-28-benzyl-36-cyclopropyl-20,23,26,29,32-pentaoxo-14-(2,5,8,11-tetraoxatridecan-13-yl)-2,5,8,11,35-pentaoxa-14,21,24,27,30,33-hexaazaheptatriacontane-37-carboxylic acid (intermediate L15-4)

**Intermediate L15-3** (301.44 mg) and **intermediate L1-25** (400 mg) were dissolved in anhydrous *N,N-*dimethylformamide (8 mL), and *O*-benzotriazole-tetramethyluronium hexafluorophosphate (88.86 mg) and *N,N-*diisopropylethylamine (113.09 mg) were sequentially added. The reaction mixture was reacted in a shaker at 25 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (500 mg).

LC-MS: m/z (ESI): 1194.6 [M+H]⁺.

### Step 5: synthesis of resin-supported (19S,28S,36S)-19-amino-28-benzyl-36-cyclopropyl-20,23,26,29,32-pentaoxo-14-(2,5,8,11-tetraoxatridecan-13-yl)-2,5,8,11,35-pentaoxa-14,21,24,27,30,33-hexaazaheptatriacontane-37-carboxylic acid (intermediate L15-5)

**Intermediate L15-4** (0.55 g) was dissolved in anhydrous N,N-dimethylformamide (2.5 mL), and piperidine (0.5 mL) was added. The reaction mixture was reacted in a shaker at 25 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (440 mg).

LC-MS: m/z (ESI): 972.6 [M+H]⁺.

### Step 6: synthesis of resin-supported (19S,28S,36S)-28-benzyl-19-(3-(2-(cyclopentylsulfonyl)-5-oxo-7,8-dihydropyrido[4,3-d]pyrimidin]-6(5H)-yl)propanamido)-36-cyclopropyl-20,23,26,29,32-pentaoxo-14-(2,5,8,11-tetraoxatridecan-13-yl)-2,5,8,11,35-pentaoxa-14,21,24,27,30,33-hexaazaheptatriacontane-37-carboxylic acid (intermediate L15-6)

**Intermediate L15-5** (400 mg) and **intermediate L3-1** (100.74 mg) were dissolved in anhydrous *N,N-*dimethylformamide (8 mL), and *O*-benzotriazole-tetramethyluronium hexafluorophosphate (48.25 mg) and *N,N-*diisopropylethylamine (122.80 mg) were sequentially added. The reaction mixture was reacted in a shaker at 25 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (458 mg).

LC-MS: m/z (ESI): 1307.5 [M+H]⁺.

### Step 7: synthesis of (19S,28S,36S)-28-benzyl-19-(3-(2-cyclopentylsulfonyl)-5-oxo-7,8-dihydropyrido[4,3-d]pyrimidin]-6(5H)-yl)propanamido)-36-cyclopropyl-20,23,26,29,32-pentaoxo-14-(2,5,8,11-tetraoxatridecan-13-yl)-2,5,8,11,35-pentaoxa-14,21,24,27,30,33-hexaazaheptatriacontane-37-carboxylic acid (intermediate L15-7)

**Intermediate L15-6** (458.00 mg) was dissolved in anhydrous dichloromethane (4 mL), and 1,1,1,3,3,3-hexafluoroisopropanol (1 mL) was added. The reaction mixture was stirred at 25 °C for reaction for 1 h. After the reaction was completed, the mixture was filtered and washed three times with dichloromethane (10 mL). The filtrate was concentrated under reduced pressure, and the residue was purified by preparative high performance liquid chromatography (C18 column, 5 µm particle size, 30 mm diameter, 150 mm length; a mixture of water (containing 0.225% formic acid) and acetonitrile with decreasing polarity used as the eluent; acetonitrile gradient: 20%-37%, elution time: 19 min) to give the title compound (25 mg).

LC-MS: m/z (ESI): 1307.5 [M+H]⁺.

### Step 8: synthesis of (S)-N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-19-(3-(2-cyclopentylsulfonyl-5-oxo-7,8-dihydropyrido[4,3-d]pyrimidin]-6(5H)-yl)propanamido)-14-(2,5,8,11-tetraoxatridecan-13-yl)-2,5,8,11-tetraoxa-14-azaeicosan-20-amide (b-L15-1)

**Intermediate L15-7** (20.00 mg) and **intermediate L1-7** (14.25 mg) were dissolved in anhydrous *N,N-*dimethylformamide (0.8 mL), and 1-(3-dimethylaminopropyl)-3-ethylcarbodiimide hydrochloride (11.73 mg), 1-hydroxybenzotriazole (8.27 mg), and pyridine (7.26 mg) were sequentially added. The mixture was purged 3 times with nitrogen. The reaction mixture was stirred at 25 °C for reaction for 1 h under a nitrogen atmosphere. After the reaction was completed, the reaction mixture was directly purified by high performance liquid chromatography (C18 column, 5 µm particle size, 30 mm diameter, 150 mm length; a mixture of water (containing 0.225% formic acid) and acetonitrile with decreasing polarity used as the eluent; acetonitrile gradient: 28%-38.5%, elution time: 15 min) to give the title compound (7.8 mg).

LC-MS: m/z (ESI): 857.4 [(M+2H)/2]⁺.

**¹H NMR (400 MHz, DMSO-d6)** δ = 9.29-9.17 (m, 1H), 8.73-8.59 (m, 2H), 8.34 (s, 2H), 8.30-8.18 (m, 2H), 8.17-8.10 (m, 1H), 8.04-7.96 (m, 1H), 7.82-7.75 (m, 1H), 7.54-7.46 (m, 1H), 7.29-7.18 (m, 5H), 7.18-7.12 (m, 1H), 6.68-6.41 (m, 1H), 5.46-5.40 (m, 3H), 4.87-4.71 (m, 2H), 4.70-4.62 (m, 1H), 4.56-4.34 (m, 2H), 4.29-4.14 (m, 2H), 3.78-3.60 (m, 11H), 3.53-3.44 (m, 32H), 3.23-3.21 (m, 6H), 3.07-3.00 (m, 1H), 2.84-2.74 (m, 1H), 2.58-2.53 (m, 4H), 2.40-2.32 (m, 2H), 1.80 (m, 6H), 1.75-1.56 (m, 5H), 1.53-1.39 (m, 1H), 1.37-1.13 (m, 4H), 1.00-0.93 (m, 1H), 0.91-0.83 (m, 3H), 0.41-0.26 (m, 4H)

### Example 37. (S)-N⁴-(26-Amino-3,6,9,12,15,18,21,24-octamethyl-2,5,8,11,14,17,20,23,26-nonaoxo-3,6,9,12,15,18,21,24-octaazahexacosan-1-yl)-N¹-((4S,12S)-12-benzyl-4-cyclopropyl-1-((S)-7-ethyl-7-hydroxy-8,11-dioxo-7,8,11,13-tetrahydro-10H-[1,3]dioxadideuteriopentacyclo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-2-(3-(2-(cyclopentylsulfonyl)-5-oxo-7,8-dihydropyrido[4,3-d]pyrimidin-6(5H)-yl)propanamido)-N⁴-methylbutanediamide (b-L12-1)

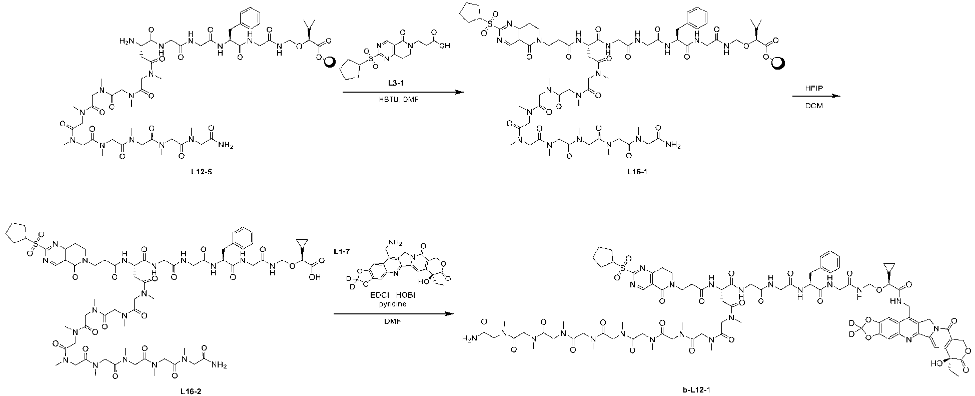

### Step 1: synthesis of resin-supported (2S,10S,19S)-48-amino-10-benzyl-19-(3-(2-(cyclopentylsulfonyl)-5-oxo-7,8-dihydropyrido[4,3-d]pyrimidin-6(5H)-yl)propanamido)-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontane-1-carboxylic acid (intermediate L16-1)

**Intermediate L12-5** (535 mg) and **intermediate L3-1** (187.41 mg) were dissolved in *N,N-*dimethylformamide (10 mL), and *O*-benzotriazole-tetramethyluronium hexafluorophosphate (270 mg) and diisopropylethylamine (309 µL) were added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (550 mg).

### Step 2: synthesis of (2S,10S,19S)-48-amino-10-benzyl-19-(3-(2-(cyclopentylsulfonyl)-5-oxo-7,8-dihydropyrido[4,3-d]pyrimidin-6(5H)-yl)propanamido)-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontane-1-carboxylic acid (intermediate L16-2)

**Intermediate L16-1** (550 mg) was added to a mixed solvent of dichloromethane (8 mL) and 1,1,1,3,3,3-hexafluoroisopropanol (2 mL), and the resulting mixture was shaken at 25 °C for 0.5 h. After the reaction was completed, the reaction mixture was filtered to remove the resin. The filtrate was concentrated to dryness under reduced pressure, and the residue was directly purified by high performance liquid chromatography (column: Welch Xtimate C18 150 × 30 mm × 5 µm; mobile phase: [A: water (0.225% formic acid), B: acetonitrile]; B%: 18%-38%, 20 min) to give the title compound (35 mg).

### Step 3: synthesis of (S)-N⁴-(26-amino-3,6,9,12,15,18,21,24-octamethyl-2,5,8,11,14,17,20,23,26-nonaoxo-3,6,9,12,15,18,21,24-octaazahexacosan-1-yl)-N¹-((4S,12S)-12-benzyl-4-cyclopropyl-1-((S)-7-ethyl-7-hydroxy-8,11-dioxo-7,8,11,13-tetrahydro-10H-[1,3]dioxadideuteriopentacyclo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-2-(3-(2-(cyclopentylsulfonyl)-5-oxo-7,8-dihydropyrido[4,3-d]pyrimidin-6(5H)-yl)propanamido)-N⁴-methylbutanediamide (b-L12-1)

Intermediate L16-2 (35 mg), intermediate L1-7 (9.54 mg), 1-(3-dimethylaminopropyl)-3-ethylcarbodiimide hydrochloride (8.64 mg), pyridine (5.35 mg), and 1-hydroxybenzotriazole (6.09 mg) were dissolved in *N,N-*dimethylformamide (1 mL), and the reaction mixture was purged 3 times with nitrogen and stirred at 25 °C for reaction for 2 h under a nitrogen atmosphere. After the reaction was completed, the reaction mixture was directly purified by high performance liquid chromatography (column: Welch Xtimate C18 150 × 30 mm × 5 µm; mobile phase: [A: water (0.225% formic acid), B: acetonitrile]; B%: 25%-45%, 20 min) to give the title compound (8.2 mg).

**¹H NMR (400 MHz, DMSO-d₆)** δ = 9.22 (s, 1H), 8.72-8.64 (m, 1H), 8.59 (d, J = 4.9 Hz, 1H), 8.35-8.21 (m, 2H), 8.09-7.91 (m, 2H), 7.80 (s, 1H), 7.52 (s, 1H), 7.35-7.19 (m, 5H), 7.18-7.15 (m, 1H), 7.11-7.04 (m, 1H), 6.53-6.49 (m, 1H), 5.44 (d, J = 9.8 Hz, 3H), 4.83-4.30 (m, 9H), 4.28-3.82 (m, 13H), 3.78-3.48 (m, 18H), 3.08-2.64 (m, 30H), 2.04-1.80 (m, 6H), 1.73-1.56 (m, 4H), 1.53-1.39 (m, 1H), 1.24 (s, 3H), 1.01-0.92 (m, 1H), 0.87 (t, J = 7.2 Hz, 3H), 0.43-0.26 (m, 4H)

MS m/z (ESI): 979.6 [(M+2H)/2]⁺.

### Example 38. N-((4S,12S,21S)-12-Benzyl-21-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (b-L17-1)

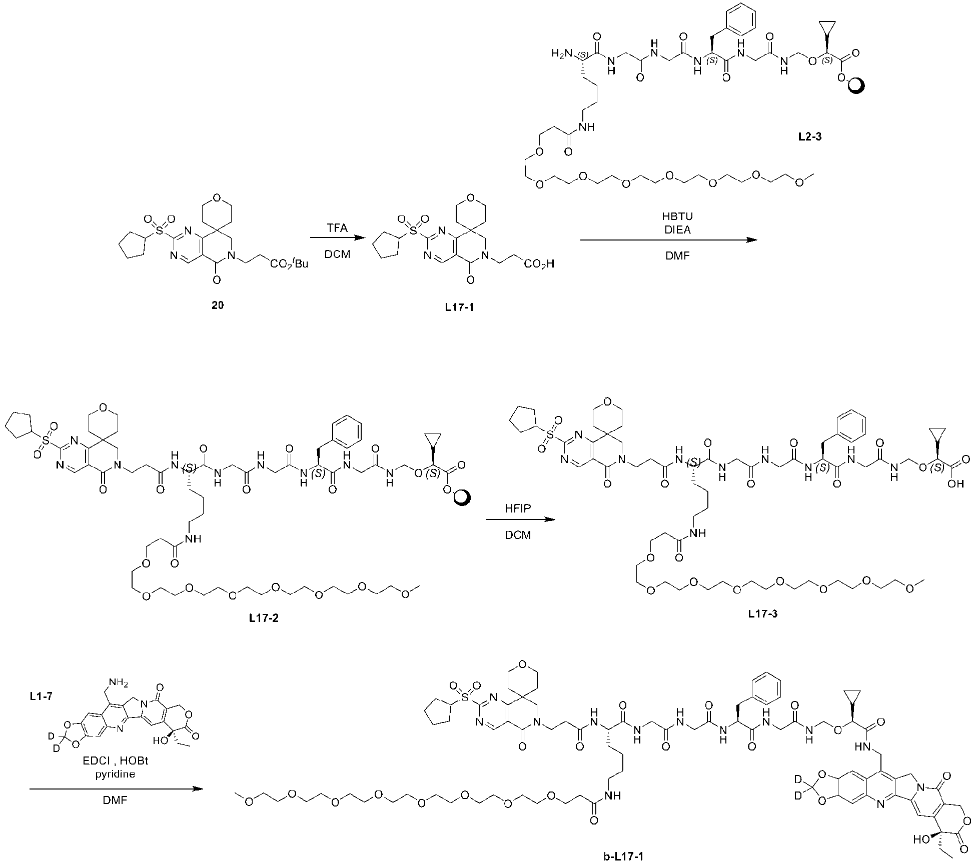

### Step 1: synthesis of 3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propionic acid (intermediate L17-1)

**Compound 20** (680 mg) was dissolved in dichloromethane (10 mL), and trifluoroacetic acid (0.68 mL) was added to the reaction mixture. The reaction mixture was stirred at 25 °C for reaction for 1 h. After the reaction was completed, the mixture was directly concentrated under reduced pressure to remove the solvent. The residue was purified by flash silica gel column chromatography (ISCO^{®}; 12 g of SepaFlash^{®} flash silica gel column, gradient 0-53% tetrahydrofuran/petroleum ether, flow rate: 40 mL/min) to give the title compound (200 mg).

MS m/z (ESI): 423.8 (M+H)⁺

### Step 2: synthesis of resin-supported (32S,41S,49S)-41-benzyl-32-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-49-cyclopropyl-26,33,36,39,42,45-hexaoxo-2,5,8,11,14,17,20,23,48-nonaoxa-27,34,37,40,43,46-hexaazapentacontane-50-carboxylic acid (intermediate L17-2)

**Intermediate L2-3** (400 mg) and **intermediate L17-1** (106.13 mg) were dissolved in *N,N-*dimethylformamide (10 mL). O-Benzotriazole-tetramethyluronium hexafluorophosphate (159.06 mg) and *N*,*N*-diisopropylethylamine (121.46 mg) were sequentially added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (420 mg).

MS m/z (ESI): 1391.5 [M+H]⁺.

### Step 3: synthesis of (32S,41S,49S)-41-benzyl-32-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-49-cyclopropyl-26,33,36,39,42,45-hexaoxo-2,5,8,11,14,17,20,23,48-nonaoxa-27,34,37,40,43,46-hexaazapentacontane-50-carboxylic acid

### (intermediate L17-3)

**Intermediate L17-2** (400 mg) was dissolved in dichloromethane (8 mL) and 1,1,1,3,3,3-hexafluoroisopropanol (2 mL), and the reaction mixture was reacted in a shaker at 25 °C for 0.25 h. After the reaction was completed, the reaction mixture was filtered and concentrated under reduced pressure to remove the solvent. The residue was purified by preparative high performance liquid chromatography (C18 column, 5 µm particle size, 30 mm diameter, 150 mm length; a mixture of water (containing 0.225% formic acid) and acetonitrile with decreasing polarity used as the eluent; acetonitrile gradient: 20%-40%, elution time: 22 min) to give the title compound (15 mg).

MS m/z (ESI): 1413.4 [M+Na]⁺.

### Step 4: synthesis of N-((4S,12S,21S)-12-benzyl-21-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17,20-hexaoxo-5-oxa-2,7,10,13,16,19-hexaazapentacosan-25-yl)-2,5,8,11,14,17,20,23-octaoxahexacosan-26-amide (b-L17-1)

**Intermediate L17-3** (15 mg) and **intermediate L1-7** (4.79 mg) were dissolved in N,N-dimethylformamide (0.5 mL), and 1-(3-dimethylaminopropyl)-3-ethylcarbodiimide hydrochloride (4.13 mg), 1-hydroxybenzotriazole (2.91 mg), and pyridine (2.56 mg) were sequentially added. The mixture was purged 3 times with nitrogen. The reaction mixture was stirred at 25 °C for reaction for 1 h under a nitrogen atmosphere. After the reaction was completed, the reaction mixture was directly purified by high performance liquid chromatography (C18 column, 5 µm particle size, 30 mm diameter, 150 mm length; a mixture of water (containing 0.225% formic acid) and acetonitrile with decreasing polarity used as the eluent; acetonitrile gradient: 28%-48%, elution time: 22 min) to give the title compound (9 mg).

MS m/z (ESI): 899.1 [(M+2H)/2]⁺.

**¹H NMR (400 MHz, DMSO-d₆)** δ = 9.28 (s, 1H), 8.66 (s, 1H), 8.62-8.56 (m, 1H), 8.30 (s, 1H), 8.21 (s, 1H), 8.17-8.13 (m, 1H), 8.13-8.08 (m, 1H), 8.01-7.95 (m, 1H), 7.82-7.75 (m, 2H), 7.53-7.50 (m, 1H), 7.26 (s, 1H), 7.24-7.21 (m, 4H), 7.19-7.13 (m, 1H), 6.48 (s, 1H), 5.44 (d, J = 8.9 Hz, 4H), 4.86-4.73 (m, 2H), 4.70-4.64 (m, 1H), 4.52-4.48 (m, 1H), 4.44-4.36 (m, 1H), 4.29 (s, 1H), 4.24-4.17 (m, 1H), 3.83-3.80 (m, 3H), 3.74-3.67 (m, 10H), 3.58-3.55 (m, 3H), 3.52-3.48 (m, 28H), 3.43-3.42 (m, 3H), 3.24 (s, 3H), 3.06-2.97 (m, 3H), 2.83-2.75 (m, 1H), 2.31-2.26 (m, 2H), 2.08-1.94 (m, 6H), 1.90-1.81 (m, 2H), 1.70-1.60 (m, 7H), 1.53-1.45 (m, 1H), 1.38-1.31 (m, 2H), 1.24 (s, 2H), 0.96 (s, 1H), 0.88 (t, J = 7.4 Hz, 3H), 0.36 (s, 4H)

### Example 39. N-((4S,12S)-12-Benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-6-(2'-(cyclopentylsulfonyl)-1-(2,5,8,11,14,17,20,23-octaoxa-26-oxohexacosan-26-yl)-5'-oxo-5'H-spiro[piperidine-4,8'-pyrido[4,3-d]pyrimidin]-6'(7'H)-yl)hexanamide (b-L18-1)

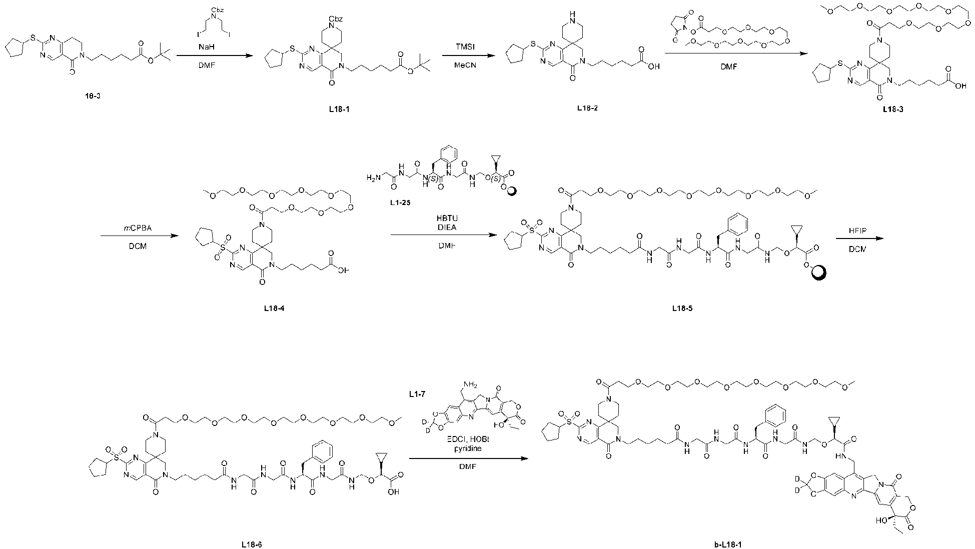

### Step 1: synthesis of benzyl 6'-(6-tert-butoxy-6-oxohexyl)-2'-(cyclopentylthio)-5'-oxo-6',7'-dihydro-5'H-spiro[piperidine-4,8'-pyrido[4,3-d]pyrimidine]-1-carboxylate (intermediate L18-1)

**Intermediate 18-3** (3 g) was dissolved in *N*,*N*-dimethylformamide (40 mL) at 0 °C under a nitrogen atmosphere, and NaH (1.72 g, 60% effective content) was added thereto. The reaction mixture was stirred at 0 °C for 15 min, and benzyl bis(2-iodoethyl)carbamate (9.85 g) was added thereto. The reaction mixture was stirred at 0 °C for 1 h under a nitrogen atmosphere. After the reaction was completed, the mixture was poured into 50 mL of ice water, and the resulting mixture was extracted with ethyl acetate (20 mL × 2). The organic phases were combined, dried over anhydrous sodium sulfate, and concentrated under reduced pressure, and the residue was separated by column chromatography (tetrahydrofuran/petroleum ether, 0-18%) to give the title compound (2.7 g).

LC-MS: m/z (ESI): 623.2 [M+H]⁺.

### Step 2: synthesis of 6-(2'-(cyclopentylthio)-5'-oxo-5'H-spiro[piperidine-4,8'-pyrido[4,3-d]pyrimidin]-6'(7'H)-yl)hexanoic acid (intermediate L18-2)

**Intermediate L18-1** (2.7 g) was dissolved in acetonitrile (5 mL), and trimethyliodosilane (1.23 mL) was added thereto. The reaction mixture was stirred at 25 °C for 0.5 h. The reaction mixture was purified by column chromatography (column: Spherical C18 20-35 µm 100 A 40 g; mobile phase: [A: water, B: acetonitrile]; B%: 10%-30%, 60 min) to give the title compound (1.4 g).

LC-MS: m/z (ESI): 433.3 [M+H]⁺.

### Step 3: synthesis of 6-(2'-(cyclopentylthio)-1-(2,5,8,11,14,17,20,23-octaoxa-26-oxohexacosan-26-yl)-5'-oxo-5'H-spiro[piperidine-4,8'-pyrido[4,3-d]pyrimidin]-6'(7'H)-yl)hexanoic acid (intermediate L18-3)

**Intermediate L18-2** (500 mg) and 2,5-dioxopyrrolidin-1-yl 2,5,8,11,14,17,20,23-octaoxahexacosan-26-oate (647.86 mg) were dissolved in anhydrous N,N-dimethylformamide (2 mL), N,N-diisopropylethylamine (302 µL) was added thereto, and the reaction mixture was stirred at 25 °C for 1 h. The reaction mixture was purified by column chromatography (column: Spherical C18 20-35 µm 100 A 40 g; mobile phase: [A: water, B: acetonitrile]; B%: 10%-30%, 60 min) to give the title compound (900 mg).

LC-MS: m/z (ESI): 827.6 [M+H]⁺.

### Step 4: synthesis of 6-(2'-(cyclopentylsulfonyl)-1-(2,5,8,11,14,17,20,23-octaoxa-26-oxohexacosan-26-yl)-5'-oxo-5'H-spiro[piperidine-4,8'-pyrido[4,3-d]pyrimidin]-6'(7'H)-yl)hexanoic acid (intermediate L18-4)

**Intermediate L18-3** (0.9 g) was dissolved in anhydrous dichloromethane (10 mL) at 0 °C, and m-chloroperoxybenzoic acid (375.58 mg) was added thereto. The reaction mixture was stirred at 25 °C for 1 h. After the reaction was completed, the mixture was concentrated at a low temperature under reduced pressure, and the residue was purified by column chromatography (column: Spherical C18 20-35 µm 100 A 40 g; mobile phase: [A: water, B: acetonitrile]; B%: 10%-24%, 60 min) to give the title compound (472 mg).

LC-MS: m/z (ESI): 859.5 [M+H]⁺.

### Step 5: synthesis of resin-supported (2S,10S)-10-benzyl-23-(2'-(cyclopentylsulfonyl)-1-(2,5,8,11,14,17,20,23-octaoxa-26-oxohexacosan-26-yl)-5'-oxo-5'H-spiro[piperidine-4,8'-pyrido[4,3-d]pyrimidin]-6'(7'H)-yl)-2-cyclopropyl-6,9,12,15,18-pentaoxo-3-oxa-5,8,11,14,17-pentaazatricosane-1-carboxylic acid (intermediate L18-5)

*N*,*N*-Dimethylformamide (6 mL) and N,N-diisopropylethylamine (213.38 µL) were added to a mixture of **intermediate L1-25** (308 mg), **intermediate L18-4** (466 mg), and *O*-(benzotriazol-1-yl)-*N*,*N*,*N*',*N*'-tetramethyluronium hexafluorophosphate (309.72 mg) under a nitrogen atmosphere, and the resulting mixture was shaken on a shaker at 20 °C for 1 h. After the reaction was completed, the resin was washed sequentially with methanol (20 mL) and dichloromethane (20 mL). This process was repeated 3 times. The resin was then filtered under vacuum until it was dry, and the filter cake was dried under vacuum to give the title compound (350 mg).

LC-MS: m/z (ESI): 653.0 [(M+2H)/2]⁺

### Step 6: synthesis of (2S,10S)-10-benzyl-23-(2'-(cyclopentylsulfonyl)-1-(2,5,8,11,14,17,20,23-octaoxa-26-oxohexacosan-26-yl)-5'-oxo-5'H-spiro[piperidine-4,8'-pyrido[4,3-d]pyrimidin]-6'(7'H)-yl)-2-cyclopropyl-6,9,12,15,18-pentaoxo-3-oxa-5,8,11,14,17-pentaazatricosane-1-carboxylic acid (intermediate L18-6)

**Intermediate L18-5** (350 mg) was dissolved in dichloromethane (10 mL) and 1,1,1,3,3,3-hexafluoroisopropanol (2.5 mL) under a nitrogen atmosphere. The resulting mixture was shaken on a shaker at 25 °C for 0.5 h. After the reaction was completed, the reaction mixture was filtered to remove the resin. The filtrate was concentrated to dryness under reduced pressure, and the residue was purified by high performance liquid chromatography (column: C18 Welch Xtimate C18 150 × 30 mm × 5 µm; mobile phase: [A: water (0.225% formic acid), B: acetonitrile]; B%: 22%-52%, 11 min) to give the title compound (10 mg).

LC-MS: m/z (ESI): 653.1 [(M+2H)/2]⁺

### Step 7: synthesis of N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-6-(2'-(cyclopentylsulfonyl)-1-(2,5,8,11,14,17,20,23-octaoxa-26-oxohexacosan-26-yl)-5'-oxo-5'H-spiro[piperidine-4,8'-pyrido[4,3-d]pyrimidin]-6'(7'H)-yl)hexanamide (b-L18-1)

*N*,*N*-Dimethylformamide (2 mL) was added to a mixture of **intermediate L18-6 (10 mg),** intermediate **L1-7 (3.25 mg),** 1-(3-dimethylaminopropyl)-3-ethylcarbodiimide hydrochloride (2.94 mg), 1-hydroxybenzotriazole (2.07 mg), and pyridine (1.85 µL) under a nitrogen atmosphere. The resulting mixture was stirred at room temperature for 1 h. After the starting materials were completely reacted as detected by LCMS, the reaction mixture was directly purified by high performance liquid chromatography (column: C18 Welch Xtimate C18 150 × 30 mm × 5 µm; mobile phase: [A: water (0.225% formic acid), B: acetonitrile]; B%: 28%-48%, 20 min) to give the title compound (7.3 mg).

LC-MS: m/z (ESI): 855.9 [(M+2H)/2]⁺

**¹H NMR (400 MHz, DMSO-d₆)** δ = 9.28 (s, 1H), 8.67 (s, 1H), 8.60 (s, 1H), 8.29 (s, 1H), 8.15-8.06 (m, 2H), 8.04-7.99 (m, 1H), 7.80 (s, 1H), 7.52 (s, 1H), 7.28-7.19 (m, 5H), 7.16 (s, 1H), 6.50 (s, 1H), 5.44 (d, J = 9.9 Hz, 4H), 4.77 (s, 2H), 4.71-4.63 (m, 1H), 4.49 (s, 1H), 4.39 (d, J = 7.0 Hz, 1H), 4.26 (t, J = 6.9 Hz, 1H), 3.95 (s, 1H), 3.77 (s, 3H), 3.71-3.61 (m, 6H), 3.54-3.45 (m, 32H), 3.44-3.40 (m, 6H), 3.04 (d, J = 10.1 Hz, 1H), 2.84-2.75 (m, 1H), 2.65-2.56 (m, 2H), 2.14 (t, J = 7.0 Hz, 2H), 2.03-1.83 (m, 8H), 1.73-1.50 (m, 10H), 1.26 (d, J = 11.8 Hz, 2H), 0.95 (d, J = 5.4 Hz, 1H), 0.87 (t, J = 7.2 Hz, 3H), 0.40-0.28 (m, 4H)

### Example 40. (S)-N⁴-(26-Amino-3,6,9,12,15,18,21,24-octamethyl-2,5,8,11,14,17,20,23,26-nonaoxo-3,6,9,12,15,18,21,24-octaazahexacosan-1-yl)-N¹-((4S,12S)-12-benzyl-4-cyclopropyl-1-((S)-7-ethyl-7-hydroxy-8,11-dioxo-7,8,11,13-tetrahydro-10H-[1,3]dioxadideuteriopentacyclo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-2-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-N⁴-methylbutanediamide (b-L19-1)

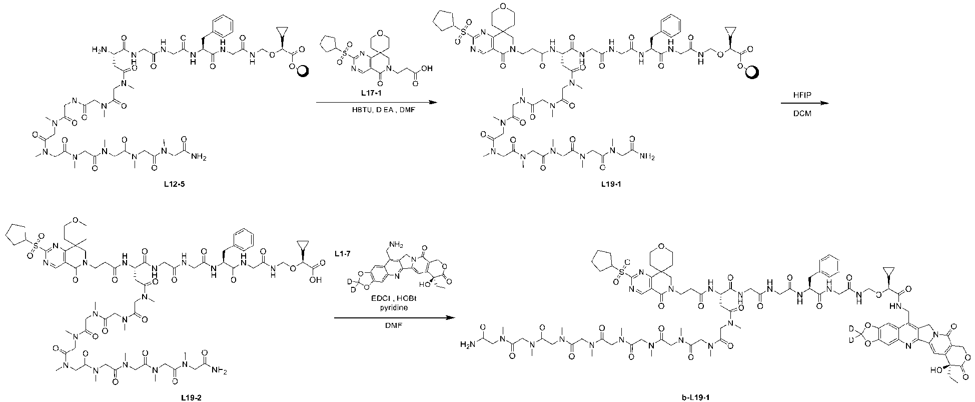

### Step 1: synthesis of resin-supported (2S,10S,19S)-48-amino-10-benzyl-19-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontane-1-carboxylic acid (intermediate L19-1)

**Intermediate L12-5** (880 mg) and **intermediate L17-1** (393.14 mg) were dissolved in *N*,*N*-dimethylformamide (15 mL), and O-benzotriazole-tetramethyluronium hexafluorophosphate (453.65 mg) and *N,N-*diisopropylethylamine (313.82 µL) were added. The reaction mixture was shaken on a shaker at 25 °C for 1 h. After the reaction was completed, the resin was washed sequentially with methanol (10 mL) and dichloromethane (10 mL). This process was repeated 3 times. The resin was then filtered under vacuum until it was dry, and the filter cake was dried under vacuum to give the title compound (920 mg).

### Step 2: synthesis of (2S,10S,19S)-48-amino-10-benzyl-19-(3-(2-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-2-cyclopropyl-22,25,28,31,34,37,40,43,46-nonamethyl-6,9,12,15,18,21,24,27,30,33,36,39,42,45,48-pentadecaoxo-3-oxa-5,8,11,14,17,22,25,28,31,34,37,40,43,46-tetradecaazaoctatetracontane-1-carboxylic acid (intermediate L19-2)

**Intermediate L19-1** (920 mg) was added to a mixed solvent of dichloromethane (8 mL) and 1,1,1,3,3,3-hexafluoroisopropanol (2 mL), and the resulting mixture was shaken at 25 °C for 0.5 h. After the reaction was completed, the reaction mixture was filtered to remove the resin. The filtrate was concentrated to dryness under reduced pressure, and the residue was directly purified by high performance liquid chromatography (column: Welch Xtimate C18 150 × 30 mm × 5 µm; mobile phase: [A phase: water (0.225% formic acid), B phase: acetonitrile]; B%: 18%-38%, 20 min) to give the title compound (65 mg).

### Step 3: synthesis of (S)-N⁴-(26-amino-3,6,9,12,15,18,21,24-octamethyl-2,5,8,11,14,17,20,23,26-nonaoxo-3,6,9,12,15,18,21,24-octaazahexacosane)-N¹-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)-3,8,11,14,17-pentaoxo-5-oxa-2,7,10,13,16-pentaazaoctadecan-18-yl)-2-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propionylamino)-N⁴-methylbutanediamide (b-L19-1)

**Intermediate L19-2** (65 mg), **intermediate L1-7** (22.10 mg), 1-(3-dimethylaminopropyl)-3-ethylcarbodiimide hydrochloride (15.36 mg), pyridine (9.51 mg), and 1-hydroxybenzotriazole (10.82 mg) were dissolved in *N,N-*dimethylformamide (3 mL), and the reaction mixture was purged 3 times with nitrogen and stirred at 25 °C for reaction for 2 h under a nitrogen atmosphere. After the reaction was completed, the reaction mixture was directly purified by high performance liquid chromatography (column: Welch Xtimate C18 150 × 30 mm × 5 µm; mobile phase: [A phase: water (0.225% formic acid), B phase: acetonitrile]; B%: 21%-51%, 11 min) to give the title compound (35 mg).

**¹H NMR (400 MHz, DMSO-d₆)** δ = 9.28 (s, 1H), 8.69-8.55 (m, 2H), 8.30-8.21 (m, 2H), 8.01 (m, 2H), 7.79 (s, 1H), 7.52 (s, 1H), 7.39-7.27 (m, 1H), 7.26-7.21 (m, 5H), 7.15 (s, 1H), 7.11-7.00 (m, 1H), 6.49 (s, 1H), 5.43 (d, J = 5.8 Hz, 4H), 4.86-4.70 (m, 2H), 4.70-4.63 (m, 1H), 4.62-4.53 (m, 1H), 4.52-4.44 (m, 1H), 4.40-4.17 (m, 10H), 4.12-3.89 (m, 9H), 3.86-3.76 (m, 5H), 3.75-3.65 (m, 9H), 3.63-3.52 (m, 2H), 3.51-3.43 (m, 2H), 3.00-2.68 (m, 32H), 2.07-1.92 (m, 6H), 1.91-1.80 (m, 2H), 1.71-1.59 (m, 6H), 1.01-0.92 (m, 1H), 0.88 (t, J = 7.3 Hz, 3H), 0.38-0.27 (m, 4H)

MS m/z (ESI): 1014.8 [(M+2)/2]⁺.

### Example 41. 4-((30S,33S,36S)-1-Amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-33-isopropyl-3,6,9,12,15,18,21,24,27,36-decamethyl-1,4,7,10,13,16,19,22,25,28,31,34-dodecaoxo-3,6,9,12,15,18,21,24,27,32,35-undecaazaheptatriacontan-37-methanamido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (Compound b-L22-3)

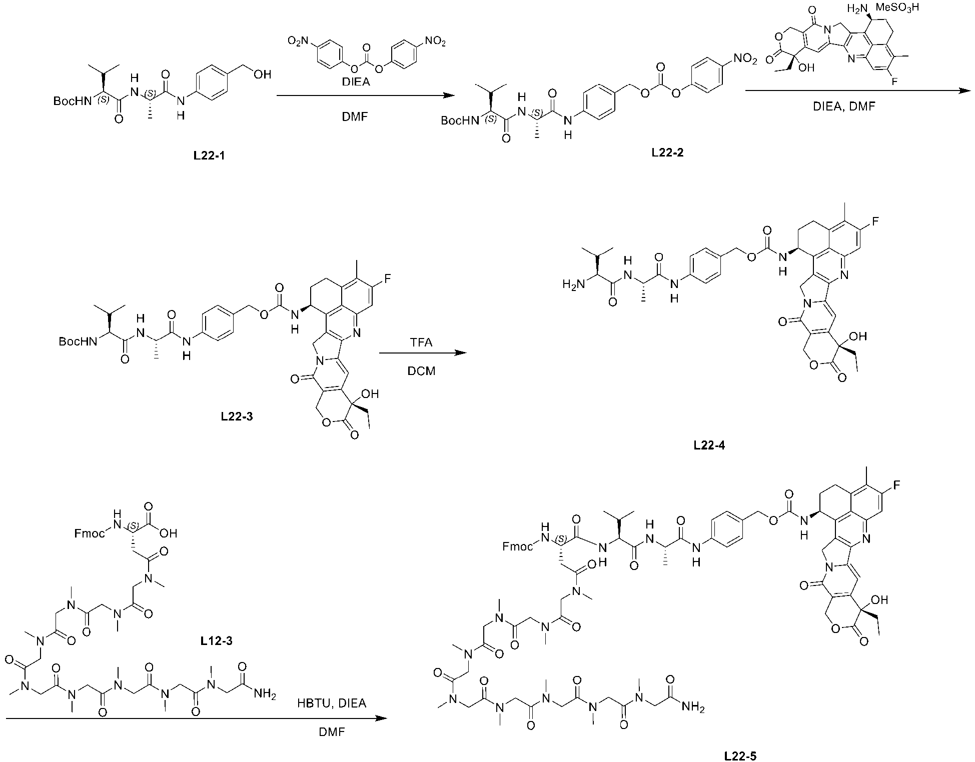

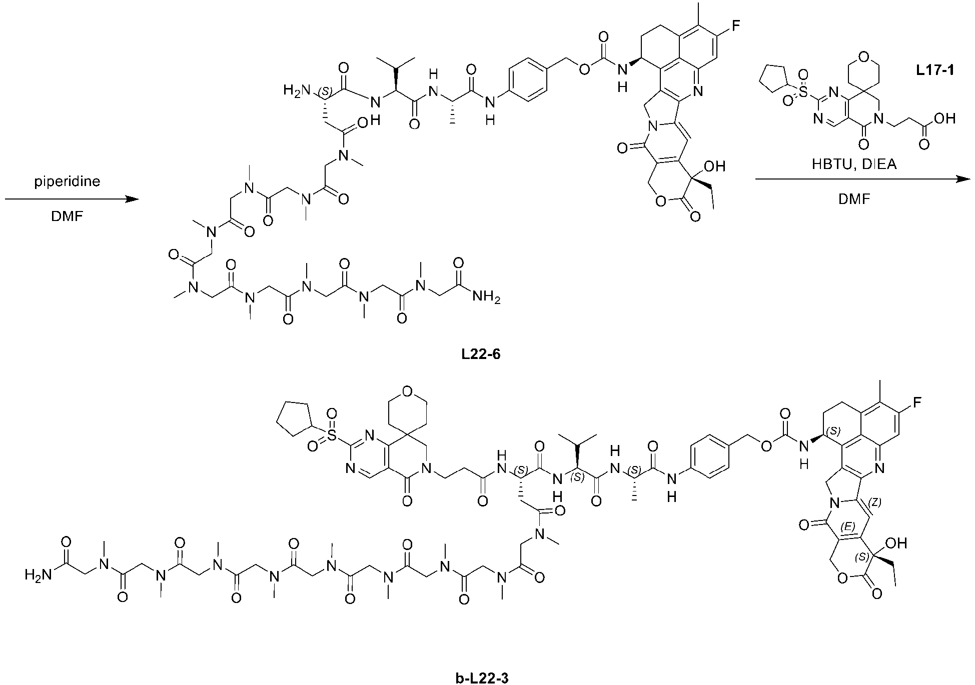

### Step 1: synthesis of tert-butyl ((S)-3-methyl-1-(((S)-1-((4-((((4-nitrophenoxy)carbonyl)oxy)methyl)phenyl) amino)-1-oxopropan-2-yl)amino)-1-oxobutan-2-yl)carbamate (intermediate L22-2)

**Intermediate L22-1** (1.9 g) and bis(4-nitrophenyl)carbonate (1.76 g) were dissolved in DMF (20 mL), DIEA (2.5 g) was added under a nitrogen atmosphere, and the reaction mixture was stirred at 25 °C for 3 h. The reaction mixture was then directly concentrated to remove the solvent, and the crude product was purified by a flash silica gel column (ISCO^{®}; 20 g of SepaFlash^{®} flash silica gel column, eluent: 0-45% tetrahydrofuran/petroleum ether, gradient @ 65 mL/min) to give the title compound (2.5 g).

MS m/z (ESI): 559.2 [M+H]⁺

### Step 2: synthesis of 4-((S)-2-((S)-2-((tert-butoxycarbonyl)amino)-3-methylbutanamido)propanamido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (intermediate L22-3)

**Intermediate L22-2** (1 g) and exatecan mesylate (999.20 mg) were dissolved in DMF (17 mL) and pyridine (3 mL), and HOBt (266.10 mg) and DIEA (277.65 mg, 374.19 µL) were added under a nitrogen atmosphere. The reaction mixture was stirred at 25 °C for 3 h. The reaction mixture was then directly purified by high performance liquid chromatography to give the title compound (1.1 g).

MS m/z (ESI): 855.2 [M+H]⁺.

### Step 3: synthesis of 4-((S)-2-((S)-2-amino-3-methylbutanamido)propanamido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (intermediate L22-4)

Trifluoroacetic acid (5 mL) was added to a solution of **intermediate L22-3 (**1.1 g) in dichloromethane (20 mL) at room temperature, and the reaction mixture was stirred at 25 °C for 1 h. The reaction mixture was then directly concentrated and dried under vacuum to give the title compound (0.9 g).

MS m/z (ESI): 755.5 [M+H]⁺

### Step 4: synthesis of 4-((30S,33S,36S)-30-((((9H-fluoren-9-yl)methoxy)carbonyl)amino)-1-amino-33-isopropyl-3,6,9,12,15,18,21,24,27,36-decamethyl-1,4,7,10,13,16,19,22,25,28,31,34-dodecaoxo-3,6,9,12,15,18,21,24,27,32,35-undecaazaheptatriacontan-37-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (intermediate L22-5)

**Intermediate L22-4** (135 mg) and **intermediate L12-3 (**97.79 mg) were dissolved in DMF (2 mL), HBTU (74.61 mg) and DIEA (38.14 mg) were added under a nitrogen atmosphere, and the reaction mixture was stirred at 25 °C for 3 h. The reaction mixture was then directly purified by a C18 reversed-phase column (acetonitrile:water = 0-50%) to give the title compound (120 mg).

MS m/z (ESI): 1732.1 [M+H]⁺

### Step 5: synthesis of 4-((30S,33S,36S)-1,30-diamino-33-isopropyl-3,6,9,12,15,18,21,24,27,36-decamethyl-1,4,7,10,13,16,19,22,25,28,31,34-dodecaoxo-3,6,9,12,15,18,21,24,27,32,35-undecaazaheptatriacontan-37-amino)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (intermediate L22-6)

**Intermediate L22-5** (60 mg) was dissolved in DMF (0.5 mL), piperidine (0.1 mL) was added under a nitrogen atmosphere, and the reaction mixture was stirred at 25 °C for 1 h. The reaction mixture was then directly purified by a C18 reversed-phase column (acetonitrile:water = 0-50%) to give the title compound (50 mg).

MS m/z (ESI): 1508.8 [M+H]⁺

### Step 6: synthesis of 4-((30S,33S,36S)-1-amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-33-isopropyl-3,6,9,12,15,18,21,24,27,36-decamethyl-1,4,7,10,13,16,19,22,25,28,31,34-dodecaoxo-3,6,9,12,15,18,21,24,27,32,35-undecaazaheptatriacontan-37-methanamido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (compound b-L22-3)

HBTU (20.11 mg) and DIEA (10.28 mg, 13.86 µL) were added to a solution of **intermediate L22-6 (**40 mg) and **intermediate L17-1 (**13.47 mg) in DMF (1 mL) at room temperature, and the reaction mixture was stirred at 25 °C for 1 h. The reaction mixture was then directly purified by high performance liquid chromatography (column: C18 150 × 30 mm × 5 µm; mobile phase: [A: water (0.225% formic acid), B: acetonitrile]; B%: 26%-56%, 20 min) to give the title compound (18.6 mg).

MS m/z (ESI): 1915.5 [M+H]⁺.

**¹H NMR (400 MHz, DMSO-d₆)** δ = 9.68-9.42 (m, 1H), 9.35-8.93 (m, 1H), 8.21-8.17 (m, 2H), 8.06-8.02 (m, 2H), 7.78 (d, *J* = 10.8 Hz, 2H), 7.66-7.62 (m, 3H), 7.37 (d, *J* = 8.7 Hz, 2H), 7.32 (s, 1H), 5.45 (s, 2H), 5.29 (s, 3H), 5.08 (s, 2H), 4.79-4.63 (m, 1H), 4.41-4.16 (m, 10H), 4.13-3.90 (m, 10H), 3.84 (s, 4H), 3.75-3.62 (m, 5H), 3.01-2.72 (m, 32H), 2.38 (s, 3H), 2.26-2.14 (m, 4H), 2.09-1.85 (m, 8H), 1.72-1.60 (m, 5H), 1.55-1.46 (m, 2H), 1.38-1.30 (m, 3H), 0.91-0.81 (m, 9H).

### Example 42. 4-((42S,45S,48S)-1-Amino-42-(3-(2'-(cyclopentylsulfonyl)-5'-oxo-2,3,5,6-tetrahydro-5'H-spiro[pyrano-4,8'-pyrido[4,3-d]pyrimidin]-6'(7'H)-yl)propanamido)-45-isopropyl-3,6,9,12,15,18,21,24,27,30,33,36,39,48-tetradecamethyl-1,4,7,10,13,16,19,22,25,28,31,34,37,40,43,46-hexadecaoxo-3,6,9,12,15,18,21,24,27,30,33,36,39,44,47-pentadecaazanonatetracontan-49-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (Compound b-L23-3)

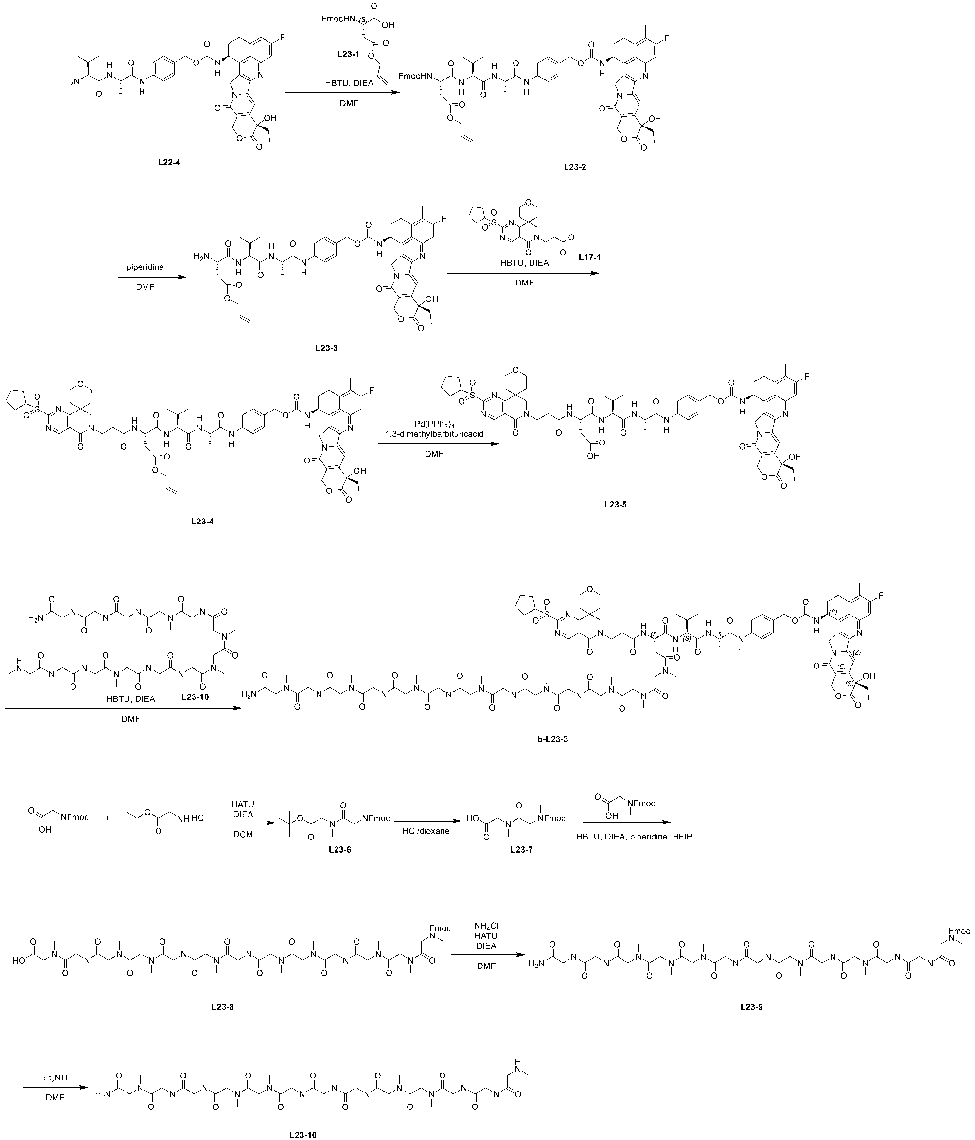

### Step 1: synthesis of allyl (S)-3-((((9H-fluoren-9-yl)methoxy)carbonyl)amino)-4-(((S)-1-(((S)-1-((4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenyl)amino)-1-oxopropan-2-yl)amino)-3-methyl-1-oxobutan-2-yl)amino)-4-oxobutanoate (intermediate L23-2)

Intermediate L22-4 (300 mg) and compound L23-1 (86.44 mg) were dissolved in DMF (4 mL), and HBTU (110.99 mg) and DIEA (84.76 mg) were added thereto. The reaction mixture was stirred at 25 °C for 1 h under a nitrogen atmosphere. The mixture was then directly purified by reversed-phase chromatography (column: C18 (12 g); mobile phase: [A: water, B: acetonitrile]; B%: 0%-100%, 60 mL/min) to give the title compound (180 mg).

MS m/z (ESI): 1132.6 [M+H]⁺.

### Step 2: synthesis of allyl (S)-3-amino-4-(((S)-1-(((S)-1-((4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenyl)amino)-1-oxopropan-2-yl)amino)-3-methyl-1-oxobutan-2-yl)amino)-4-oxobutanoate (intermediate L23-3)

**Intermediate L23-2** (170 mg) was dissolved in DMF (1.5 mL) and piperidine (0.3 mL), and the mixture was stirred at 25 °C for 1 h under a nitrogen atmosphere. The mixture was then directly purified by reversed-phase chromatography (column: C18 (40 g); mobile phase: [A: water, B: acetonitrile]; B%: 0%-40%, 60 mL/min) to give the title compound (100 mg).

MS m/z (ESI): 910.4 [M+H]⁺.

### Step 3: synthesis of allyl (S)-3-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-4-(((S)-1-(((S)-1-((4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenyl)amino)-1-oxopropan-2-yl)amino)-3-methyl-1-oxobutan-2-yl)amino)-4-oxobutanoate (intermediate L23-4)

**Intermediate L23-3** (30 mg) and **intermediate L17-1** (15.36 mg) were dissolved in DMF (1 mL), and HBTU (25.01 mg) and DIEA (12.78 mg) were added thereto. The reaction mixture was stirred at 25 °C for 1 h under a nitrogen atmosphere. The mixture was then directly purified by reversed-phase chromatography (column: C18 (12 g); mobile phase: [A: water, B: acetonitrile]; B%: 0%-100%, 40 mL/min) to give the title compound (15 mg).

MS m/z (ESI): 1315.5 [M+H]⁺.

### Step 4: synthesis of (S)-3-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-4-(((S)-1-(((S)-1-((4-(((((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamoyl)oxy)methyl)phenyl)amino)-1-oxopropan-2-yl)amino)-3-methyl-1-oxobutan-2-yl)amino)-4-oxobutanoic acid (intermediate L23-5)

Intermediate L23-4 (14 mg) was dissolved in DMF (0.5 mL), and tetrakis(triphenylphosphine)palladium(0) (3.69 mg) and 1,3-barbituric acid (2.49 mg) were added thereto. The reaction mixture was stirred at 25 °C for 1 h under a nitrogen atmosphere. The mixture was then directly purified by reversed-phase chromatography (column: C18 (12 g); mobile phase: [A: water, B: acetonitrile]; B%: 0%-90%, 40 mL/min) to give the title compound (13 mg).

MS m/z (ESI): 1275.2 [M+H]⁺.

### Step 5: synthesis of tert-butyl N-(N-(((9H-fluoren-9-yl)methoxy)carbonyl)-N-methylglycyl)-N-methylglycinate (intermediate L23-6)

Fmoc-sarcosine (50 g) was dissolved in dichloromethane (500 mL), and HATU (66.65 g) and DIEA (83.92 mL) were added to the reaction mixture. The resulting mixture was stirred at 25 °C for 1 h, and *tert*-butyl *N-*methylglycinate hydrochloride (29.17 g) was added to the reaction mixture. The reaction mixture was stirred at 25 °C for 2 h. The mixture was then diluted with 200 mL of water, and the resulting mixture was extracted with dichloromethane (100 mL × 3). The organic phases were combined, dried over anhydrous sodium sulfate, and concentrated under reduced pressure to give the title compound (115 g).

LC-MS: m/z (ESI): 439.2 [M+H]⁺

### Step 6: synthesis of N-(N-(((9H-fluoren-9-yl)methoxy)carbonyl)-N-methylglycyl)-N-methylglycine (intermediate L23-7)

**Intermediate L23-6** (110 g) was dissolved in a solution of hydrochloric acid in dioxane (400 mL), and the reaction mixture was stirred at 25 °C for 16 h. After the reaction was completed, the mixture was concentrated under reduced pressure, and the residue was separated by column chromatography (methanol/dichloromethane, 0-9%) to give the title compound (40 g).

LC-MS: m/z (ESI): 383.2 [M+H]⁺

### Step 7: synthesis of 1-(9H-fluoren-9-yl)-4,7,10,13,16,19,22,25,28,31,34,37,40-tridecamethyl-3,6,9,12,15,18,21,24,27,30,33,36,39-tridecaoxo-2-oxa-4,7,10,13,16,19,22,25,28,31,34,37,40-tridecaazadotetracontan-42-oic acid (intermediate L23-8)

The compound was synthesized by a polypeptide solid-phase synthesis method, and the steps are as follows:
1) Dichloromethane was added to a mixture of CTC resin (1.17 mmol/g, 15 g) and *N*-(((9*H*-fluoren-9-yl)methoxy)carbonyl)-*N*-methylglycine (5.5 g) under a nitrogen atmosphere;
2) diisopropylethylamine (DIEA, 40 mL) was added dropwise, and the mixture was mixed and stirred for 16 h;
3) the resin was filtered and washed three times with methanol (100 mL) and DCM (100 mL);
4) a 20% piperidine/DMF solution was added, and the mixture was reacted for 1 h;
5) the resin was filtered and washed three times with methanol (100 mL) and DCM (100 mL);
6) according to the weight of the resin, intermediate L23-7 (2.0 eq.) was added, followed by the addition of HBTU (3 eq.) and DIEA (6.0 eq.). The reaction was carried out under nitrogen bubbling for 1 h;
7) steps 3-6 were repeated five times.

After the solid phase was prepared, a 20% HFIP/DCM solution (12.5 mL) was added to the resulting mixture, and the mixture was stirred for reaction for 1 h. The reaction mixture was then filtered, and the filtrate was collected and concentrated to give the title compound (150 mg).

MS m/z (ESI): 1164.8 [M+H]⁺.

### Step 8: synthesis of (9H-fluoren-9-yl)methyl (38-amino-3,6,9,12,15,18,21,24,27,30,33,36-dodecamethyl-2,5,8,11,14,17,20,23,26,29,32,35,38-tridecaoxo-3,6,9,12,15,18,21,24,27,30,33,36-dodecaazaoctatriacontyl)(methyl)carbamate (intermediate L23-9)

**Intermediate L23-8** (130 mg) was dissolved in DMF (2 mL), and ammonium chloride (17.92 mg), HATU (84.25 mg), and DIEA (11.97 µL) were sequentially added. The reaction mixture was stirred at 25 °C for 1 h. After the reaction was completed, the mixture was purified by a reversed-phase column (column: Spherical C18 20-35 µm 100 A20 g; mobile phase: [A: water, B: acetonitrile]; B%: 10%-30%, 60 min) to give the title compound (80 mg). MS m/z (ESI): 1163.6 [M+H]⁺.

### Step 9: synthesis of N-(2-amino-2-oxoethyl)-N-methyl-2-(N,5,8,11,14,17,20,23,26,29,32-undecamethyl-4,7,10,13,16,19,22,25,28,31-decaoxo-2,5,8,11,14,17,20,23,26,29,32-undecaazatetratriacontane-34-amido)acetamide (intermediate L23-10)

**Intermediate L23-9** (70 mg) was dissolved in DMF (0.5 mL), and diethylamine (30.99 µL) was added. The reaction mixture was stirred at 25 °C for 2 h. After the reaction was completed, the mixture was purified by a reversed-phase column (column: Spherical C18 20-35 µm 100 A 20 g; mobile phase: [A: water, B: acetonitrile]; B%: 10%-30%, 60 min) to give the title compound (32 mg).

LC-MS: m/z (ESI): 941.3 [M+H]⁺

### Step 10: synthesis of 4-((42S,45S,48S)-1-amino-42-(3-(2'-(cyclopentylsulfonyl)-5'-oxo-2,3,5,6-tetrahydro-5'H-spiro[pyrano-4,8'-pyrido[4,3-d]pyrimidin]-6'(7'H)-yl)propanamido)-45-isopropyl-3,6,9,12,15,18,21,24,27,30,33,36,39,48-tetradecamethyl-1,4,7,10,13,16,19,22,25,28,31,34,37,40,43,46-hexadecaoxo-3,6,9,12,15,18,21,24,27,30,33,36,39,44,47-pentadecaazanonatetracontan-49-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (compound b-L23-3)

**Intermediate L23-5** (13 mg) and **intermediate L23-10** (9.59 mg) were dissolved in DMF (0.5 mL), and HBTU (7.73 mg) and DIEA (3.95 mg) were added thereto. The reaction mixture was stirred at 25 °C for 1 h under a nitrogen atmosphere. After the reaction was completed, the reaction mixture was filtered and purified by preparative high performance liquid chromatography (Welch Xtimate C18 column, 5 µm silica, 30 mm diameter, 150 mm length; a mixture of water (containing 0.225% formic acid) and acetonitrile with decreasing polarity used as the eluent; acetonitrile gradient: 26%-56%, elution time: 11 min) to give the title compound (2.7 mg).

MS m/z (ESI): 1099.9 [(M+2H)/2]⁺

**¹H NMR (400 MHz, DMSO-d₆)** δ = 9.67-9.42 (m, 1H), 9.29 (s, 1H), 8.46-8.24 (m, 1H), 8.16-8.02 (m, 2H), 7.78 (d, *J =* 11.0 Hz, 1H), 7.69-7.61 (m, 2H), 7.37 (d, *J =* 7.9 Hz, 2H), 7.31 (s, 1H), 6.53 (s, 1H), 5.45 (s, 2H), 5.29 (s, 3H), 5.08 (s, 2H), 4.78-4.64 (m, 1H), 4.42-4.19 (m, 14H), 4.12-3.89 (m, 14H), 3.84 (s, 5H), 3.74-3.62 (m, 4H), 3.18-3.05 (m, 2H), 3.00-2.65 (m, 45H), 2.38 (s, 3H), 2.25-2.14 (m, 2H), 2.11-1.76 (m, 9H), 1.72-1.61 (m, 5H), 1.54-1.40 (m, 1H), 1.37-1.29 (m, 3H), 0.93-0.80 (m, 10H).

### Example 43. 4-((30S,33S,36S)-1-Amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-33-isopropyl-3,6,9,12,15,18,21,24,27-nonamethyl-1,4,7,10,13,16,19,22,25,28,31,34-dodecaoxo-36-(3-ureidopropyl)-3,6,9,12,15,18,21,24,27,32,35-undecaazaheptatriacontan-37-amido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (Compound b-L26-4)

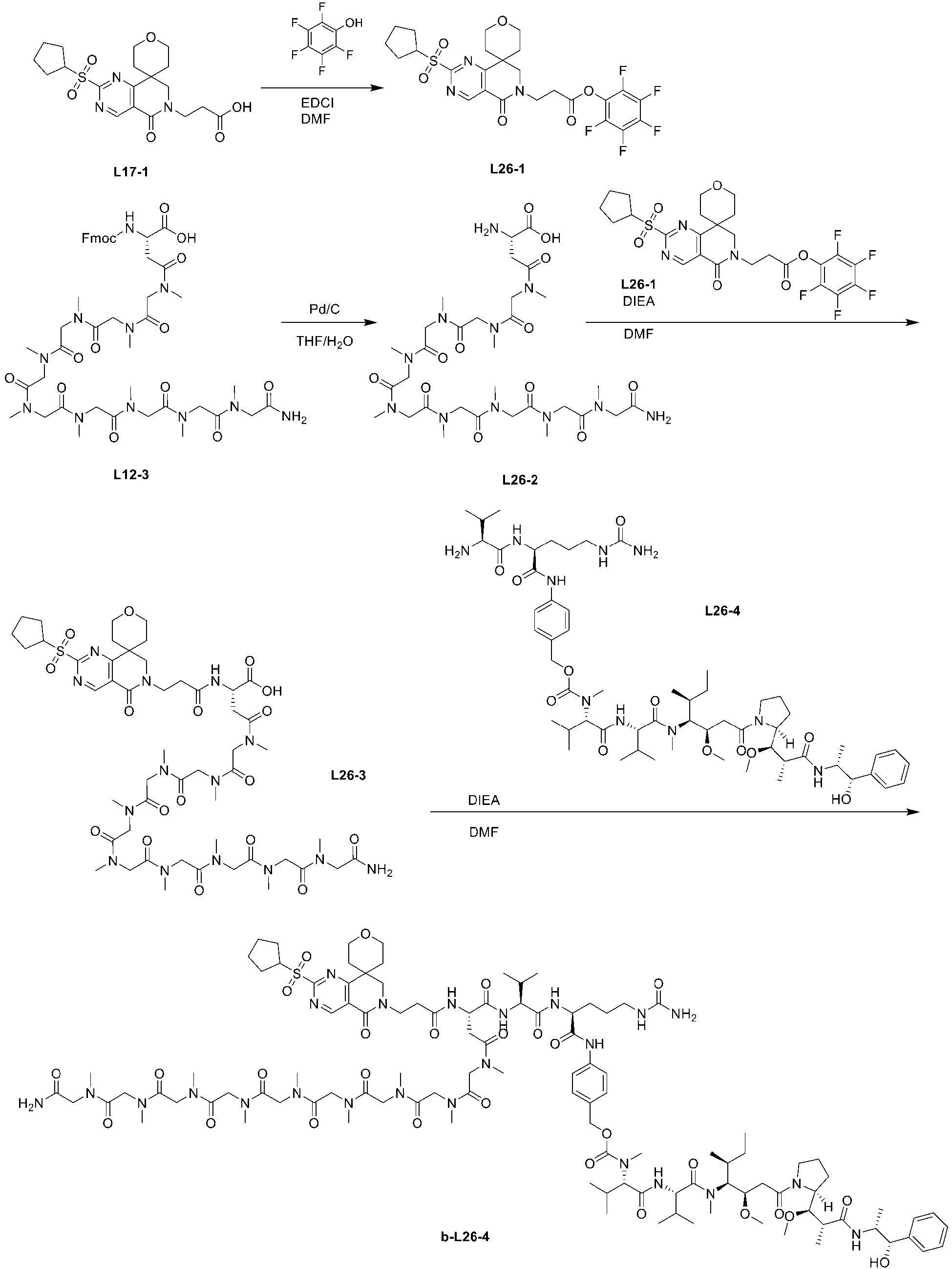

### Step 1: synthesis of perfluorophenyl 3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanoate (intermediate L26-1)

**Intermediate L17-1** (0.5 g) and pentafluorophenol (325.98 mg) were dissolved in DMF (5 mL), EDCI (425.68 mg) was added under a nitrogen atmosphere, and the reaction mixture was stirred at 25 °C for 1 h. The reaction mixture was then directly purified by a C18 reversed-phase column (100% acetonitrile) to give the title compound (600 mg).

MS m/z (ESI): 590.3 [M+H]⁺

### Step 2: synthesis of (S)-1,30-diamino-3,6,9,12,15,18,21,24,27-nonamethyl-1,4,7,10,13,16,19,22,25,28-decaoxo-3,6,9,12,15,18,21,24,27-nonaazahentriacontan-31-oic acid (intermediate L26-2)

**Intermediate L12-3** (1 g) was dissolved in tetrahydrofuran (10 mL) and water (5 mL), and wet palladium on carbon (244.36 mg) was added. Then, the reaction mixture was stirred at 25 °C for 16 h under a hydrogen atmosphere. The reaction mixture was then directly filtered and concentrated to give the title compound (0.7 g).

MS m/z (ESI): 772.3 [M+H]⁺.

### Step 3: synthesis of (S)-1-amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-3,6,9,12,15,18,21,24,27-nonamethyl-1,4,7,10,13,16,19,22,25,28-decaoxo-3,6,9,12,15,18,21,24,27-nonaazahentriacontan-31-oic acid (intermediate L26-3)

**Intermediate L26-2** (300 mg) and **intermediate L26-1** (206.23 mg) were dissolved in DMF (3 mL), DIEA (150.71 mg) was added under a nitrogen atmosphere, and the reaction mixture was stirred at 25 °C for 1 h. The reaction mixture was then directly purified by high performance liquid chromatography (column: C18 150 × 30 mm × 5 µm; mobile phase: [A: water (0.225% formic acid), B: acetonitrile]; B%: 13%-33%, 10 min) to give the title compound (100 mg).

MS m/z (ESI): 1177.5 [M+H]⁺

### Step 4: synthesis of 4-((30S,33S,36S)-1-amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-33-isopropyl-3,6,9,12,15,18,21,24,27-nonamethyl-1,4,7,10,13,16,19,22,25,28,31,34-dodecaoxo-36-(3-ureidopropyl)-3,6,9,12,15,18,21,24,27,32,35-undecaazaheptatriacontan-37-amido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (compound b-L26-4)

**Intermediate L26-3 (**20 mg) and **compound L26-4** (19.08 mg) were dissolved in DMF (0.5 mL) at room temperature, and butylphosphonic anhydride (T4P) (24.48 mg, 50% effective content) and DIEA (8.78 mg, 11.84 µL) were added under a nitrogen atmosphere. The reaction mixture was stirred at 25 °C for 1 h. The reaction mixture was then directly purified by high performance liquid chromatography (column: C18 150 × 40 mm; mobile phase: [A: water (0.225% formic acid), B: acetonitrile]; B%: 20%-60%, 9 min) to give the title compound (11 mg).

MS m/z (ESI): 1142.6 [(M+2H)/2]⁺

**¹H NMR (400 MHz, DMSO-d₆)** δ = 9.80-9.51 (m, 1H), 9.32-9.23 (m, 1H), 8.40-8.27 (m, 1H), 8.18-7.95 (m, 2H), 7.89 (d, *J* = 9.0 Hz, 1H), 7.62 (s, 2H), 7.33-7.23 (m, 6H), 7.17 (s, 2H), 5.97 (s, 1H), 5.40 (s, 3H), 5.15-4.91 (m, 2H), 4.79-4.57 (m, 2H), 4.49 (d, *J* = 5.9 Hz, 2H), 4.37-4.19 (m, 10H), 4.13-3.66 (m, 22H), 3.61-3.54 (m, 1H), 3.25-3.17 (m, 9H), 3.12 (s, 2H), 3.02-2.71 (m, 34H), 2.31-2.23 (m, 1H), 2.16-1.90 (m, 11H), 1.84-1.57 (m, 12H), 1.56-1.20 (m, 8H), 1.06-0.97 (m, 6H), 0.91-0.72 (m, 26H).

### Example 44. 4-((30S,33S,36S)-1-Amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-33-isopropyl-3,6,9,12,15,18,21,24,27-nonamethyl-1,4,7,10,13,16,19,22,25,28,31,34-dodecaoxo-36-(3-ureidopropyl)-3,6,9,12,15,18,21,24,27,32,35-undecaazaheptatriacontan-37-amido)benzyl (2-(2-chloro-4-(3-((2-(2,6-dioxopiperidin-3-yl)-1-oxoisoindolin-5-yl)methyl)ureido)phenethoxy)ethyl)(methyl)carbamate (Compound b-L27-5)

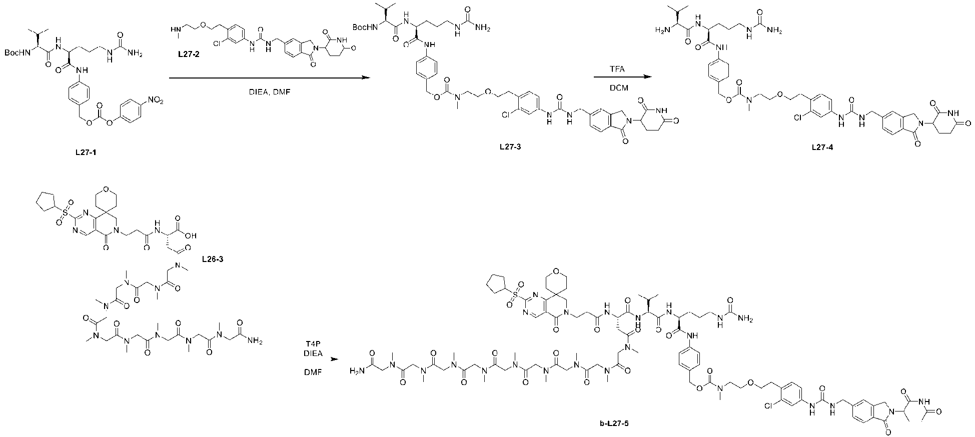

### Step 1: synthesis of 4-((S)-2-((S)-2-((tert-butoxycarbonyl)amino)-3-methylbutanamido)-5-ureidopentanamido) benzyl (2-(2-chloro-4-(3-((2-(2,6-dioxopiperidin-3-yl)-1-oxoisoindolin-5-yl)methyl)ureido)phenethoxy)ethyl) (methyl)carbamate (intermediate L27-3)

**Compound L27-1** (100 mg) and compound **L27-2** (90 mg, synthesized by referring to the method reported in patent WO2021198965) were dissolved in DMF (1 mL), and DIEA (60.08 mg) was added under a nitrogen atmosphere. The reaction mixture was stirred at 25 °C for 1 h. The reaction mixture was then diluted with ethyl acetate (30 mL) and washed with water (30 mL × 3). The organic phases were combined and dried over anhydrous sodium sulfate. After filtration, the filtrate was concentrated under reduced pressure, and the residue was purified by a flash silica gel column (ISCO^{®}; 20 g of SepaFlash^{®} flash silica gel column, eluent: 0-100% tetrahydrofuran/petroleum ether, gradient @ 40 mL/min) to give the title compound (100 mg).

MS m/z (ESI): 1033.3 [M+H]⁺.

### Step 2: synthesis of 4-((S)-2-((S)-2-amino-3-methylbutanamido)-5-ureidopentanamido)benzyl (2-(2-chloro-4-(3-((2-(2,6-dioxopiperidin-3-yl)-1-oxoisoindolin-5-yl)methyl)ureido)phenethoxy)ethyl)(methyl)carbamate (intermediate L27-4)

Trifluoroacetic acid (0.2 mL) was added to a solution of **intermediate L27-3** (100 mg) in dichloromethane (1 mL) at 0 °C, and the reaction mixture was stirred at 25 °C for 1 h. The reaction mixture was then directly concentrated, dried under vacuum, and directly purified by high performance liquid chromatography (column: C18 150 × 30 mm; mobile phase: [A: water (0.00025% hydrochloric acid), B: acetonitrile]; B%: 12%-52%, 9 min) to give the title compound (13 mg).

MS m/z (ESI): 933.6 [M+H]⁺

### Step 3: synthesis of 4-((30S,33S,36S)-1-amino-30-(3-(2'-(cyclopentylsulfonyl)-5'-oxo-2,3,5,6-tetrahydro-5'H-spiro[pyran-4,8'-pyrido[4,3-d]pyrimidin]-6'(7'H)-yl)propanamido)-33-isopropyl-3,6,9,12,15,18,21,24,27-nonamethyl-1,4,7,10,13,16,19,22,25,28,31,34-dodecaoxo-36-(3-ureidopropyl)-3,6,9,12,15,18,21,24,27,32,35-undecaazaheptatriacontan-37-amido)benzyl (2-(2-chloro-4-(3-((2-(2,6-dioxopiperidin-3-yl)-1-oxoisoindolin-5-yl)methyl)ureido)phenethoxy)ethyl)(methyl)carbamate (compound b-L27-5)

Butylphosphonic anhydride (12.73 mg, 50% content) and DIEA (4.57 mg, 6.16 µL) were added to a solution of **intermediate L27-4 (**11 mg) and **compound L26-3 (**13.87 mg) in DMF (0.5 mL) at room temperature, and the reaction mixture was stirred at 25 °C for 1 h. The reaction mixture was then directly purified by high performance liquid chromatography (column: C18 150 × 30 mm; mobile phase: [A: water (0.225% formic acid), B: acetonitrile]; B%: 22%-62%, 9 min) to give the title compound (6.5 mg).

MS m/z (ESI): 1046.9 [(M+2H)/2]⁺

**¹H NMR (400 MHz, DMSO-d₆)** δ = 10.99 (s, 1H), 9.79-9.52 (m, 1H), 9.29 (s, 1H), 8.79 (s, 1H), 8.41-8.21 (m,

1H), 8.10-7.90 (m, 2H), 7.73-7.58 (m, 4H), 7.52 (s, 1H), 7.44 (d, *J =* 8.1 Hz, 1H), 7.30 (s, 2H), 7.23-7.05 (m, 3H), 6.81 (t, *J =* 5.6 Hz, 1H), 5.98 (d, *J = 2.3* Hz, 1H), 5.42 (s, 2H), 5.13-5.09 (m, 1H), 4.98 (s, 2H), 4.81-4.65 (m, 1H), 4.41 (d, *J =* 8.0 Hz, 3H), 4.37-4.19 (m, 10H), 4.14-3.99 (m, 6H), 3.97-3.86 (m, 4H), 3.85-3.74 (m, 6H), 3.73-3.65 (m, 3H), 3.59-3.45 (m, 8H), 3.02-2.89 (m, 12H), 2.89-2.73 (m, 22H), 2.64-2.57 (m, 2H), 2.41-2.37 (m, 1H), 2.09-1.91 (m, 8H), 1.84-1.75 (m, 1H), 1.70-1.61 (m, 6H), 1.48-1.44 (m, 3H), 1.39-1.31 (m, 2H), 0.89-0.78 (m, 8H).

### Example 45. Preparation or Source of Drug-Linker Controls 1-5

For the preparation of drug-linker control 1, reference was made to patent document CN111065621A, 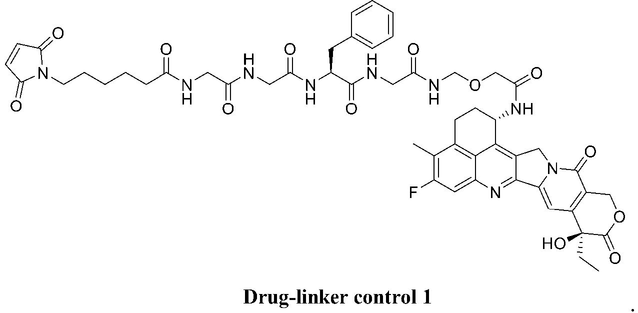 Drug-linker control 2 was prepared by referring to the method reported in Example 22 and patent document CN111065621A, 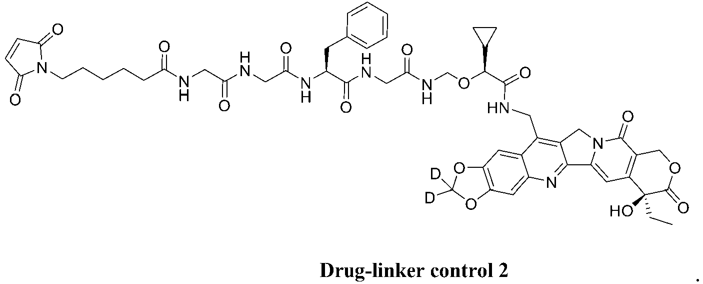 Drug-linker control 3 was purchased from MCE (Cat. No. HY-15575), 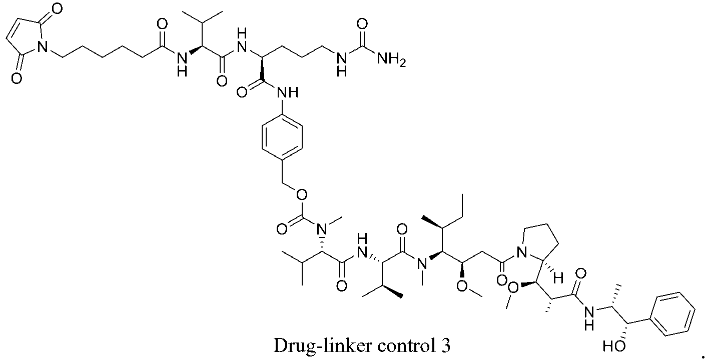 For the preparation of drug-linker control 4, reference was made to patent document WO2021198965A1, 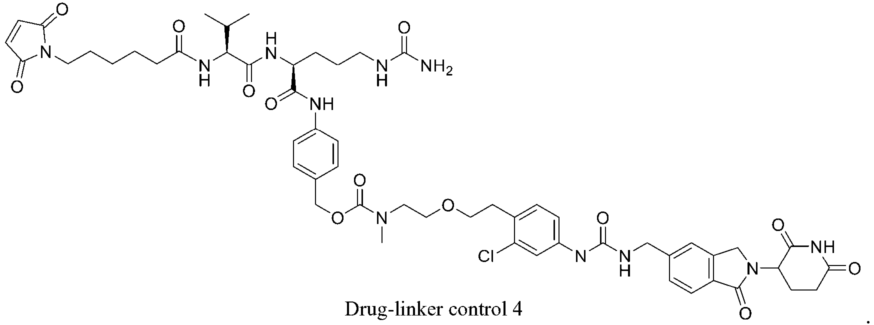 Drug-linker control 5 was purchased from MCE (Cat. No. HY-147270),

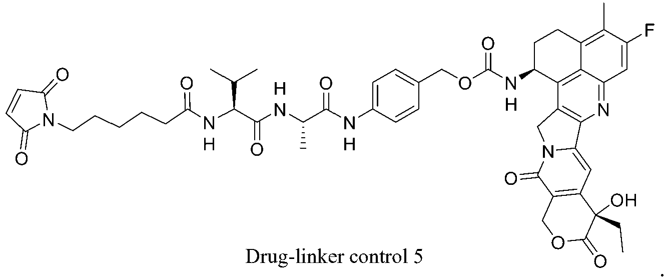

### Test Example 1. Physicochemical Property Test of Antibody Conjugates

### 1.1 Antibody:

### 1.1.1 Construction and production of anti-human HER2 monoclonal antibody

The sequences of the anti-human HER2 monoclonal antibody are shown in Table 1 below. The nucleotide sequences of the heavy and light chains were separately cloned into a pTT5 vector (purchased from Youbio), and plasmids were prepared according to established conventional standard molecular biology methods. The expression vector and the transfection reagent PEI (Polysciences, Cat. No. 24765-1) were added to OPTI-MEM (Gibco, Cat. No. 11058021). The mixture was well mixed and allowed to stand for 15 min. The Expi293F cells (manufacturer: Thermofisher, Cat. No. A14527) were added to the mixture, and the system was incubated on a shaker at 37 °C in 5% CO₂ at 120 rpm. On day 2 of the transfection, OPM-293 ProFeed (Shanghai OPM Biosciences Co., Ltd., Cat. No. F081918-001) and 6 g/L glucose (manufacturer: Sigma, Cat. No. G7528) were added. On day 6 of the transfection, the cell supernatant was collected.

**Table 1. Amino acid sequence information of anti-human HER2 antibody trastuzumab and CDR analysis thereof (according to Kabat numbering scheme)**

| Antibody | Sequence No. | | Sequence information |
|---|---|---|---|
| Trastuzumab-VH | SEQ ID NO:1 | | |
| Trastuzumab-VL | SEQ ID NO:2 | | |
| human CH1-Fc | SEQ ID NO:3 | | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTV LHQDWLNGKEYKCKVSNKALPAPIEKTISKAKGQPREPQVYTLPPSRE EMTKNQVSLTCLVKGFYPSDIAVEWESNGQPENNYKTTPPVLDSDGSF |
| | | FLYSKLTVDKSRWQQGNVFSCSVMHEALHNHYTQKSLSLSPGK | |
| human CL | SEQ ID NO:4 | | |

| Antibody | CDR1 | CDR2 | CDR3 |
|---|---|---|---|
| Trastuzumab-VH | DTYIH (SEQ ID NO:5) | RIYPTNGYTRYADSVKG (SEQ ID NO:6) | WGGDGFYAMDY (SEQ ID NO:7) |
| Trastuzumab-VL | RASQDVNTAVA (SEQ ID NO:8) | SASFLYS (SEQ ID NO:9) | QQHYTTPPT (SEQ ID NO:10) |

### 1.1.2 Construction and production of anti-human EGFR and cMet bispecific antibody

Referring to the method in Example **1.1.1,** nucleic acid sequences encoding the VHs and VLs of the antibody were constructed into a plasmid (sequence source: WO2024002938A1, see Table 2) and transfected into Expi293F cells to give an expression supernatant.

**Table 2. Sequence information of anti-human EGFR-cMet bispecific antibody EGFR-cMet-Ab**

| Antibody | Sequence No. | Sequence information |
|---|---|---|
| EGFR-cMet-Ab-EGFR HC | SEQ ID NO:11 | QVQLVQSGAEVKKPGSSVKVSCKASGGTFSDNDFSWVRQ APGQGLEWMGAIVAVFRTETYAQKFQDRVKITADISTRTT YMELSSLRSEDTAVYYCARRLMSAISGPGAPLLMWGQGT LVTVSSASTKGPSVCPLAPSSKSTSGGTAALGCLVKDYFPE PVTVSWNSGALTSGVHTFPAVLQSSGLYSLSSVVTVPSSSL GTQTYICNVNHKPSNTKVDKRVEPKSVDKTHTCPPCPAPE FEGGPSVFLFPPKPKDTLMISRTPEVTCVVVDVSHEDPEV KFNWYVDGVEVHNAKTKPREEQYNSTYRVVSVLTVLHQ DWLNGKEYKCKVSNKALPASIEKTISKAKGQPREPQVYT LPPCREEMTKNQVSLWCLVKGFYPSDIAVEWESNGQPEN NYKTTPPVLDSDGSFFLYSKLTVDKSRWQQGNVFSCSVM HEALHNHYTQKSLSLSPGK |
| EGFR-cMet-Ab-cMet HC | SEQ ID NO:12 | QVQLVQSGAEVKKPGASVKVSCKASGYTFTDYYIHWVR QATGQGLEWMGWMNPNSGNTGYAQKFQGRVTMTRDTSI STAYMELSSLRSEDTAVYYCARGQGYTHSWGQGTMVTVS SASTKGPSVFPLAPSSKSTSGGTAALGCLVKDYFPEPVTVS WNSGALTSGVHTFPAVLQSSGLYSLSSVVTVPSSSLGTQTY ICNVNHKPSNTKVDKRVEPKSCDKTHTCPPCPAPEFEGGP SVFLFPPKPKDTLMISRTPEVTCVVVDVSHEDPEVKFNWY VDGVEVHNAKTKPREEQYNSTYRVVSVLTVLHQDWLNG KEYKCKVSNKALPASIEKTISKAKGQPREPQVCTLPPSREE MTKNQVSLSCAVKGFYPSDIAVEWESNGQPENNYKTTPP VLDSDGSFFLVSKLTVDKSRWQQGNVFSCSVMHEALHNH YTQKSLSLSPGK |
| EGFR-cMet-Ab-EGFR LC | SEQ ID NO:13 | QSALTQPRSVSGSPGQSVTISCTGTSSDVGGYNYVSWYQQ HPGKAPKLMIYDVSKRPSGVPDRFSGSKSGNTASLTISGLQ AEDEADYYCSSYTSSDTLEIFGGGTKLTVLGQPKAAPSVT LFPPCSEELQANKATLVCLISDFYPGAVTVAWKADSSPVK AGVETTTPSKQSNNKYAASSYLSLTPEQWKSHRSYSCQVT HEGSTVEKTVAPTEVS |
| EGFR-cMet-Ab-cMet LC | SEQ ID NO:14 | DIQMTQSPSTLSASVGDRVTITCRASEGIYHWLAWYQQKP GKAPKLLIYKASSLASGVPSRFSGSGSGTEFTLTISSLQPDD FATYYCQQYSNYPPTFGGGTKLEIKRTVAAPSVFIFPPSDE QLKSGTASVVCLLNNFYPREAKVQWKVDNALQSGNSQE SVTEQDSKDSTYSLSSTLTLSKADYEKHKVYACEVTHQGL SSPVTKSFNRGEC |

### 1.1.3 Construction and production of anti-human CDH6 antibody

The anti-human CDH6 antibody CDH6-Ab-1 was produced by immunization of mice. The immunogens were human CDH6-hFc protein and HEK293T cells overexpressing human CDH6. Spleen lymphocytes of mice with a high antibody titer in serum were selected to prepare hybridoma cells. Positive hybridoma clones were obtained by screening through ELISA, FACS, and other conventional methods in the field. The antibody was further prepared by a serum-free cell culture method, and the antibody was purified and sequenced to give a murine anti-human CDH6 antibody and a variable region sequence thereof. By comparing the IMGT database (http://imgt.cines.fr) for germline genes from heavy and light chain variable regions of human antibodies with the Molecular Operating Environment (MOE) software, germline genes, with high homology with murine antibodies, from heavy chain and light chain variable regions were respectively selected as templates, and CDRs of murine antibodies were respectively grafted into corresponding human templates to form a variable region sequence of FR1-CDR1-FR2-CDR2-FR3-CDR3-FR4. Based on this, back mutation was performed as needed to maintain the original affinity and/or hotspot mutation was performed to eliminate the risk of molecular modifications. The VH and the VL corresponding to the finally optimized humanized antibody CDH6-Ab-1 and the CDR sequences divided according to the Kabat form are shown in Table 3.

**Table 3. Sequence information of anti-human CDH6 antibody CDH6-Ab-1 and CDR sequences thereof (according to Kabat numbering scheme)**

| Antibody | Sequence No. | Sequence information | | |
|---|---|---|---|---|
| CDH6-Ab-1-VH | SEQ ID NO:15 | EVQLVQSGAEVKKPGSSVKVSCKASGYTFTNYWMHWV RQAPGQGLEWMGDIDPSHSETHYTQKFKDRVTITVDKS TSTAYMELSSLRSEDTAVYYCARGDLGRFDYWGQGTTV TVSS DIQMTQSPSSLSASVGDRVTITCRASESVSIHGTHLMHW YQQKPGKPPKLLIYAASNLESGVPSRFSGSGSETDFTFTIS SLQPEDIATYYCQQSFEDPWTFGGGTKVEIK | | |
| CDH6-Ab-1-VL | SEQ ID NO:16 | [?]PSSLSASVGDRVTITCRASE [?]PPKLLIYAASNLESGVPSR [?]TYYCQQSFEDPWTFGGGT | | |

| Antibody | CDR1 | | CDR2 | CDR3 |
|---|---|---|---|---|
| CDH6-Ab-1-VH | NYWMH (SEQ ID NO: 17) | | DIDPSHSETHYTQKFKD (SEQ ID NO:18) | GDLGRFDY (SEQ ID NO:19 ) |
| CDH6-Ab-1-VL | RASESVSIHGTHLMH(SEQ ID NO: 20) | | AASNLES (SEQ ID NO: 21) | QQSFEDPWT(SEQ ID NO: 22) |

Referring to the method in Example **1.1.1,** nucleic acid sequences encoding the VHs and VLs of the antibody were constructed into a plasmid and transfected into Expi293F cells to give an expression supernatant.

### 1.2 Purification of antibodies

The antibodies described above were purified from the cell culture supernatants using ProteinA affinity chromatography (Cytiva, Cat. No. 17549802). The Protein A affinity column was washed with 3-5 column volumes of 0.5 M NaOH and then with 3-5 column volumes of pure water. The chromatographic column was equilibrated with 3-5 column volumes of, for example, a 1× PBS (pH 7.4) buffer system as an equilibration buffer. The cell supernatants were loaded at a low flow rate for binding, and after the binding was completed, the chromatographic column was washed with 3-5 column volumes of 1× PBS (pH 7.4) until the UV absorbance fell back to baseline. The samples were eluted with an eluent (50 mM acetic acid buffer, pH 3.5), and the elution peaks were collected according to UV monitoring. The eluted products were rapidly adjusted to pH 5-6 with 1 M Tris-HCl (pH 8.0) and temporarily stored. The buffer system was replaced using ultrafiltration, dialysis, a desalting column, etc., or the polymer components in the eluted products were removed using, for example, molecular sieves, to improve the sample purity. The purified proteins meeting the purity requirements were subjected to sterile filtration through a 0.22 µm filter (Millipore, Cat. No. SLGVR13SL), aliquoted after passing the SEC-HPLC purity test, and stored at -80 °C for later use.

### 1.3.1 Preparation method 1 for antibody-drug conjugate

10-fold molar equivalents of TCEP (tris(2-carboxyethyl)phosphine hydrochloride) were added to a 2-10 mg/mL antibody solution (1× PBS, 1 mM EDTA, pH 7.4), and the mixture was well mixed and incubated at 37 °C for 2 h. Then 15-fold molar equivalents of the drug-linker compound (prepared according to Examples 22-45 or commercially acquired) were added, and the mixture was well mixed and reacted at 25 °C for 4 h. After the reaction was completed, the mixture was subjected to buffer exchange by ultrafiltration 3 times with a 20 mM histidine buffer (pH 6.0) to remove residual unreacted free small molecules. The purity of the sample was detected by SEC-HPLC, and the conjugation was detected by LC-MS mass spectrometry.

**1.3.2 Preparation method 2 for antibody-drug conjugate** (suitable for preparing ADC molecules with DAR of about 4)

2.5-fold molar equivalents of TCEP (tris(2-carboxyethyl)phosphine hydrochloride) were added to a 2-10 mg/mL antibody solution (1× PBS, 1 mM EDTA, pH 7.4), and the mixture was well mixed and incubated at 37 °C for 2 h. Then 8-fold molar equivalents of the drug-linker compound (prepared according to Examples 22-45 or commercially acquired) were added, and the mixture was well mixed and reacted at 25 °C for 4 h. After the reaction was completed, the mixture was subjected to buffer exchange by ultrafiltration 3 times with a 20 mM histidine buffer (pH 6.0) to remove residual unreacted free small molecules. The purity of the sample was detected by SEC-HPLC, and the conjugation was detected by LC-MS mass spectrometry.

### 1.4 Purity measurement of antibody-drug conjugate (SEC-HPLC)

The protein sample to be tested was analyzed using an SEC-HPLC method, the molecular size uniformity of the recombinant protein was characterized, and the purity of the recombinant protein was determined. The HPLC used in this method was Agilent 1260, the chromatographic column was TSKgel G3000SWXL from Tosoh Bioscience, the mobile phase was a 200 mM phosphate buffer (pH 7.0, containing 10% isopropanol), the detection temperature was 25 °C, the flow rate was 0.5 mL/min, the detection wavelength was 280 nm, the loading amount of the target protein was 50 µg, and the analysis time was 40 min. For SEC-HPLC data, the chromatograms were analyzed by manual integration, and the protein purity was calculated according to the area normalization method. The main peak was considered as monomer, the chromatographic peaks before the main peak were referred to as aggregates, and the chromatographic peaks after the main peak were referred to as fragments. The measurement results are shown in Table 4. The SEC purity of ADC-L26-4 (DAR8), ADC-L27-5 (DAR8), ADC-L22-3 (DAR8), and ADC-L23-3 was significantly improved compared with that of control ADC7, control ADC8, and control ADC9.

**Table 4. Analysis of purity of antibody-drug conjugates by SEC-HPLC**

| Conjugate No. | Conjugation reaction substrate | | Monomer (%) |
|---|---|---|---|
| | Antibody | Drug-linker | |
| ADC-L1-1 | Trastuzumab | Example 22 | 97.37 |
| ADC-L2-1 | Trastuzumab | Example 23 | 98.58 |
| ADC-L2-1^{a} | Trastuzumab | Example 24 | 98.75 |
| ADC-L2-1^{b} | Trastuzumab | Example 25 | 98.85 |
| ADC-L5-1 | Trastuzumab | Example 26 | 98.73 |
| ADC-L6-1 | Trastuzumab | Example 27 | 98.50 |
| ADC-L7-1 | Trastuzumab | Example 28 | 98.63 |
| ADC-L8-1 | Trastuzumab | Example 29 | 98.77 |
| ADC-L9-1 | Trastuzumab | Example 30 | 98.45 |
| ADC-L10-1 | Trastuzumab | Example 31 | 98.83 |
| ADC-L6-1^{a} | Trastuzumab | Example 32 | 98.70 |
| ADC-L12-1 | Trastuzumab | Example 33 | 98.70 |
| ADC-L13-1 | Trastuzumab | Example 34 | 98.04 |
| ADC-L14-1 | Trastuzumab | Example 35 | 98.40 |
| ADC-L15-1 | Trastuzumab | Example 36 | 95.98 |
| ADC- L12-1^{a} | Trastuzumab | Example 37 | 98.81 |
| ADC-L17-1 | Trastuzumab | Example 38 | 98.86 |
| ADC-L18-1 | Trastuzumab | Example 39 | 98.83 |
| ADC-L19-1 | Trastuzumab | Example 40 | 99.10 |
| Control ADC1 | Trastuzumab | Drug-linker control 1 | 97.76 |
| Control ADC2 | Trastuzumab | Drug-linker control 2 | 97.44 |
| Control ADC-ISO-1 | Anti-FITC | Drug-linker control 2 | 95.98 |
| Control ADC-ISO-2 | Anti-FITC | Example 40 | 98.76 |
| Control ADC3 | Trastuzumab | Drug-linker control 3 | 99.88 |
| Control ADC4 | Trastuzumab | Drug-linker control 4 | 90.48 |
| Control ADC5 | Trastuzumab | Drug-linker control 5 | 95.87 |
| Control ADC6 | Trastuzumab | Drug-linker control 2 | 99.93 |
| Control ADC7 | Trastuzumab | Drug-linker control 3 | 65.30 |
| Control ADC8 | Trastuzumab | Drug-linker control 4 | 21.52 |
| Control ADC9 | Trastuzumab | Drug-linker control 5 | 37.03 |
| ADC-L26-4(DAR4) | Trastuzumab | Example 43 | 99.86 |
| ADC-L27-5(DAR4) | Trastuzumab | Example 44 | 99.88 |
| ADC-L22-3(DAR4) | Trastuzumab | Example 41 | 99.86 |
| ADC-L19-1(DAR4) | Trastuzumab | Example 40 | 99.91 |
| ADC-L26-4(DAR8) | Trastuzumab | Example 43 | 99.85 |
| ADC-L27-5(DAR8) | Trastuzumab | Example 44 | 99.84 |
| ADC-L22-3(DAR8) | Trastuzumab | Example 41 | 99.81 |
| ADC-L23-3 | Trastuzumab | Example 42 | 99.87 |
| Control ADC10 | EGFR-cMet-Ab | Drug-linker control 2 | 96.42 |
| ADC2-L19-1 | EGFR-cMet-Ab | Example 40 | 96.42 |
| Control ADC11 | CDH6-Ab-1 | Drug-linker control 2 | 96.2 |
| ADC3-L19-1 | CDH6-Ab-1 | Example 40 | 98.23 |
| ADC3-L12-1 | CDH6-Ab-1 | Example 37 | 98.51 |

### 1.5 Measurement of DAR values of antibody-drug conjugates (LC-MS mass spectrometry)

The ADC samples to be tested were analyzed by LC-MS mass spectrometry to characterize their DAR values. About 50 µg of ADC sample was taken, and 1 µL of PNGase F was added. The mixture was incubated at 37 °C for 4 h, and DTT at a final concentration of 20 mM was added. The mixture was incubated at 37 °C for 1 h, and the sample was centrifuged for testing. The HPLC used in this method was Thermo Vanquish, the chromatographic column was Protein BEH SEC (Waters), the mobile phase was a 25% acetonitrile solution (containing 0.1% formic acid and 0.05% TFA), the flow rate was 0.2 mL/min, the detection wavelength was 280 nm, and the analysis time was 30 min. The high-resolution mass spectrum used in this method was Q Exactive Plus. The mass spectrometry parameters were as follows: Spray Voltage: 3.8 kV; Capillary Temp (capillary temperature): 300 °C; S-lens: 50; Sheath Gas (sheath gas flow rate): 35 arb; Aux Gas (auxiliary gas flow rate): 10 arb; Probe Heater Temp (probe heater temperature): 300 °C; Full MS Scan range (precursor ion scan range): 810-3500 m/z; Resolution: 17500; AGC (automatic gain control target): 3e⁶; Maximum IT (maximum ion injection time): 200 ms; Microscans: 10; In-source CID: 20 eV. The original mass spectrometry data were analyzed using the mass spectrometry data analysis software Biopharma Finder 4.1 and deconvoluted using the general Respect algorithm to calculate the molecular weight information of the light and heavy chain mass spectrometry peaks and the mass spectrometry response signals of each component, and the DAR value of the sample was calculated according to the corresponding mass spectrometry response values of the conjugated small molecules of the sample.

According to calculation, the measurement results of the ADC samples are shown in Table 5:

**Table 5. Measurement of DAR values of antibody-drug conjugates by LC-MS mass spectrometry**

| Conjugate No. | DAR |
|---|---|
| ADC-L1-1 | 7.90 |
| ADC-L2-1^{b} | 7.94 |
| ADC-L5-1 | 7.68 |
| ADC-L6-1^{a} | 7.70 |
| ADC-L7-1 | 7.92 |
| ADC-L8-1 | 7.78 |
| ADC-L9-1 | 7.78 |
| ADC-L10-1 | 7.26 |
| ADC-L12-1 | 7.78 |
| ADC-L13-1 | 7.86 |
| ADC-L14-1 | 8.2 |
| ADC-L15-1 | 7.92 |
| ADC-L12-1^{a} | 7.82 |
| ADC-L17-1 | 8.04 |
| ADC-L18-1 | 8.12 |
| ADC-L19-1 | 8.04 |
| Control ADC1 | 8.00 |
| Control ADC2 | 7.90 |
| Control ADC-ISO-1 | 8.48 |
| Control ADC-ISO-2 | 7.92 |
| Control ADC3 | 3.74 |
| Control ADC4 | 3.66 |
| Control ADC5 | 3.92 |
| Control ADC6 | 3.66 |
| Control ADC7 | 7.00 |
| Control ADC8 | 8.00 |
| Control ADC9 | 8.00 |
| ADC-L26-4(DAR4) | 3.52 |
| ADC-L27-5(DAR4) | 3.66 |
| ADC-L22-3(DAR4) | 3.92 |
| ADC-L19-1(DAR4) | 3.56 |
| ADC-L26-4(DAR8) | 7.94 |
| ADC-L27-5(DAR8) | 8.00 |
| ADC-L22-3(DAR8) | 8.16 |
| ADC-L23-3 | 7.85 |
| Control ADC10 | 6.00 |
| ADC2-L19-1 | 6.00 |
| Control ADC11 | 7.84 |
| ADC3-L19-1 | 8.06 |
| ADC3-L12-1 | 7.98 |

| | |
|---|---|
| ADC-L2-1^{b} was an ADC sample prepared by conjugating trastuzumab with Example 25. ADC-L6-1^{a} was an ADC sample prepared by conjugating trastuzumab with Example 32. ADC-L12-1^{a} was an ADC sample prepared by conjugating trastuzumab with Example 37. | |

### 1.6 Hydrophobicity measurement of antibody-drug conjugates (HIC-HPLC)

The samples to be tested were analyzed by the HIC-HPLC method to characterize their hydrophobicity. The HPLC used in this method was Agilent 1260, the chromatographic column was TSKgel Butyl-NPR from Tosoh Bioscience, the mobile phase A was 1.5 M ammonium sulfate and a 50 mM phosphate buffer (pH 7.0), the mobile phase B was a 25 mM phosphate buffer (pH 7.0, containing 20% isopropanol), the detection temperature was 25 °C, the flow rate was 0.5 mL/min, and the detection wavelength was 280 nm. The sample to be tested was first concentrated to 1 mg/mL, and then 2 M ammonium sulfate and a 100 mM phosphate buffer (pH 7.0) were added to make the concentration of ammonium sulfate to be 0.8 M. The mixture was well mixed, and the supernatant was taken. The loading amount was 40 µg, the mobile phase gradient was set as mobile phase B increasing from 0% to 70% in 35 min, and the analysis time was 50 min. For HIC-HPLC data, the chromatograms were analyzed by manual integration, and the hydrophobicity was determined according to the peak time of the antibody. The measurement results are shown in Table 6:

**Table 6. Hydrophobicity measurement of antibody-drug conjugates by HIC-HPLC**

| Conjugate No. | Elution time (min) |
|---|---|
| Trastuzumab | 16.891 |
| ADC-L1-1 | 23.389 |
| ADC-L2-1 | 23.352 |
| ADC-L2-1a | 23.402 |
| ADC-L2-1b | 23.321 |
| ADC-L5-1 | 23.577 |
| ADC-L6-1 | 24.328 |
| ADC-L7-1 | 23.604 |
| ADC-L8-1 | 23.282 |
| ADC-L9-1 | 23.403 |
| ADC-L10-1 | 24.862 |
| ADC-L6-1a | 24.186 |
| ADC-L12-1 | 20.083 |
| ADC-L13-1 | 27.197 |
| ADC-L14-1 | 24.409 |
| ADC-L15-1 | 21.399 |
| ADC- L12-1a | 20.155 |
| ADC-L17-1 | 24.155 |
| ADC-L18-1 | 23.241 |
| ADC-L19-1 | 20.365 |
| Control ADC1 | 24.171 |
| Control ADC2 | 23.443 |
| ADC-L26-4(DAR8) | 33.502 |
| ADC-L27-5(DAR8) | 24.244 |
| ADC-L22-3(DAR8) | 21.788 |
| ADC-L23-3 | 20.389 |
| EGFR-cMet-Ab | 19.566 |
| Control ADC10 | 24.509 |
| ADC2-L19-1 | 22.264 |

### Test Example 2. Plasma Stability Test for Antibody Conjugates

### 2.1 Plasma stability test 1 for antibody conjugates

A certain amount of ADC sample was taken and added to sterile human plasma from which IgG had been removed, such that the final concentration of ADC was 0.5 mg/mL. The mixture was incubated in an incubator at 37 °C for 0 d, 3 d, and 7 d, and after the incubation was completed, the mixture was stored in a refrigerator at -80 °C. 50 µL of ProteinA filler (MabSelect SuRe) was added to each tube, and the mixture was adsorbed by shaking in a mixer for 1 h. After washing, elution, and Tris-HCl neutralization, an incubated ADC sample was obtained. The change in DAR value was measured by LC-MS to determine the plasma stability of the sample. The test results are shown in Table 7.

**Table 7. In vitro plasma stability of antibody-drug conjugates**

| Conjugate No. | Incubation condition | DAR value (LC-MS) |
|---|---|---|
| Control ADC 1 | Plasma incubation for 0 d | 7.92 |
| | Plasma incubation for 3 d | 5.00 |
| | Plasma incubation for 7 d | 3.50 |
| Control ADC3 | Plasma incubation for 0 d | 3.3 |
| | Plasma incubation for 3 d | 1.48 |
| | Plasma incubation for 7 d | 0.96 |
| Control ADC4 | Plasma incubation for 0 d | 3.3 |
| | Plasma incubation for 3 d | 1.62 |
| | Plasma incubation for 7 d | 1.02 |
| ADC-L19-1 | Plasma incubation for 0 d | 8.00 |
| | Plasma incubation for 3 d | 8.00 |
| | Plasma incubation for 7 d | 7.96 |
| ADC-L26-4(DAR4) | Plasma incubation for 0 d | 3.72 |
| | Plasma incubation for 3 d | 3.5 |
| | Plasma incubation for 7 d | 3.4 |
| ADC-L27-5(DAR4) | Plasma incubation for 0 d | 3.84 |
| | Plasma incubation for 3 d | 2.96 |
| | Plasma incubation for 7 d | 3.28 |
| ADC2-L19-1 | Plasma incubation for 0 d | 6.00 |
| | Plasma incubation for 3 d | 6.00 |
| | Plasma incubation for 7 d | 5.98 |

The experimental results show that compared with the control ADCs, the ADCs of the present disclosure had no significant change in DAR values during plasma incubation, and had better stability in plasma.

### 2.2 Plasma stability test 2 for antibody-drug conjugates

A certain amount of ADC sample was taken and added to sterile human plasma from which IgG had been removed, such that the final concentration of ADC was 0.5 mg/mL. The mixture was incubated in an incubator at 37 °C for 0 d, 3 d, and 7 d, and after the incubation was completed, the mixture was stored in a refrigerator at -80 °C. 50 µL of ProteinA filler (MabSelect SuRe) was added to each tube, and the mixture was adsorbed by shaking in a mixer for 1 h. After washing, elution, and Tris-HCl neutralization, an incubated ADC sample was obtained. The change in purity was measured by SEC-HPLC. The test results are shown in Table 8.

**Table 8. In vitro plasma stability of antibody-drug conjugates**

| Conjugate No. | Incubation condition | Monomer (%) | Aggregate (%) | Fragment (%) |
|---|---|---|---|---|
| Control ADC 1 | Plasma incubation for 0 d | 94.114 | 4.375 | 1.511 |
| | Plasma incubation for 3 d | 53.911 | 45.218 | 0.871 |
| | Plasma incubation for 7 d | 37.382 | 61.814 | 0.904 |
| Control ADC3 | Plasma incubation for 0 d | 92.168 | 2.513 | 5.319 |
| | Plasma incubation for 3 d | 68.161 | 29.098 | 2.741 |
| | Plasma incubation for 7 d | 70.437 | 26.506 | 3.057 |
| Control ADC4 | Plasma incubation for 0 d | 95.729 | 4.271 | - |
| | Plasma incubation for 3 d | 83.623 | 15.033 | 1.345 |
| | Plasma incubation for 7 d | 76.876 | 23.124 | - |
| ADC-L19-1 | Plasma incubation for 0 d | 99.317 | 0.683 | - |
| | Plasma incubation for 3 d | 98.827 | 1.173 | - |
| | Plasma incubation for 7 d | 98.12 | 1.88 | - |
| ADC-L26-4(DAR4) | Plasma incubation for 0 d | 97.293 | 2.707 | - |
| | Plasma incubation for 3 d | 94.61 | 5.39 | - |
| | Plasma incubation for 7 d | 92.281 | 5.953 | 1.766 |
| ADC-L27-5(DAR4) | Plasma incubation for 0 d | 97.75 | 1.935 | 0.316 |
| | Plasma incubation for 3 d | 97.692 | 2.096 | 0.212 |
| | Plasma incubation for 7 d | 97.644 | 2.2 | 0.156 |

| | | | | |
|---|---|---|---|---|
| "-" represents "not detected". | | | | |

The experimental results show that compared with the control groups, the ADCs of the present disclosure had no significant change in SEC monomer purity during plasma incubation, and had better stability in plasma.

### Test Example 3. Test of Biological Activity and Related Properties of Antibody Conjugates

### 3.1 Test of anti-proliferative activity of antibody-drug conjugates against tumor cells

### 3.1.1 Test of anti-proliferative activity of anti-Her2-ADCs against tumor cells

**Cells and materials: The human breast cancer** cell line **SKBR3 with high HER2 expression** was purchased from ATCC, EMEM medium (ATCC#30-2003) was purchased from ATCC, McCoy's 5a medium (Gibco#16600-082), 1640 medium (Gibco#A10491-01), penicillin-streptomycin (Gibco#15140-122), and 0.25% Trypsin-EDTA (Gibco#25200-056) were purchased from Gibco (USA), human insulin (Solarbio# 40112ES25) was purchased from Solarbio, 96-well plates (Greiner Bio-one#655098) were purchased from Corning (USA), and Cell-Titer Glo reagent (Promega#G7568) was purchased from Promega (USA).

**Cell culture:** SKBR3 cells were cultured with a McCoy's 5a culture medium containing 10% fetal bovine serum + 1% penicillin-streptomycin at 37 °C with 5% CO₂. Cells at the logarithmic growth phase were available for the experiment.

**Cell proliferation activity test:** The inhibitory activity of the ADCs on the proliferation of the SKBR3 cell line was measured using the Cell-Titer Glo reagent. SKBR3 cells were digested and pipetted in cell culture flasks, and resuspended using the corresponding fresh medium. The cell density was adjusted. SKBR3 cells were seeded on a 96-well plate at 5000 cells/90 µL/well and incubated at 37 °C with 5% CO₂ overnight. The ADC concentration was diluted to 5000 nM with a complete medium and subjected to 5-fold gradient dilution to give a total of 8 concentration gradients, and then 10 µL of an ADC diluted solution was transferred to the 96-well plate, i.e., the initial final concentration of the ADC was 500 nM. The 96-well plate was incubated at 37 °C with 5% CO₂ for 3 days. The Cell-Titer Glo reagent was added, and the cell activity was measured.

A negative control group and a positive control group were set as Bottom and Top, respectively. The negative control group was not added with cells, with only the culture medium in the same volume added, and other operations were consistent with those of the experimental groups. The positive control group was not added with the test antibodies, and other operations were consistent with those of the experimental groups.

### Data analysis:

%Inhibition was calculated and fitted to give compound IC₅₀. $% Inhibition = 1 - 100 % \times \left(Signal-Bottom\right) / \left(Top-Bottom\right) .$
Signal referred to the signal value of the experimental group, Bottom referred to the mean signal value of the negative control group, and Top referred to the mean signal value of the positive control group.

### Experimental results:

Under the conditions of this experiment, the ADCs of the present disclosure exhibited relatively strong inhibitory activity against the proliferation of the human breast cancer cell line SKBR3 with high Her2 expression.

**Table 9. Anti-tumor cell proliferation activity of ADCs (SKBR3 cell line)**

| Conjugate No. | SKBR3 | |
|---|---|---|
| | IC₅₀ (nM) | Maximum inhibition rate (%) |
| ADC-L1-1 | 0.597 | 75.71 |
| ADC-L2-1^{b} | 0.353 | 76.58 |
| ADC-L5-1 | 0.5208 | 76.68 |
| ADC-L6-1^{a} | 0.3033 | 74.55 |
| ADC-L7-1 | 0.4223 | 76.91 |
| ADC-L8-1 | 0.6384 | 74.29 |
| ADC-L9-1 | 0.5258 | 76.45 |
| ADC-L10-1 | 0.2266 | 77.88 |
| ADC-L12-1 | 0.2877 | 73.65 |
| Control ADC 1 | 3.424 | 68.49 |
| Control ADC2 | 1.275 | 71.94 |

| | | |
|---|---|---|
| ADC-L2-1^{b} was an ADC sample prepared by conjugating trastuzumab with Example 25. ADC-L6-1^{a} was an ADC sample prepared by conjugating trastuzumab with Example 32. | | |

### 3.1.2 Test of anti-proliferative activity of anti-Her2-ADCs against tumor cells NCI-N87

**Cells and materials:** The human gastric cancer cell line NCI-N87 with high HER2 expression was purchased from the Cell Bank of Chinese Academy of Sciences, 1640 medium (Gibco#A10491-01), penicillin-streptomycin (Gibco#15140-122), and 0.25% Trypsin-EDTA (Gibco#25200-056) were purchased from Gibco (USA), 96-well plates (Greiner Bio-one#655098) were purchased from Corning (USA), and Cell-Titer Glo reagent (Promega#G7568) was purchased from Promega (USA).

**Cell culture:** NCI-N87 cells were cultured with a 1640 culture medium containing 10% fetal bovine serum + 1% penicillin-streptomycin. The cells were cultured at 37 °C with 5% CO₂ and were available for the experiment when the cells were in the logarithmic growth phase.

**Cell proliferation activity test:** The inhibitory activity of the ADCs on the proliferation of the NCI-N87 cell line was measured using the Cell-Titer Glo reagent. NCI-N87 cells were digested and pipetted in cell culture flasks, and resuspended using the corresponding fresh medium. The cell density was adjusted. NCI-N87 cells were seeded on a 96-well plate at 6000 cells/90 µL/well and incubated at 37 °C with 5% CO₂ overnight. The ADC concentration was diluted to 5000 nM with a complete medium and subjected to 5-fold gradient dilution to give a total of 8 concentration gradients, and then 10 µL of an ADC diluted solution was transferred to the 96-well plate, i.e., the initial final concentration of the ADC was 500 nM. The 96-well plate was incubated at 37 °C with 5% CO₂ for 3 days. The Cell-Titer Glo reagent was added, and the cell activity was measured.

A negative control group and a positive control group were set as Bottom and Top, respectively. The negative control group was not added with cells, with only the culture medium in the same volume added, and other operations were consistent with those of the experimental groups. The positive control group was not added with the test antibodies, and other operations were consistent with those of the experimental groups.

### Data analysis:

%Inhibition was calculated and fitted to give compound IC₅₀.
$% Inhibition = 1 - 100 % \times \left(Signal-Bottom\right) / \left(Top-Bottom\right) .$

Signal referred to the signal value of the experimental group, Bottom referred to the mean signal value of the negative control group, and Top referred to the mean signal value of the positive control group.

Graphpad curve fitting was performed, and the drug concentration at an inhibition score of 50% was calculated as the absolute IC₅₀.

### Experimental results:

Under the conditions of this experiment, the ADCs of the present disclosure exhibited relatively strong inhibitory activity against the proliferation of the human gastric cancer cell line NCI-N87 with high Her2 expression.

**Table 10. Anti-tumor cell proliferation activity of ADCs (NCI-N87 cell line)**

| Conjugate No. | NCI-N87 | |
|---|---|---|
| | IC₅₀ (nM) | Maximum inhibition rate (%) |
| ADC-L1-1 | 1.57 | 70.9 |
| ADC-L2-1^{b} | 2.56 | 67.9 |
| ADC-L5-1 | 1.74 | 65.8 |
| ADC-L6-1 | 1.30 | 72.5 |
| ADC-L7-1 | 2.30 | 66.1 |
| ADC-L8-1 | 3.19 | 63.9 |
| ADC-L9-1 | 1.82 | 72.3 |
| ADC-L10-1 | 2.09 | 68.0 |
| ADC-L13-1 | 1.34 | 71.8 |
| ADC-L14-1 | 2.94 | 70.1 |
| ADC-L15-1 | 2.85 | 68.4 |
| ADC-L12-1^{a} | 1.18 | 72.3 |
| ADC-L17-1 | 1.82 | 68.7 |
| ADC-L18-1 | 2.09 | 65.5 |
| ADC-L19-1 | 1.95 | 79.1 |
| Control ADC1 | 39.06 | 56.3 |
| Control ADC2 | 7.19 | 65.4 |

| | | |
|---|---|---|
| ADC-L2-1^{b} was an ADC sample prepared by conjugating trastuzumab with Example 25. ADC-L12-1^{a} was an ADC sample prepared by conjugating trastuzumab with Example 37. | | |

### 3.1.3 Test of anti-proliferative activity of anti-Her2-ADCs against tumor cells SKBR3

**Cells and materials:** The human breast cancer cell line SKBR3 with high Her2 expression was purchased from ATCC, McCoy's 5a medium (Gibco#16600-082), penicillin-streptomycin (Gibco#15140-122), and 0.25% Trypsin-EDTA (Gibco#25200-056) were purchased from Gibco (USA), 96-well plates (Greiner Bio-one#655098) were purchased from Corning (USA), and Cell-Titer Glo reagent (Promega#G7568) was purchased from Promega (USA). **Cell culture:** SKBR3 cells were cultured with a McCoy's 5a culture medium containing 10% fetal bovine serum + 1% penicillin-streptomycin at 37 °C with 5% CO₂. Cells at the logarithmic growth phase were available for the experiment.

**Cell proliferation activity test:** The inhibitory activity of the ADCs on the proliferation of the SKBR3 cell line was measured using the Cell-Titer Glo reagent. SKBR3 cells were digested and pipetted in cell culture flasks, and resuspended using the corresponding fresh medium. The cell density was adjusted. SKBR3 cells were seeded on a 96-well plate at 5000 cells/90 µL/well and incubated at 37 °C with 5% CO₂ overnight. The ADC concentration was diluted to 100 nM with a complete medium and subjected to 3-fold gradient dilution to give a total of 8 concentration gradients, and then 10 µL of an ADC diluted solution was transferred to the 96-well plate, i.e., the initial final concentration of the ADC was 10 nM. The 96-well plate was incubated at 37 °C with 5% CO₂ for 3 days. The Cell-Titer Glo reagent was added, and the cell activity was measured.

A negative control group and a positive control group were set as Bottom and Top, respectively. The negative control group was not added with cells, with only the culture medium in the same volume added, and other operations were consistent with those of the experimental groups. The positive control group was not added with the test antibodies, and other operations were consistent with those of the experimental groups.

### Data analysis:

%Inhibition was calculated and fitted to give compound IC₅₀.
$% Inhibition = 1 - 100 % \times \left(Signal-Bottom\right) / \left(Top-Bottom\right) .$

Signal referred to the signal value of the experimental group, Bottom referred to the mean signal value of the negative control group, and Top referred to the mean signal value of the positive control group.

### Experimental results:

Under the conditions of this experiment, the ADCs of the present disclosure exhibited relatively strong inhibitory activity against the proliferation of the human breast cancer cell line SKBR3 with high Her2 expression.

**Table 11. Anti-tumor cell proliferation activity of ADCs (SKBR3 cell line)**

| Conjugate No. | SKBR3 | |
|---|---|---|
| | IC₅₀ (nM) | Maximum inhibition rate (%) |
| ADC-L26-4(DAR4) | 0.025 | 83.6 |
| ADC-L26-4(DAR8) | 0.01 | 83.4 |
| ADC-L27-5(DAR4) | 0.042 | 83.9 |
| ADC-L27-5(DAR8) | 0.021 | 87.4 |

### 3.1.4 Test of anti-proliferative activity of anti-Her2-ADCs against tumor cells NCI-N87

**Cells and materials:** The human gastric cancer cell line NCI-N87 with high Her2 expression was purchased from the Cell Bank of Chinese Academy of Sciences, 1640 medium (Gibco#A10491-01), penicillin-streptomycin (Gibco#15140-122), and 0.25% Trypsin-EDTA (Gibco#25200-056) were purchased from Gibco (USA), 96-well plates (Greiner Bio-one#655098) were purchased from Corning (USA), and Cell-Titer Glo reagent (Promega#G7568) was purchased from Promega (USA).

**Cell culture:** NCI-N87 cells were cultured with a 1640 culture medium containing 10% fetal bovine serum + 1% penicillin-streptomycin. The cells were cultured at 37 °C with 5% CO₂ and were available for the experiment when the cells were in the logarithmic growth phase.

**Cell proliferation activity test:** The inhibitory activity of the ADCs on the proliferation of the NCI-N87 cell line was measured using the Cell-Titer Glo reagent. NCI-N87 cells were digested and pipetted in cell culture flasks, and resuspended using the corresponding fresh medium. The cell density was adjusted. NCI-N87 cells were seeded on a 96-well plate at 3000 cells/90 µL/well and incubated at 37 °C with 5% CO₂ overnight. The ADC concentration was diluted to 250 nM with a complete medium and subjected to 4-fold gradient dilution to give a total of 8 concentration gradients per ADC, and then 10 µL of an ADC diluted solution was transferred to the 96-well plate, i.e., the initial final concentration of the ADC was 25 nM. The 96-well plate was incubated at 37 °C with 5% CO₂ for 5 days. The Cell-Titer Glo reagent was added, and the cell activity was measured.

A negative control group and a positive control group were set as Bottom and Top, respectively. The negative control group was not added with cells, with only the culture medium in the same volume added, and other operations were consistent with those of the experimental groups. The positive control group was not added with the test antibodies, and other operations were consistent with those of the experimental groups.

### Data analysis:

%Inhibition was calculated and fitted to give compound IC₅₀.
$% Inhibition = 1 - 100 % \times \left(Signal-Bottom\right) / \left(Top-Bottom\right) .$

Signal referred to the signal value of the experimental group, Bottom referred to the mean signal value of the negative control group, and Top referred to the mean signal value of the positive control group.

Graphpad curve fitting was performed, and the drug concentration at an inhibition score of 50% was calculated as the absolute IC₅₀.

### Experimental results:

Under the conditions of this experiment, the ADCs of the present disclosure exhibited relatively strong inhibitory activity against the proliferation of the human gastric cancer cell line NCI-N87 with high Her2 expression.

**Table 12. Anti-tumor cell proliferation activity of ADCs (NCI-N87 cell line)**

| Conjugate No. | NCI-N87 | |
|---|---|---|
| | IC₅₀ (nM) | Maximum inhibition rate (%) |
| ADC-L26-4(DAR4) | 0.03 | 86 |
| ADC-L26-4(DAR8) | 0.012 | 88.9 |
| ADC-L27-5(DAR4) | 0.098 | 91.6 |
| ADC-L27-5(DAR8) | 0.053 | 92.7 |
| Control ADC5 | >25 | 46.7 |
| ADC-L22-3(DAR4) | 1.846 | 54.6 |
| ADC-L22-3(DAR8) | 0.45 | 70.5 |

### 3.1.5 Test of anti-proliferative activity of anti-EGFR-cMet-ADC against tumor cells

**Cells and materials:** HCT116 cells (Cat. No. CCL-247) with medium EGFR expression and medium cMEt expression were purchased from ATCC, and the medium was a McCoy's 5a medium containing 10% FBS. Capan-1 cells (Cat. No. CBP60543) with low EGFR expression and low cMEt expression were purchased from Nanjing Cobioer Biosciences Co., Ltd., and the medium was an IMDM medium containing 20% FBS. PC9-hEGFR-Del19-T790M-C797S (PC9-DTC) cells with high EGFR expression and high cMEt expression were constructed by Crown Bioscience (Taicang) Co., Ltd., and the medium was an RPMI1640 medium containing 10% FBS and 0.5 µg/mL puromycin. HCC827 cells (Cat. No. CRL-2868) with high EGFR expression and medium cMet expression were purchased from ATCC, and the medium was an RPMI 1640 medium containing 10% FBS.

**Cell proliferation activity test:** HCT116, Capan-1, PC9-DTC, and HCC827 cells were digested with trypsin (purchased from Gibco, Cat. No. 25200072), and then resuspended using their respective corresponding media. The cell density was adjusted. The cells were seeded on a 96-well plate (purchased from corning, Cat. No. 3610) at the following cell densities: 0.6 × 10³ cells/90 µL for HCT116 cells, 4.0 × 10³ cells/90 µL for Capan-1 cells, 1.8 × 10³ cells/90 µL for PC9-DTC cells, and 3.0 × 10³ cells/90 µL for HCC827 cells, and the plate was incubated overnight in an incubator at 37 °C with 5% CO₂. The next day, the antibody-drug conjugate was serially diluted with the complete medium according to the experimental design, and 10 µL of the diluted antibody-drug conjugate was transferred to the corresponding well plate. The cell plate was incubated in an incubator at 37 °C with 5% CO₂ for 5 days. After 5 days, 50 µL of KeyTec^{®} Luminescent Cell Viability Detection Kit detection reagent (purchased from vkey-bio, Cat. No. A2010003N, see the product instructions for use) was added to each well, and the fluorescence values were read on an Envision instrument (purchased from PerkinElmer, model: Envision 2105) to measure the cell viability.

A positive control group and a negative control group were set as 0% and 100% killing controls, respectively. The positive control group was not added with test drugs, and other operations were consistent with those in the experimental group. The negative control group was not added with cells, with only the culture medium in the same volume added, and other operations were consistent with those in the experimental group. The formula for calculating the cell proliferation inhibition rate: inhibition rate = ((positive control - sample well reading)/(positive control - negative well reading)) × 100%. The inhibition rate of the corresponding drug on different cells was obtained by this formula, where the maximum inhibition rate was expressed as Emax. Plotting was performed using the GraphPad Prism 9.0 software, and IC₅₀ was calculated.

The results are shown in Table 13. The anti-EGFR-cMet-ADC drugs had relatively good proliferation inhibitory activity against tumor cells expressing EGFR and cMET *in vitro.*

**Table 13. Inhibition of tumor cell proliferation with varying EGFR and cMET expression levels**

| Conjugate No. | HCT116 | | Capan-1 | | PC9-DTC | | HCC827 | |
|---|---|---|---|---|---|---|---|---|
| | IC₅₀ (nM) | Emax (%) | IC₅₀ (nM) | Emax (%) | IC₅₀ (nM) | Emax (%) | IC₅₀ (nM) | Emax (%) |
| ADC2-L19-1 | 6.25 | 80.10 | 16.72 | 69.27 | 12.98 | 68.32 | >100 | 47.51 |
| Control ADC-ISO-2 | >100 | 30.98 | >100 | 12.03 | >100 | 2.51 | >100 | 1.76 |

### 3.2 Evaluation of in vivo efficacy of anti-EGFR-cMet-ADC

### 3.2.1 Evaluation of efficacy of anti-EGFR-cMet-ADC in HCC827 subcutaneous tumor model

### Experimental reagents:

Lung cancer HCC827 cells: with high EGFR and cMet expression, from Chinese Academy of Sciences, Shanghai RPMI-1640 culture medium: Gibco; Cat No.: A104910
Fetal bovine serum: Gibco; Cat No.: 10099-141C
0.25% trypsin-EDTA: Gibco, Cat No.: 25200-072
D-PBS (phosphate-buffered saline without calcium and magnesium ions): Hyclone, Cat. No.: SH30256.01
Matrigel: Corning, Cat. No.: 356237

### Procedures:

Animal information: Balb/c nude mice, female, 5-6 weeks, body weight about 14-20 g, purchased from Beijing Vital River Biotechnology Co., Ltd. The mice were bred in an SPF-rated environment with air exhaust for each cage individually. All animals had free access to standard certified commercial laboratory diets and water.

Cell culture: The lung cancer HCC827 cell line was cultured *in vitro* under the conditions of RPMI-1640 (cell culture medium) added with 10% fetal bovine serum and 1% Pen Strep, and an incubator at 37 °C with 5% CO₂. The cells were digested with a 0.25% trypsin-EDTA digestive solution twice a week for passaging as per conventional practice. When the cell saturation was 80%-90% and a required amount was achieved, the cells were collected and counted.

Cell inoculation: a 0.2 mL/(containing 1 × 10⁷) HCC827 cell suspension (RPMI-1640: Matrigel, volume ratio 1:1) was inoculated subcutaneously into the lumbar dorsal part of each mouse. On day 8 after inoculation of the cells, the mice were randomly grouped and administered based on the tumor volume. The day when random grouping was performed was day 0.

Administration: ADC2-L19-1, control ADC10, and control ADC-ISO-2 were each administered at a dose of 1.5 mg/kg via a single intravenous injection (IV). Each group had 6 mice.

### Tumor measurement and experimental indices:

Tumor diameters were measured twice a week using a vernier caliper. The tumor volume was calculated using the following formula: V = 0.5a × b², where a and b represent the long diameter and short diameter of the tumor, respectively. Mouse body weight was measured twice a week.

The anti-tumor therapeutic effect of the compound was evaluated by tumor growth inhibition rate TGI (%). TGI (%) = [(1 - (mean tumor volume at the end of administration in a treatment group - mean tumor volume at the start of administration of the treatment group))/(mean tumor volume at the end of treatment of the solvent control group - mean tumor volume at the start of treatment of the solvent control group)] × 100%.

### Experimental results:

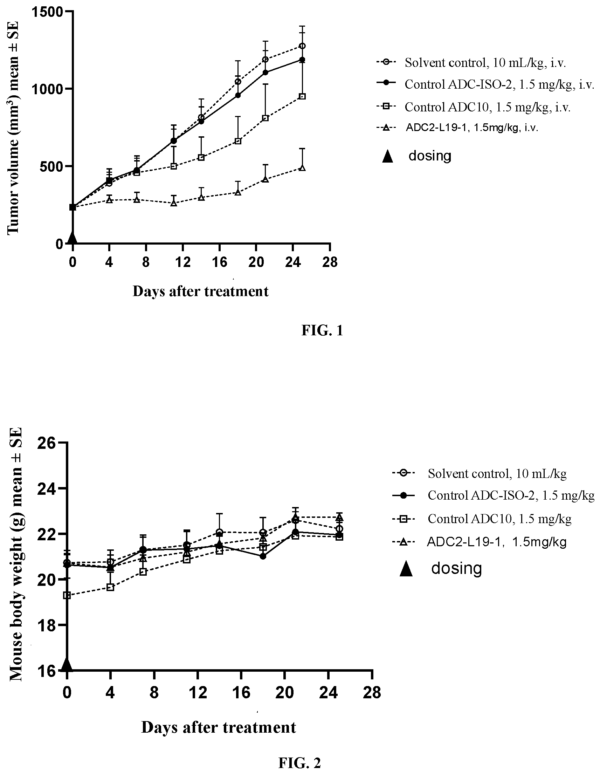

See Table 14, FIG. 1, and FIG. 2.

**Table 14. HCC827 subcutaneous tumor model tumor volume**

| **Test compound** | **Dose** | **Administration regimen** | **Administration frequency** | **Mean tumor volume (mm³)** | | | | | | | | **TGI (%)** |
|---|---|---|---|---|---|---|---|---|---|---|---|---|
| | **mg/kg** | | | **Day 0** | **Day 4** | **Day 7** | **Day 11** | **Day 14** | **Day 18** | **Day 21** | **Day 25** | |
| Solvent control | / | IV | Single administration | 234.8 | 388.72 | 473.72 | 664.44 | 816.91 | 1046.12 | 1189.86 | 1277.59 | / |
| Control ADC10 | 1.5 | IV | Single administration | 234.5 3 | 407.0 7 | 457.9 9 | 499.4 5 | 557.0 4 | 661.94 | 810. 87 | 950.58 | 31.33 |
| ADC2-L19-1 | 1.5 | IV | Single administration | 234.7 5 | 281.0 3 | 284.4 4 | 262.1 6 | 298.6 4 | 330.3 | 415. 69 | 489.11 | 75.61 |
| Control ADC-ISO-2 | 1.5 | IV | Single administration | 234.8 9 | 411.4 7 | 476.7 3 | 665.2 6 | 788.1 | 958.04 | 1105. 85 | 1189.1 1 | 8.49 |

### Experimental conclusion:

In the mouse subcutaneous xenograft tumor HCC827 model, the compound ADC2-L19-1 of the present disclosure had a significant inhibitory effect on tumor growth (P < 0.0001) after a single administration at 1.5 mg/kg via an intravenous injection and was significantly superior to control ADC10 and control ADC-ISO-2 at the same dose (P < 0.05, P < 0.0001). The ADC molecules of the present disclosure were not found to affect the body weight of the mice or cause any death of the mice at the test dose described above, and the mice could tolerate the dose.

### 3.2.2 Evaluation of efficacy of anti-EGFR-cMet-ADC in HCT116 subcutaneous tumor model

### Experimental reagents:

Intestinal cancer HCT116 cells: ATCC
McCOY' 5A culture medium: Gbico; Cat No.: 16600-082
Fetal bovine serum: Gibco; Cat No.: 10099-141C
0.25% trypsin-EDTA: Gibco, Cat No.: 25200-072
D-PBS (phosphate-buffered saline without calcium and magnesium ions): Hyclone, Cat. No.: SH30256.01
Matrigel: Corning, Cat. No.: 356237

### Procedures:

Animal information: Balb/c nude mice, female, 5-6 weeks, body weight about 14-20 g, purchased from Beijing Vital River Biotechnology Co., Ltd. The mice were bred in an SPF-rated environment with air exhaust for each cage individually. All animals had free access to standard certified commercial laboratory diets and water.

Cell culture: The intestinal cancer HCT116 cell line was cultured *in vitro* under the conditions of McCOY' 5A (cell culture medium) added with 10% fetal bovine serum and 1% Pen Strep, and an incubator at 37 °C with 5% CO₂.

The cells were digested with a 0.25% trypsin-EDTA digestive solution twice a week for passaging as per conventional practice. When the cell saturation was 80%-90% and a required amount was achieved, the cells were collected and counted.

Cell inoculation: a 0.1 mL/(containing 2.5 × 10⁶) HCT116 cell suspension (RPMI-1640: Matrigel, volume ratio 1: 1) was inoculated subcutaneously into the lumbar dorsal part of each mouse. On day 7 after inoculation of the cells, the mice were randomly grouped and administered based on the tumor volume. The day when random grouping was performed was day 0.

Administration: Control ADC10 and ADC2-L19-1 were administered at a dose of 1.5 mg/kg via an intravenous injection (IV) on day 0 and day 14. Each group had 6 mice.

### Tumor measurement and experimental indices:

Tumor diameters were measured twice a week using a vernier caliper. The tumor volume was calculated using the following formula: V = 0.5a × b², where a and b represent the long diameter and short diameter of the tumor, respectively. Mouse body weight was measured twice a week.

The anti-tumor therapeutic effect of the compound was evaluated by tumor growth inhibition rate TGI (%). TGI (%) = [(1 - (mean tumor volume at the end of administration in a treatment group - mean tumor volume at the start of administration of the treatment group))/(mean tumor volume at the end of treatment of the solvent control group - mean tumor volume at the start of treatment of the solvent control group)] × 100%.

### Experimental results:

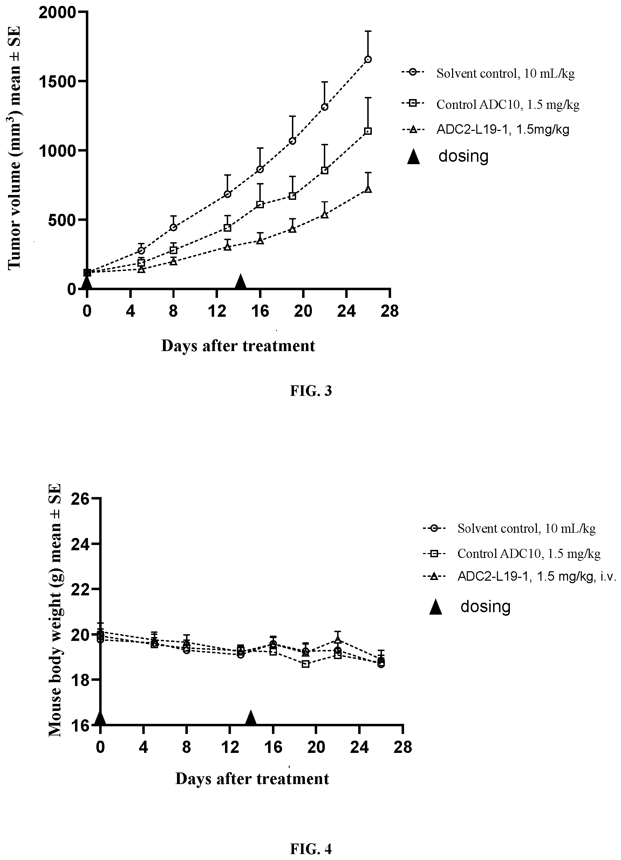

See Table 15, FIG. 3, and FIG. 4.

**Table 15. HCT116 subcutaneous tumor model tumor volume**

| **Test compound** | **Dose** | **Administration regimen** | **Administration frequency** | **Mean tumor volume (mm³)** | | | | | | | | **TGI (%)** |
|---|---|---|---|---|---|---|---|---|---|---|---|---|
| | **mg/kg** | | | **Day 0** | **Day 5** | **Day 8** | **Day 13** | **Day 16** | **Day 19** | **Day 22** | **Day 26** | |
| Solvent control | / | IV | Day 0 | 117.0 8 | 276.2 2 | 443.9 4 | 684.1 3 | 862.1 7 | 1067.9 7 | 1313.0 3 | 1656.7 6 | / |
| | | | Day 14 | | | | | | | | | |
| Control ADC10 | 1.5 | IV | Day 0 | 117.5 5 | 186.5 4 | 278.4 | 440.6 4 | 607.8 9 | 669.18 | 855.5 | 1139.4 4 | 33.6 3 |
| | | | Day 14 | | | | | | | | | |
| ADC2-L19-1 | 1.5 | IV | Day 0 | 117.67 | 142.23 | 196.75 | 302.96 | 348.11 | 433.9 | 536.37 | 719.61 | 60.9 |
| | | | Day 14 | | | | | | | | | |

### Experimental conclusion:

In the mouse subcutaneous xenograft tumor HCT1 16 model, control ADC10 and ADC2-L19-1 both had a significant inhibitory effect on tumor growth (P < 0.01, P < 0.0001) after two administrations at 1.5 mg/kg via an intravenous injection (day 0 and day 14), and ADC2-L19-1 was significantly superior to control ADC10 (P < 0.05). The ADC molecules of the present disclosure were not found to affect the body weight of the mice or cause any death of the mice at the test dose described above, and the mice could tolerate the dose.

### 3.2.3 Evaluation of efficacy of anti-EGFR-cMet-ADC in PC9-6 subcutaneous tumor model

### Experimental reagents:

Human lung cancer PC9-6 cells: (PC9-6 was engineered from PC9 blast cells by Crown Bioscience, hEGFR-Del19-T790M-C797S)
RPMI-1640 culture medium: Gbico; Cat No.: A104910
Fetal bovine serum: Gibco; Cat No.: 10099-141C
0.25% trypsin-EDTA: Gibco, Cat No.: 25200-072
D-PBS (phosphate-buffered saline without calcium and magnesium ions): Hyclone, Cat. No.: SH30256.01 Matrigel: Corning, Cat. No.: 356237

### Procedures:

Animal information: Balb/c nude mice, female, 5-6 weeks, body weight about 14-20 g, purchased from Beijing Vital River Biotechnology Co., Ltd. The mice were bred in an SPF-rated environment with air exhaust for each cage individually. All animals had free access to standard certified commercial laboratory diets and water.

Cell culture: The human lung cancer PC9-6 cell line was cultured *in vitro* under the conditions of RPMI-1640 (cell culture medium) containing 10% fetal bovine serum, 1% Pen Strep, and 0.5 µg/mL puromycin, and an incubator at 37 °C with 5% CO2. The cells were digested with a 0.25% trypsin-EDTA digestive solution twice a week for passaging as per conventional practice. When the cell saturation was 80%-90% and a required amount was achieved, the cells were collected and counted.

Cell inoculation: a 0.1 mL/(containing 5 × 10⁶) PC9-6 cell suspension (RPMI-1640: Matrigel, volume ratio 1:1) was inoculated subcutaneously into the lumbar dorsal part of each mouse. On day 7 after inoculation of the cells, the mice were randomly grouped and administered based on the tumor volume. The day when random grouping was performed was day 0.

Administration: Control ADC10 and ADC2-L19-1 were administered at a dose of 1.5 mg/kg via an intravenous injection (IV) on day 0, day 8, and day 14 after grouping. Each group had 6 mice.

### Tumor measurement and experimental indices:

Tumor diameters were measured twice a week using a vernier caliper. The tumor volume was calculated using the following formula: V = 0.5a × b², where a and b represent the long diameter and short diameter of the tumor, respectively. Mouse body weight was measured twice a week.

The anti-tumor therapeutic effect of the compound was evaluated by tumor growth inhibition rate TGI (%). TGI (%) = [(1 - (mean tumor volume at the end of administration in a treatment group - mean tumor volume at the start of administration of the treatment group))/(mean tumor volume at the end of treatment of the solvent control group - mean tumor volume at the start of treatment of the solvent control group)] × 100%.

### Experimental results:

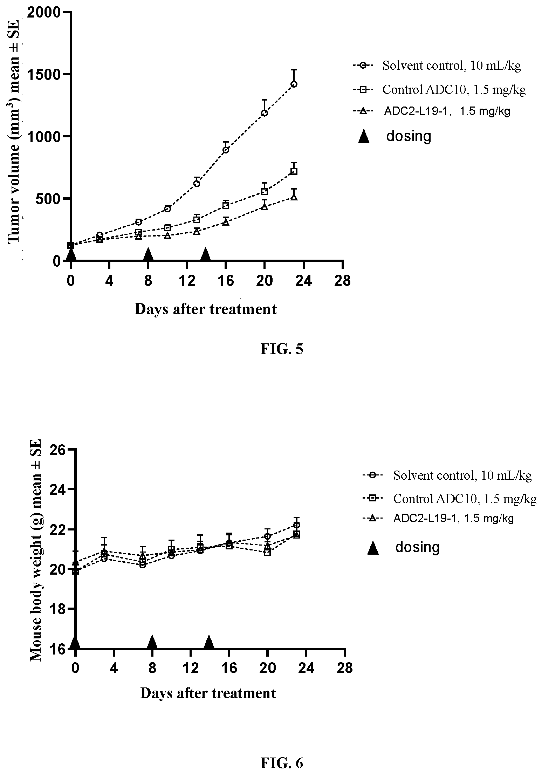

See Table 16, FIG. 5, and FIG. 6.

**Table 16. PC9-6 subcutaneous tumor model tumor volume**

| **Test compound** | **Dose** | **Administration regimen** | **Administration frequency** | **Mean tumor volume (mm³)** | | | | | | | | **TGI (%)** |
|---|---|---|---|---|---|---|---|---|---|---|---|---|
| | **mg/kg** | | | **Day 0** | **Day 3** | **Day 7** | **Day 10** | **Day 13** | **Day 16** | **Day 20** | **Day 23** | |
| Solvent control | / | IV | Day 0 | 123.42 | 204.26 | 309.64 | 415.86 | 618.8 4 | 890.5 5 | 1184.7 4 | 1418.0 7 | / |
| | | | Day 8 | | | | | | | | | |
| | | | Day 14 | | | | | | | | | |
| Control ADC10 | 1.5 | IV | Day 0 | 123.44 | 170.94 | 230.02 | 263.57 | 327.7 6 | 442.7 7 | 555.2 | 718.84 | 54.01 |
| | | | Day 8 | | | | | | | | | |
| | | | Day 14 | | | | | | | | | |
| ADC2-L19-1 | 1.5 | IV | Day 0 | 123.56 | 167.92 | 195.17 | 202.49 | 234.8 | 309.0 1 | 432.97 | 511.13 | 70.06 |
| | | | Day 8 | | | | | | | | | |
| | | | Day 14 | | | | | | | | | |

### Experimental conclusion:

In the mouse subcutaneous xenograft tumor PC9-6 model, in the case of 3 administrations via an intravenous injection at 1.5 mg/kg (day 0, day 8, and day 14), the compound ADC2-L19-1 of the present disclosure and control ADC10 both had a significant inhibitory effect on tumor growth (P < 0.0001, P < 0.0001), and ADC2-L19-1 was significantly superior to control ADC 10 (P < 0.05). The ADC molecules of the present disclosure were not found to affect the body weight of the mice or cause any death of the mice at the test administration dose described above, and the mice could tolerate the dose.

### 3.3 Evaluation of efficacy of CDH-6 ADC in PA-1 subcutaneous tumor model

**Animal information:** BALB/c nude mice, female, 6-8 weeks, body weight about 16-22 g, purchased from Beijing Vital River Biotechnology Co., Ltd. The mice were bred in an SPF-rated environment with air exhaust for each cage individually. All animals had free access to standard certified commercial laboratory diets and water.

**Cell information:** The human ovarian cancer PA-1 cell line with medium human CDH6 expression (from Nanjing Cobioer) was cultured *in vitro* under the conditions of MEM (Gbico; Cat No.: 32561-037) added with 10% fetal bovine serum (Gbico; Cat No.: 10091-148), 1% Pen Strep, 1% NEAA, and 1 mM sodium pyruvate (Gbico; Cat No.: 11360-070), and an incubator at 37 °C with 5% CO₂. The cells were digested with a versene digestive solution (Gbico; Cat No.: 15040-066) once a week for passaging as per conventional practice. When the cell saturation was 80%-90% and a required amount was achieved, the cells were collected and counted.

**Cell inoculation:** a 0.2 mL/(containing 1 × 10⁷) PA-1 cell suspension (MEM: Matrigel, volume ratio 1:1) was inoculated subcutaneously into the axilla of each mouse. On day 14 after inoculation of the cells, the mice were randomly grouped and administered based on the tumor volume. The day when random grouping was performed was day 0.

**Administration:** ADC3-L19-1, ADC3-L12-1, control ADC11, control ADC-ISO-1, and control ADC-ISO-2 were administered at a dose of 1 mg/kg via a single intravenous injection on day 0, with 6 mice in each group.

**Tumor measurements and experimental indices:** Tumor diameters were measured twice a week using a vernier caliper. The tumor volume was calculated using the following formula: V = 0.5a × b², where a and b represent the long diameter and short diameter of the tumor, respectively. Mouse body weight was measured twice a week.

The anti-tumor therapeutic effect of the compound was evaluated by tumor growth inhibition rate TGI (%). TGI (%) = [(1 - (mean tumor volume at the end of administration in a treatment group - mean tumor volume at the start of administration of the treatment group))/(mean tumor volume at the end of treatment of the solvent control group - mean tumor volume at the start of treatment of the solvent control group)] × 100%.

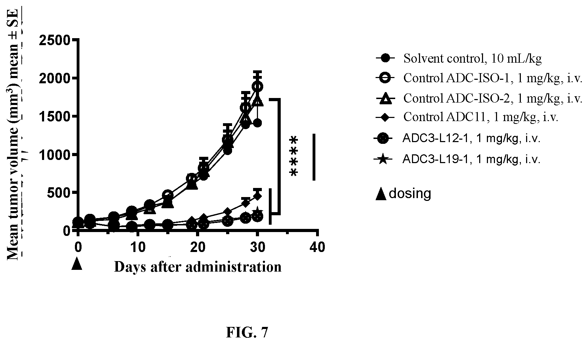

**Experimental results:** As shown in Table 17 and FIG. 7, in the mouse subcutaneous xenograft tumor PA-1 model, compared with the solvent control group, control ADC11, ADC3-L19-1, and ADC3-L12-1 after a single administration via an intravenous injection at a dose of 1 mg/kg all had a significant inhibitory effect on tumor growth (P < 0.0001, P < 0.0001, P < 0.0001); compared with control ADC11, ADC3-L19-1 and ADC3-L12-1 had better TGI on day 30 and showed greater tumor inhibition potential. The ADC molecules of the present disclosure were not found to affect the body weight of the mice or cause any death of the mice at the test administration dose described above, and the mice could tolerate the dose.

**Table 17. PA-1 subcutaneous tumor model tumor volume**

| Administration group | Administration dose and frequency | **Day 30** | | | |
|---|---|---|---|---|---|
| | | **Mean tumor size (mm³)** | **TGI (%)** | **P value** | **Change in body weight of experimental animal (%)** |
| Solvent control | NA | 1412.80 | NA | NA | 12.95 |
| Control ADC-ISO-1 | 1 mpk, single administration | 1890.13 | -36.59 | **** | 18.52 |
| Control ADC-ISO-2 | 1 mpk, single administration | 1704.16 | -22.32 | **** | 20.02 |
| Control ADC11 | 1 mpk, single administration | 451.20 | 73.71 | **** | 9.81 |
| ADC3-L12-1 | 1 mpk, single administration | 186.38 | 94.06 | **** | 12.72 |
| ADC3-L19-1 | 1 mpk, single administration | 241.50 | 89.79 | **** | 10.40 |

## Claims

1. A ligand-drug conjugate having a structural general formula of Pc-(L-D)ₙ or a pharmaceutically acceptable salt thereof, wherein
Pc is a ligand unit;
D is a drug unit;
L is a linker unit of the following structure, wherein the a-terminus is linked to the ligand unit, 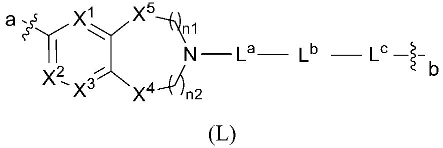
wherein
X¹, X², and X³ are each independently selected from N or CR^{a1}, and at least one of X¹, X², and X³ is selected from N, wherein R^{a1} is selected from H, halogen, CN, C₁-C₃ alkyl, C₂-C₃ alkenyl, C₂-C₃ alkynyl, C₃-C₆ cycloalkyl, or 4-to 7-membered heterocyclyl;
n1 and n2 are each independently selected from 0, 1, or 2;
X⁴ and X⁵ are each independently selected from C(=O), C(R^{b1})(R^{b2}), O, S, S(=O), S(=O)₂, NR^{b3}, or any combination thereof, wherein R^{b1}, R^{b2}, and R^{b3} are each independently selected from H or R^{c1}, or R^{b1} and R^{b2}, together with the carbon atom to which they are attached, form C₃-C₆ cycloalkyl or 4- to 7-membered heterocyclyl, wherein the C₃-C₆ cycloalkyl or 4- to 7-membered heterocyclyl is optionally substituted with one or more R^{c1};
each R^{c1} is independently selected from halogen, CN, C₁-C₆ alkyl, C₃-C₆ cycloalkyl, or a hydrophilic group;
L^{a} is selected from 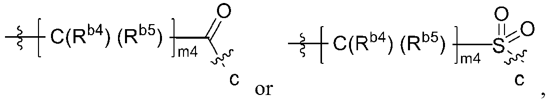
wherein R^{b4} and R^{b5} are each independently selected from H, halogen, CN, C₁-C₃ alkyl, OH, O(C₁-C₃ alkyl), NH₂, NH(C₁-C₃ alkyl), or N(C₁-C₃ alkyl)₂, or R^{b4} and R^{b5}, together with the carbon atom to which they are attached, form C₃-C₆ cycloalkyl or 4- to 7-membered heterocyclyl, m4 is selected from integers of 1-10, and the c-terminus of L^{a} is linked to L^{b};
L^{b} is selected from a chemical bond or 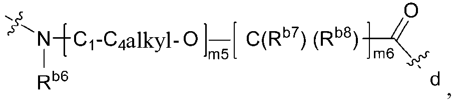
wherein m5 is selected from integers of 0-24, m6 is selected from integers of 1-10, each R^{b6}, each R^{b7}, and each R^{b8} are each independently selected from H or R^{c6}, wherein R^{c6} is selected from halogen, CN, C₁-C₆ alkyl, C₃-C₆ cycloalkyl, or a hydrophilic group, and the d-terminus of L^{b} is linked to L^{c};
L^{c} is selected from Y¹-Y²-Y³, wherein Y¹ is selected from a chemical bond or 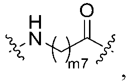
m7 is selected from integers of 1-6, Y² is selected from a peptide residue consisting of 2-10 amino acids, Y³ is selected from 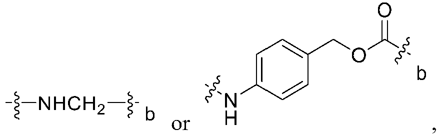 and the b-terminus of L^{c} is linked to drug unit D;
n is a real number of 1-16.

2. The ligand-drug conjugate or the pharmaceutically acceptable salt thereof according to claim 1, wherein X¹ and X² are both selected from N, and X³ is selected from CH.

3. The ligand-drug conjugate or the pharmaceutically acceptable salt thereof according to claim 1 or 2, wherein n1 and n2 are each independently selected from 0 or 1; or, n1 is selected from 1, and n2 is selected from 0.

4. The ligand-drug conjugate or the pharmaceutically acceptable salt thereof according to any one of claims 1-3, wherein X⁴ and X⁵ are each independently selected from C(=O) or C(R^{b1})(R^{b2}), wherein R^{b1} and R^{b2} are each independently selected from H or R^{c1}, or R^{b1} and R^{b2}, together with the carbon atom to which they are attached, form C₃-C₆ cycloalkyl or 4- to 7-membered heterocyclyl, wherein the C₃-C₆ cycloalkyl or 4- to 7-membered heterocyclyl is optionally substituted with one or more R^{c1}; or,
X⁴ is selected from C(=O), and X⁵ is selected from C(R^{b1})(R^{b2}), wherein R^{b1} and R^{b2} are each independently selected from H or R^{c1}, or R^{b1} and R^{b2}, together with the carbon atom to which they are attached, form C₃-C₆ cycloalkyl or 4- to 7-membered heterocyclyl, wherein the C₃-C₆ cycloalkyl or 4- to 7-membered heterocyclyl is optionally substituted with one or more R^{c1}; or,
X⁴ is selected from C(=O), and X⁵ is selected from C(R^{b1})(R^{b2}), wherein R^{b1} and R^{b2} are each independently selected from H or R^{c1}, or R^{b1} and R^{b2}, together with the carbon atom to which they are attached, form C₄-C₆ cycloalkyl or 4- to 6-membered heterocyclyl containing one O atom or one N atom, wherein the 4- to 6-membered heterocyclyl containing one O atom or one N atom is optionally substituted with one R^{c1}.

5. The ligand-drug conjugate or the pharmaceutically acceptable salt thereof according to any one of claims 1-4,
wherein L^{b} is selected from a chemical bond, 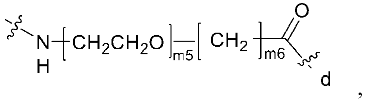 or 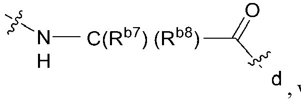
wherein m5 is selected from integers of 1-16, or, m5 is selected from 1, 2, 3, 4, 5, 6, 7, 8, 9, 10, 11, 12, 13, 14, 15, or 16; m6 is selected from integers of 1-6, or, m6 is selected from 1, 2, 3, 4, 5, or 6, or, m6 is 2; R^{b7} and R^{b8} are each independently selected from H or R^{c6}; the d-terminus of L^{b} is linked to L^{c}; or,
L^{b} is selected from a chemical bond or 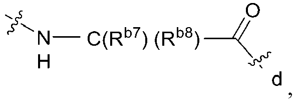 wherein R^{b7} is H, R^{b8} is R^{c6}, and the d-terminus of L^{b} is linked to L°.

6. The ligand-drug conjugate or the pharmaceutically acceptable salt thereof according to any one of claims 1-5, wherein each R^{c1} and each R^{c6} are each independently selected from halogen, CN, C₁-C₆ alkyl, C₃-C₆ cycloalkyl, or a hydrophilic group comprising one or more of the following atomic groups or structural fragments: C(=O), OH, O(C₁-C₃ alkyl), NH₂, NH(C₁-C₃ alkyl), N(C₁-C₃ alkyl)₂, C₁-C₄ alkyl-O, NHC(=O), N(CH₃)C(=O), C(=O)NH₂, S(=O)₂NH₂, C(=O)NH(C₁-C₃ alkyl), S(=O)₂NH(C₁-C₃ alkyl), C(=O)N(C₁-C₃ alkyl)₂, S(=O)₂N(C₁-C₃ alkyl)₂, 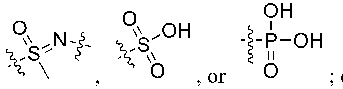 or, each R^{c1} and each R^{c6} are independently selected from halogen, CN, C₁-C₆ alkyl, C₃-C₆ cycloalkyl, or W¹-W²-W³-W⁴-W⁵, wherein each W¹, each W², each W³, and each W⁴ are each independently selected from a chemical bond, A-(C₁-C₄ alkyl-O)ₘ₁, or [A-(CR^{d1}R^{d2})ₘ₂-NR^{d3}]ₘ₃-B, wherein each A and each B are independently selected from a chemical bond, C₁-C₁₀ alkylene, C₃-C₆ cycloalkylene, 4- to 7-membered heterocyclylene, O, S, NR^{d4}, C=O,
C(=O)NR^{d5}, S(=O)₂, S(=O)₂NR^{d6}, S(=O)(=NR^{d7}), 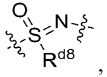
or any combination thereof, wherein R^{d1}, R^{d2}, R^{d3}, R^{d4}, R^{d5}, R^{d6}, R^{d7}, and R^{d8} are each independently selected from H, C₁-C₆ alkyl, C₃-C₆ cycloalkyl, 4- to 7-membered heterocyclyl, or (C₁-C₄ alkyl-O)ₖ₁-C₁-C₆ alkyl, wherein the C₁-C₆ alkyl, C₃-C₆ cycloalkyl, or 4- to 7-membered heterocyclyl is optionally substituted with halogen, CN, OH, O(C₁-C₃ alkyl), NH₂, NH(C₁-C₃ alkyl), N(C₁-C₃ alkyl)₂, C(=O)NH₂, NHC(=O)NH₂, or COOH; m1 is selected from integers of 0-30, m2 is selected from integers of 1-5, and m3 and k1 are each independently selected from integers of 1-30; W⁵ is selected from H, C₁-C₆ alkyl, C₃-C₆ cycloalkyl, 4- to 7-membered heterocyclyl, OH, O(C₁-C₃ alkyl), NH₂, NH(C₁-C₃ alkyl), N(C₁-C₃ alkyl)₂, C(=O)NH₂, S(=O)₂NH₂, C(=O)NH(C₁-C₃ alkyl), S(=O)₂NH(C₁-C₃ alkyl), C(=O)N(C₁-C₃ alkyl)₂, S(=O)₂N(C₁-C₃
alkyl)₂, 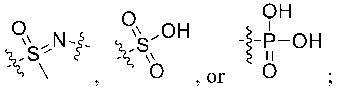 or, each R^{c1} and each R^{c6} are independently selected from W¹-W²-W³-W⁴-W⁵, wherein W¹ and W² are both selected from a chemical bond, W³ is selected from a chemical bond or A-(C₁-C₄ alkyl-O)ₘ₁, and W⁴ is selected from A-(C₁-C₄ alkyl-O)ₘ₁ or [A-(CR^{d1}R^{d2} )ₘ₂-NR^{d3}]ₘ₃-B, wherein each A and each B are independently selected from a chemical bond, C₁-C₁₀ alkylene, C₃-C₆ cycloalkylene, 4- to 7-membered heterocyclylene, O, S, NR^{d4}, C=O, C(=O)NR^{d5},
S(=O)₂, S(=O)₂NR^{d6}, S(=O)(-NR^{d7}), 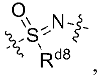
or any combination thereof, wherein R^{d1}, R^{d2}, R^{d3}, R^{d4}, R^{d5}, R^{d6}, R^{d7}, and R^{d8} are each independently selected from H, C₁-C₆ alkyl, C₃-C₆ cycloalkyl, 4- to 7-membered heterocyclyl, or (C₁-C₄ alkyl-O)ₖ₁-C₁-C₆ alkyl, wherein the C₁-C₆ alkyl, C₃-C₆ cycloalkyl, or 4- to 7-membered heterocyclyl is optionally substituted with halogen, CN, OH, O(C₁-C₃ alkyl), NH₂, NH(C₁-C₃ alkyl), N(C₁-C₃ alkyl)₂, C(=O)NH₂, NHC(=O)NH₂, or COOH; m1 is selected from integers of 0-30, m2 is selected from integers of 1-5, and m3 and k1 are each independently selected from integers of 1-30; W⁵ is selected from H, C₁-C₆ alkyl, C₃-C₆ cycloalkyl, 4- to 7-membered heterocyclyl, OH, O(C₁-C₃ alkyl), NH₂, NH(C₁-C₃ alkyl), N(C₁-C₃ alkyl)₂, C(=O)NH₂, S(=O)₂NH₂,
C(=O)NH(C₁-C₃ alkyl), S(=O)₂NH(C₁-C₃ alkyl), C(=O)N(C₁-C₃ alkyl)₂, S(=O)₂N(C₁-C₃ alkyl)₂, 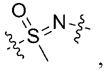 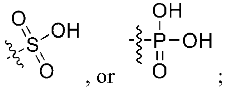 or, each R^{c1} is independently selected from A-(C₁-C₄ alkyl-O)ₘ₁-W⁵ or [C(=O)-CH₂-NR^{d3}]ₘ₃-B-W⁵, wherein A is selected from a chemical bond or C=O, R^{d3} is selected from C₁-C₆ alkyl, B is selected from C₁-C₁₀ alkylene, C=O, or a combination thereof C(=O)-C₁-C₁₀ alkylene, W⁵ is selected from C₁-C₆ alkyl, C(=O)NH₂, or S(=O)₂NH₂, and m1 and m3 are independently selected from integers of 1-24; or,
each R^{c1} is independently selected from 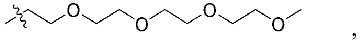 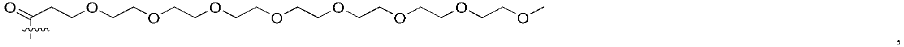 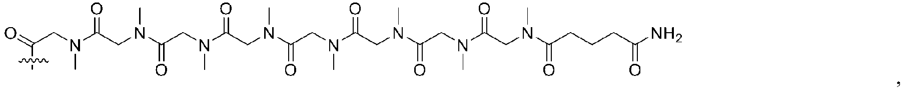 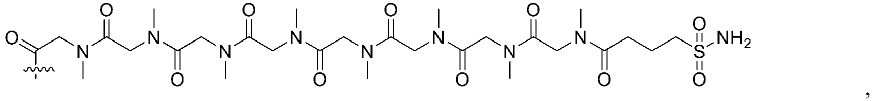
or 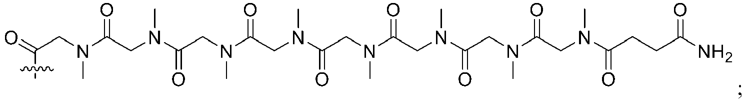
or, each R^{c1} is independently selected from 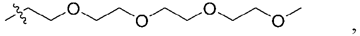 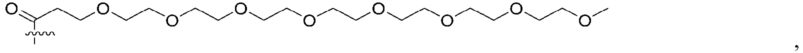
or 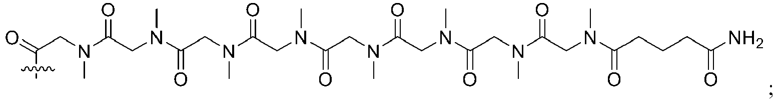
or, each R^{c6} is independently selected from W¹-W²-W³-W⁴-W⁵, wherein W¹ and W² are both selected from a chemical bond, W³ is selected from a chemical bond or A-(C₁-C₄ alkyl-O)ₘ₁, and W⁴ is selected from A-(C₁-C₄ alkyl-O)ₘ₁ or [C(=O)-CH₂-NR^{d3}]ₘ₃-B, wherein each A and each B are independently selected from a chemical bond, C₁-C₆
alkylene, NR^{d4}, C(=O), C(=O)NH, S(=O)₂, S(=O)₂NR^{d6}, S(=O)(-NR^{d7}), 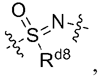
or any combination thereof, wherein R^{d1}, R^{d2}, R^{d3}, R^{d4}, R^{d6}, R^{d7}, and R^{d8} are each independently selected from H, C₁-C₆ alkyl, or (C₁-C₄ alkyl-O)ₖ₁-C₁-C₆ alkyl; m1 is selected from integers of 0-24, m2 is selected from integers of 1-5, and m3 and k1 are each
independently selected from integers of 1-24; W⁵ is selected from C₁-C₆ alkyl, NH₂, 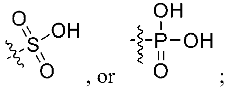
or, R^{c6} is selected from 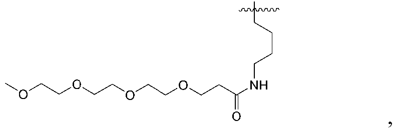 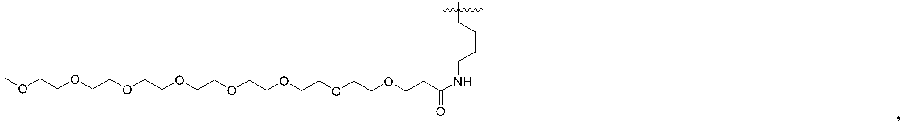 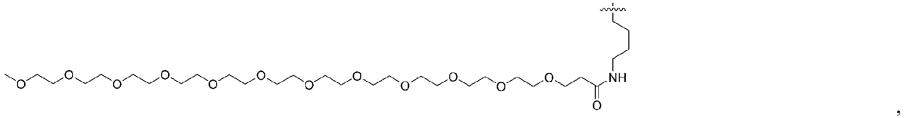 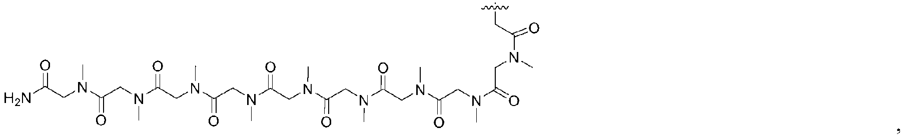 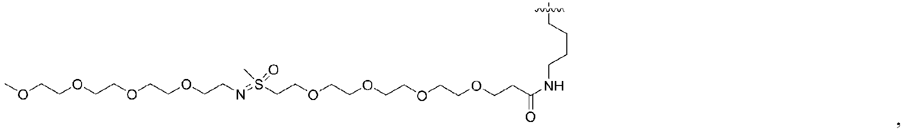 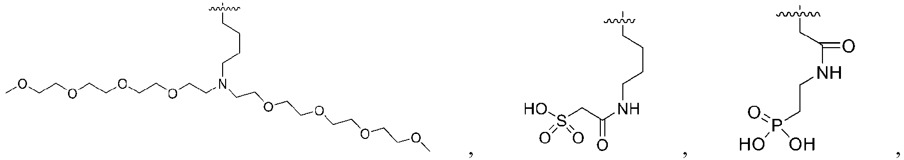 or

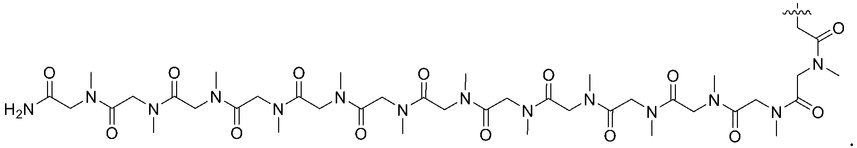

7. The ligand-drug conjugate or the pharmaceutically acceptable salt thereof according to any one of claims 1-6,
wherein L^{a} is selected from 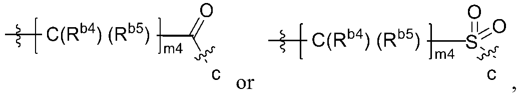 wherein R^{b4} and R^{b5} are each independently selected from H, m4 is selected from integers of 1-6, and the c-terminus of L^{a} is linked to L^{b}; or,
L^{a} is selected from 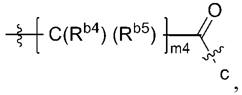
R^{b4} and R^{b5} are each independently selected from H, m4 is selected from integers of 1-6, and the c-terminus of L^{a} is linked to L^{b}.

8. The ligand-drug conjugate or the pharmaceutically acceptable salt thereof according to any one of claims 1-7,
wherein L° is selected from Y¹-Y²-Y³, wherein Y¹ is selected from a chemical bond or 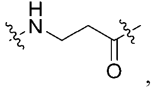 Y² is selected from dipeptide, tripeptide, or tetrapeptide residues of Val-Cit, Val-Ala, Gly-Gly-Phe-Gly, and Ala-Ala-Ala,
Y³ is selected from or 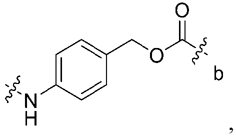
and the b-terminus of L^{c} is linked to drug unit D; or, L^{c} is selected from 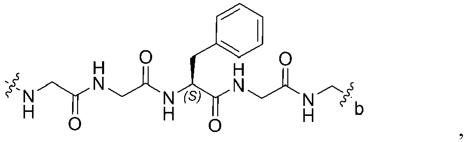 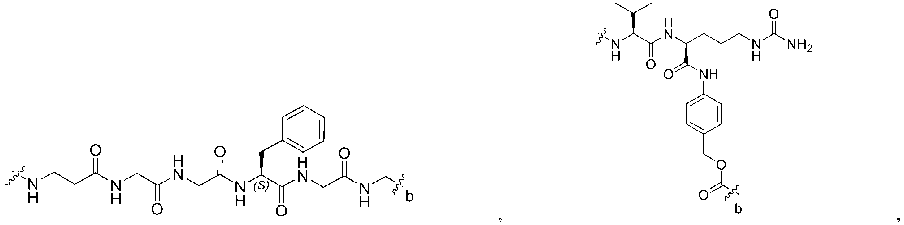 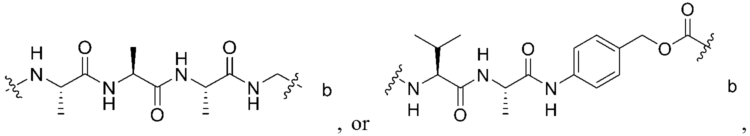 and the b-terminus of L° is linked to drug unit D.

9. The ligand-drug conjugate or the pharmaceutically acceptable salt thereof according to claim 1, wherein linker unit L is selected from the following structure: 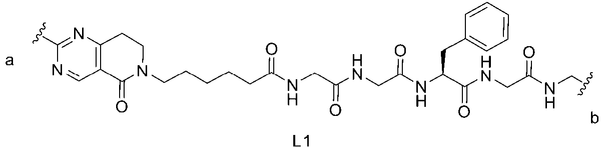 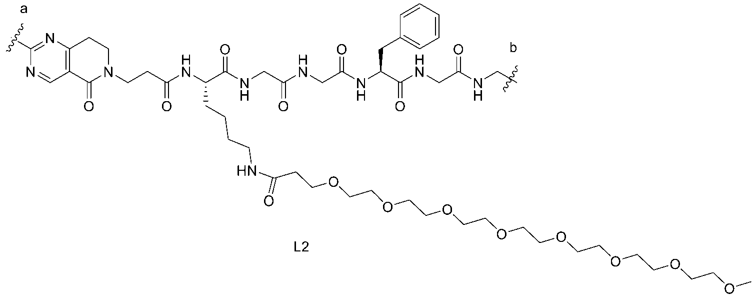 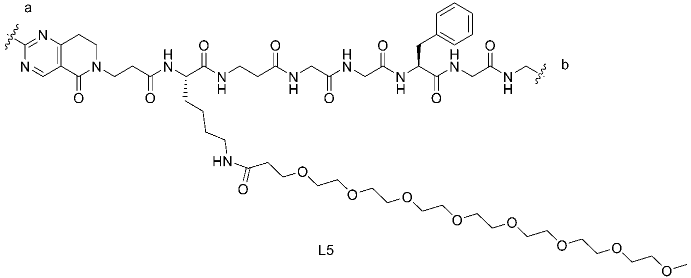 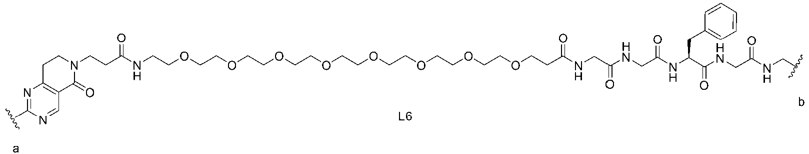 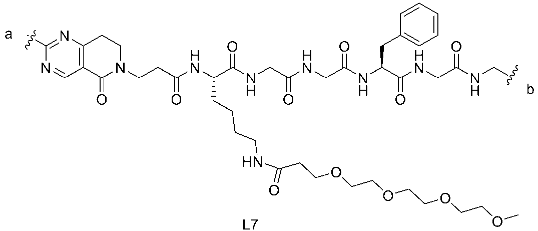 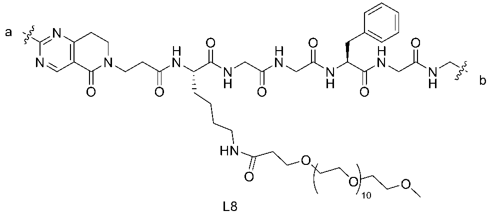 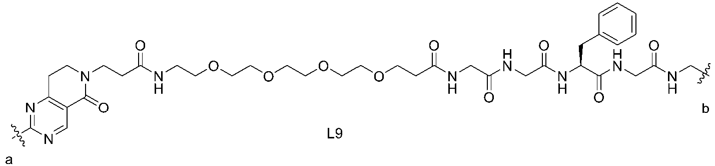 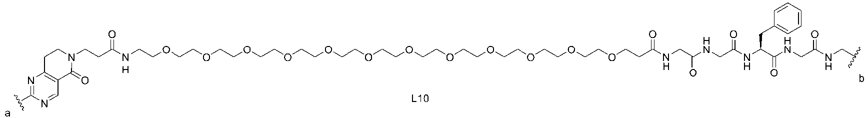 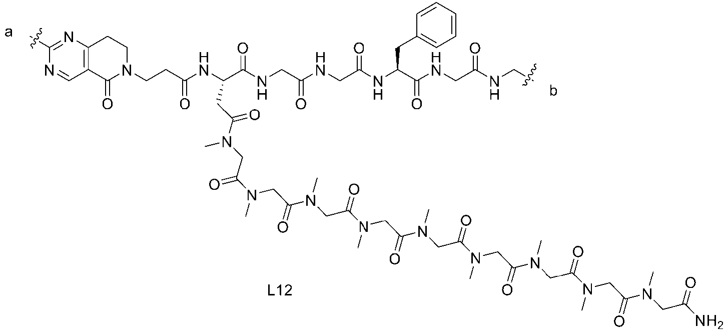 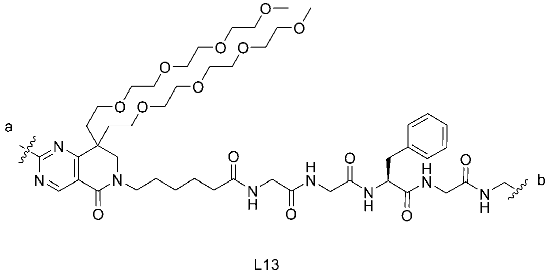 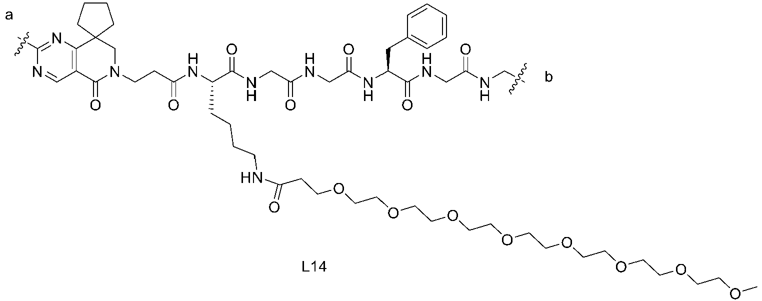 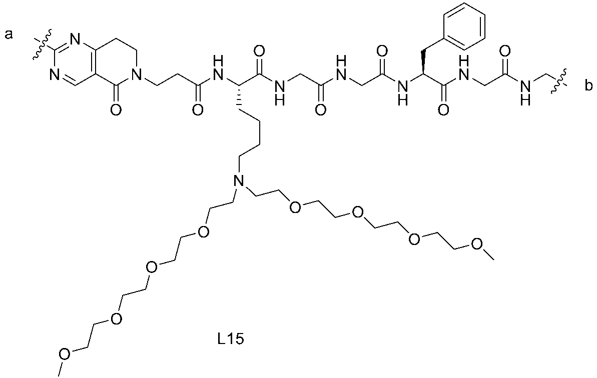 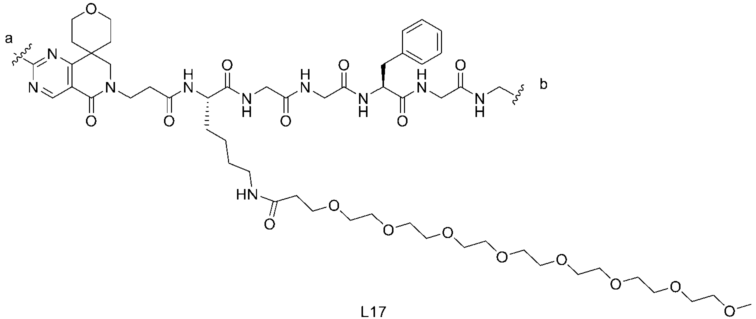 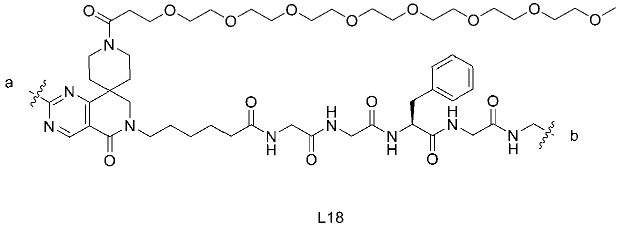 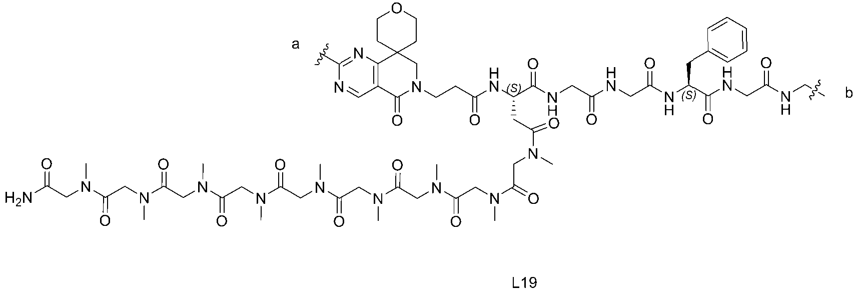 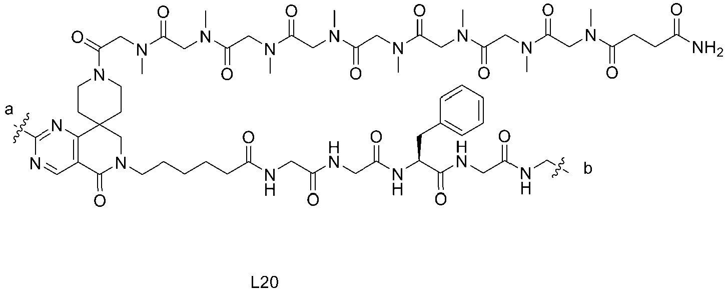 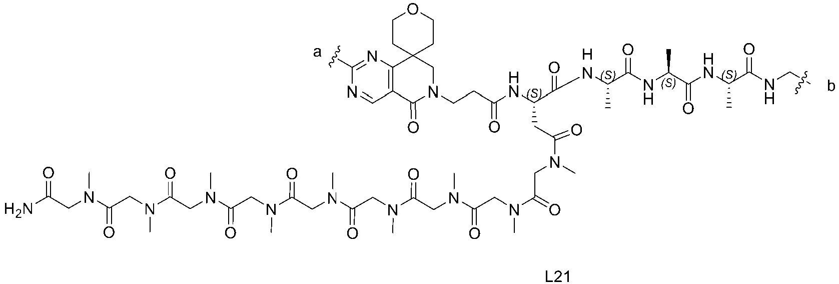 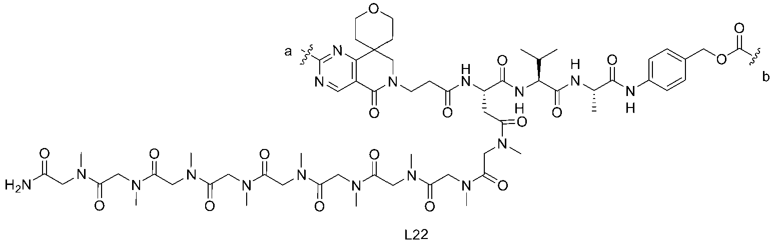 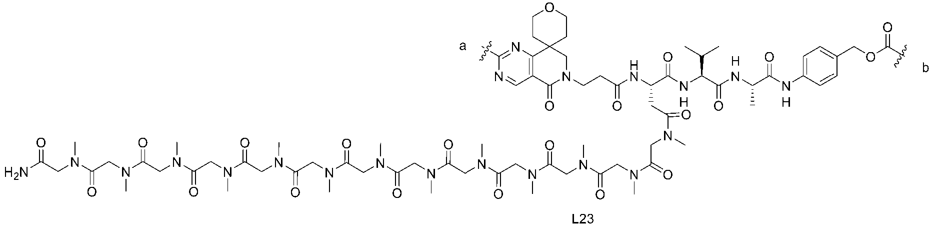 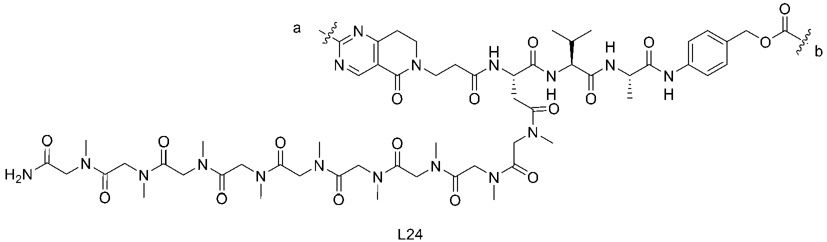 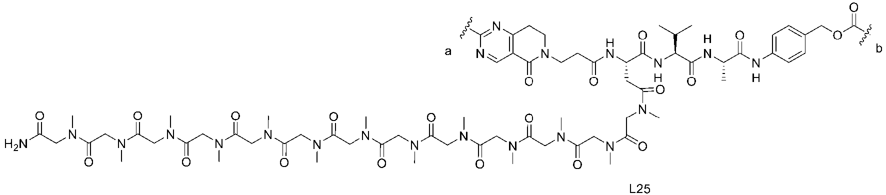 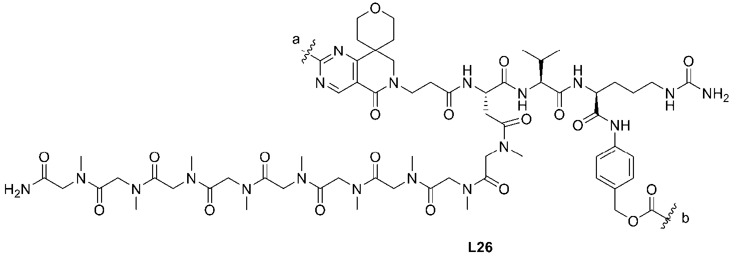 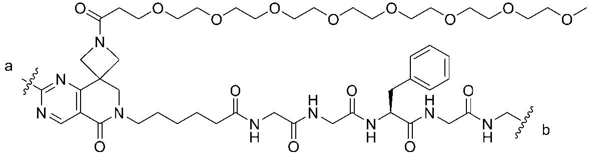 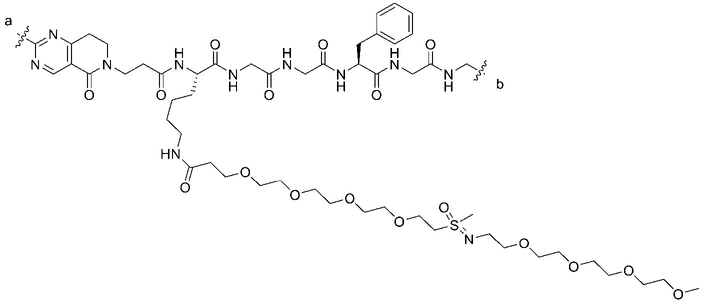 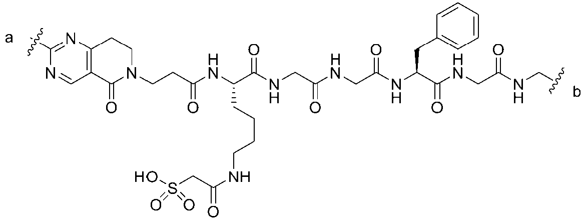 or

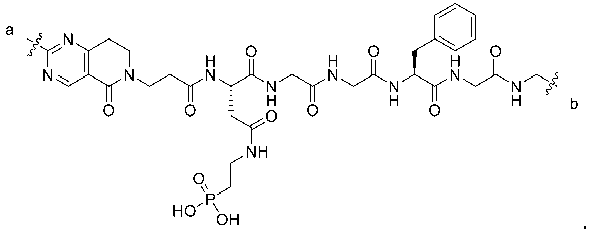

10. The ligand-drug conjugate or the pharmaceutically acceptable salt thereof according to any one of claims 1-9, wherein drug unit D is selected from a cytotoxic drug or a protein degrader.

11. The ligand-drug conjugate or the pharmaceutically acceptable salt thereof according to claim 10, wherein the cytotoxic drug is selected from a tubulin inhibitor, a DNA damaging agent, or a topoisomerase inhibitor, wherein the tubulin inhibitor is, for example, a dolastatin, auristatin, maytansine, tubulysin, or cryptomycin drug, the DNA damaging agent is, for example, a PBD, duocarmycin, or calicheamicin drug, and the topoisomerase inhibitor is, for example, a camptothecin drug; or,
the cytotoxic drug is selected from camptothecin, hydroxycamptothecin, 9-aminocamptothecin, SN-38, irinotecan, topotecan, belotecan, rubitecan, actinomycin D, doxorubicin, duocarmycin, daunorubicin, mitoxantrone, podophyllotoxin, etoposide, vincristine, vinblastine, paclitaxel, docetaxel, cabazitaxel, maytansine, maytansinoid, DM1, DM3, DM4, calicheamicin, MMAE, MMAF, or tubulysin D; or,
the cytotoxic drug is selected from a topoisomerase I inhibitor or an auristatin drug; or,
the protein degrader is selected from a GSPT1 protein degrader; or,
drug unit D is selected from 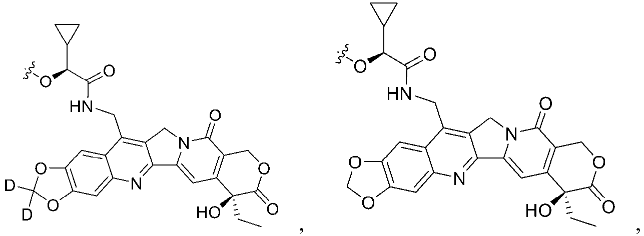 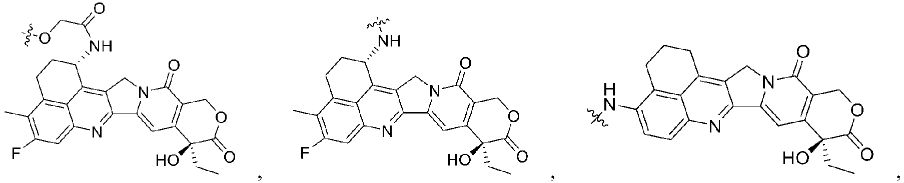 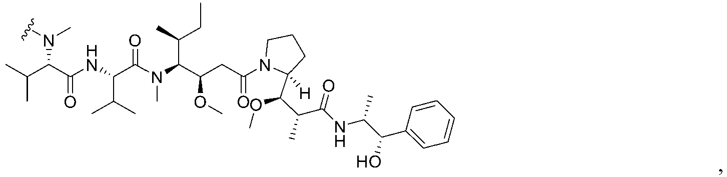 or 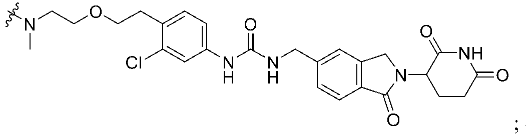 or,
drug unit D is selected from 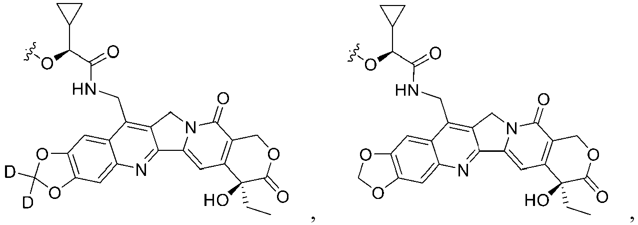 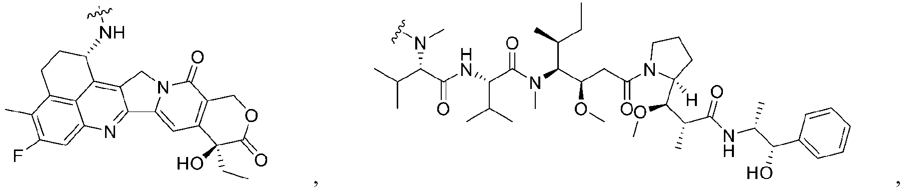
or

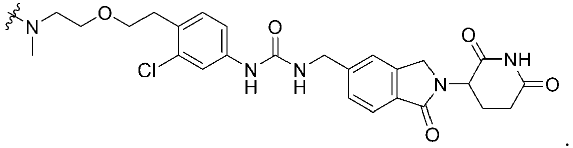

12. The ligand-drug conjugate or the pharmaceutically acceptable salt thereof according to claim 1, wherein the ligand-drug conjugate of general formula Pc-(L-D)ₙ or the pharmaceutically acceptable salt thereof is selected from the following compound or a pharmaceutically acceptable salt thereof: 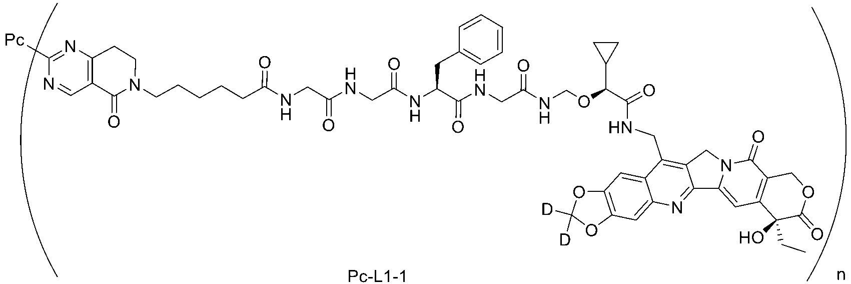 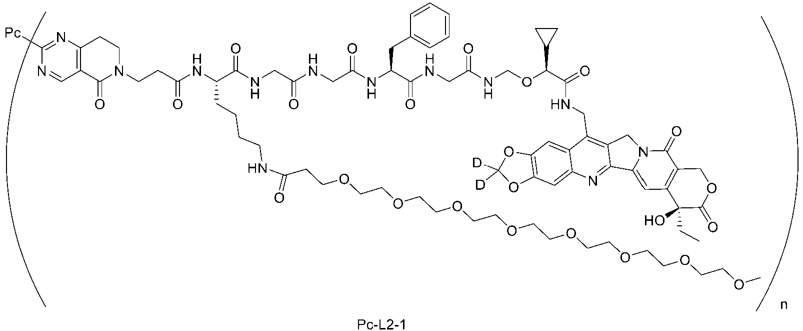 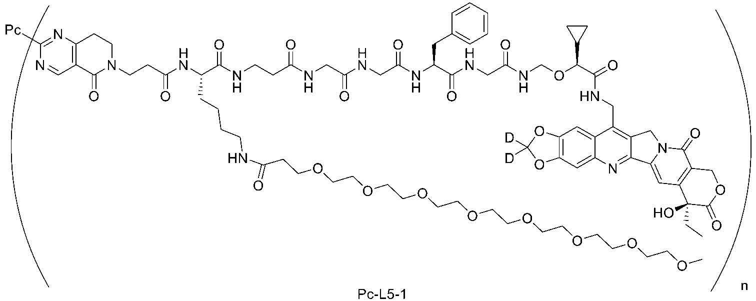 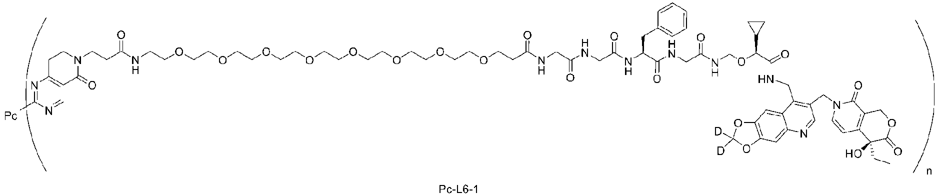 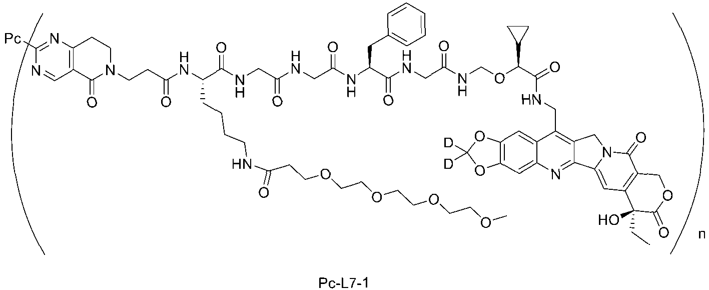 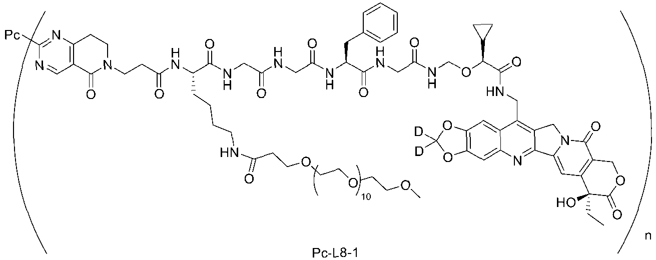 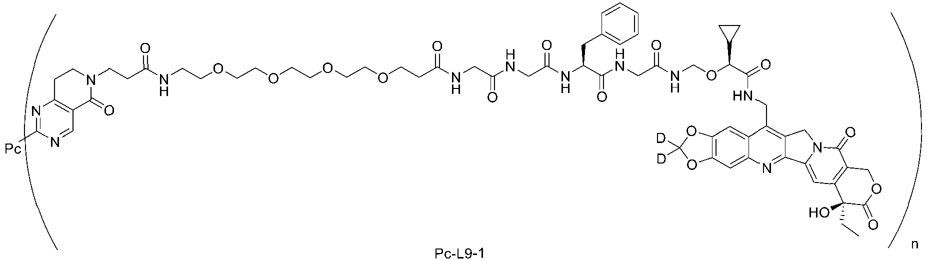 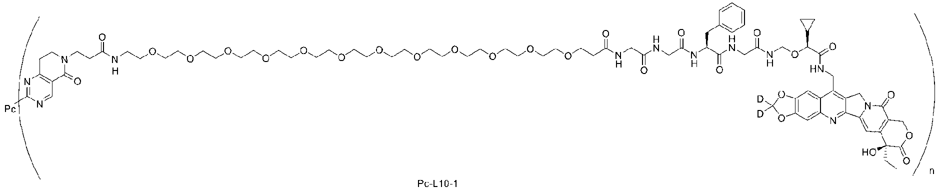 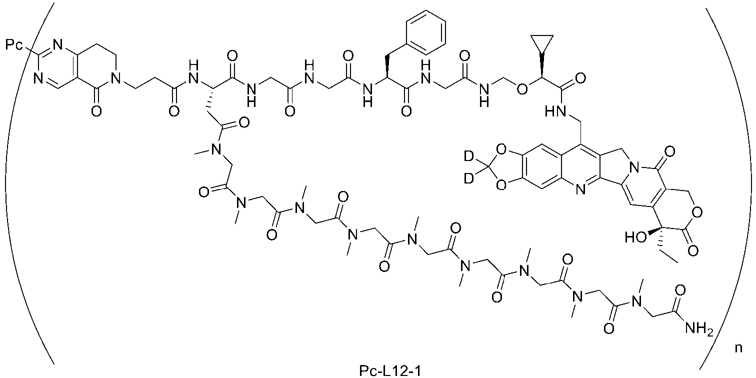 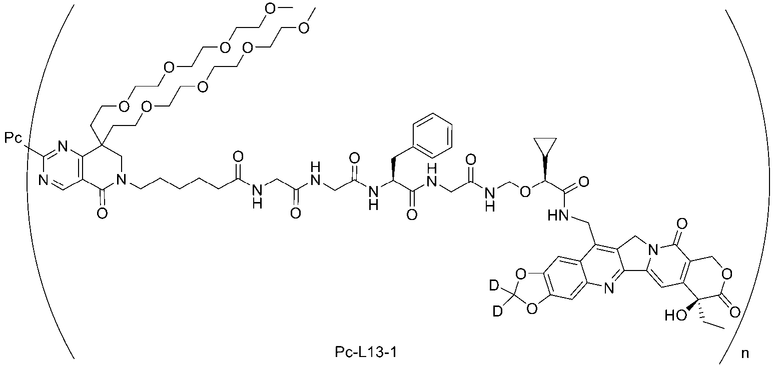 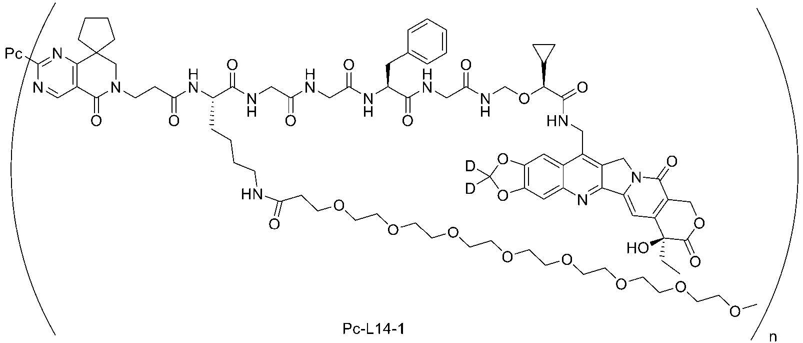 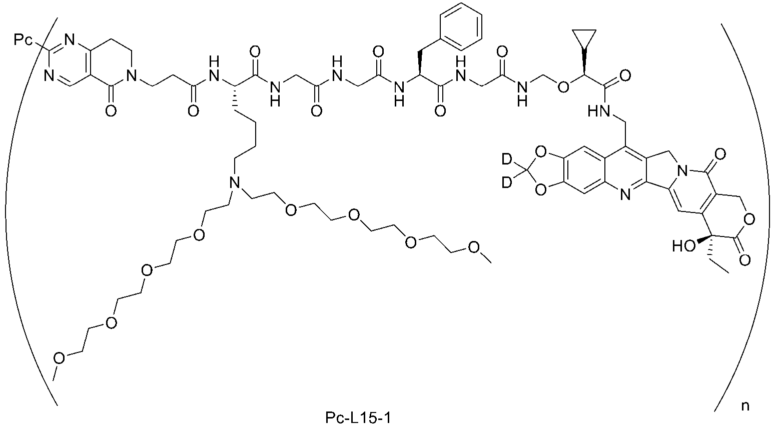 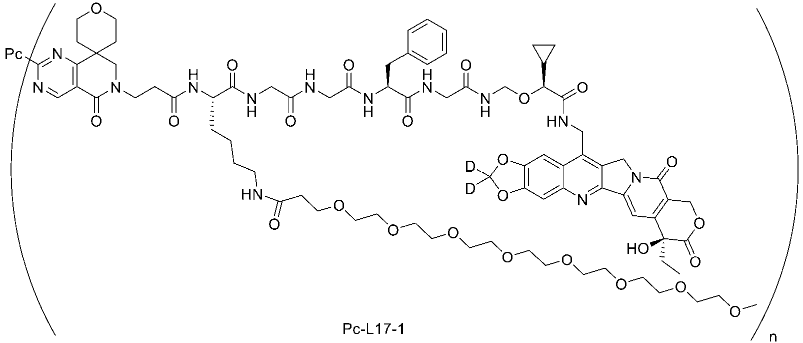 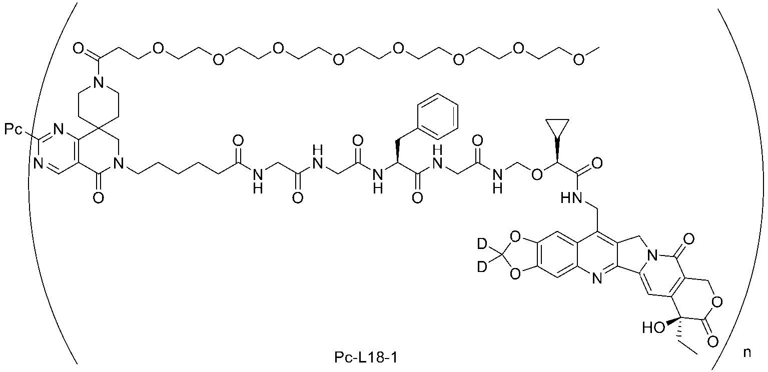 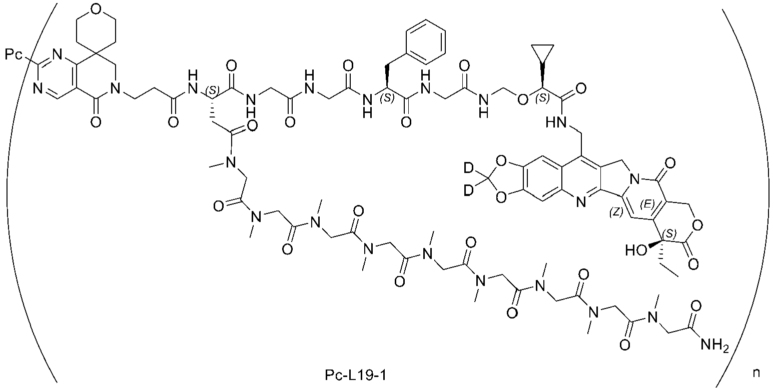 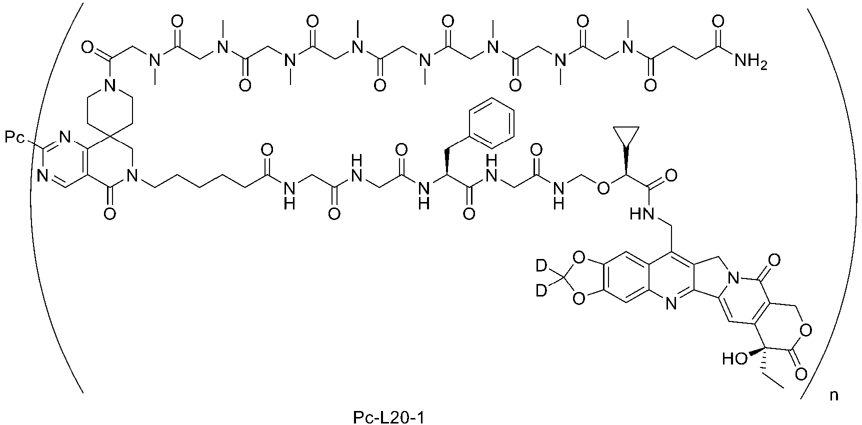 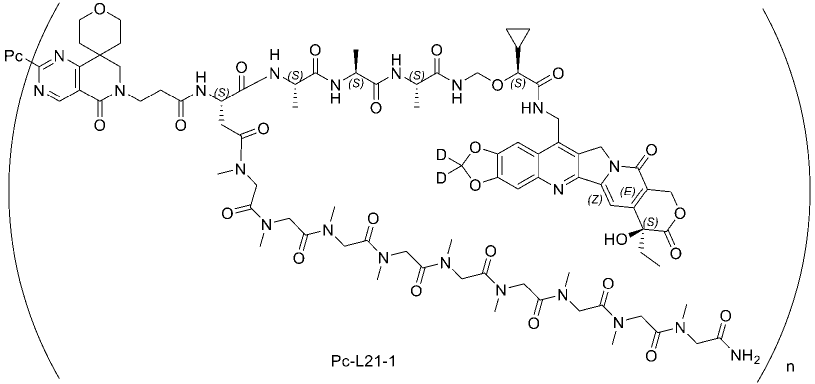 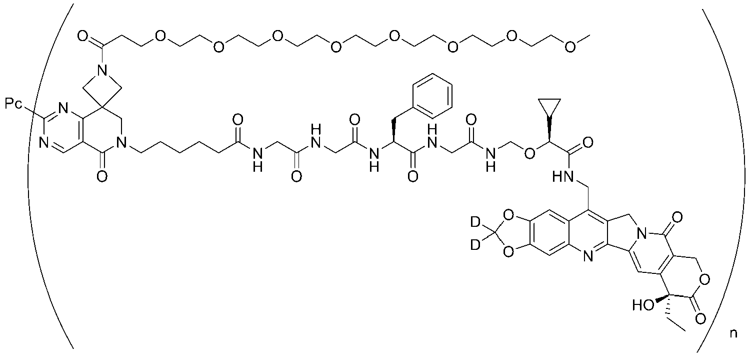 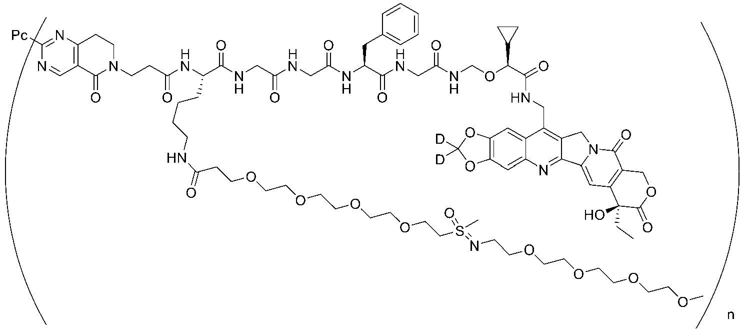 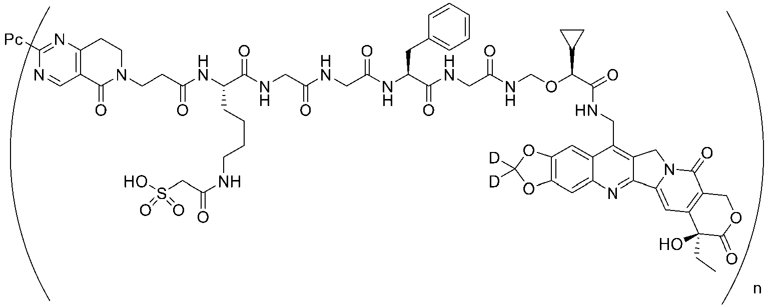 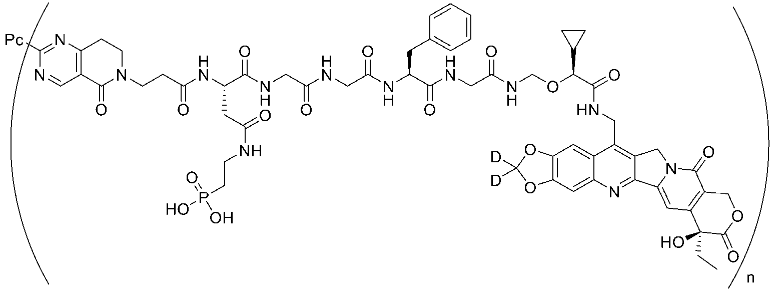 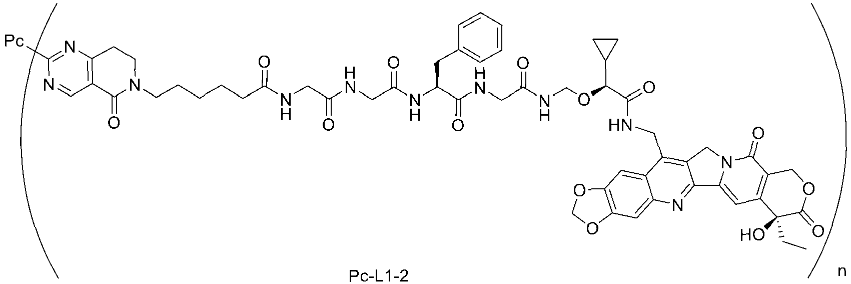 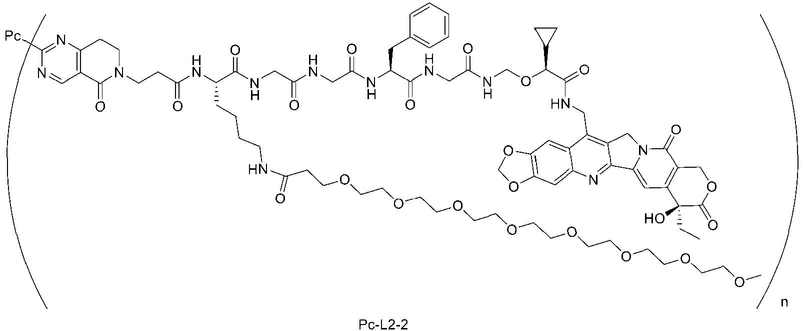 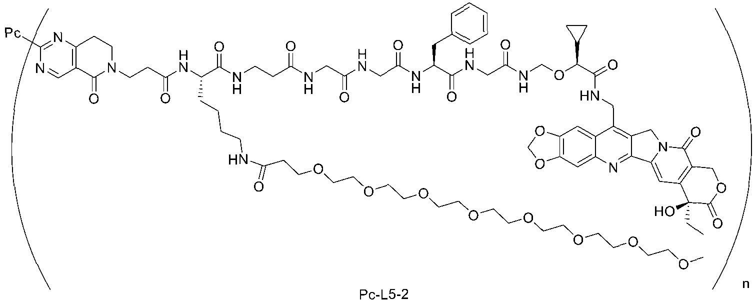 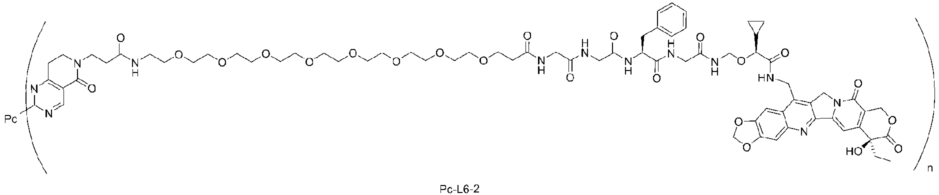 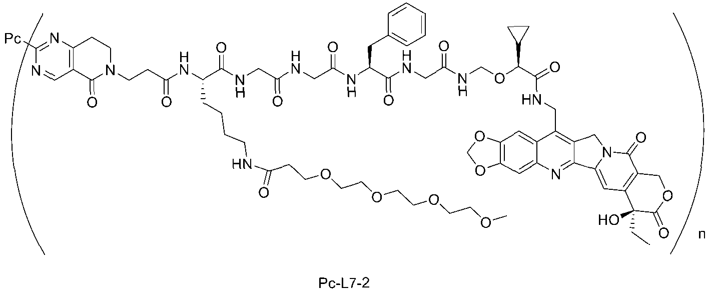 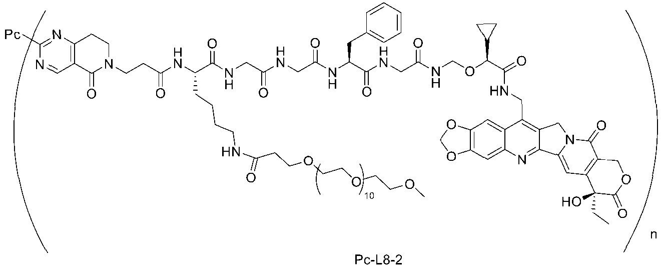 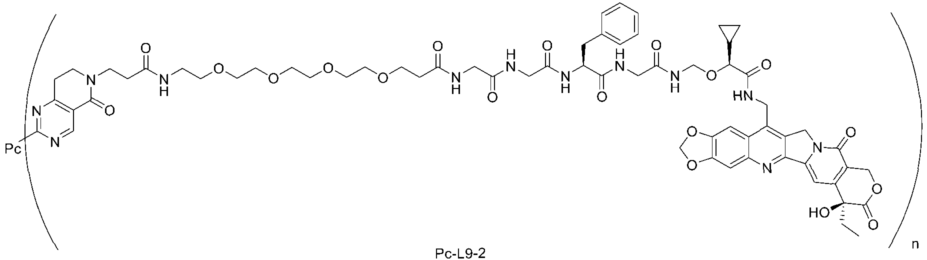 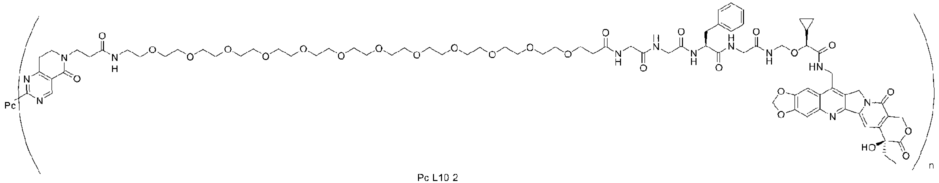 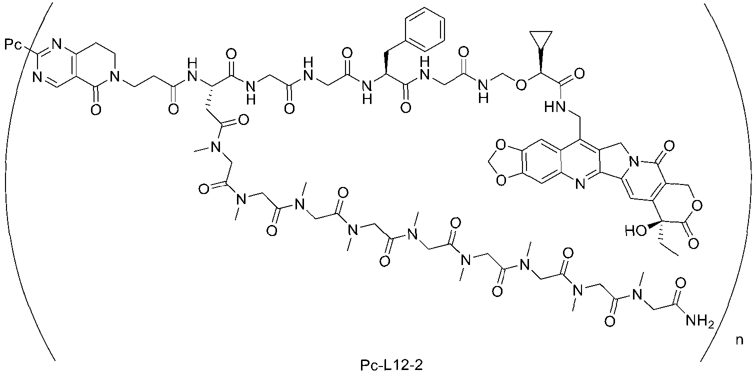 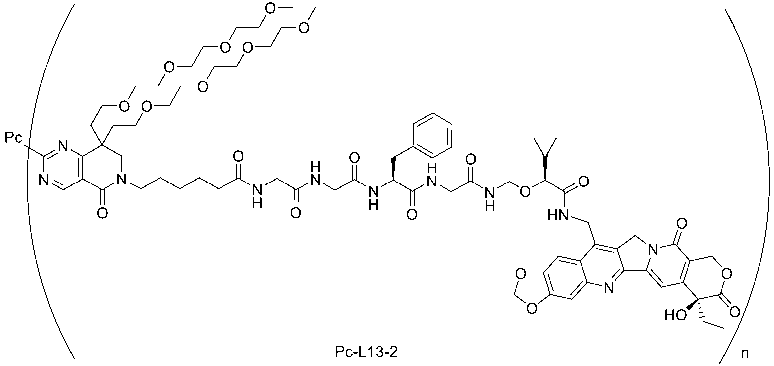 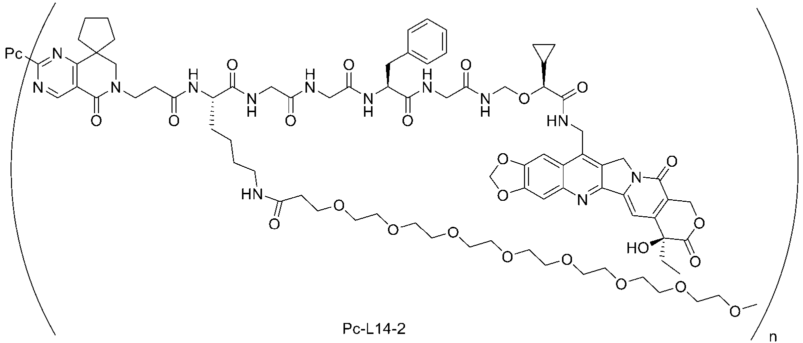 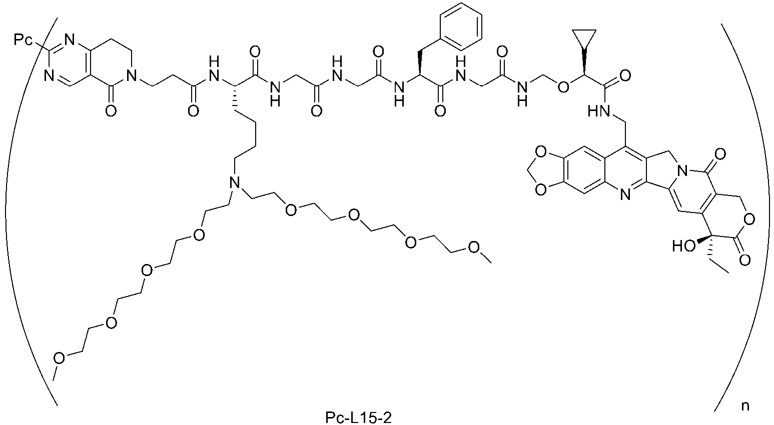 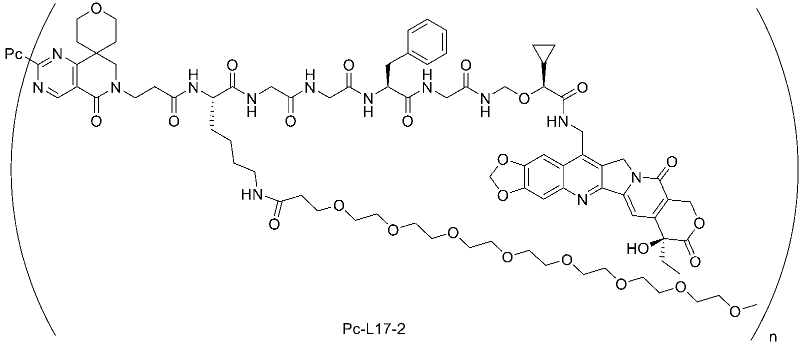 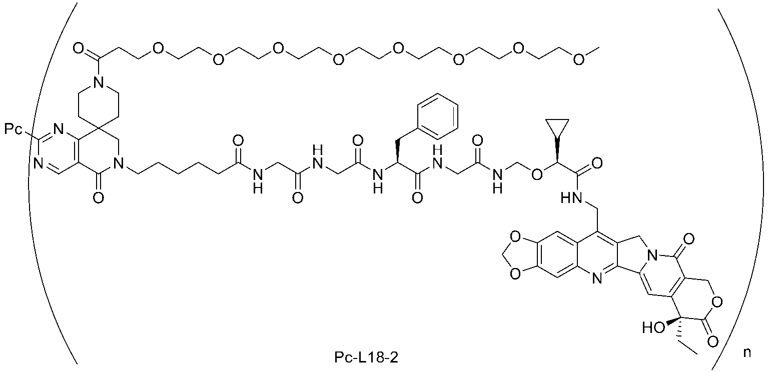 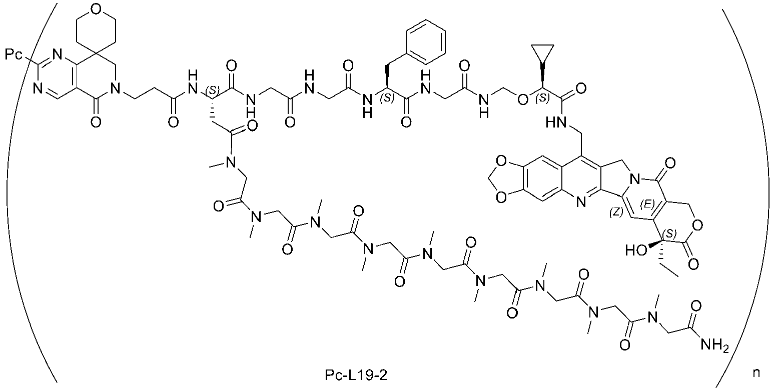 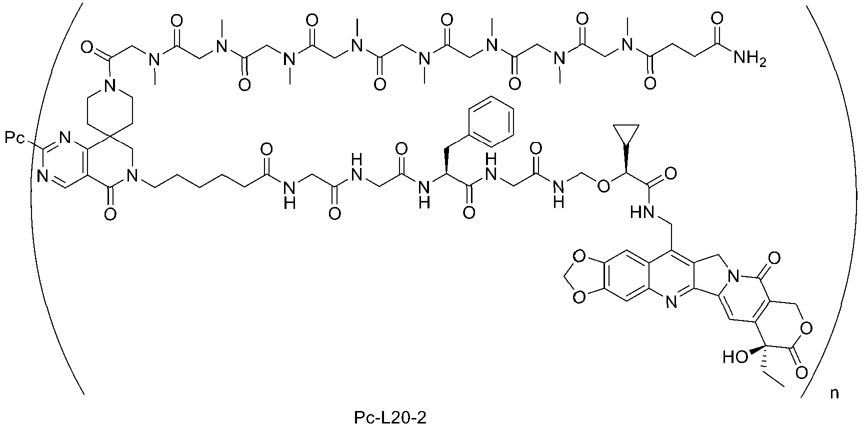 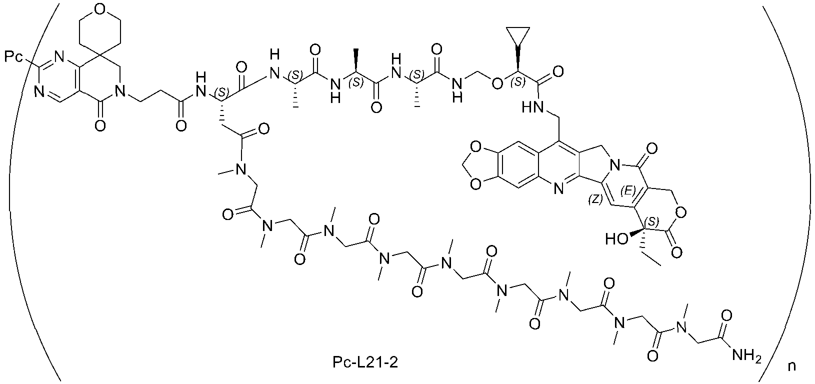 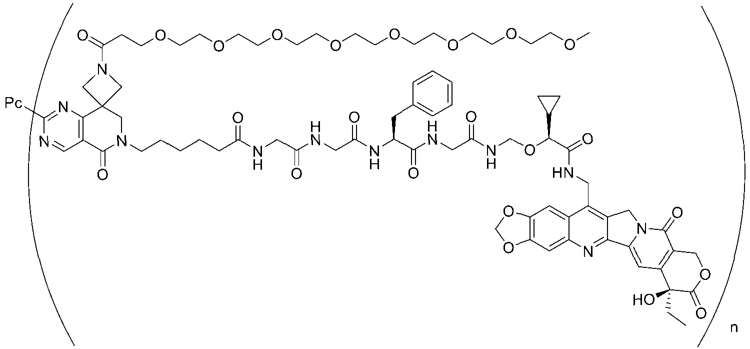 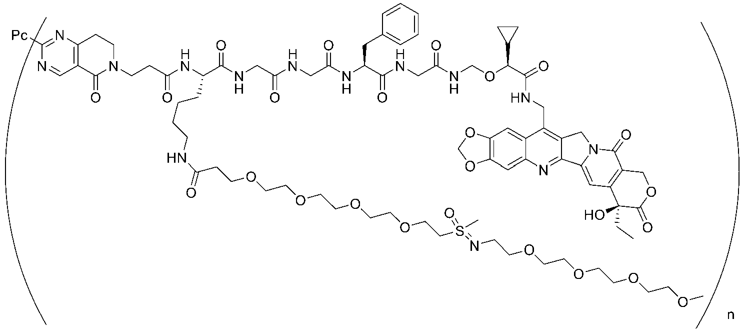 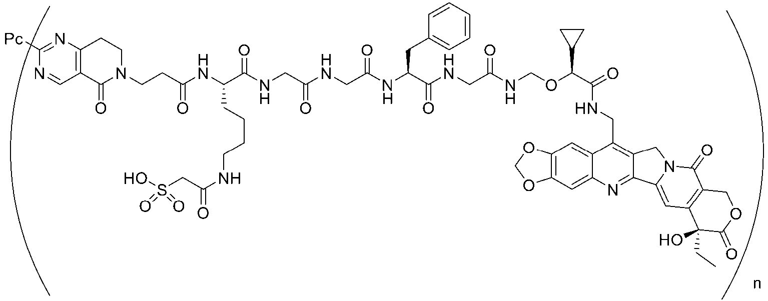 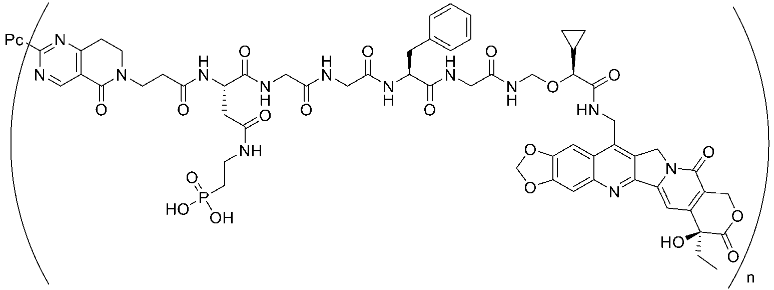 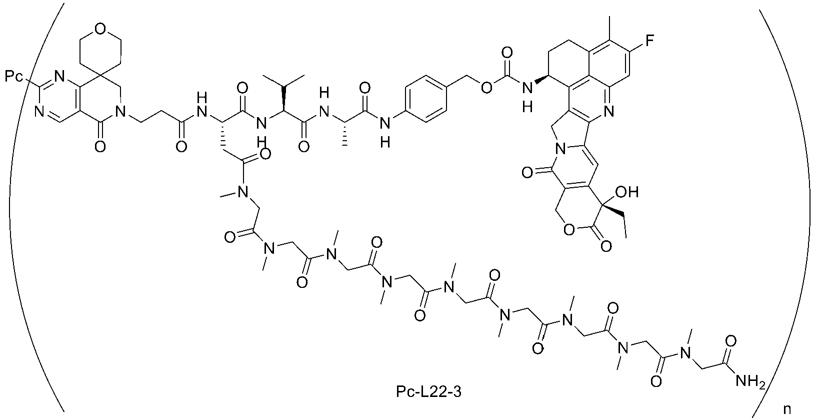 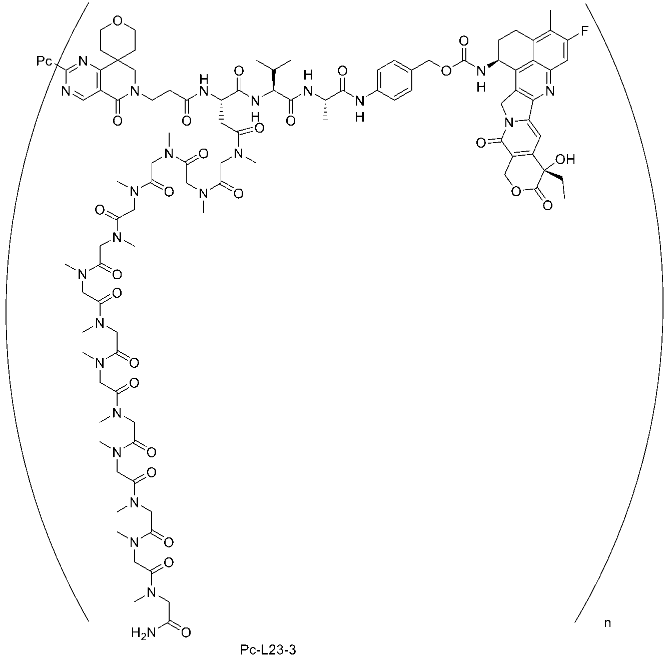 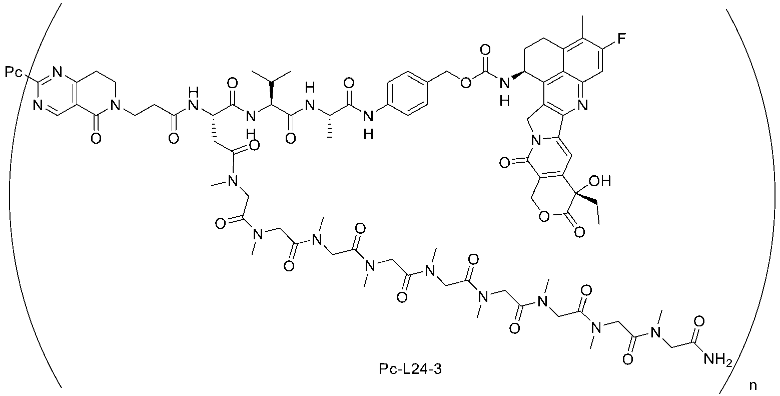 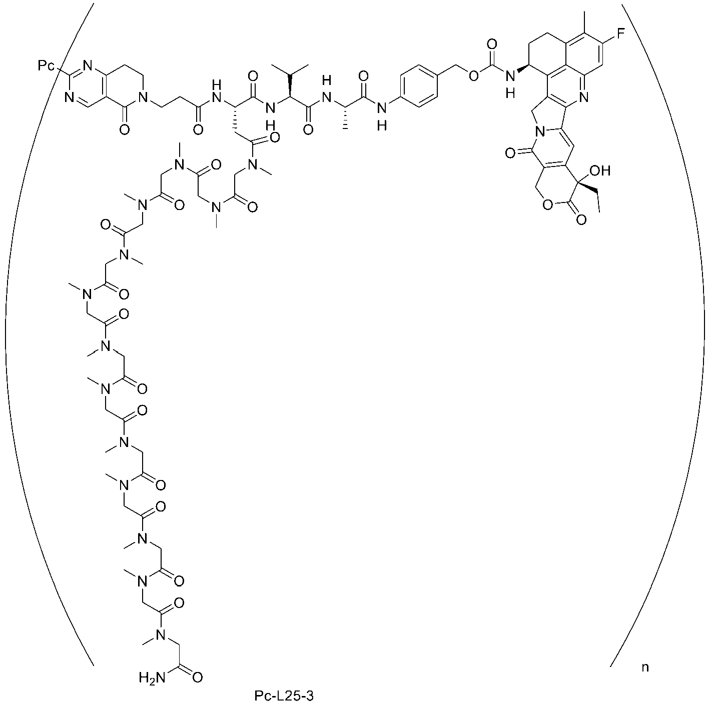 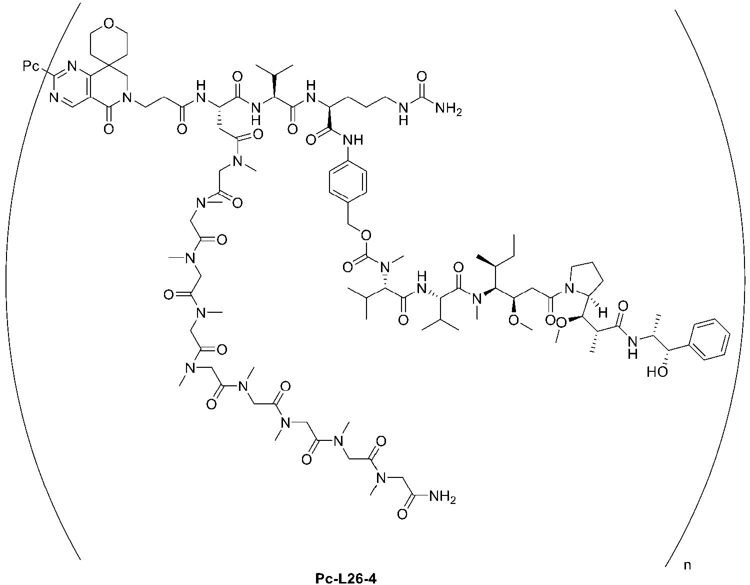 or

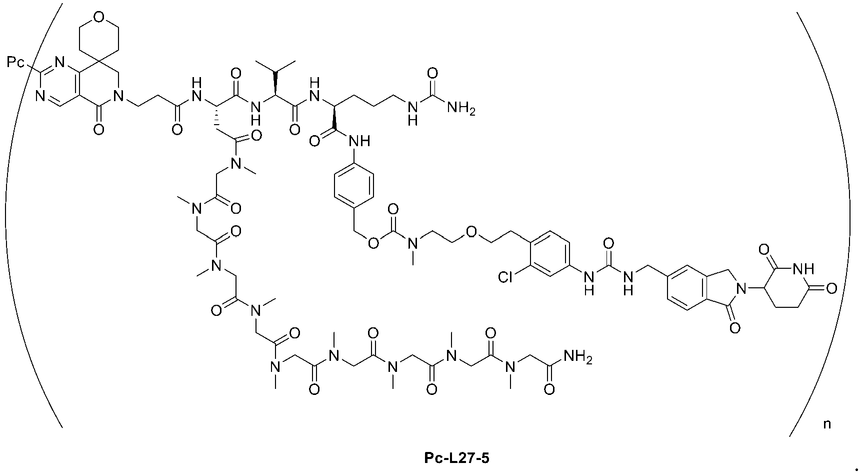

13. The ligand-drug conjugate or the pharmaceutically acceptable salt thereof according to any one of claims 1-12, wherein ligand unit Pc is selected from a polypeptide, an antibody, or an antigen-binding fragment thereof; or ligand unit Pc specifically binds to one or more antigens selected from the group consisting of: HER2 (ErbB2), p95HER2, HER3 (ErbB3), CD3, CD16, ROR1, DLL3, CDH6, CD70, CD5, CD20, BCMA, EGFR, VEGF, Trop-2, Claudin6, Claudin18.2, c-MET, CD30, MUC1, Nectin-4, CD22, CD74, CD19, CD79b, MSLN, TOPO2, EpCAM, CEACAM-5, Mesothelin, PD-L1, PSMA, ROR1, ROR2, CD25, CD33, CD123, FLT3, CD174, CD166, CD326, CD71, LIV-1, MUC16, ENPP3, TDGF1, ETBR, TIM1, TIM3, LRRC15, CanAg/AFP, FAP, SLITRK6, KIT/CD117, STEAP1, SLAMF7/CS1, NaPi2B/SLC34A2, GPNMB, AXL, B7-H3 (CD276), PTK7/CCK4, PRLR, EFNA4, 5T4, NOTCH3, CD142, CA6, GPR20, EphA2, LYPD3, FGFR2, FGFR3, FRα, CEACAMs, GCC, IntegrinAv, CAIX, P-cadherin, GD3, cadherin6, LAMP1, CD56, CD37, CD47, CD138, or CD352.

14. The ligand-drug conjugate or the pharmaceutically acceptable salt thereof according to any one of claims 1-13, wherein n is selected from real numbers of 1-8; or, n is selected from real numbers of 2-8; or, n is selected from real numbers of 6-8.

15. A linker unit of formula (L), wherein the linker unit is used for linking a ligand unit and a drug unit to give a ligand-drug conjugate, 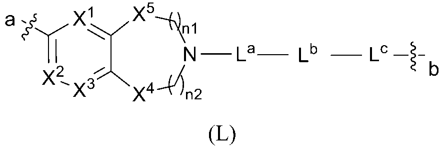 wherein X¹, X², X³, X⁴, X⁵, L^{a}, L^{b}, L^{c}, n1, and n2 are as defined in any one of claims 1-8, the a-terminus of L is linked to the ligand unit, and the b-terminus is linked to the drug unit.

16. The linker unit according to claim 15, wherein the linker unit of formula (L) is selected from the following structural unit: 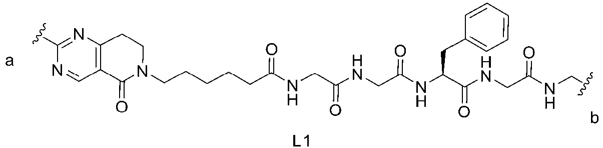 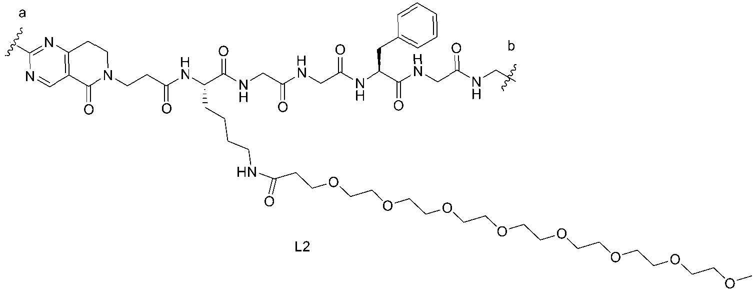 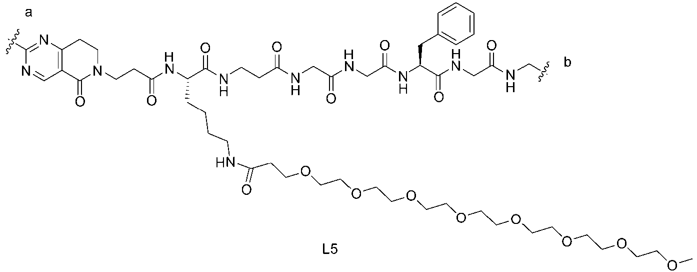 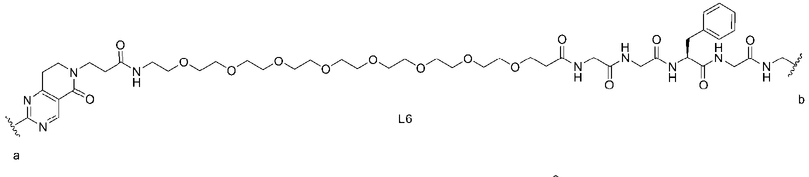 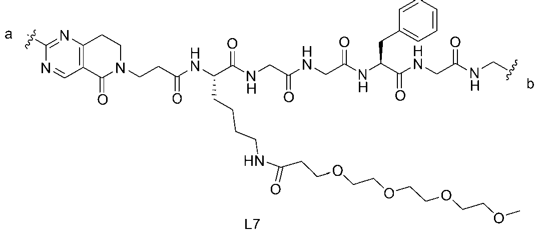 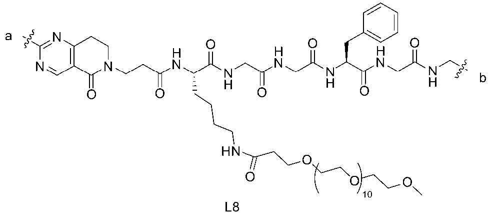 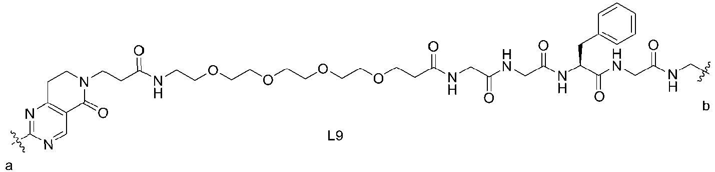 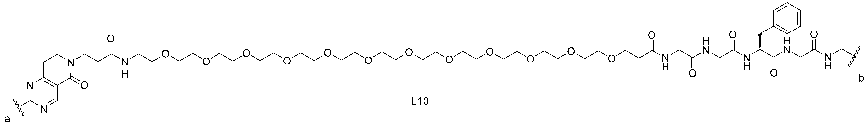 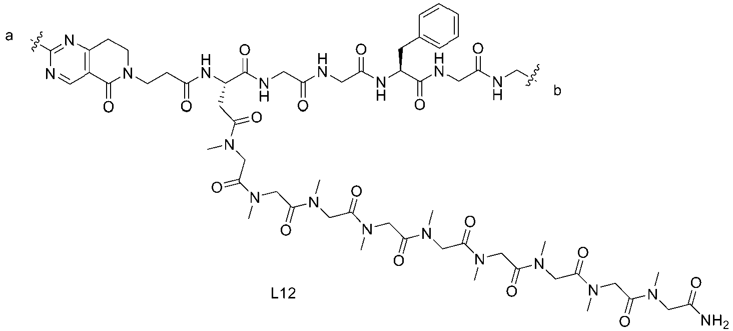 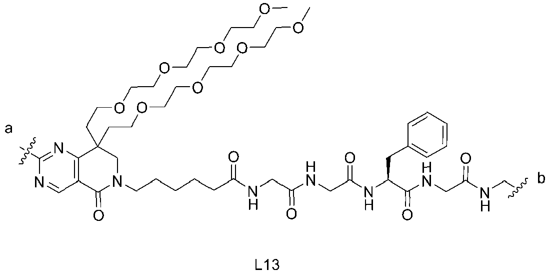 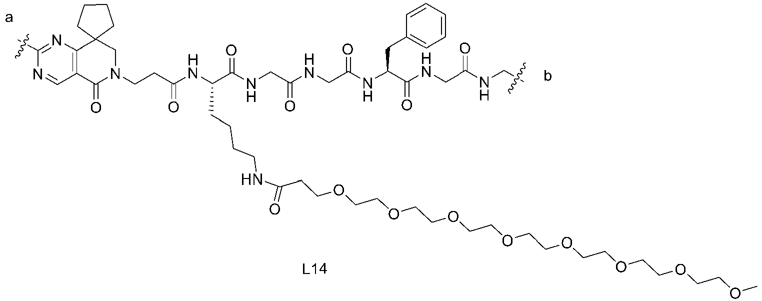 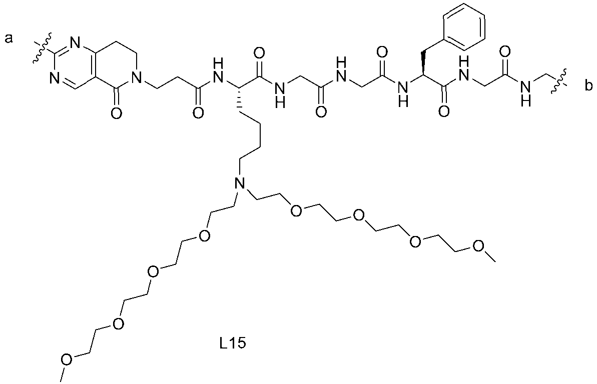 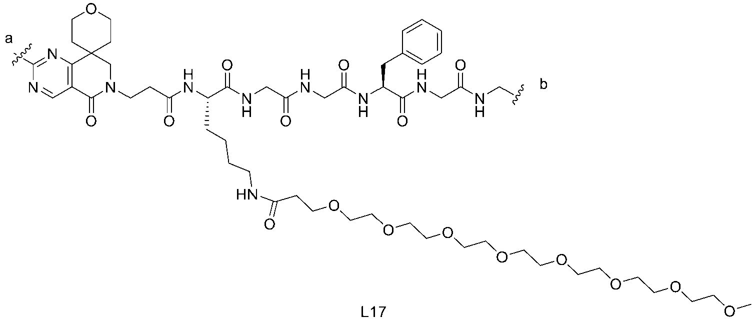 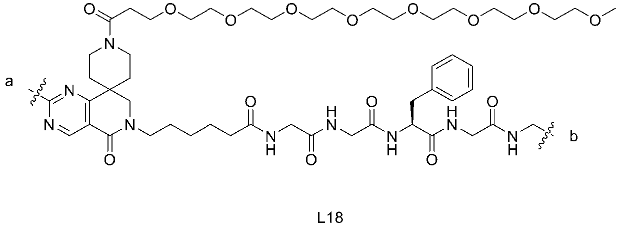 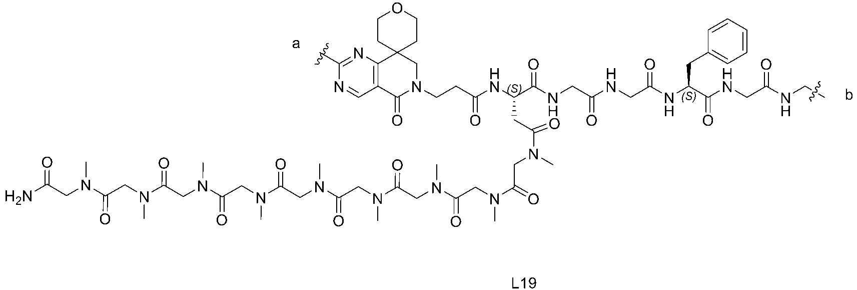 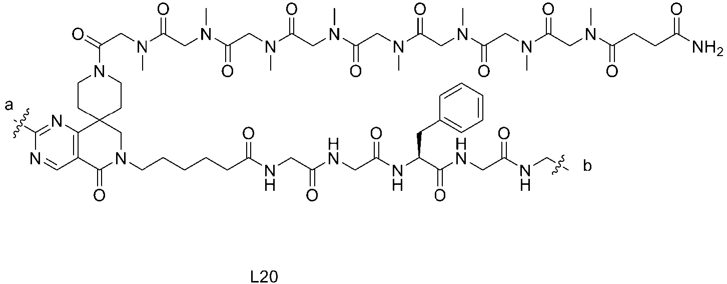 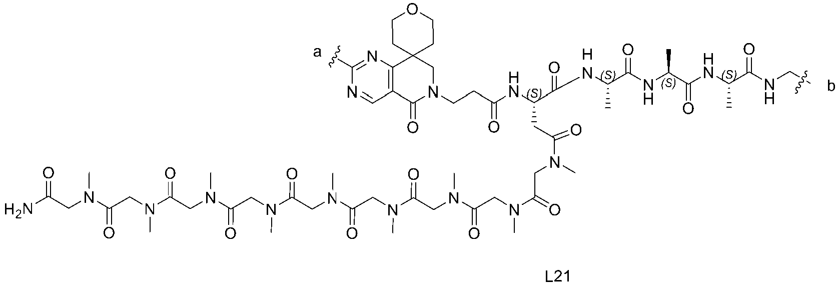 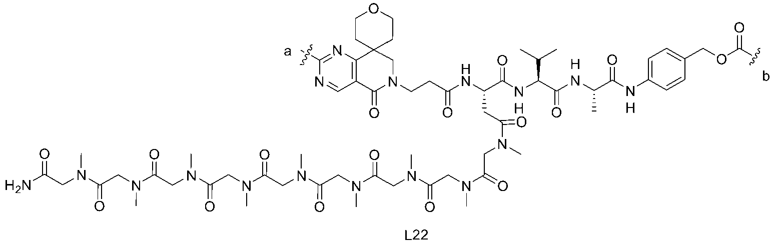 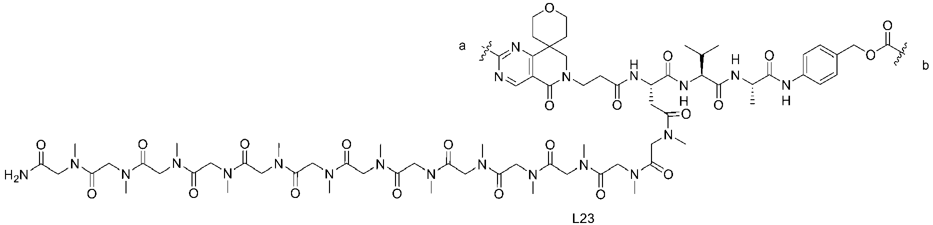 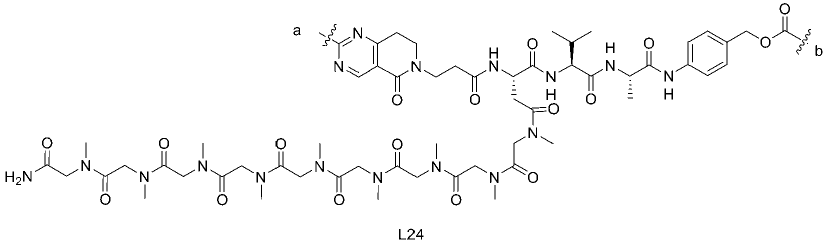 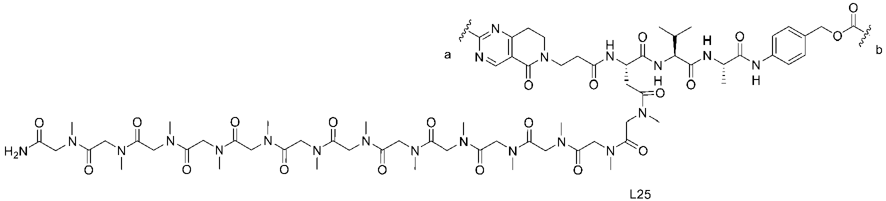 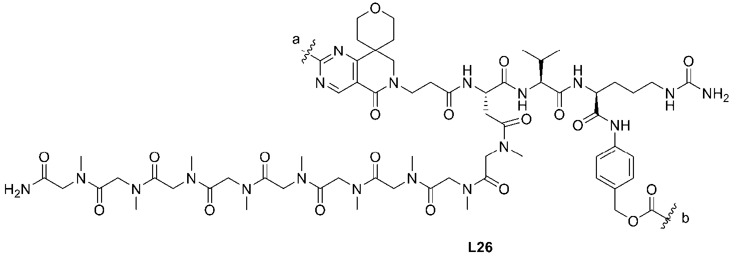 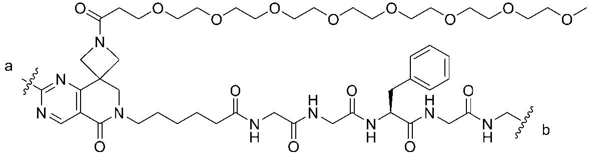 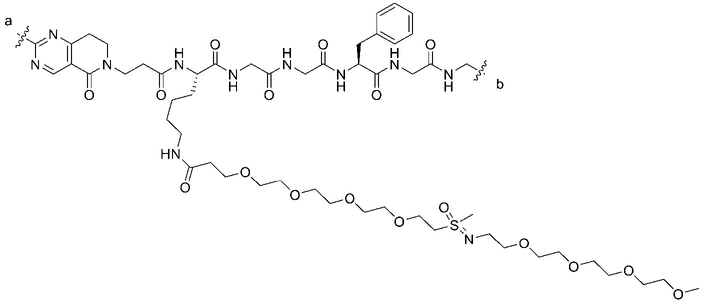 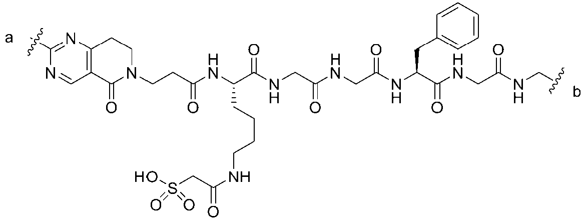 or

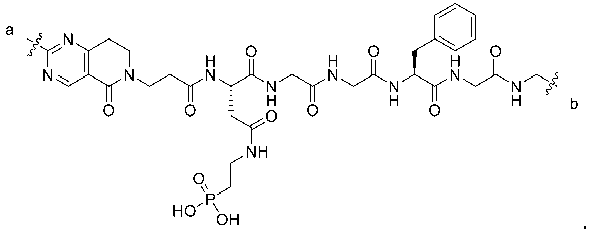

17. A drug-linker compound of formula (II) or a pharmaceutically acceptable salt thereof, 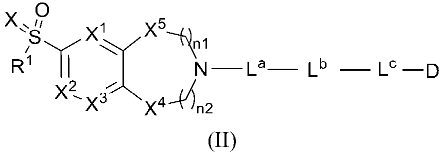 wherein X¹, X², X³, X⁴, X⁵, L^{a}, L^{b}, L^{c}, n1, and n2 are as defined in any one of claims 1-8, and drug unit D is as defined in any one of claims 10-11;
X is selected from being absent, O, or NR^{e1};
R¹ is selected from N(R^{e2})(R^{e3}), C₁-C₁₀ alkyl, C₃-C₁₀ cycloalkyl, 4- to 14-membered heterocyclyl, C₆-C₁₀ aryl, or 5-to 10-membered heteroaryl, wherein the C₁-C₁₀ alkyl, C₃-C₁₀ cycloalkyl, 4- to 14-membered heterocyclyl, C₆-C₁₀ aryl, or 5- to 10-membered heteroaryl is optionally substituted with one or more R^{f}, wherein each R^{f} is independently selected from halogen, CN, =O, C₁-C₃ alkyl, OH, O(C₁-C₃ alkyl), NH₂, NH(C₁-C₃ alkyl), or N(C₁-C₃ alkyl)₂; and R^{e1}, R^{e2}, and R^{e3} are each independently selected from H, C₁-C₆ alkyl, C₃-C₆ cycloalkyl, or 4- to 7-membered heterocyclyl.

18. The drug-linker compound or the pharmaceutically acceptable salt thereof according to claim 17, wherein
X is selected from O; and/or
R¹ is selected from C₁-C₆ alkyl or C₃-C₆ cycloalkyl, wherein the C₁-C₆ alkyl or C₃-C₆ cycloalkyl is optionally substituted with one or more R^{f}; or, R¹ is selected from methyl, tert-butyl, or cyclopentyl.

19. The drug-linker compound or the pharmaceutically acceptable salt thereof according to claim 17, wherein the drug-linker compound of formula (II) or the pharmaceutically acceptable salt thereof is selected from the following compounds or pharmaceutically acceptable salts thereof:

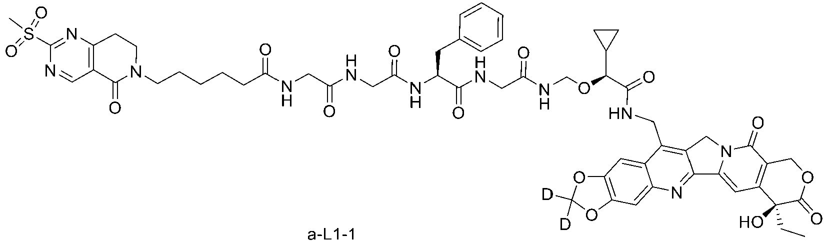

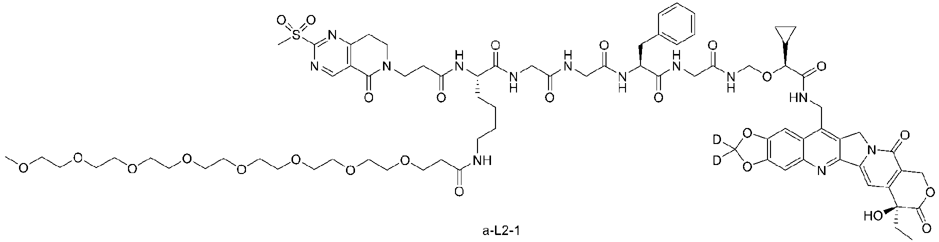

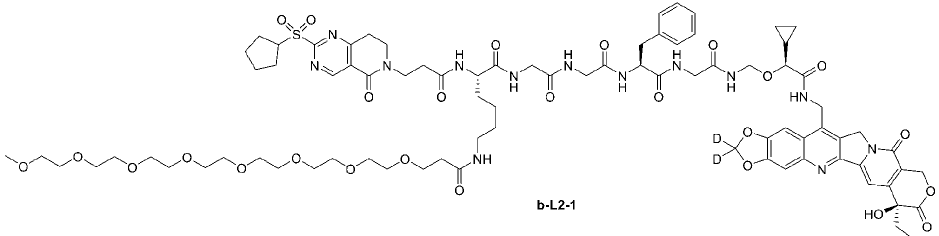

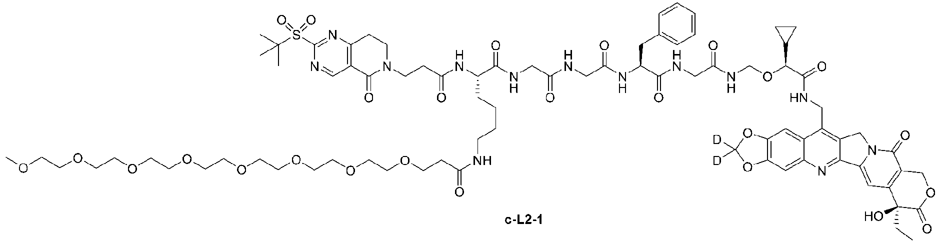

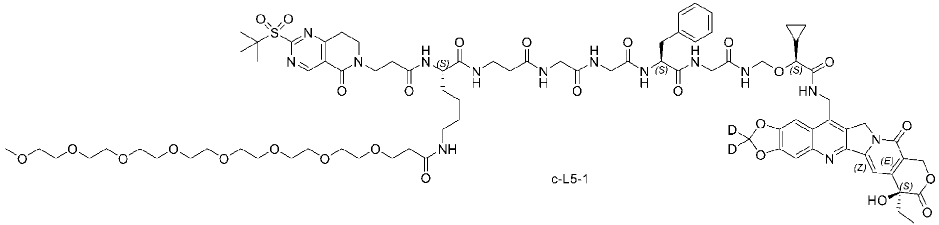

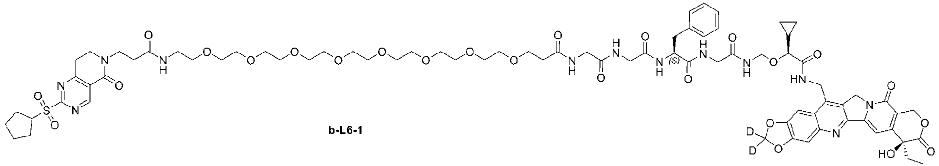

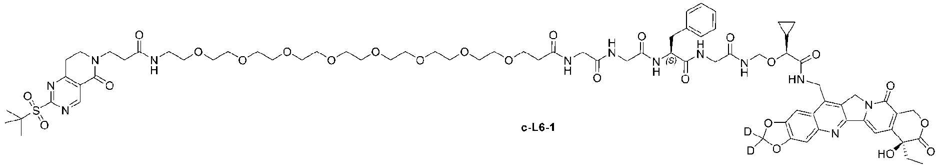

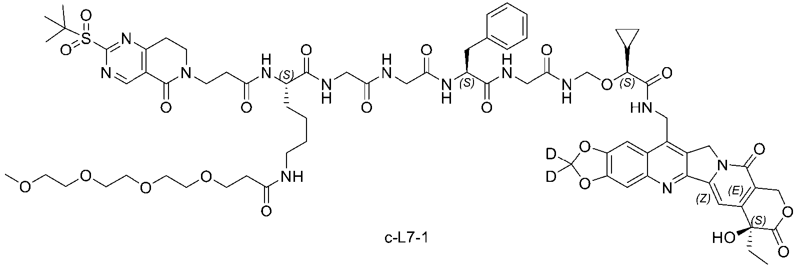

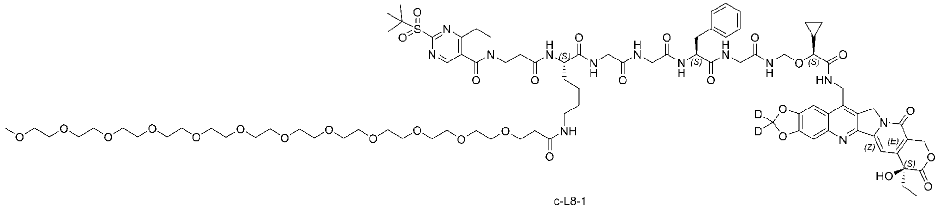

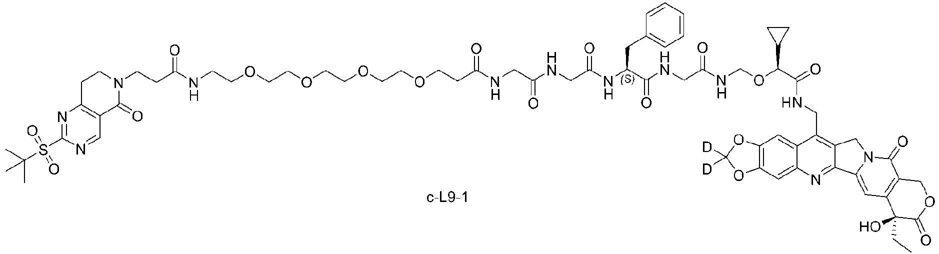

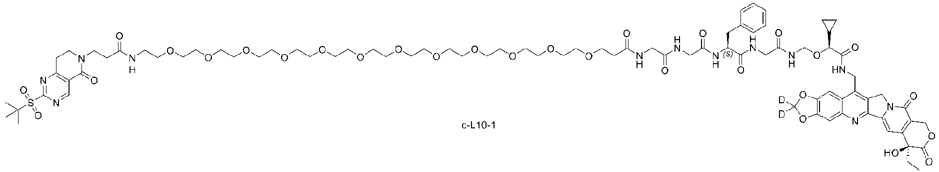

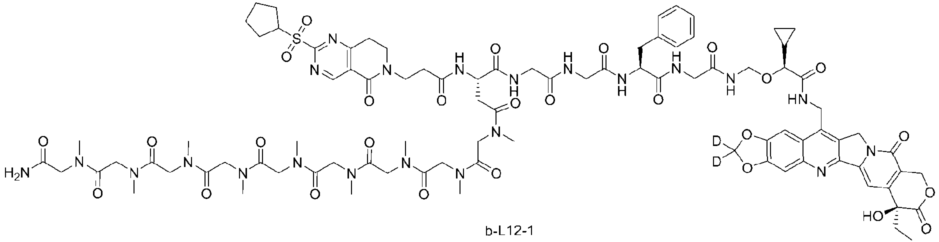

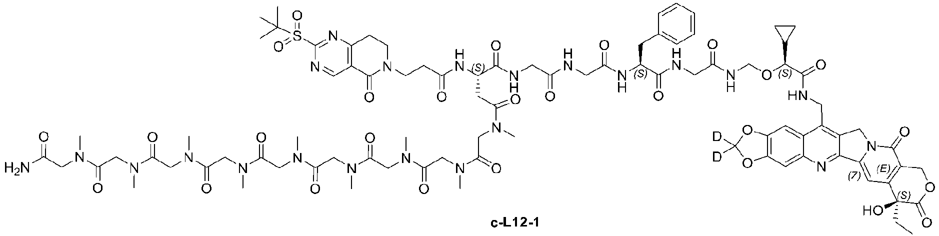

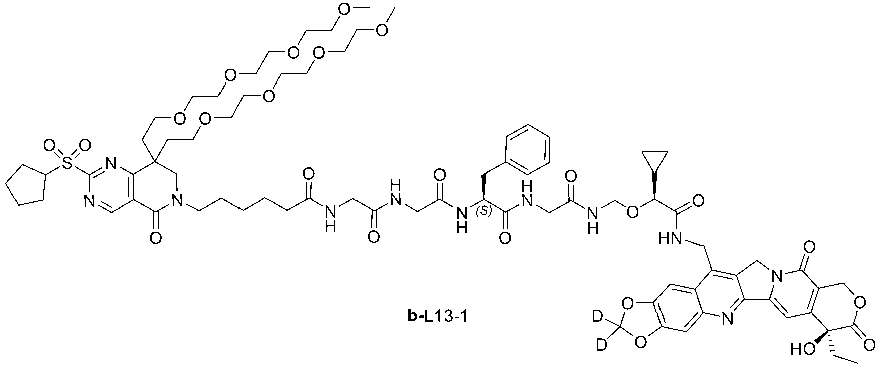

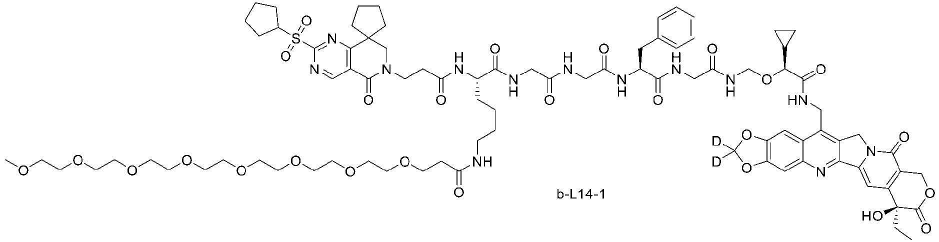

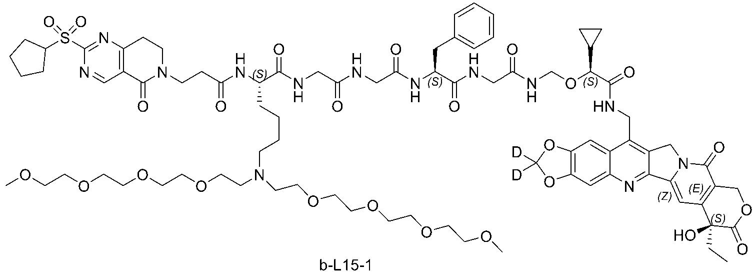

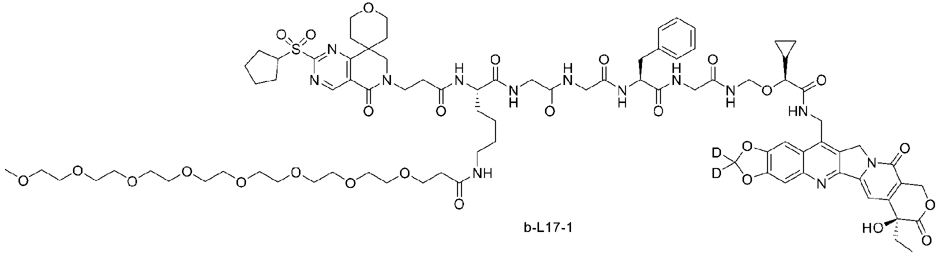

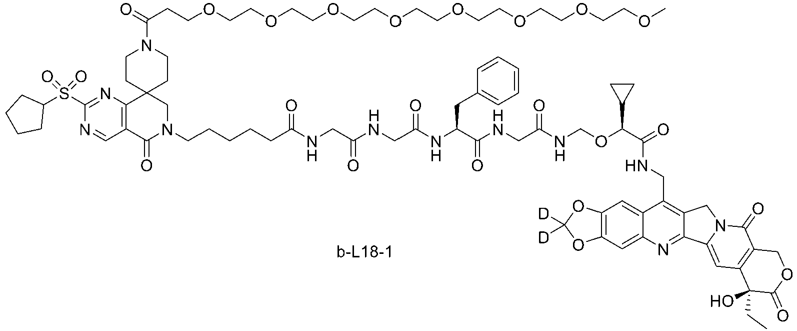

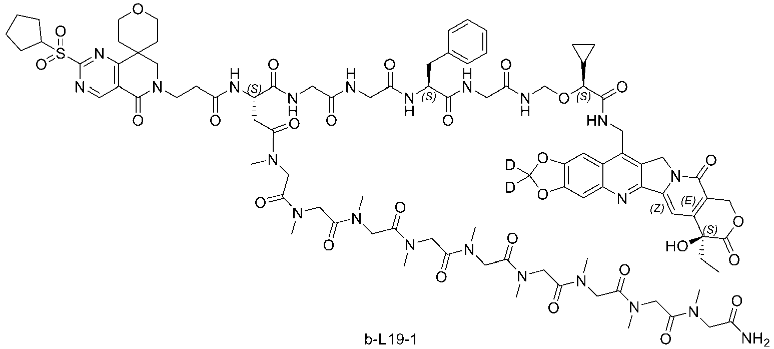

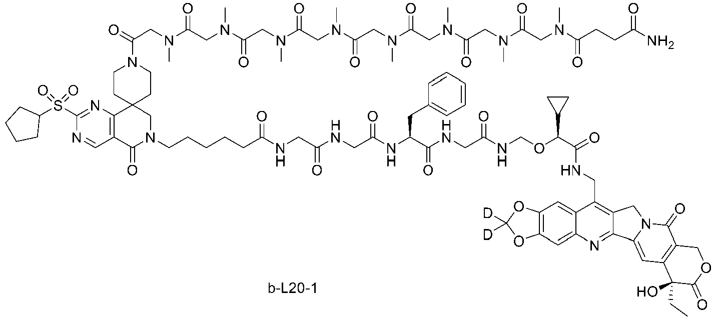

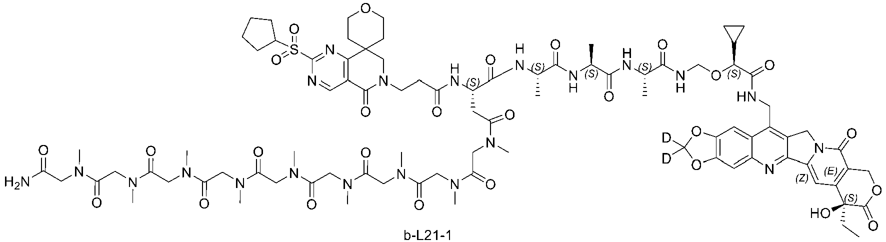

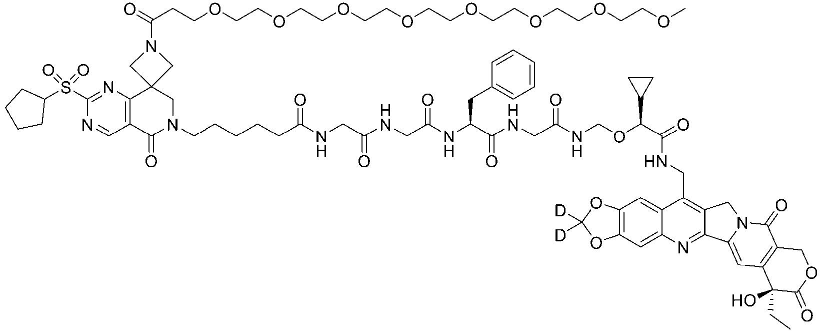

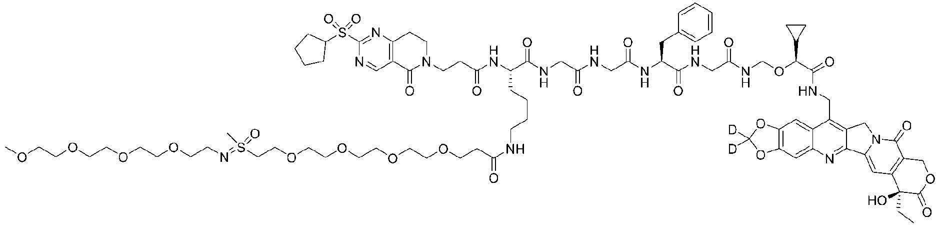

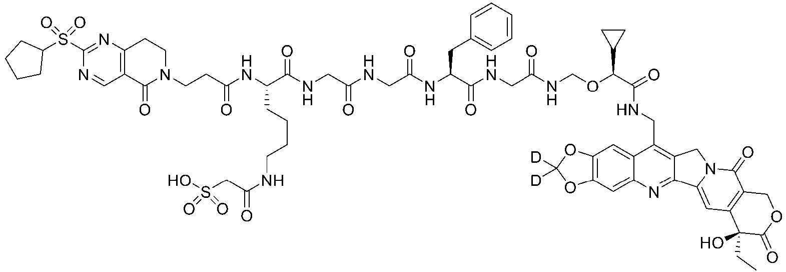

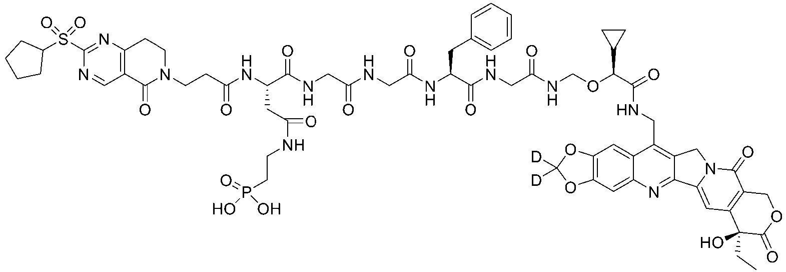

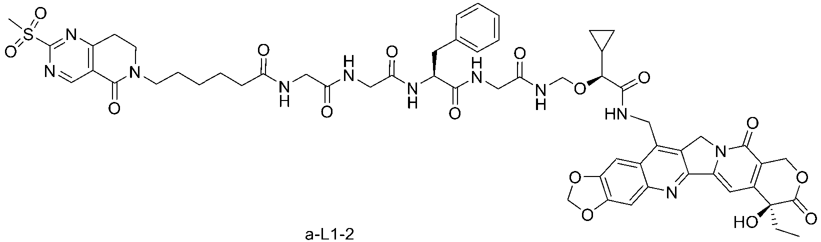

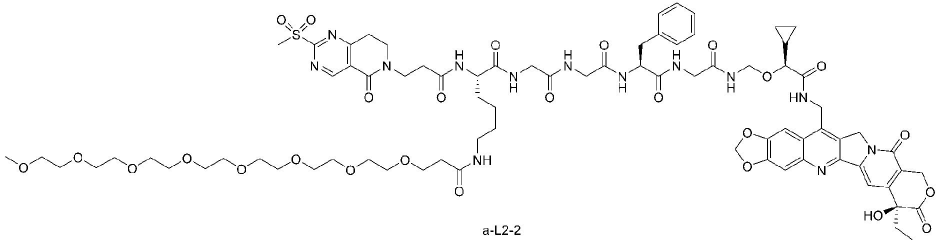

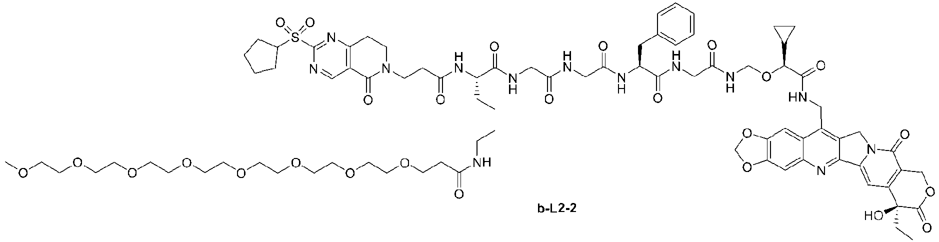

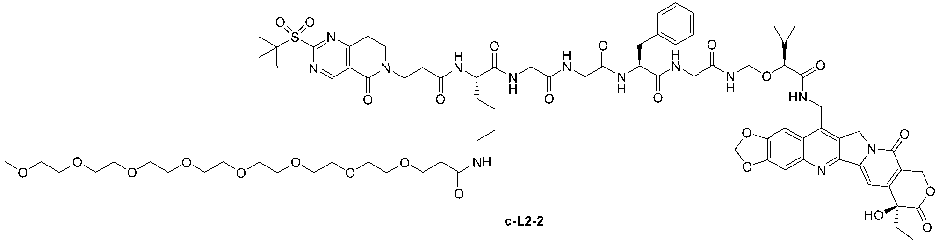

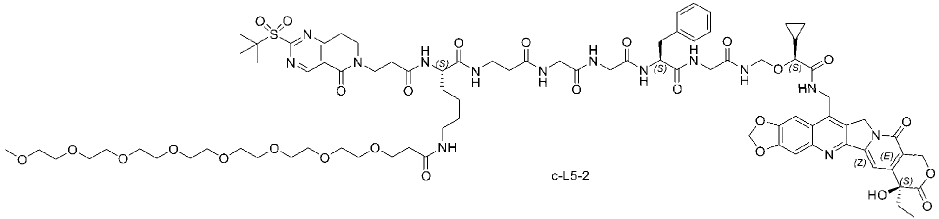

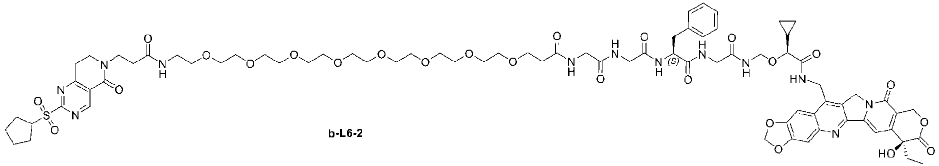

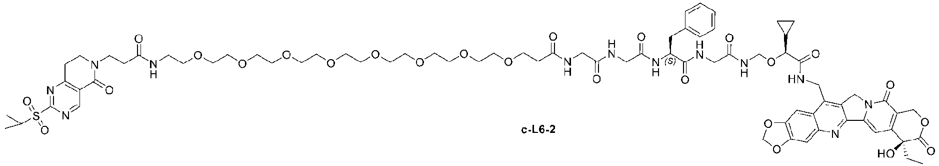

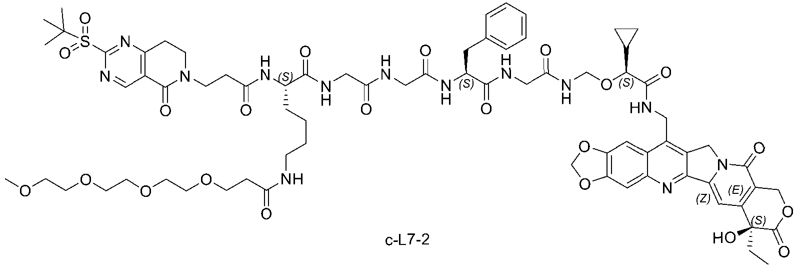

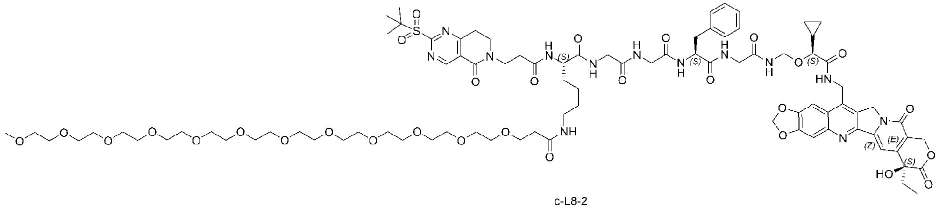

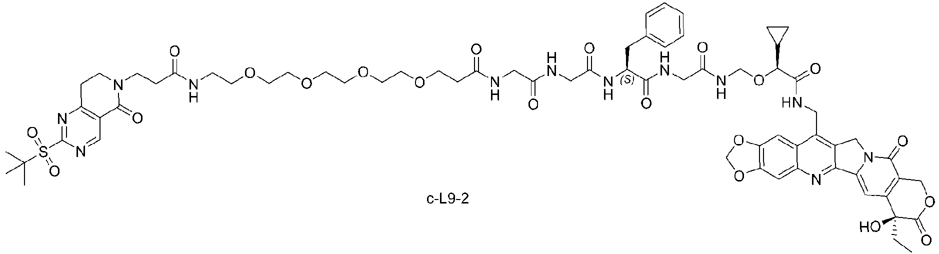

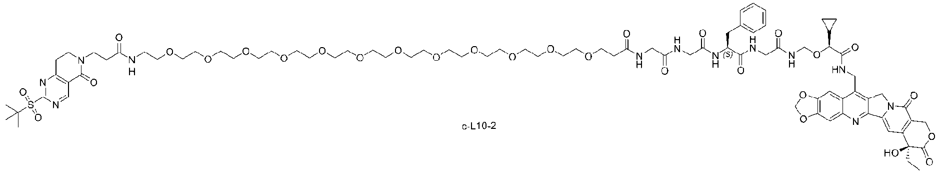

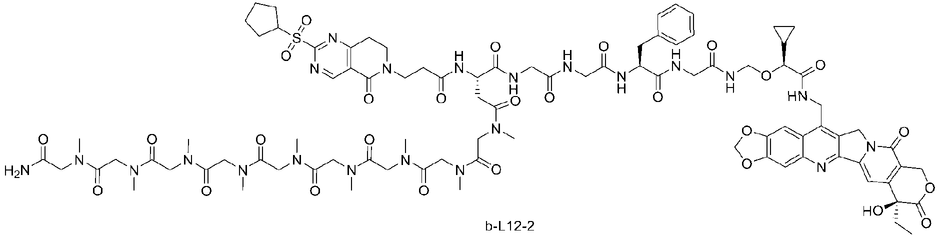

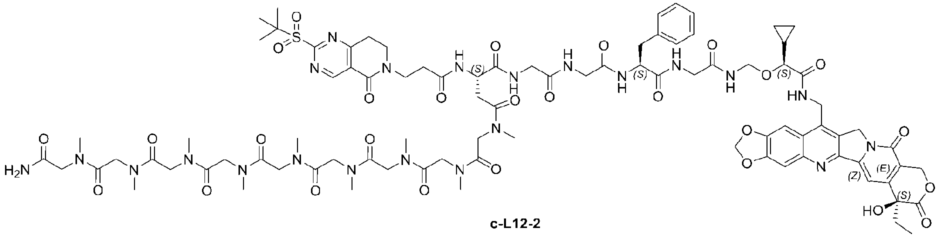

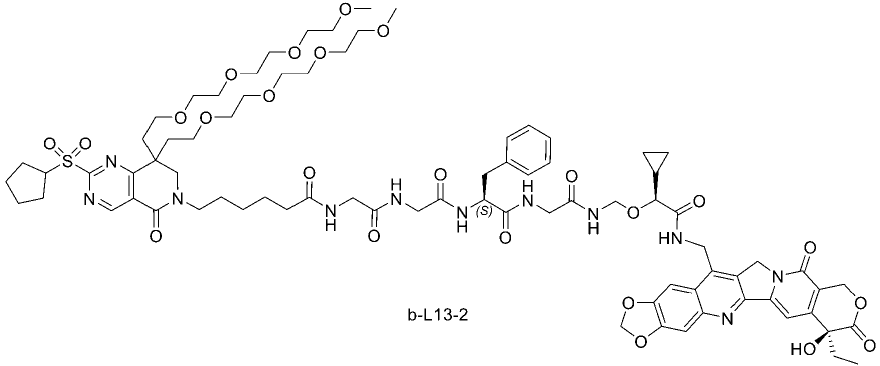

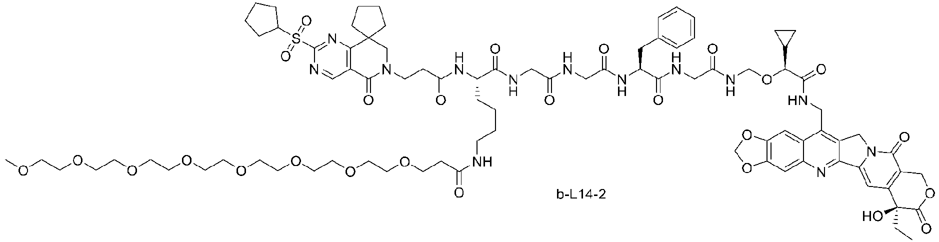

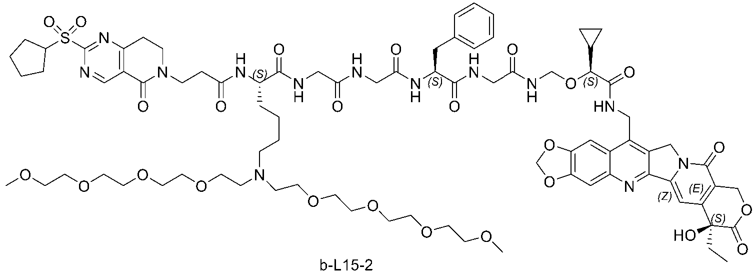

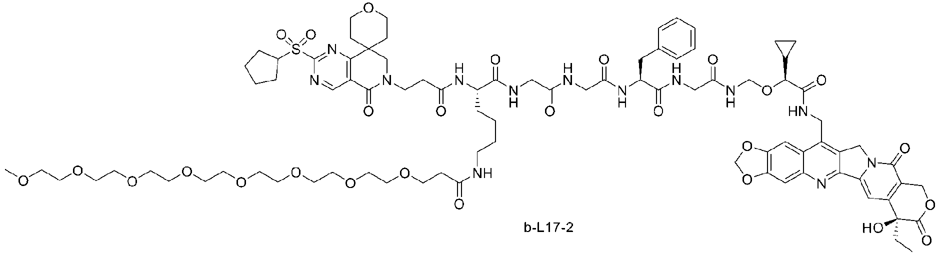

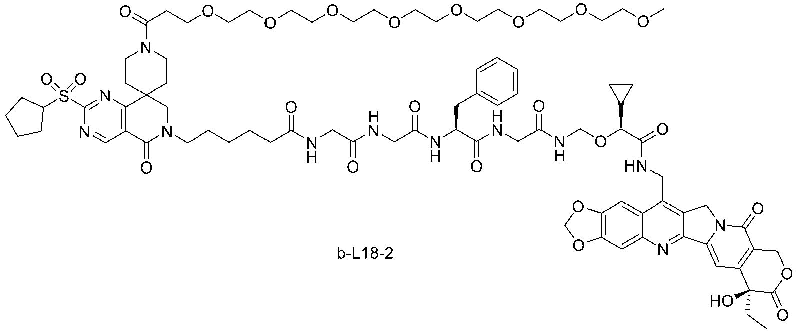

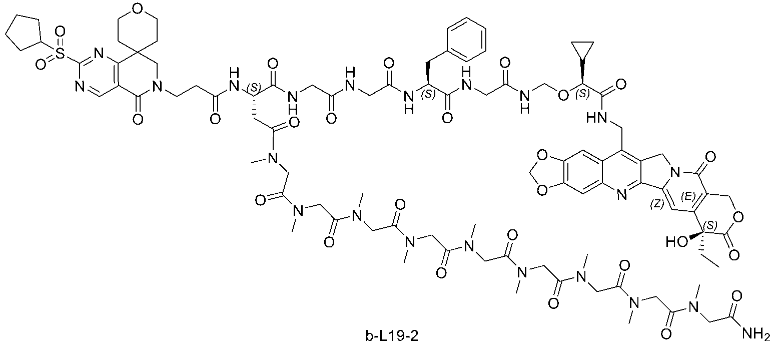

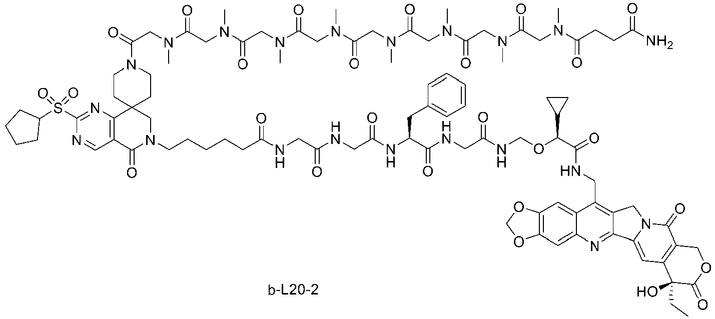

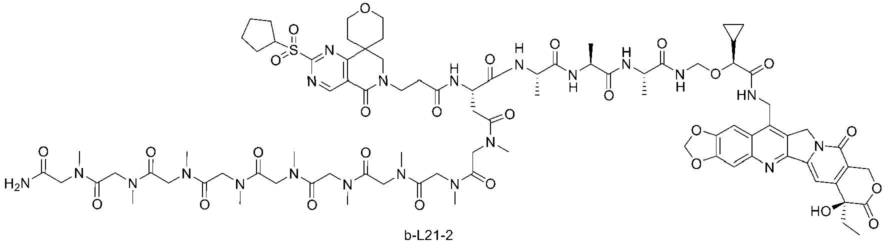

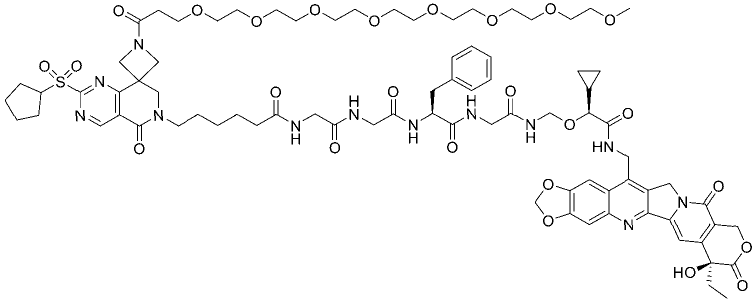

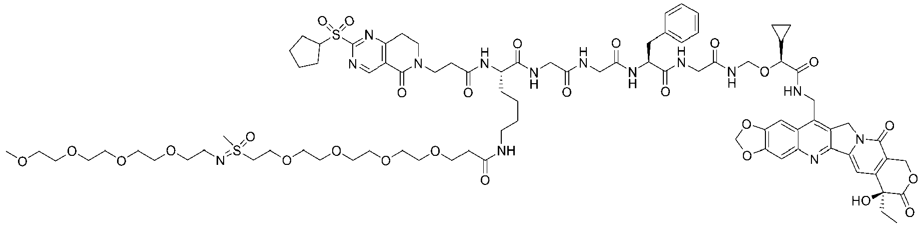

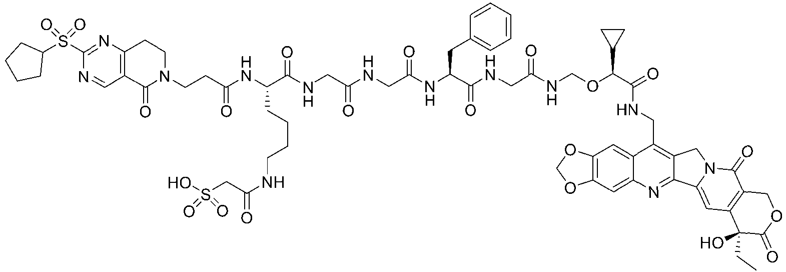

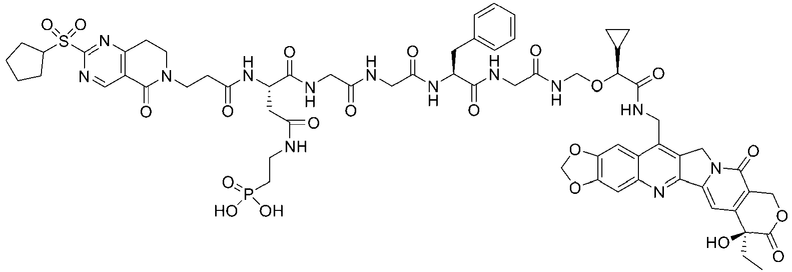

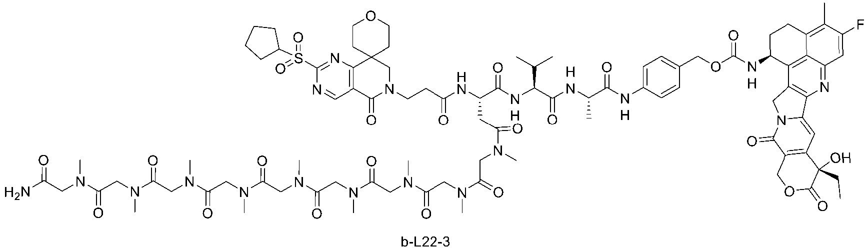

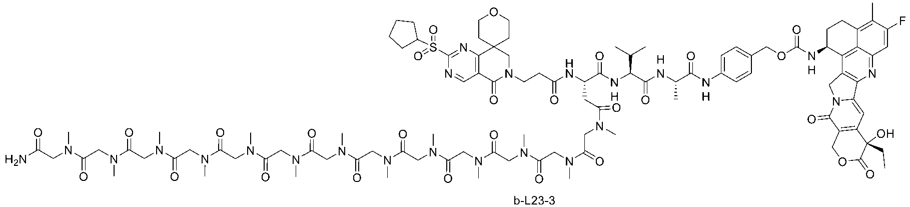

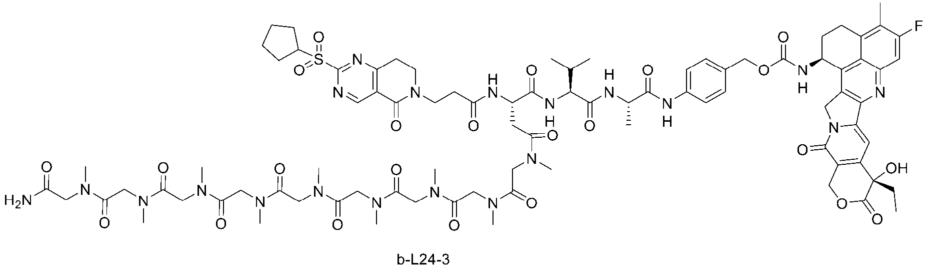

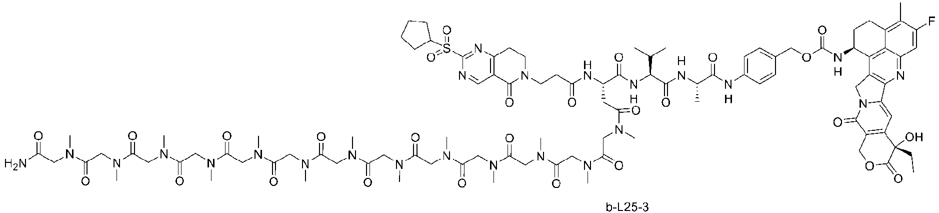

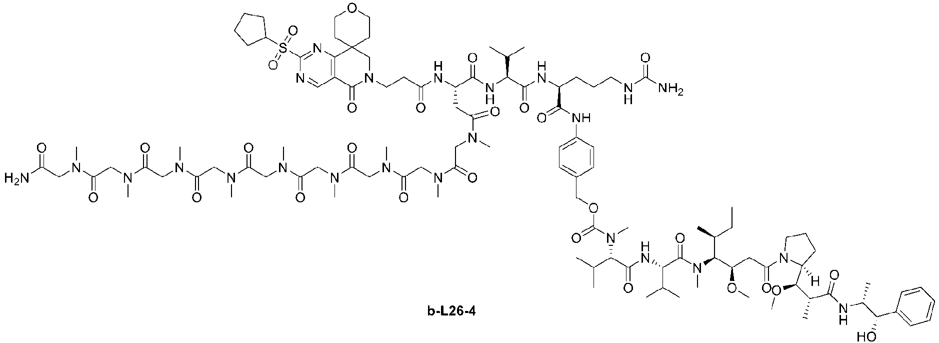

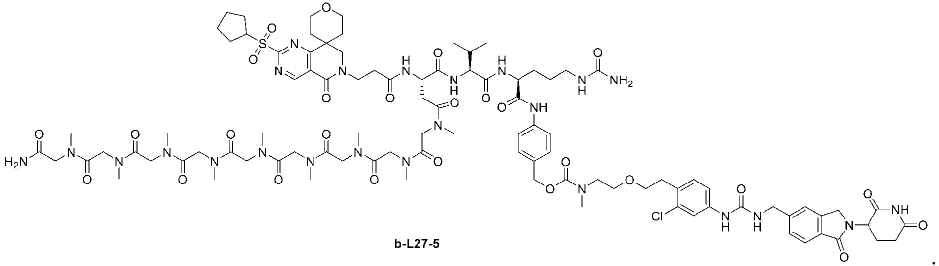

20. A pharmaceutical composition, comprising the ligand-drug conjugate or the pharmaceutically acceptable salt thereof according to any one of claims 1-14 and a pharmaceutically acceptable excipient.

21. A method for treating a mammalian tumor, comprising administering to a mammal, preferably a human, in need of the treatment a therapeutically effective amount of the ligand-drug conjugate or the pharmaceutically acceptable salt thereof according to any one of claims 1-14, or a therapeutically effective amount of the pharmaceutical composition according to claim 20.

22. Use of the ligand-drug conjugate or the pharmaceutically acceptable salt thereof according to any one of claims 1-14, or the pharmaceutical composition according to claim 20 in the manufacture of a medicament for treating a tumor.

23. The ligand-drug conjugate or the pharmaceutically acceptable salt thereof according to any one of claims 1-14 or the pharmaceutical composition according to claim 20 for use in treating a tumor.
